# EUROPEAN PATENT APPLICATION

(11) **EP 1 533 312 A1**
(43) Date of publication of application: **25.05.2005**
(21) Application number: 03760954.2
(22) Date of filing: 25.06.2003
(51) Int. Cl.: C07D 471/04, A61K 31/437, A61K 31/445, A61K 31/4453, A61K 31/4545, A61K 31/496, A61K 31/519, A61K 31/5377, A61K 31/55, A61P 3/10, A61P 9/10, A61P 13/12, A61P 25/00, A61P 43/00, C07D 487/04

(54) **HETERO-BICYCLIC COMPOUNDS**

(30) Priority: 25.06.2002 JP 2002185281
(71) Applicant: KYOWA HAKKO KOGYO CO., LTD., Chiyoda-ku, Tokyo 100-8185 (JP)
(72) Inventor: MACHII, Daisuke, Pharm. Res. Inst., Kyowa Hakko, Sunto-gun, Shizuoka 411-8731 (JP); SUZUKI, Koji, Sakai Res. Lab., Sakai-shi, Osaka 590-8554 (JP); MATSUMURA, Tsutomu, Sakai Res. Lab., Sakai-shi, Osaka 590-8554 (JP); ARAI, Hitoshi, Pharm. Res. Inst., Kyowa Hakko, Sunto-gun, Shizuoka 411-8731 (JP); IWASE, Miho, Pharm. Res. Inst., Kyowa Hakko, Sunto-gun, Shizuoka 411-8731 (JP); YANO, Hiroshi, Pharm. Res. Inst., Kyowa Hakko, Sunto-gun, Shizuoka 411-8731 (JP); NAKANISHI, S. Head Office, Chiyoda-ku, Tokyo 100-8185 (JP); OGINO, Fumiko, Tsukuba Res. Lab., Tsukuba-shi, Ibaraki 305-0841 (JP); TAKASAKI, Kotaro, Pharm. Res. Inst., Kyowa Hakko, Sunto-gun, Shizuoka 411-8731 (JP); KUSAKA, Hideaki, Pharm. Res. Inst., Kyowa Hakko, Sunto-gun, Shizuoka 411-8731 (JP)
(74) Representative: Casalonga, Axel
(86) International application number: PCT/JP2003/008045
(87) International publication number: WO 2004/000841

(57) **Abstract**

The present invention provides a compound represented by formula (I): 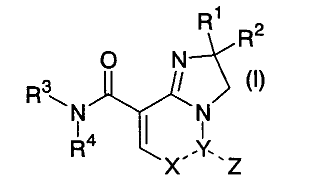 [wherein X--Y--Z represents R⁵N-C=O (wherein R⁵ represents a hydrogen atom, substituted or unsubstituted lower alkyl, substituted or unsubstituted aralkyl, substituted or unsubstituted lower alkenyl, or substituted or unsubstituted lower alkynyl), etc;
R¹ and R² may be the same or different and each represents a hydrogen atom, substituted or unsubstituted lower alkyl, etc; and
R³ and R⁴ may be the same or different and each represents a hydrogen atom, substituted or unsubstituted lower alkyl, etc], or a pharmaceutically acceptable salt thereof.

## Description

### Technical Field

The present invention relates to heterobicyclic compounds or pharmaceutically acceptable salts thereof which have glucose concentration-dependent insulin secretion promoting action or suitable hypoglycemic action and is useful as a therapeutic agent for diabetes.

### Background Art

Diabetes is caused by a metabolic abnormality mainly of glycometabolism, resulting from insufficient insulin secretion, decreased sensitivity of target cells of insulin or the like, and principally characterized by noticeable hyperglycemia. If the hyperglycemia continues for a long period of time, serious complications arise in various organs and nerves, such as retinopathy, nephropathy, or neuropathy, which are caused mainly by vascular lesion. Therefore, for the treatment of diabetes, it is extremely important to control and maintain a blood glucose level at a normal level, and methods for that purpose have been studied since old days.

For a type of diabetes where onset is gradual and insulin therapy is not necessarily required for life support (non-insulin dependent diabetes mellitus: NIDDM), a blood glucose level can be controlled by combination of exercise therapy and drug therapy. As the drugs, insulin secretion promoters, one of orally available hypoglycemic agents, have widely been used clinically. However, since currently available insulin secretion promoters all promote insulin secretion independent of a glucose level, they cause problems of severe hypoglycemia or insufficient control of blood glucose if doses are not appropriate, and are not fully satisfactory drugs. If a hypoglycemic agent can be provided that is capable of promoting insulin secretion depending on a blood glucose level, the agent is expected to be extremely useful for blood glucose control of patients suffering from diabetes because the risk of hypoglycemia due to an excess dosage can be avoided.

Japanese Published Unexamined Patent Application (Kokai) No. 91-204880; Journal of Medicinal Chemistry (J. Med. Chem.), vol.35, p.3578, 1992; Journal of Medicinal Chemistry (J. Med. Chem.), vol.36, p.2508, 1993; WO98/15555 and WO00/1388 disclose that compounds including a 2,3-dihydroimidazo[1,2-c]pyrimidine or 2,3-dihydroimidazo[1,2-α]pyridine structure as a part of their structure have diuretic action, antiasthmatic action, antidemential action, bronchodilatation action, antiallergic action, antiulcer action, or hypoglycemic action.

Also WO01/47931 discloses that condensed purine derivatives have insulin secretion promoting action and hypoglycemic action.

### Disclosure of the Invention

An object of the present invention is to provide a compound useful for preventive and/or therapeutic treatment of diabetes or complications of diabetes.

The present invention thus relates to the following (1) to (24).
(1) A compound represented by Formula (I): 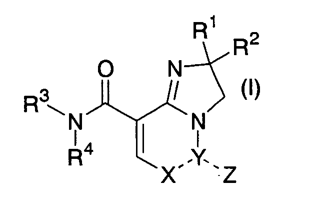 {wherein X--Y--Z represents R⁵N-C=O (wherein R⁵ represents a hydrogen atom, substituted or unsubstituted lower alkyl, substituted or unsubstituted aralkyl, substituted or unsubstituted lower alkenyl, or substituted or unsubstituted lower alkynyl), N=C-NR⁶R⁷ [wherein R⁶ and R⁷ may be the same or different and each represents a hydrogen atom, substituted or unsubstituted lower alkyl, substituted or unsubstituted aralkyl, substituted or unsubstituted lower alkenyl, substituted or unsubstituted lower alkynyl, substituted or unsubstituted aryl, or a substituted or unsubstituted heterocyclic group; or R⁶ and R⁷, are combined together with the adjacent nitrogen atom thereto to form: 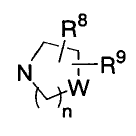 (wherein n represents an integer of 1 to 3; W represents -CH₂-, -CH=CH-, -NH-, a sulfur atom, or an oxygen atom; and R⁸ and R⁹ may be the same or different and each represents a hydrogen atom, substituted or unsubstituted lower alkyl, substituted or unsubstituted aralkyl, substituted or unsubstituted aryl, a substituted or unsubstituted heterocyclic group, substituted or unsubstituted lower alkoxycarbonyl, substituted or unsubstituted lower alkanoyl, substituted or unsubstituted aroyl, substituted or unsubstituted heteroaroyl, a halogen atom, amino, nitro, cyano, hydroxy, oxo, carboxy, carbamoyl, mono or di(substituted or unsubstituted lower alkyl)amino, substituted or unsubstituted lower alkanoylamino, substituted or unsubstituted lower alkoxy, substituted or unsubstituted aralkyloxy, or substituted or unsubstituted lower alkanoyloxy)], or C=C-NR^{6a}R^{7a} (wherein R^{6a} and R^{7a} have the same meanings as R⁶ and R⁷ defined above, respectively); R¹ and R² may be the same or different and each represents a hydrogen atom, substituted or unsubstituted lower alkyl, substituted or unsubstituted aralkyl, substituted or unsubstituted lower alkenyl, substituted or unsubstituted lower alkynyl, substituted or unsubstituted aryl, a substituted or unsubstituted heterocyclic group, a halogen atom, carboxy, substituted or unsubstituted lower alkoxycarbonyl, or C(=O)-NR^{6b}R^{7b} (wherein R^{6b} and R^{7b} have the same meanings as R⁶ and R⁷ defined above, respectively); and R³ and R⁴ may be the same or different and each represents a hydrogen atom, substituted or unsubstituted lower alkyl, substituted or unsubstituted lower alkenyl, substituted or unsubstituted aralkyl, or a substituted or unsubstituted heterocyclic group; or R³ and R⁴ are combined together with the adjacent nitrogen atom thereto to form: 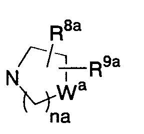 (wherein na, W^{a}, R^{8a}, and R^{9a} have the same meanings as n, W, R⁸, and R⁹ defined above, respectively)}, or a pharmaceutically acceptable salt thereof.
(2) The compound or the pharmaceutically acceptable salt thereof according to (1), wherein X--Y--Z is R⁵N-C=O (wherein R⁵ has the same meaning as defined above).
(3) The compound or the pharmaceutically acceptable salt thereof according to (1), wherein X--Y--Z is N=C-NR⁶R⁷ (wherein R⁶ and R⁷ have the same meanings as defined above, respectively).
(4) The compound or the pharmaceutically acceptable salt thereof according to (3), wherein R⁶ and R⁷ are not hydrogen atoms simultaneously.
(5) The compound or the pharmaceutically acceptable salt thereof according to (3), wherein R⁶ is a hydrogen atom and R⁷ is substituted or unsubstituted pyrrolidinyl.
(6) The compound or the pharmaceutically acceptable salt thereof according to (1), wherein X--Y--Z is C=C-NR^{6a}R^{7a} (wherein R^{6a} and R^{7a} have the same meanings as defined above, respectively).
(7) The compound or the pharmaceutically acceptable salt thereof according to (6), wherein R^{6a} and R^{7a} are not hydrogen atoms simultaneously.
(8) The compound or the pharmaceutically acceptable salt thereof according to any one of (1) to (7), wherein either R¹ or R² is a hydrogen atom and the other is substituted or unsubstituted lower alkyl, or substituted or unsubstituted aralkyl; or R¹ and R² may be the same or different and each is substituted or unsubstituted lower alkyl.
(9) The compound or the pharmaceutically acceptable salt thereof according to any one of (1) to (7), wherein R¹ is a hydrogen atom and R² is aralkyl.
(10) The compound or the pharmaceutically acceptable salt thereof according to any one of (1) to (9), wherein R³ is a hydrogen atom and R⁴ is lower alkyl.
(11) The compound or the pharmaceutically acceptable salt thereof according to any one of (1) to (9), wherein R³ is a hydrogen atom and R⁴ is substituted or unsubstituted aralkyl.
(12) A pharmaceutical composition which comprises the compound or the pharmaceutically acceptable salt thereof according to any one of (1) to (11).
(13) A preventive and/or therapeutic agent for diabetes, which comprises the compound or the pharmaceutically acceptable salt thereof according to any one of (1) to (11).
(14) A preventive and/or therapeutic agent for complication of diabetes, which comprises the compound or the pharmaceutically acceptable salt thereof according to any one of (1) to (11).
(15) An insulin secretion promoter which comprises the compound or the pharmaceutically acceptable salt thereof according to any one of (1) to (11).
(16) A hypoglycemic agent which comprises the compound or the pharmaceutically acceptable salt thereof according to any one of (1) to (11).
(17) A method for preventing and/or treating diabetes, which comprises administering an effective amount of the compound or the pharmaceutically acceptable salt thereof according to any one of (1) to (11).
(18) A method for preventing and/or treating a complication of diabetes, which comprises administering an effective amount of the compound or the pharmaceutically acceptable salt thereof according to any one of (1) to (11).
(19) A method for promoting insulin secretion, which comprises administering an effective amount of the compound or the pharmaceutically acceptable salt thereof according to any one of (1) to (11).
(20) A method for decreasing blood glucose level, which comprises administering an effective amount of the compound or the pharmaceutically acceptable salt thereof according to any one of (1) to (11).
(21) Use of the compound or the pharmaceutically acceptable salt thereof according to any one of (1) to (11) for the manufacture of a preventive and/or therapeutic agent for diabetes.
(22) Use of the compound or the pharmaceutically acceptable salt thereof according to any one of (1) to (11) for the manufacture of a preventive and/or therapeutic agent for a complication of diabetes.
(23) Use of the compound or the pharmaceutically acceptable salt thereof according to any one of (1) to (11) for the manufacture of an insulin secretion promoter.
(24) Use of the compound or the pharmaceutically acceptable salt thereof according to any one of (1) to (11) for the manufacture of a hypoglycemic agent.

Hereinafter, the compounds represented by Formula (I) are referred to as Compound (I). The same shall apply to the compounds of the other formula numbers.

Explanations of terminologies used to define each group in formula (I) are as follows:

Examples of the lower alkyl and the lower alkyl moieties of the lower alkoxy, lower alkoxycarbonyl, mono or di(lower alkyl)amino, lower alkanoyl, lower alkanoyloxy, and lower alkanoylamino include, for example, straight-chain, branched, or cyclic alkyl, as well as a combination thereof, which have 1 to 10 carbon atoms, such as methyl, ethyl, propyl, isopropyl, cyclopropylmethyl, butyl, sec-butyl, *tert*-butyl, cyclobutyl, pentyl, neopentyl, cyclopentyl, hexyl, cyclohexyl, cyclohexylmethyl, heptyl, cycloheptyl, octyl, cyclooctyl, nonyl, decyl and the like. Two lower alkyl moieties of the di(lower alkyl)amino may be the same or different.

Examples of the lower alkenyl include, for example, straight-chain, branched, or cyclic alkenyl, as well as a combination thereof, which have 3 to 10 carbon atoms, such as allyl, 2-butenyl, 3-butenyl, 4-pentenyl, 2-(1-cyclohexenyl)ethyl, 6-octenyl, 2,6-octadienyl, decenyl and the like.

Examples of the lower alkynyl include, for example, straight-chain or branched alkynyl which have 3 to 6 carbon atoms, such as propargyl, 3-butynyl, 5-hexynyl and the like.

Examples of the aryl and the aryl moiety of the aroyl include, for example, 5- to 14-membered monocyclic, bicyclic, or tricyclic aryl, such as phenyl, naphthyl, indenyl, anthranyl and the like.

Examples of the aralkyl and the aralkyl moiety of the aralkyloxy include, for example, aralkyl which have 7 to 13 carbon atoms, such as benzyl, phenethyl, phenylpropyl, benzhydryl, naphthylmethyl and the like. Bicyclic aralkyl formed by aryl bonded to branched alkyl at two positions, such as indanyl, 1,2,3,4-tetrahydronaphthyl, 6,7,8,9-tetrahydro-5*H*-benzocycloheptyl and the like, are also included thereto.

Examples of the heterocyclic group include an alicyclic heterocyclic group and an aromatic heterocyclic group. Examples of the alicyclic heterocyclic group include, for example, a 3- to 8-membered monocyclic alicyclic heterocyclic group containing at least one atom selected from a nitrogen atom, an oxygen atom, and a sulfur atom; and a bicyclic or tricyclic condensed alicyclic heterocyclic group in which 3- to 8-membered rings are condensed and containing at least one atom selected from a nitrogen atom, an oxygen atom, and a sulfur atom. More specific examples include tetrahydropyranyl, tetrahydrofuranyl, pyranyl, thiopyranyl, pyrrolidinyl, piperidino, piperidyl, perhydroazepinyl, perhydroazocinyl, morpholino, morpholinyl, thiomorpholino, thiomorpholinyl, piperadinyl, homopiperadinyl, dioxolanyl, imidazolidinyl, imidazolinyl, pyrazolidinyl, indolinyl, isoindolinyl, 2-pyrrolinyl, 2-pyrrolidonyl, 3-pyrrolidonyl, 2-piperidonyl, 3-piperidonyl, 4-piperidonyl, perhydro-2-azepinonyl, perhydro-3-azepinonyl, perhydro-4-azepinonyl, 2-thiazolidonyl, 4-thiazolidonyl, 2-oxazolidonyl, 4-oxazolidonyl, succinimido, phthalimido, glutarimido, maleimido, hydantoinyl, thiazolidinedionyl, oxazolidinedionyl, and the like. Examples of the aromatic heterocyclic group include, for example, a 3- to 8-membered monocyclic aromatic heterocyclic group containing at least one atom selected from a nitrogen atom, an oxygen atom, and a sulfur atom; and a bicyclic or tricyclic condensed aromatic heterocyclic group in which 3- to 8-membered rings are condensed and containing at least one atom selected from a nitrogen atom, an oxygen atom, a sulfur atom. More specific examples include furyl, thienyl, pyrrolyl, imidazolyl, pyrazolyl, triazolyl, tetrazolyl, oxazolyl, isooxazolyl, thiazolyl, isothiazolyl, 1,2,3-oxadiazolyl, 1,2,4-oxadiazolyl, 1,3,4-oxadiazolyl, 1,2,3-thiadiazolyl, 1,2,4-thiadiazolyl, 1,3,4-thiadiazolyl, furazanyl, pyridyl, pyrimidinyl, triazinyl, pyrazinyl, pyridazinyl, quinolyl, isoquinolyl, quinazolinyl, phthalazinyl, purinyl, benzodioxolyl, benzoxazolyl, benzothiazolyl, benzimidazolyl, indolyl, isoindolyl, 2-pyridonyl, 4-pyridonyl, uracilyl, and the like.

The heterocyclic moiety of the heteroaroyl has the same meaning as the above-defined aromatic heterocyclic group.

The halogen atom represents a fluorine atom, a chlorine atom, a bromine atom, and an iodine atom.

Examples of substituents of the substituted lower alkenyl, substituted lower alkoxycarbonyl, substituted lower alkanoyl, mono or di(substituted lower alkyl)amino, substituted lower alkanoylamino, substituted lower alkoxy, substituted lower alkynyl, substituted lower alkanoyloxy, substituted aralkyl, substituted aryl, a substituted heterocyclic group, substituted aralkyloxy, substituted aroyl, and substituted heteroaroyl, which may be the same or different and, for example, in number of 1 to 3 include a hydroxy, carboxy, nitro, cyano, amino, lower alkyl, trifluoromethyl, lower alkoxy, (lower alkoxy)lower alkyl, lower alkenyl, lower alkynyl, lower alkoxycarbonyl, lower alkanoyl, trifluoroacetyl, lower alkanoyloxy, lower alkanoylamino, lower alkylaminocarbonyl, mono or di(lower alkyl)amino, carbamoyl, methylenedioxy, lower alkylsulfinyl, lower alkylsulfonyl, mercapto, lower alkylthio, a halogen atom, aryl, aralkyl, aryloxy, aralkyloxy, aroyl, and a heterocyclic group. These substituents may contain other functional groups, such as, chlorophenyl, methylcarbamoyl, chlorobenzyl, and lower alkoxybenzyl. Herein, lower alkyl, lower alkoxy, lower alkenyl, lower alkynyl, lower alkoxycarbonyl, lower alkanoyl, lower alkanoyloxy, lower alkanoylamino, mono or di(lower alkyl)amino, aryl, aralkyl, aralkyloxy, aroyl, a heterocyclic group, and a halogen atom have the same meaning as defined above. Lower alkyl moieties of the lower alkylaminocarbonyl, lower alkylsulfinyl, lower alkylsulfonyl, lower alkylthio, and lower alkoxybenzyl are the same as the above-defined lower alkyl. Aryl moieties of the aryloxy are the same as the above-defined aryl. Lower alkyl moiety of the terminal lower alkoxy moiety in the (lower alkoxy)lower alkyl are the same as the above-defined lower alkyl, and alkylene moiety correspond to that obtained by eliminating one hydrogen atom from the above defined lower alkyl.

Examples of substituents of the substituted lower alkyl, which may be the same or different, and in number of 1 to 3 include , for example, hydroxy, carboxy, nitro, cyano, amino, carbamoyl, methylenedioxy, lower alkoxy, (lower alkoxy)lower alkyl, (lower alkoxy)lower alkoxy, lower alkenyl, lower alkynyl, lower alkoxycarbonyl, lower alkanoyl, trifluoroacetyl, lower alkanoyloxy, lower alkanoylamino, lower alkylaminocarbonyl, mono or di(lower alkyl)amino, lower alkylsulfinyl, lower alkylsulfonyl, mercapto, lower alkylthio, a halogen atom, aryl, aralkyl, aryloxy, aralkyloxy, aroyl, or a substituted or unsubstituted heterocyclic group. These substituents may contain other functional groups, such as chlorophenyl, methylcarbamoyl, chlorobenzyl, lower alkoxybenzyl, and the like. Herein, lower alkoxy, (lower alkoxy)lower alkyl, lower alkenyl, lower alkynyl, lower alkoxycarbonyl, lower alkanoyl, lower alkanoyloxy, lower alkanoylamino, lower alkylaminocarbonyl, mono or di(lower alkyl)amino, lower alkylsulfinyl, lower alkylsulfonyl, lower alkylthio, aryl, aralkyl, aryloxy, aralkyloxy, aroyl, a heterocyclic group, a halogen atom, and lower alkoxybenzyl have the same meaning as defined above and lower alkyl moiety of the terminal lower alkoxy moiety is the same as the above-defined lower alkyl, and alkylene moiety correspond to that obtained by eliminating one hydrogen atom from the above defined lower alkyl in (lower alkoxy)lower alkoxy. Substituents of the substituted heterocyclic group are the same as the above-defined substituents of the substituted heterocyclic group.

Examples of the pharmaceutically acceptable salts of Compound (I) include, for example, metal salts, ammonium salts, organic amine addition salts, amino acid addition salts, acid addition salts and the like. Examples of the pharmaceutically acceptable metal salts include, for example, alkaline metal salts such as sodium salts and potassium salts, alkaline earth metal salts such as magnesium salts and calcium salts, as well as aluminum salts, zinc salts and the like. Examples of pharmaceutically acceptable ammonium salts include salts of an ammonium salt, tetramethylammonium salt and the like. Examples of pharmaceutically acceptable organic amine addition salts include, for example, addition salts such as morpholine and piperidine. Examples of pharmaceutically acceptable amino acid addition salts include, for example, addition salts with amino acids such as lysine, glycine, phenylalanine and the like. Examples of pharmaceutically acceptable acid addition salts include, for example, inorganic acid salts such as hydrochlorides, sulfates, and phosphates; and organic acid salts such as acetates, maleates, fumarates, tartrates, and citrates.

Compound (I) of the present invention may have a stereoisomer, a regioisomer, a tautomer, or the like. Any possible isomers and mixtures thereof fall within the scope of the present invention in any mixing ratio.

Compound (I) can be produced, for example, by the following preparation method.

When any defined group changes under a given reaction condition or is not suitable for carrying out a reaction process, in the preparation method mentioned below, preparation may be readily carried out by applying methods commonly used in the field of synthetic organic chemistry such as protection and deprotection of a functional group(see, for example, T. W. Greene, Protective Groups in Organic Synthesis, third edition, John Wiley & Sons, Inc. (1999) and the like). The order of the reaction steps such as introduction of the substituents can be changed, if necessary.

Production Method 1: Compound (I-A) which corresponds to Compound (I) wherein X--Y--Z represents R⁵N-C=O (wherein R⁵ has the same meaning as defined above) can be synthesized according to Steps 1 to 5 described below: 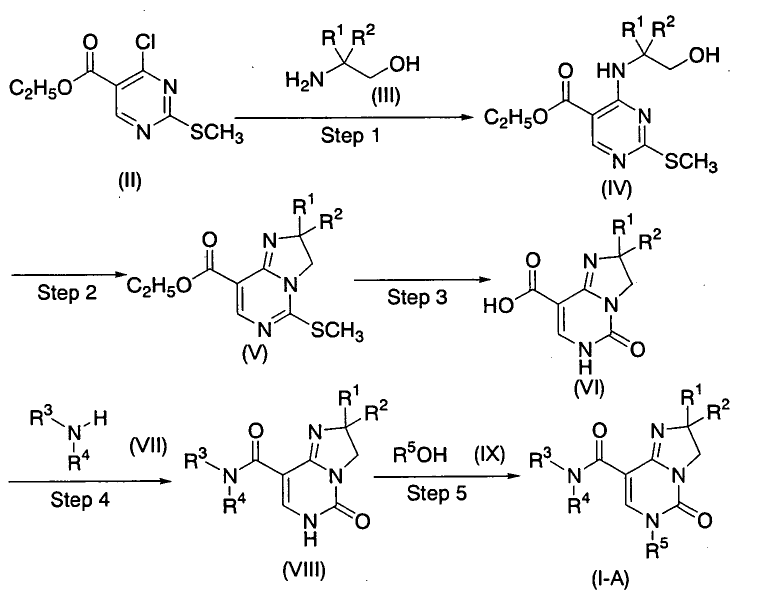 (wherein R¹, R², R³, R⁴ and R⁵ have the same meanings as defined above, respectively)

### [Step 1]

Compound (IV) can be obtained by reacting commercially available Compound (II) with 1 equivalent to large excess, preferably 1 to 3 equivalents of Compound (III) in an inert solvent. Compound (III) is commercially available or can be synthesized by known methods. The reaction is usually performed at a temperature between 0°C and 100°C, preferably at a temperature between 0°C and 50°C, for about 10 minutes to 24 hours. Any solvent can be used, so long as it is inert to the reaction. Examples of the inert solvent include tetrahydrofuran, dioxane, diethyl ether, diisopropyl ether, benzene, toluene, xylene, ethyl acetate, acetonitrile, dichloromethane, chloroform, dichloroethane, dimethylformamide, dimethylacetamide, *N*-methylpyrrolidone, dimethyl sulfoxide, pyridine, and any mixture thereof, preferably, tetrahydrofuran, chloroform, and mixtures thereof, although there is no limitation thereto. The reaction may be perfomed by adding a base with, 1 equivalent to large excess, preferably, 1 to 10 equivalents. Examples of the base include, for example, various organic or inorganic bases such as triethylamine, diisopropylethylamine, 1,8-diazabicyclo[5.4.0]-7-undecene (DBU), *N,N*-dimethylaniline, pyridine, quinoline, potassium carbonate, sodium carbonate, lithium carbonate, sodium hydrogen carbonate, potassium hydroxide, sodium hydroxide, lithium hydroxide, potassium *tert*-butoxide and the like; various basic anion exchange resins such as Amberlyst A-21 (ROHM AND HAAS), AG 1-X8 (Bio-Rad Laboratories), and the like; and various bases immobilized on solid-phases such as polyvinylpyridine morpholinomethyl polystyrene and the like, preferably, triethylamine.

### [Step 2]

Compound (V) can be obtained by treating Compound (IV) synthesized in Step 1 with 1 to 5 equivalents of a chlorinating agent such as thionyl chloride and phosphorus oxychloride in an inert solvent. The reaction is usually performed at a temperature between room temperature and 200°C, preferably at a temperature between 50°C and 100°C, for about 1 to 50 hours. Any solvent can be used, so long as it is inert to the reaction. Examples of the inert solvent include tetrahydrofuran, dioxane, diisopropyl ether, benzene, toluene, xylene, ethyl acetate, dichloromethane, chloroform, dichloroethane, pyridine, triethylamine, and any mixture thereof preferably, chloroform, although there is no limitation thereto. The reaction may be performed, by adding 1 equivalent to large excess, preferably, 1 to 10 equivalents of a base. Examples of the base include various organic bases such as triethylamine, diisopropylethylamine, DBU, *N,N*-dimethylaniline, pyridine, quinoline and the like; and various bases immobilized on solid-phases such as polyvinylpyridine, morpholinomethyl polystyrene and the like, preferably, polyvinylpyridine.

### [Step 3]

Compound (IV) can be obtained by treating Compound (V) synthesized in Step 2 with 1 equivalent to large excess, preferably 3 to 10 equivalents of a base in a protic solvent. The reaction is usually performed at a temperature between room temperature and 200°C, preferably at a temperature between 50°C and 120°C, for 1 to 100 hours. Examples of the solvent for the reaction include, for example, protic solvents such as water, methanol, ethanol, propanol, butanol and the like, used each alone or as a mixture thereof; or a mixture of any of these protic solvents and non-protic solvents such as tetrahydrofuran, dioxane, diethyl ether, diisopropyl ether, benzene, toluene, xylene, ethyl acetate, acetonitrile, dichloromethane, chloroform, dichloroethane, dimethylformamide, dimethylacetamide, *N*-methylpyrrolidone, dimethyl sulfoxide, pyridine and the like; and preferably, a water-chloroform mixture and a water-ethanol mixture. Examples of the base include, for example, potassium carbonate, sodium carbonate, sodium hydrogen carbonate, lithium carbonate, potassium hydroxide, sodium hydroxide, lithium hydroxide, potassium tert-butoxide, Amberlyst A-21 (ROHM AND HAAS), AG 1-X8 (Bio-Rad Laboratories) and the like.

### [Step 4]

Compound (VIII) can be obtained by reacting Compound (VI) synthesized in Step 3 with 1 to 5 equivalents of Compound (VII) in the presence of 1 to 10 equivalents of a condensing agent in an inert solvent. Compound (VII) is commercially available or can be synthesized by known methods. The reaction is usually performed at a temperature between 0°C and 150°C, preferably at a temperature between room temperature and 80°C, for about 1 to 120 hours. Any solvent can be used, so long as it is inert to the reaction. Examples of the inert solvent include dichloromethane, chloroform, dichloroethane, dimethylformamide, dimethylacetamide, *N*-methylpyrrolidone, dimethyl sulfoxide, tetrahydrofuran, dioxane, diethyl ether, diisopropyl ether, benzene, toluene, xylene, ethyl acetate, acetonitrile, and any mixture thereof, preferably, chloroform, tetrahydrofuran and any mixtures thereof, although there is no limitation thereto. Examples of the condensing agent include, for example, dicyclohexylcarbodiimide, diisopropylcarbodiimide, *N*-ethyl-*N*'-(3-dimethylaminopropyl)carbodiimide or its hydrochloride, *N*-ethyl-*N*'-(3-dimethylaminopropyl)carbodiimide supported on polystyrene, *N*-benzyl-*N*'-cyclohexylcarbodiimide supported on polystyrene, benzotriazol-1-yl-tris(dimethylamino)phosphonium hexafluorophosphide salt, and diphenylphosphoryl azide, preferably, *N*-ethyl-*N*'-(3-dimethylaminopropyl)carbodiimide or its hydrochloride, and *N*-ethyl-*N*'-(3-dimethylaminopropyl)carbodiimide supported on polystyrene. The reaction may be performed in the coexistence of 1 to 5 equivalents of an additive, and examples of which include, for example, *N*-hydroxysuccinimide, 1-hydroxybenzotriazole, 3-hydroxy-4-oxo-3,4-dihydro-1,2,3-benzotriazine and the like, preferably, 1-hydroxybenzotriazole.

### [Step 5]

Compound (I-A) can be obtained by subjecting Compound (VIII) synthesized in Step 4 with 1 to 5 equivalents of Compound (IX) to a Mitsunobu reaction in an inert solvent. Compound (IX) is commercially available or can be synthesized by known methods. The reaction is usually performed at a temperature between 0°C and 100°C, preferably at a temperature between room temperature and 60°C, for about 1 to 50 hours. Any solvent can be used, so long as it is inert to the reaction. Examples of the inert solvent include dichloromethane, chloroform, dichloroethane, dimethylformamide, dimethylacetamide, *N*-methylpyrrolidone, dimethyl sulfoxide, tetrahydrofuran, dioxane, diethyl ether, diisopropyl ether, benzene, toluene, xylene, ethyl acetate, acetonitrile, and mixtures thereof, preferably, tetrahydrofuran, although there is no limitation thereto. Condensing agents necessary for the Mitsunobu reaction may be any agents generally used in the reaction, such as a combination of 1 to 10 equivalents of dialkyl azodicarboxylate and 1 to 10 equivalents of triphenylphosphine or trialkylphosphine; and 1 to 10 equivalents of (cyanomethylene)triphenylphosphorane and the like, preferably, a combination of 1 to 3 equivalents of diethylazodicarboxylate (DEAD) and 1 to 3 equivalents of triphenyl phosphine.

Preparation Method 2: Compound (I-B) which corresponds to Compound (I) wherein X--Y--Z represents N=C-NR⁶R⁷ (wherein R⁶ and R⁷ have the same meanings as defined above, respectively) can be synthesized according to Steps 6 to 9 described below: 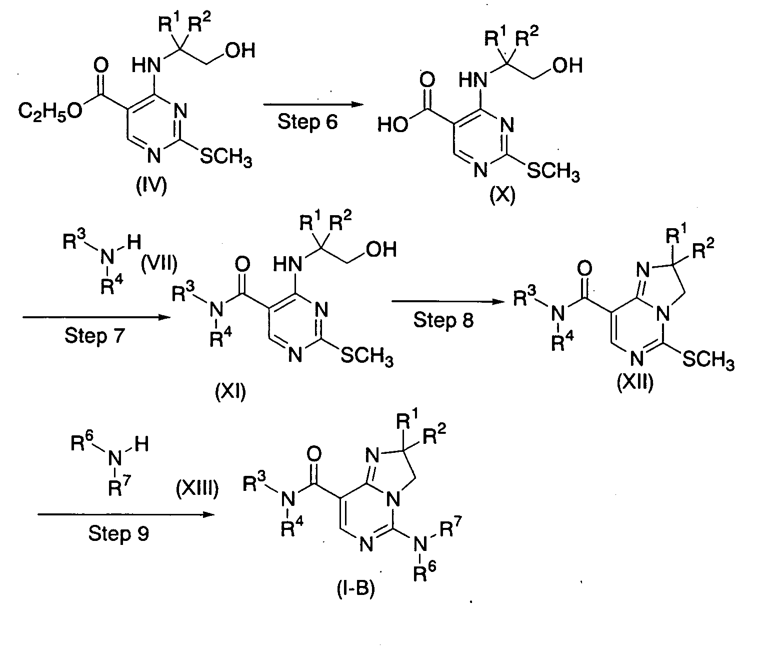 (wherein R¹, R², R³, R⁴, R⁶ and R⁷ have the same meanings as defined above, respectively)

### [Step 6]

Compound (X) can be obtained by treating Compound (IV) synthesized in Step 1 of the Preparation Method 1 with 1 equivalent to large excess, preferably 3 to 10 equivalents of a base in a protic solvent. The reaction is usually performed at a temperature between 0°C and 100°C, preferably at a temperature between room temperature and 80°C, for 1 to 100 hours. Examples of the solvent for the reaction include, for example, protic solvents such as water, methanol, ethanol, propanol, butanol and the like used each alone or a mixture thereof; or a mixture of any of these protic solvents and non-protic solvents such as tetrahydrofuran, dioxane, diethyl ether, diisopropyl ether, benzene, toluene, xylene, ethyl acetate, acetonitrile, dichloromethane, chloroform, dichloroethane, dimethylformamide, dimethyl acetamide, *N*-methylpyrrolidone, dimethyl sulfoxide, pyridine and the like, preferably, a water-ethanol mixture. Examples of the base include, for example, potassium carbonate, sodium carbonate, sodium hydrogen carbonate, lithium carbonate, potassium hydroxide, sodium hydroxide, lithium hydroxide, potassium *tert*-butoxide, Amberlyst A-21 (ROHM AND HAAS), AG1-X8 (Bio-Rad Laboratories), and the like.

### [Step 7]

Compound (XI) can be obtained by reacting Compound (X) synthesized in Step 6 with 1 to 5 equivalents of Compound (VII) in the presence of 1 to 10 equivalents of a condensing agent in an inert solvent. Compound (VII) is commercially available or can be synthesized by known methods. The reaction is usually performed at a temperature between 0°C and 150°C, preferably at a temperature between room temperature and 60°C, for about 1 to 120 hours. Any solvent can be used, so long as it is inert to the reaction. Examples of the inert solvent include dichloromethane, chloroform, dichloroethane, dimethylformamide, dimethylacetamide, *N*-methylpyrrolidone, dimethyl sulfoxide, tetrahydrofuran, dioxane, diethyl ether, diisopropyl ether, benzene, toluene, xylene, ethyl acetate, acetonitrile, and any mixture thereof preferably, chloroform, tetrahydrofuran, and mixtures thereof, although there is no limitation thereto. Examples of the condensing agent include, for example, dicyclohexylcarbodiimide, diisopropylcarbodiimide, *N*-ethyl-*N*'-(3-dimethylaminopropyl)carbodiimide or its hydrochloride, *N*-ethyl-*N*'-(3-dimethylaminopropyl)carbodiimide supported on polystyrene, *N*-benzyl-*N*'-cyclohexylcarbodiimide supported on polystyrene, benzotriazol-1-yl-tris(dimethylamino)phosphonium hexafluorophosphide salt, diphenylphosphoryl azide and the like, preferably, *N*-ethyl-*N*'-(3-dimethylaminopropyl)carbodiimide or its hydrochloride, *N*-ethyl-*N*'-(3-dimethylaminopropyl)carbodiimide supported on polystyrene. The reaction may be performed in the coexistence of 1 to 5 equivalents of an additive, examples of which include *N*-hydroxysuccinimide, 1-hydroxybenzotriazole, 3-hydroxy-4-oxo-3,4-dihydro-1,2,3-benzotriazine and the like, preferably, 1-hydroxybenzotriazole.

### [Step 8]

Compound (XII) can be obtained by treating Compound (XI) synthesized in Step 7 with 1 to 5 equivalents of a chlorinating agent such as thionyl chloride, phosphorus oxychloride and the like, in an inert solvent. The reaction is usually performed at a temperature between room temperature and 200°C, preferably at a temperature between 50°C and 100°C, for about 1 to 50 hours. Any Solvent can be used, so long as it is inert to the reaction. Examples of the inert solvent include tetrahydrofuran, dioxane, diisopropyl ether, benzene, toluene, xylene, ethyl acetate, dichloromethane, chloroform, dichloroethane, pyridine, triethylamine, and any mixture thereof, preferably, chloroform, although there is no limitation thereto. In the reaction, 1 equivalent to large excess, preferably 1 to 10 equivalents of a base may be added. Examples of the base include, for example, various organic bases such as triethylamine, diisopropylethylamine, DBU, *N,N*-dimethylaniline, pyridine, quinoline and the like; and various bases immobilized on solid-phases such as polyvinylpyridine morpholinomethyl polystyrene and the like, preferably, polyvinylpyridine.

### [Step 9]

Compound (I-B) can be obtained by reacting Compound (XII) synthesized in Step 8 with 1 to 10 equivalents, preferably 2 to 5 equivalents of Compound (XIII) in an inert solvent. The reaction is usually performed at a temperature between room temperature and 200°C, preferably at a temperature between 50°C and 100°C, for about 1 to 100 hours. Any solvent can be used, so long as it is inert to the reaction. Examples of the inert solvent include tetrahydrofuran, dioxane, diethyl ether, diisopropyl ether, benzene, toluene, xylene, ethyl acetate, acetonitrile, dichloromethane, chloroform, dichloroethane, dimethylformamide, dimethylacetamide, *N*-methylpyrrolidone, dimethyl sulfoxide, pyridine, and any mixture thereof, preferably, tetrahydrofuran, chloroform, and mixtures thereof, although there is no limitation thereto. In the reaction, 1 equivalent to large excess, preferably 1 to 10 equivalents of a base may be added. Examples of the base include various organic and inorganic bases such as triethylamine, diisopropylethylamine, DBU, *N,N*-dimethylaniline, pyridine, quinoline, potassium carbonate, sodium carbonate, lithium carbonate, sodium hydrogen carbonate, potassium hydroxide, sodium hydroxide, lithium hydroxide, potassium *tert*-butoxide and the like; various basic anion exchange resins such as Amberlyst A-21 (ROHM AND HAAS) AG1-X8 (Bio-Rad Laboratories) and the like; and various bases immobilized on solid-phases such as polyvinylpyridine morpholinomethyl polystyrene and the like preferably, polyvinylpyridine.

Preparation Method 3: Compound (I-C), which corresponds to Compound (I) wherein X--Y--Z represents C=C-NR^{6a}R^{7a} (wherein R^{6a} and R^{7a} have the same meanings as defined above, respectively) can be synthesized according to Steps 10 to 13 described below: 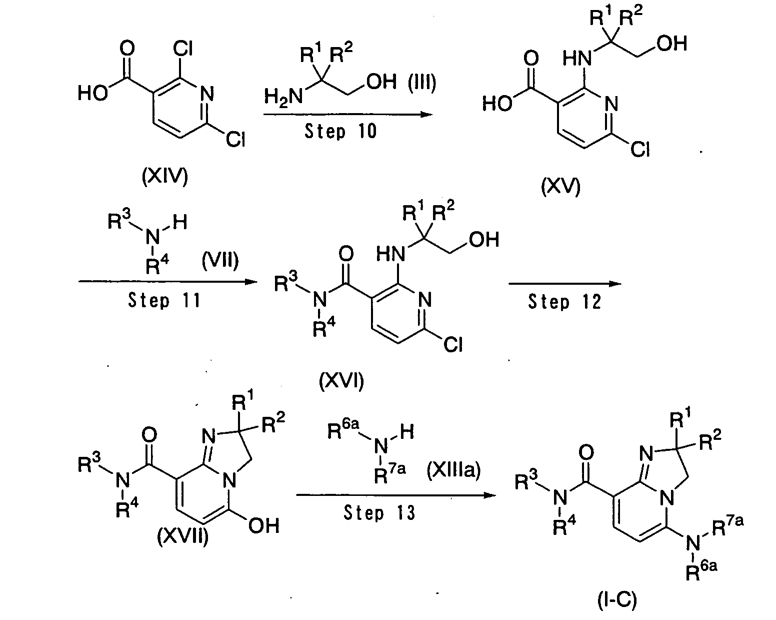 (wherein R¹, R², R³, R⁴, R^{6a} and R^{7a} have the same meanings as defined above, respectively)

### [Step 10]

Compound (XV) can be obtained by reacting commercially available Compound (XIV) with 1 equivalent to large excess, preferably 1 to 3 equivalents of Compound (III) in an inert solvent. Compound (III) is commercially available or can be synthesized by known methods. The reaction is usually performed at a temperature between 50°C and 200°C, preferably at a temperature between 80°C and 150°C, for about 1 hour to 10 days. Any solvent can be used, so long as it is inert to the reaction. Examples of the inert solvent include tetrahydrofuran, dioxane, diethyl ether, diisopropyl ether, benzene, toluene, xylene, ethyl acetate, acetonitrile, dichloromethane, chloroform, dichloroethane, dimethylformamide, dimethylacetamide, *N*-methylpyrrolidone, dimethyl sulfoxide, pyridine, and any mixture thereof, preferably, dioxane, although there is no limitation thereto. In the reaction, 1 equivalent to large excess, preferably 1 to 10 equivalents of a base may be added. Examples of the base include various organic or inorganic bases such as triethylamine, diisopropylethylamine, DBU, *N,N*-dimethylaniline, pyridine, quinoline, potassium carbonate, sodium carbonate, lithium carbonate, sodium hydrogen carbonate, potassium hydroxide, sodium hydroxide, lithium hydroxide, potassium tert-butoxide and the like; various basic anion exchange resins such as Amberlyst A-21 (ROHM AND HAAS), AG1-X8 (Bio-Rad Laboratories) and the like; and various bases immobilized on solid-phases such as polyvinylpyridine, morpholinomethyl polystyrene, and the like preferably, triethylamine.

### [Step 11]

Compound (XVI) can be obtained by reacting Compound (XV) synthesized in Step 10, in a similar manner described in Step 7, with 1 to 5 equivalents of Compound (VII) in the presence of 1 to 10 equivalents of a condensing agent in an inert solvent at a temperature between 0°C and 150°C, preferably at a temperature between room temperature and 60°C for 1 to 120 hours.

### [Step 12]

Compound (XVII) can be obtained by treating Compound (XVI) synthesized in Step 11, in a similar manner described in Step 8, with 1 to 5 equivalents of a chlorinating agent in the presence or absence of a base in an inert solvent at a temperature between room temperature and 200°C, preferably at a temperature between 50°C and 120°C for 1 to 50 hours.

### [Step 13]

Compound (I-C) can be obtained by reacting Compound (XVII) synthesized in Step 12 with 1 to 5 equivalents, preferably 2 to 5 equivalents of Compound (XIIIa) in the presence of 1 to 10 equivalents, preferably 2 to 5 equivalents of a condensing agent in an inert solvent. The reaction is usually performed at a temperature between 0°C and 100°C, preferably at a temperature between room temperature and 80°C, for about 1 to 100 hours. Any solvent can be used, so long as it is inert to the reaction. Examples of the inert solvent include dichloromethane, chloroform, dichloroethane, dimethylformamide, dimethylacetamide, *N*-methylpyrrolidone, dimethyl sulfoxides, tetrahydrofuran, dioxane, diethyl ether, diisopropyl ether, benzene, toluene, xylene, ethyl acetate, acetonitrile, and any mixture thereof, preferably, tetrahydrofuran, although there is no limitation thereto. Examples of the condensing agent include, for example, a combination of dialkylazodicarboxylate and triphenylphosphine or trialkylphosphine, (cyanomethylene)triphenylphosphorane and the like, preferably, (cyanomethylene)triphenylphosphorane.

Intermediates and target compounds obtained in the aforementioned preparation methods can be isolated and purified by separation and purification methods ordinarily used in the field of synthetic organic chemistry, such as, filtration, extraction, washing, drying, concentration, recrystallization, various chromatographic techniques, and the like. Furthermore, these compounds may be purified by purification methods that are generally used in typical parallel synthesis processes with, for example, a scavenger resin or an ion exchange resin. Intermediates may also be used for subsequent reactions without particular purification. For the preparation of salts of Compound (I), the final products may be purified without modification when they are obtained as salts in the aforementioned reaction processes. When the final products are obtained as compounds in a free form, they may be dissolved or suspended in a suitable solvent with addition of an acid or a base to form a salt which may be isolated and purified. It is also possible to convert a final product obtained in a form of a salt into a compound in a free form and then convert the resulting product into a desired salt.

Specific examples of compounds suitable as medicinally active ingredients of the present invention in Compound (I) are shown below. These examples, however, are not intended to limit the scope of the present invention.

Partial structures of the compounds are listed in Tables 1 through 3, wherein Me and Et represent methyl and ethyl, respectively.

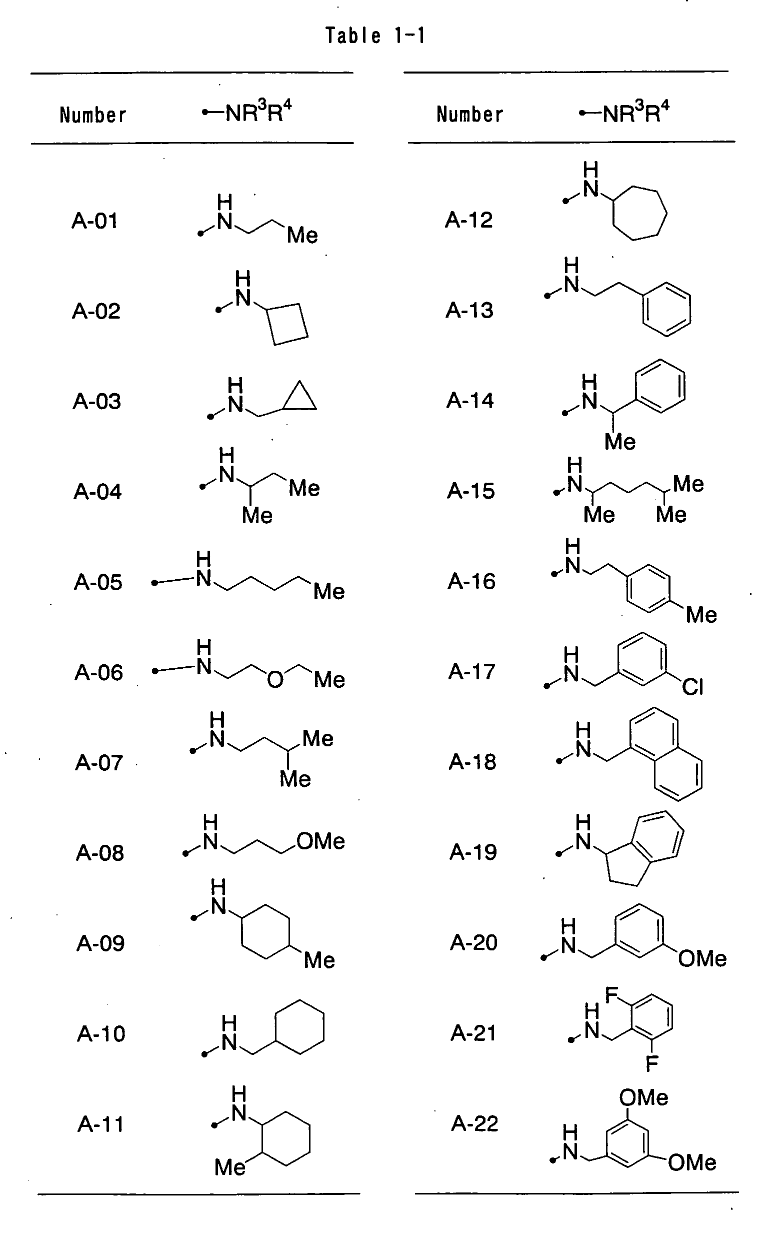

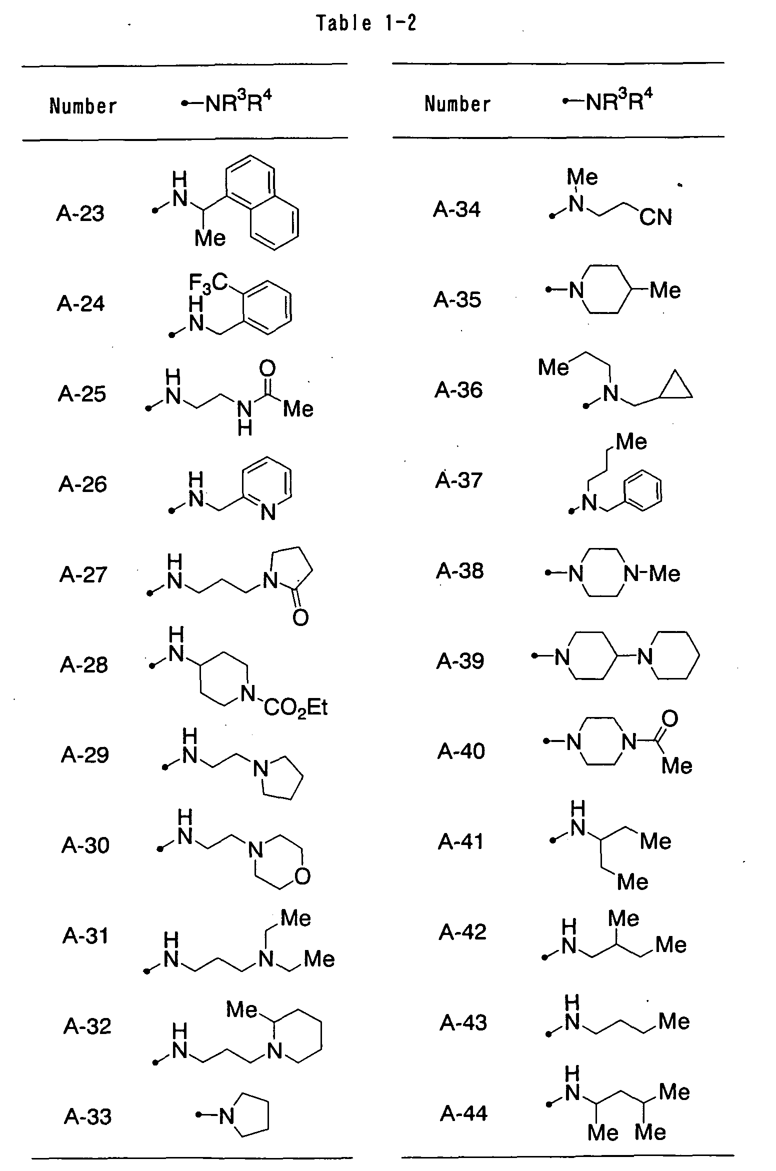

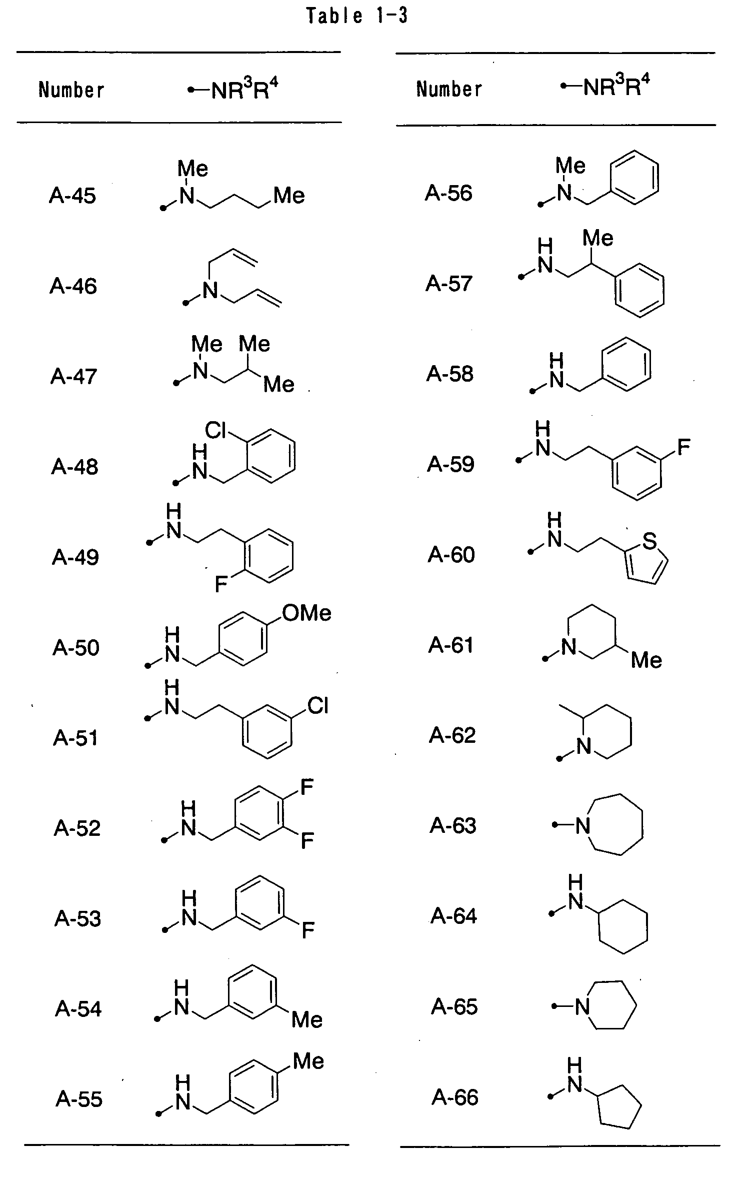

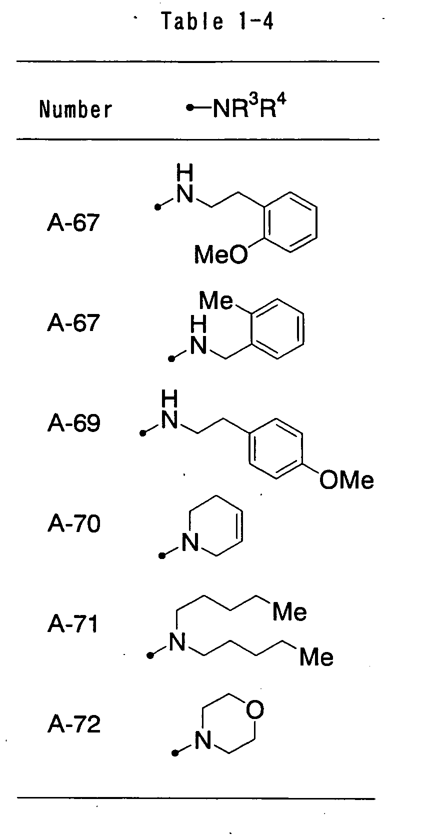

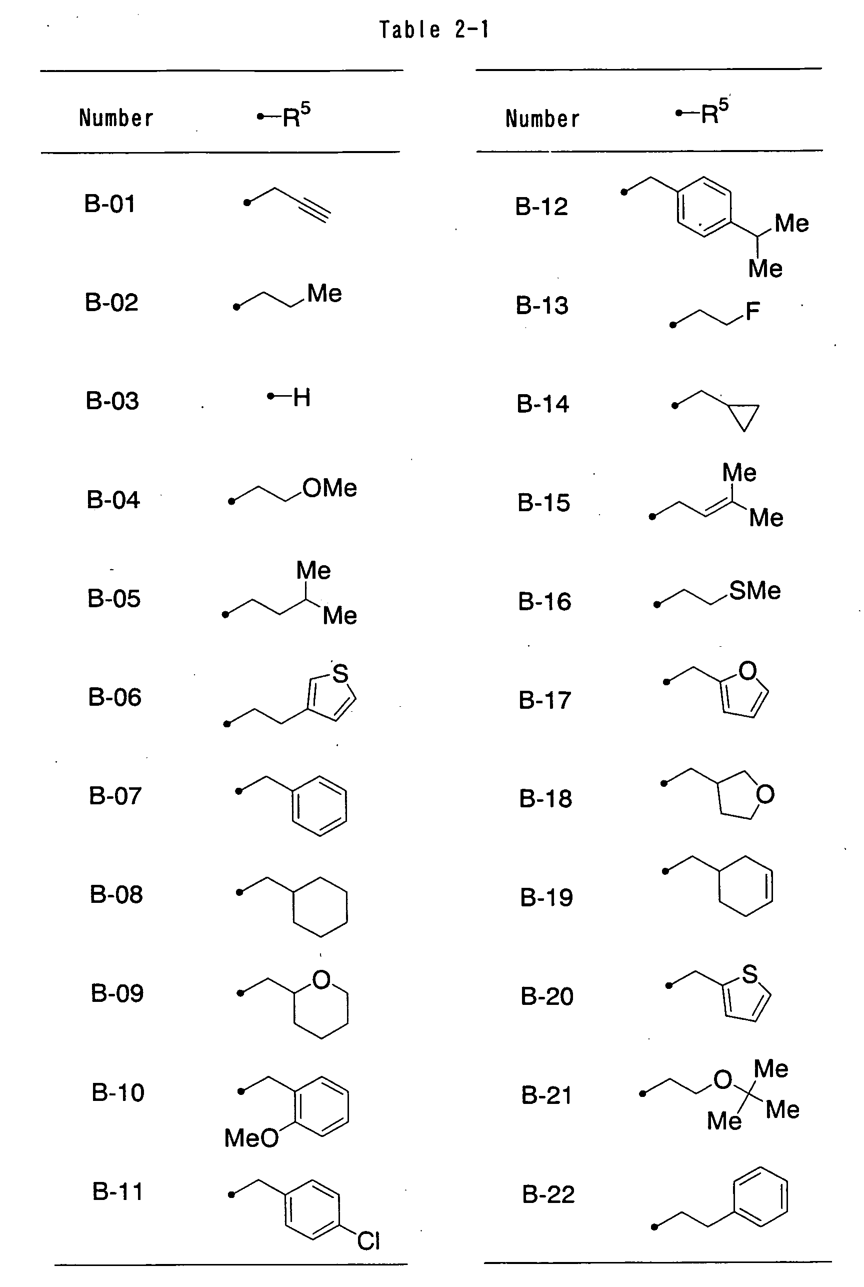

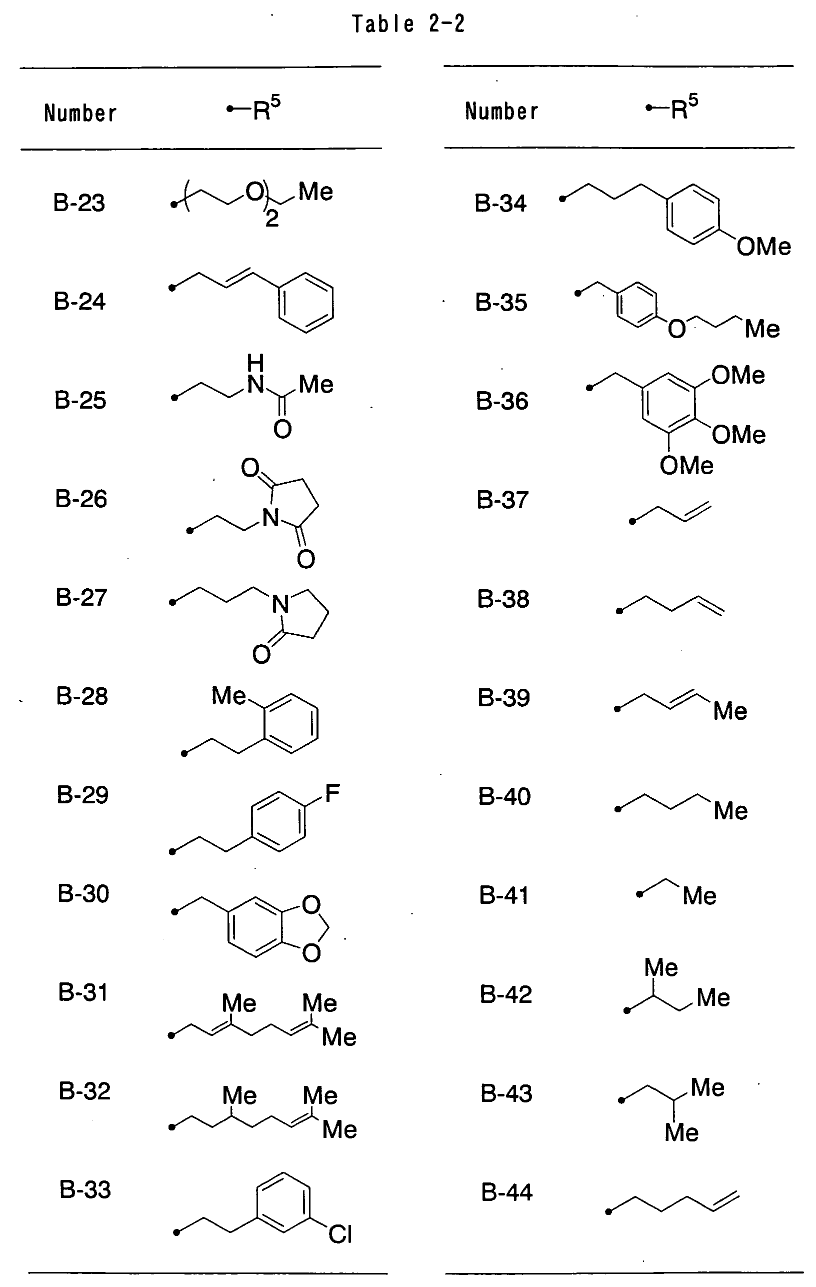

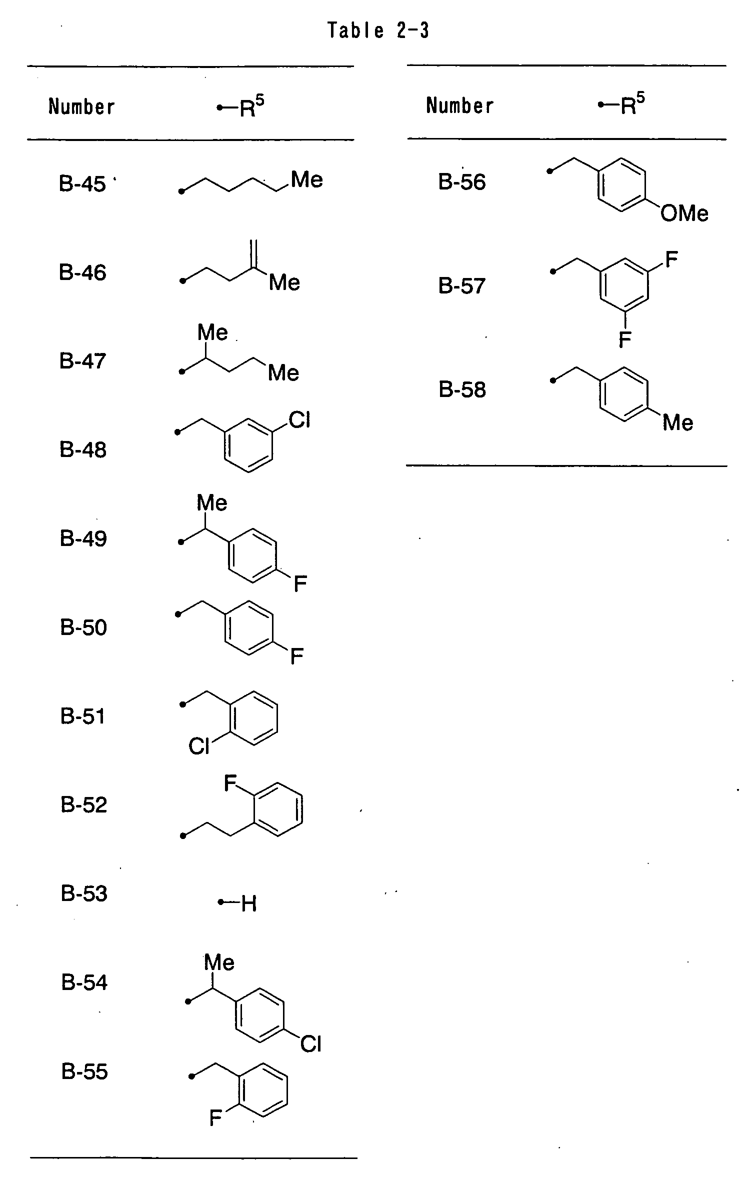

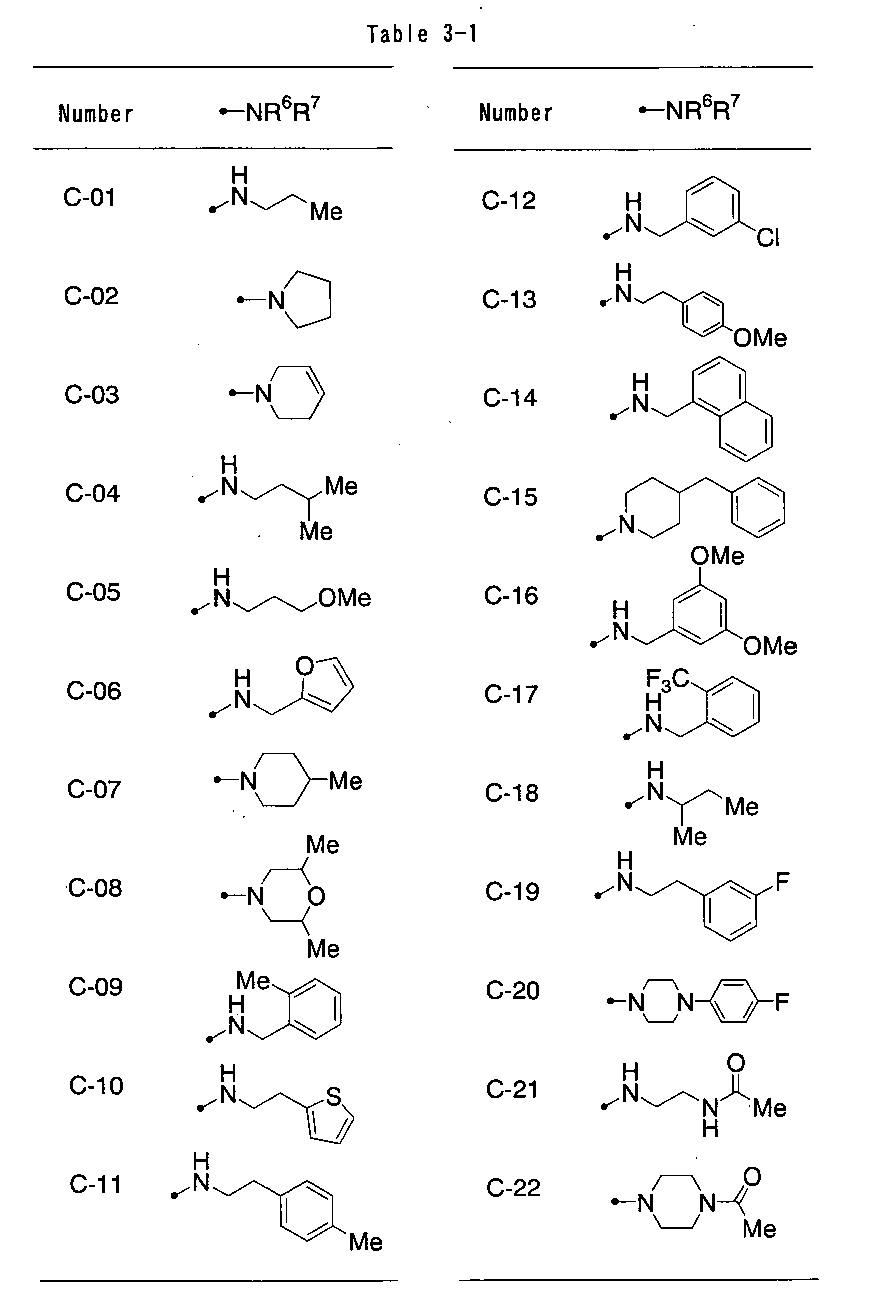

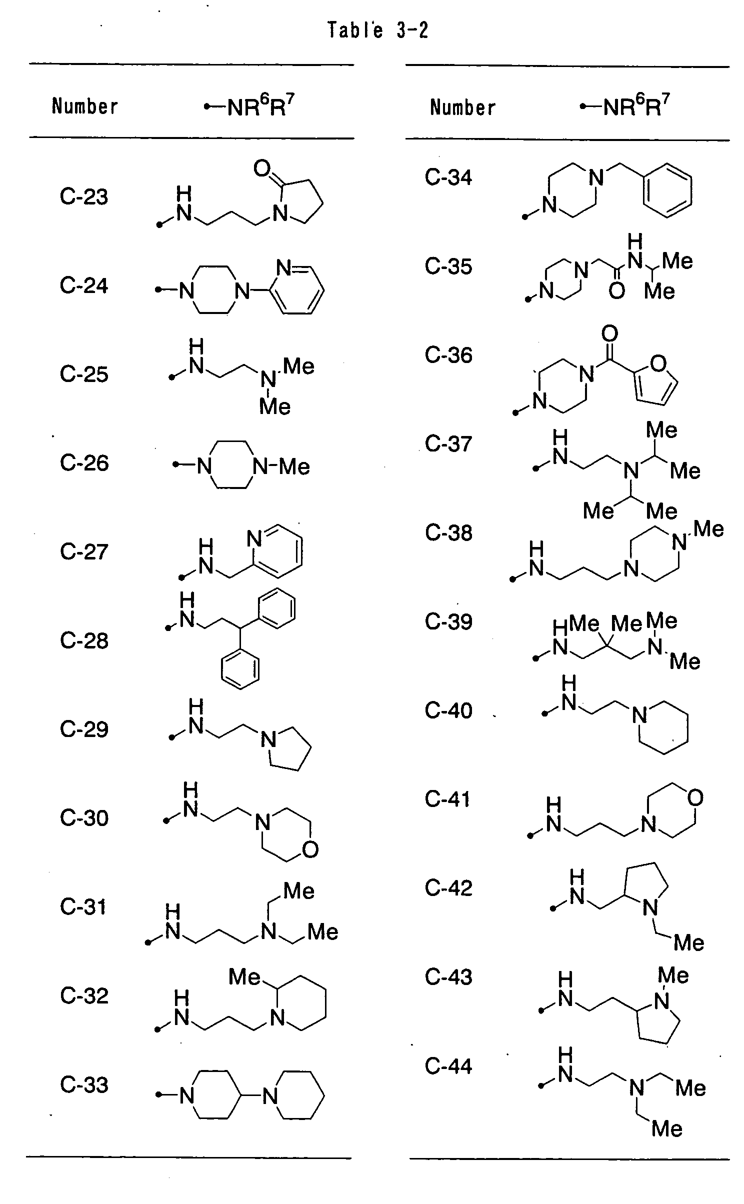

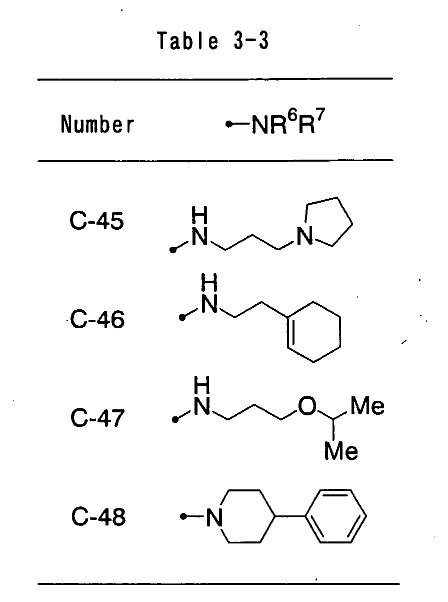

(a) The examples of compounds described in 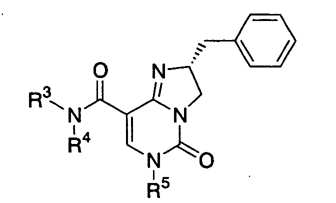 which are included in Compound (I), are listed below in order of compound number, -NR³R⁴, -R⁵ and spectrum data thereof.
   1-1; A-01; B-01; MS m/z 351 (M + H)⁺
   1-2; A-01; B-02; MS m/z 355 (M + H)⁺
   1-3; A-01; B-03; MS m/z 313 (M + H)⁺
   1-4; A-01; B-04; MS m/z 371 (M + H)⁺
   1-5; A-01; B-05; MS m/z 383 (M + H)⁺
   1-6; A-01; B-06; MS m/z 423 (M + H)⁺
   1-7; A-01; B-07; MS m/z 403 (M + H)⁺
   1-8; A-01; B-08; MS m/z 409 (M + H)⁺
   1-9; A-01: B-09; MS m/z 411 (M + H)⁺
   1-10; A-01; B-10; MS m/z 433 (M + H)⁺
   1-11; A-01; B-11; MS m/z 437 (M + H)⁺
   1-12; A-01; B-12; MS m/z 445 (M + H)⁺
   1-13; A-01; B-13; MS m/z 359 (M + H)⁺
   1-14; A-01; B-14; MS m/z 367 (M + H)⁺
   1-15; A-01; B-15; MS m/z 381 (M + H)⁺
   1-16; A-01; B-16; MS m/z 387 (M + H)⁺
   1-17; A-01; B-17; MS m/z 393 (M + H)⁺
   1-18; A-01; B-18; MS m/z 397 (M + H)⁺
   1-19; A-01; B-19; MS m/z 407 (M + H)⁺
   1-20; A-01; B-20; MS m/z 409 (M + H)⁺
   1-21; A-01; B-21; MS m/z 413 (M +. H)⁺
   1-22; A-01; B-22; MS m/z 417 (M + H)⁺
   1-23; A-01; B-23; MS m/z 429 (M + H)⁺
   1-24; A-01; B-24; MS m/z 429 (M + H)⁺
   1-25; A-01; B-25; MS m/z 398 (M + H)⁺
   1-26; A-01; B-26; MS m/z 438 (M + H)⁺
   1-27; A-01; B-27; MS m/z 438 (M + H)⁺
   1-28; A-01; B-28; MS m/z 431 (M + H)⁺
   1-29; A-01; B-29; MS m/z 435 (M + H)⁺
   1-30; A-01; B-30; MS m/z 447 (M + H)⁺
   1-31; A-01; B-31; MS m/z 449 (M + H)⁺
   1-32; A-01; B-32; MS m/z 451 (M + H)⁺
   1-33; A-01; B-33; MS m/z 451 (M + H)⁺
   1-34; A-01; B-34; MS m/z 461 (M + H)⁺
   1-35; A-01; B-35; MS m/z 475 (M + H)⁺
   1-36; A-01; B-36; MS m/z 493 (M + H)⁺
   1-37; A-02; B-01; MS m/z 363 (M + H)⁺
   1-38; A-02; B-02; MS m/z 367 (M + H)⁺
   1-39; A-02; B-03; MS m/z 325 (M + H)⁺
   1-40; A-02; B-04; MS m/z 383 (M + H)⁺
   1-41; A-02; B-05; MS m/z 395 (M + H)⁺
   1-42; A-02; B-06; MS m/z 435 (M + H)⁺
   1-43; A-02; B-07; MS m/z 415 (M + H)⁺
   1-44; A-02; B-08; MS m/z 421 (M + H)⁺
   1-45; A-02; B-09; MS m/z 423 (M + H)⁺
   1-46; A-02; B-10; MS m/z 445 (M + H)⁺
   1-47; A-02; B-11; MS m/z 449 (M + H)⁺
   1-48; A-02; B-12; MS m/z 457 (M + H)⁺
   1-49; A-02; B-13; MS m/z 371 (M + H)⁺
   1-50; A-02; B-14; MS m/z 379 (M + H)⁺
   1-51; A-02; B-15; MS m/z 393 (M + H)⁺
   1-52; A-02; B-16; MS m/z 399 (M + H)⁺
   1-53; A-02; B-17; MS m/z 405 (M + H)⁺
   1-54; A-02; B-18; MS m/z 409 (M + H)⁺
   1-55; A-02; B-19; MS m/z 419 (M + H)⁺
   1-56; A-02; B-20; MS m/z 421 (M + H)⁺
   1-57; A-02; B-21; MS m/z 425 (M + H)⁺
   1-58; A-02; B-22; MS m/z 429 (M + H)⁺
   1-59; A-02; B-23; MS m/z 441 (M + H)⁺
   1-60; A-02; B-24; MS m/z 441 (M + H)⁺
   1-61; A-02; B-25; MS m/z 410 (M + H)⁺
   1-62; A-02; B-26; MS m/z 450 (M + H)⁺
   1-63; A-02; B-27; MS m/z 450 (M + H)⁺
   1-64; A-02; B-28; MS m/z 443 (M + H)⁺
   1-65; A-02; B-29; MS m/z 447 (M + H)⁺
   1-66; A-02; B-30; MS m/z 459 (M + H)⁺
   1-67; A-02; B-31; MS m/z 461 (M + H)⁺
   1-68; A-02; B-32; MS m/z 463 (M + H)⁺
   1-69; A-02; B-33; MS m/z 463 (M + H)⁺
   1-70; A-02; B-34; MS m/z 473 (M + H)⁺
   1-71; A-02; B-35; MS m/z 487 (M + H)⁺
   1-72; A-02; B-36; MS m/z 505 (M + H)⁺
   1-73; A-03; B-01; MS m/z 363 (M + H)⁺
   1-74; A-03; B-02; MS m/z 367 (M + H)⁺
   1-75; A-03; B-03; MS m/z 325 (M + H)⁺
   1-76; A-03; B-04; MS m/z 383 (M + H)⁺
   1-77; A-03; B-05; MS m/z 395 (M + H)⁺
   1-78; A-03; B-06; MS m/z 435 (M + H)⁺
   1-79; A-03; B-07; MS m/z 415 (M + H)⁺
   1-80; A-03; B-08; MS m/z 421 (M + H)⁺
   1-81; A-03; B-09; MS m/z 423 (M + H)⁺
   1-82; A-03; B-10; MS m/z 445 (M + H)⁺
   1-83; A-03; B-11; MS m/z 449 (M + H)⁺
   1-84; A-03; B-12; MS m/z 457 (M + H)⁺
   1-85; A-03; B-13; MS m/z 371 (M + H)⁺
   1-86; A-03; B-14; MS m/z 379 (M + H)⁺
   1-87; A-03; B-15; MS m/z 393 (M + H)⁺
   1-88; A-03; B-16; MS m/z 399 (M + H)⁺
   1-89; A-03; B-17; MS m/z 405 (M + H)⁺
   1-90; A-03; B-18; MS m/z 409 (M + H)⁺
   1-91; A-03; B-19; MS m/z 419 (M + H)⁺
   1-92; A-03; B-20; MS m/z 421 (M + H)⁺
   1-93; A-03; B-21; MS m/z 425 (M + H)⁺
   1-94; A-03; B-22; MS m/z 429 (M + H)⁺
   1-95; A-03; B-23; MS m/z 441 (M + H)⁺
   1-96; A-03; B-24; MS m/z 441 (M + H)⁺
   1-97; A-03; B-25; MS m/z 410 (M + H)⁺
   1-98; A-03; B-26; MS m/z 450 (M + H)⁺
   1-99; A-03; B-27; MS m/z 450 (M + H)⁺
   1-100; A-03; B-28; MS m/z 443 (M + H)⁺
   1-101; A-03; B-29; MS m/z 447 (M + H)⁺
   1-102; A-03; B-30; MS m/z 459 (M + H)⁺
   1-103; A-03; B-31; MS m/z 461 (M + H)⁺
   1-104; A-03; B-32; MS m/z 463 (M + H)⁺
   1-105; A-03; B-33; MS m/z 463 (M + H)⁺
   1-106; A-03; B-34; MS m/z 473 (M + H)⁺
   1-107; A-03; B-35; MS m/z 487 (M + H)⁺
   1-108; A-03; B-36; MS m/z 505 (M + H)⁺
   1-109; A-04; B-01; MS m/z 365 (M + H)⁺
   1-110; A-04; B-02; MS m/z 369 (M + H)⁺
   1-111; A-04; B-03; MS m/z 327 (M + H)⁺
   1-112; A-04; B-04; MS m/z 385 (M + H)⁺
   1-113; A-04; B-05; MS m/z 397 (M + H)⁺
   1-114; A-04; B-06; MS m/z 437 (M + H)⁺
   1-115; A-04; B-07; MS m/z 417 (M + H)⁺
   1-116; A-04; B-08; MS m/z 423 (M + H)⁺
   1-117; A-04; B-09; MS m/z 425 (M + H)⁺
   1-118; A-04; B-10; MS m/z 447 (M + H)⁺
   1-119; A-04; B-11; MS m/z 451 (M + H)⁺
   1-120; A-04; B-12; MS m/z 459 (M + H)⁺
   1-121; A-04; B-13; MS m/z 373 (M + H)⁺
   1-122; A-04; B-14; MS m/z 381 (M + H)⁺
   1-123; A-04; B-15; MS m/z 395 (M + H)⁺
   1-124; A-04; B-16; MS m/z 401 (M + H)⁺
   1-125; A-04; B-17; MS m/z 407 (M + H)⁺
   1-126; A-04; B-18; MS m/z 411 (M + H)⁺
   1-127; A-04; B-19; MS m/z 421 (M + H)⁺
   1-128; A-04; B-20; MS m/z 423 (M + H)⁺
   1-129; A-04; B-21; MS m/z 427 (M + H)⁺
   1-130; A-04; B-22; MS m/z 431 (M + H)⁺
   1-131; A-04; B-23; MS m/z 443 (M + H)⁺
   1-132; A-04; B-24; MS m/z 443 (M + H)⁺
   1-133; A-04; B-25; MS m/z 412 (M + H)⁺
   1-134; A-04; B-26; MS m/z 452 (M + H)⁺
   1-135; A-04; B-27; MS m/z 452 (M + H)⁺
   1-136; A-04; B-28; MS m/z 445 (M + H)⁺
   1-137; A-04; B-29; MS m/z 449 (M + H)⁺
   1-138; A-04; B-30; MS m/z 461 (M + H)⁺
   1-139; A-04; B-31; MS m/z 463 (M + H)⁺
   1-140; A-04; B-32; MS m/z 465 (M + H)⁺
   1-141; A-04; B-33; MS m/z 465 (M + H)⁺
   1-142; A-04; B-34; MS m/z 475 (M + H)⁺
   1-143; A-04; B-35; MS m/z 489 (M + H)⁺
   1-144; A-04; B-36; MS m/z 507 (M + H)⁺
   1-145; A-05; B-01; MS m/z 379 (M + H)⁺
   1-146; A-05; B-02; MS m/z 383 (M + H)⁺
   1-147; A-05; B-03; MS m/z 341 (M + H)⁺
   1-148; A-05; B-04; MS m/z 399 (M + H)⁺
   1-149; A-05; B-05; MS m/z 411 (M + H)⁺
   1-150; A-05; B-06; MS m/z 451 (M + H)⁺
   1-151; A-05; B-07; MS m/z 431 (M + H)⁺
   1-152; A-05; B-08; MS m/z 437 (M + H)⁺
   1-153; A-05; B-09; MS m/z 439 (M + H)⁺
   1-154; A-05; B-10; MS m/z 461 (M + H)⁺
   1-155; A-05; B-11; MS m/z 465 (M + H)⁺
   1-156; A-05; B-12; MS m/z 473 (M + H)⁺
   1-157; A-05; B-13; MS m/z 387 (M + H)⁺
   1-158; A-05; B-14; MS m/z 395 (M + H)⁺
   1-159; A-05; B-15; MS m/z 409 (M + H)⁺
   1-160; A-05; B-16; MS m/z 415 (M + H)⁺
   1-161; A-05; B-17; MS m/z 421 (M + H)⁺
   1-162; A-05; B-18; MS m/z 425 (M + H)⁺
   1-163; A-05; B-19; MS m/z 435 (M + H)⁺
   1-164; A-05; B-20; MS m/z 437 (M + H)⁺
   1-165; A-05; B-21; MS m/z 441 (M + H)⁺
   1-166; A-05; B-22; MS m/z 445 (M + H)⁺
   1-167; A-05; B-23; MS m/z 457 (M + H)⁺
   1-168; A-05; B-24; MS m/z 457 (M + H)⁺
   1-169; A-05; B-25; MS m/z 426 (M + H)⁺
   1-170; A-05; B-26; MS m/z 466 (M + H)⁺
   1-171; A-05; B-27; MS m/z 466 (M + H)⁺
   1-172; A-05; B-28; MS m/z 459 (M + H)⁺
   1-173; A-05; B-29; MS m/z 463 (M + H)⁺
   1-174; A-05; B-30; MS m/z 475 (M + H)⁺
   1-175; A-05; B-31; MS m/z 477 (M + H)⁺
   1-176; A-05; B-32; MS m/z 479 (M + H)⁺
   1-177; A-05; B-33; MS m/z 479 (M + H)⁺
   1-178; A-05; B-34; MS m/z 489 (M + H)⁺
   1-179; A-05; B-35; MS m/z 503 (M + H)⁺
   1-180; A-05; B-36; MS m/z 521 (M + H)⁺
   1-181; A-06; B-01; MS m/z 381 (M + H)⁺
   1-182; A-06; B-02; MS m/z 385 (M + H)⁺
   1-183; A-06; B-03; MS m/z 343 (M + H)⁺
   1-184; A-06; B-04; MS m/z 401 (M + H)⁺
   1-185; A-06; B-05; MS m/z 413 (M + H)⁺
   1-186; A-06; B-06; MS m/z 453 (M + H)⁺
   1-187; A-06; B-07; MS m/z 433 (M + H)⁺
   1-188; A-06; B-08; MS m/z 439 (M + H)⁺
   1-189; A-06; B-09; MS m/z 441 (M + H)⁺
   1-190; A-06; B-10; MS m/z 463 (M + H)⁺
   1-191; A-06; B-11; MS m/z 467 (M + H)⁺
   1-192; A-06; B-12; MS m/z 475 (M + H)⁺
   1-193; A-06; B-13; MS m/z 389 (M + H)⁺
   1-194; A-06; B-14; MS m/z 397 (M + H)⁺
   1-195; A-06; B-15; MS m/z 411 (M + H)⁺
   1-196; A-06; B-16; MS m/z 417 (M + H)⁺
   1-197; A-06; B-17; MS m/z 423 (M + H)⁺
   1-198; A-06; B-18; MS m/z 427 (M + H)⁺
   1-199; A-06; B-19; MS m/z 437 (M + H)⁺
   1-200; A-06; B-20; MS m/z 439 (M + H)⁺
   1-201; A-06; B-21; MS m/z 443 (M + H)⁺
   1-202; A-06; B-22; MS m/z 447 (M + H)⁺
   1-203; A-06; B-23; MS m/z 459 (M + H)⁺
   1-204; A-06; B-24; MS m/z 459 (M + H)⁺
   1-205; A-06; B-25; MS m/z 428 (M + H)⁺
   1-206; A-06; B-26; MS m/z 468 (M + H)⁺
   1-207; A-06; B-27; MS m/z 468 (M + H)⁺
   1-208; A-06; B-28; MS m/z 461 (M + H)⁺
   1-209; A-06; B-29; MS m/z 465 (M + H)⁺
   1-210; A-06; B-30; MS m/z 477 (M + H)⁺
   1-211; A-06; B-31; MS m/z 479 (M + H)⁺
   1-212; A-06; B-32; MS m/z 481 (M + H)⁺
   1-213; A-06; B-33; MS m/z 481 (M + H)⁺
   1-214; A-06; B-34; MS m/z 491 (M + H)⁺
   1-215; A-06; B-35; MS m/z 505 (M + H)⁺
   1-216; A-06; B-36; MS m/z 523 (M + H)⁺
   1-217; A-07; B-01; MS m/z 379 (M + H)⁺
   1-218; A-07; B-02; MS m/z 383 (M + H)⁺
   1-219; A-07; B-03; MS m/z 341 (M + H)⁺
   1-220; A-07; B-04; MS m/z 399 (M + H)⁺
   1-221; A-07; B-05; MS m/z 411 (M + H)⁺
   1-222; A-07; B-06; MS m/z 451 (M + H)⁺
   1-223; A-07; B-07; MS m/z 431 (M + H)⁺
   1-224; A-07; B-08; MS m/z 437 (M + H)⁺
   1-225; A-07; B-09; MS m/z 439 (M + H)⁺
   1-226; A-07; B-10; MS m/z 461 (M + H)⁺
   1-227; A-07; B-11; MS m/z 465 (M + H)⁺
   1-228; A-07; B-12; MS m/z 473 (M + H)⁺
   1-229; A-07; B-13; MS m/z 387 (M + H)⁺
   1-230; A-07; B-14; MS m/z 395 (M + H)⁺
   1-231; A-07; B-15; MS m/z 409 (M + H)⁺
   1-232; A-07; B-16; MS m/z 415 (M + H)⁺
   1-233; A-07; B-17; MS m/z 421 (M + H)⁺
   1-234; A-07; B-18; MS m/z 425 (M + H)⁺
   1-235; A-07; B-19; MS m/z 435 (M + H)⁺
   1-236; A-07; B-20; MS m/z 437 (M + H)⁺
   1-237; A-07; B-21; MS m/z 441 (M + H)⁺
   1-238; A-07; B-22; MS m/z 445 (M + H)⁺
   1-239; A-07; B-23; MS m/z 457 (M + H)⁺
   1-240; A-07; B-24; MS m/z 457 (M + H)⁺
   1-241; A-07; B-25; MS m/z 426 (M + H)⁺
   1-242; A-07; B-26; MS m/z 466 (M + H)⁺
   1-243; A-07; B-27; MS m/z 466 (M + H)⁺
   1-244; A-07; B-28; MS m/z 459 (M + H)⁺
   1-245; A-07; B-29; MS m/z 463 (M + H)⁺
   1-246; A-07; B-30; MS m/z 475 (M + H)⁺
   1-247; A-07; B-31; MS m/z 477 (M + H)⁺
   1-248; A-07; B-32; MS m/z 479 (M + H)⁺
   1-249; A-07; B-33; MS m/z 479 (M + H)⁺
   1-250; A-07; B-34; MS m/z 489 (M + H)⁺
   1-251; A-07; B-35; MS m/z 503 (M + H)⁺
   1-252; A-07; B-36; MS m/z 521 (M + H)⁺
   1-253; A-08; B-01; MS m/z 381 (M + H)⁺
   1-254; A-08; B-02; MS m/z 385 (M + H)⁺
   1-255; A-08; B-03; MS m/z 343 (M + H)⁺
   1-256; A-08; B-04; MS m/z 401 (M + H)⁺
   1-257; A-08; B-05; MS m/z 413 (M + H)⁺
   1-258; A-08; B-06; MS m/z 453 (M + H)⁺
   1-259; A-08; B-07; MS m/z 433 (M + H)⁺
   1-260; A-08; B-08; MS m/z 439 (M + H)⁺
   1-261; A-08; B-09; MS m/z 441 (M + H)⁺
   1-262; A-08; B-10; MS m/z 463 (M + H)⁺
   1-263; A-08; B-11; MS m/z 467 (M + H)⁺
   1-264; A-08; B-12; MS m/z 475 (M + H)⁺
   1-265; A-08; B-13; MS m/z 389 (M + H)⁺
   1-266; A-08; B-14; MS m/z 397 (M + H)⁺
   1-267; A-08; B-15; MS m/z 411 (M + H)⁺
   1-268; A-08; B-16; MS m/z 417 (M + H)⁺
   1-269; A-08; B-17; MS m/z 423 (M + H)⁺
   1-270; A-08; B-18; MS m/z 427 (M + H)⁺
   1-271; A-08; B-19; MS m/z 437 (M + H)⁺
   1-272; A-08; B-20; MS m/z 439 (M + H)⁺
   1-273; A-08; B-21; MS m/z 443 (M + H)⁺
   1-274; A-08; B-22; MS m/z 447 (M + H)⁺
   1-275; A-08; B-23; MS m/z 459 (M + H)⁺
   1-276; A-08; B-24; MS m/z 459 (M + H)⁺
   1-277; A-08; B-25; MS m/z 428 (M + H)⁺
   1-278; A-08; B-26; MS m/z 468 (M + H)⁺
   1-279; A-08; B-27; MS m/z 468 (M + H)⁺
   1-280; A-08; B-28; MS m/z 461 (M + H)⁺
   1-281; A-08; B-29; MS m/z 465 (M + H)⁺
   1-282; A-08; B-30; MS m/z 477 (M + H)⁺
   1-283; A-08; B-31; MS m/z 479 (M + H)⁺
   1-284; A-08; B-32; MS m/z 481 (M + H)⁺
   1-285; A-08; B-33; MS m/z 481 (M + H)⁺
   1-286; A-08; B-34; MS m/z 491 (M + H)⁺
   1-287; A-08; B-35; MS m/z 505 (M + H)⁺
   1-288; A-08; B-36; MS m/z 523 (M + H)⁺
   1-289; A-09; B-01; MS m/z 405 (M + H)⁺
   1-290; A-09; B-02; MS m/z 409 (M + H)⁺
   1-291; A-09; B-03; MS m/z 367 (M + H)⁺
   1-292; A-09; B-04; MS m/z 425 (M + H)⁺
   1-293; A-09; B-05; MS m/z 437 (M + H)⁺
   1-294; A-09; B-06; MS m/z 477 (M + H)⁺
   1-295; A-09; B-07; MS m/z 457 (M + H)⁺
   1-296; A-09; B-08; MS m/z 463 (M + H)⁺
   1-297; A-09; B-09; MS m/z 465 (M + H)⁺
   1-298; A-09; B-10; MS m/z 487 (M + H)⁺
   1-299; A-09; B-11; MS m/z 491 (M + H)⁺
   1-301; A-09; B-12; MS m/z 499 (M + H)⁺
   1-302; A-09; B-13; MS m/z 413 (M + H)⁺
   1-303; A-09; B-14; MS m/z 421 (M + H)⁺
   1-304; A-09; B-15; MS m/z 435 (M + H)⁺
   1-305; A-09; B-16; MS m/z 441 (M + H)⁺
   1-306; A-09; B-17; MS m/z 447 (M + H)⁺
   1-307; A-09; B-18; MS m/z 451 (M + H)⁺
   1-308; A-09; B-19; MS m/z 461 (M + H)⁺
   1-309; A-09; B-20; MS m/z 463 (M + H)⁺
   1-310; A-09; B-21; MS m/z 467 (M + H)⁺
   1-311; A-09; B-22; MS m/z 471 (M + H)⁺
   1-312; A-09; B-23; MS m/z 483 (M + H)⁺
   1-313; A-09; B-24; MS m/z 483 (M + H)⁺
   1-314; A-09; B-25; MS m/z 452 (M + H)⁺
   1-315; A-09; B-26; MS m/z 492 (M + H)⁺
   1-316; A-09; B-27; MS m/z 492 (M + H)⁺
   1-317; A-09; B-28; MS m/z 485 (M + H)⁺
   1-318; A-09; B-29; MS m/z 489 (M + H)⁺
   1-319; A-09; B-30; MS m/z 501 (M + H)⁺
   1-320; A-09; B-31; MS m/z 503 (M + H)⁺
   1-321; A-09; B-32; MS m/z 505 (M + H)⁺
   1-322; A-09; B-33; MS m/z 505 (M + H)⁺
   1-323; A-09; B-34; MS m/z 515 (M + H)⁺
   1-324; A-09; B-35; MS m/z 529 (M + H)⁺
   1-325; A-09; B-36; MS m/z 547 (M + H)⁺
   1-326; A-09; B-48; MS m/z 477 (M + H)⁺
   1-327; A-09; B-49; MS m/z 475 (M + H)⁺
   1-328; A-09; B-50; MS m/z 461 (M + H)⁺
   1-329; A-09; B-51; MS m/z 477 (M + H)⁺
   1-330; A-09; B-52; MS m/z 475 (M + H)⁺
   1-331; A-09; B-53; MS m/z 463 (M + H)⁺
   1-332; A-09; B-54; MS m/z 491 (M + H)⁺
   1-333; A-09; B-55; MS m/z 461 (M + H)⁺
   1-334; A-09; B-56; MS m/z 473 (M + H)⁺
   1-335; A-09; B-57; MS m/z 479 (M + H)⁺
   1-336; A-09; B-58; MS m/z 457 (M + H)⁺
   1-337; A-10; B-01; MS m/z 405 (M + H)⁺
   1-338; A-10; B-02; MS m/z 409 (M + H)⁺
   1-339; A-10; B-03; MS m/z 367 (M + H)⁺
   1-340; A-10; B-04; MS m/z 425 (M + H)⁺
   1-341; A-10; B-05; MS m/z 437 (M + H)⁺
   1-342; A-10; B-06; MS m/z 477 (M + H)⁺
   1-343; A-10; B-07; MS m/z 457 (M + H)⁺
   1-344; A-10; B-08; MS m/z 463 (M + H)⁺
   1-345; A-10; B-09; MS m/z 465 (M + H)⁺
   1-346; A-10; B-10; MS m/z 487 (M + H)⁺
   1-347; A-10; B-11; MS m/z 491 (M + H)⁺
   1-348; A-10; B-12; MS m/z 499 (M + H)⁺
   1-349; A-10; B-13; MS m/z 413 (M + H)⁺
   1-350; A-10; B-14; MS m/z 421 (M + H)⁺
   1-351; A-10; B-15; MS m/z 435 (M + H)⁺
   1-352; A-10; B-16; MS m/z 441 (M + H)⁺
   1-353; A-10; B-17; MS m/z 447 (M + H)⁺
   1-354; A-10; B-18; MS m/z 451 (M + H)⁺
   1-355; A-10; B-19; MS m/z 461 (M + H)⁺
   1-356; A-10; B-20; MS m/z 463 (M + H)⁺
   1-357; A-10; B-21; MS m/z 467 (M + H)⁺
   1-358; A-10; B-22; MS m/z 471 (M + H)⁺
   1-359; A-10; B-23; MS m/z 483 (M + H)⁺
   1-360; A-10; B-24; MS m/z 483 (M + H)⁺
   1-361; A-10; B-25; MS m/z 452 (M + H)⁺
   1-362; A-10; B-26; MS m/z 492 (M + H)⁺
   1-363; A-10; B-27; MS m/z 492 (M + H)⁺
   1-364; A-10; B-28; MS m/z 485 (M + H)⁺
   1-365; A-10; B-29; MS m/z 489 (M + H)⁺
   1-366; A-10; B-30; MS m/z 501 (M + H)⁺
   1-367; A-10; B-31; MS m/z 503 (M + H)⁺
   1-368; A-10; B-32; MS m/z 505 (M + H)⁺
   1-369; A-10; B-33; MS m/z 505 (M + H)⁺
   1-370; A-10; B-34; MS m/z 515 (M + H)⁺
   1-371; A-10; B-35; MS m/z 529 (M + H)⁺
   1-372; A-10; B-36; MS m/z 547 (M + H)⁺
   1-373; A-11; B-01; MS m/z 405 (M + H)⁺
   1-374; A-11; B-02; MS m/z 409 (M + H)⁺
   1-375; A-11; B-03; MS m/z 367 (M + H)⁺
   1-376; A-11; B-04; MS m/z 425 (M + H)⁺
   1-377; A-11; B-05; MS m/z 437 (M + H)⁺
   1-378; A-11; B-06; MS m/z 477 (M + H)⁺
   1-379; A-11; B-07; MS m/z 457 (M + H)⁺
   1-380; A-11; B-08; MS m/z 463 (M + H)⁺
   1-381; A-11; B-09; MS m/z 465 (M + H)⁺
   1-382; A-11; B-10; MS m/z 487 (M + H)⁺
   1-383: A-11; B-11; MS m/z 491 (M + H)⁺
   1-384; A-11; B-12; MS m/z 499 (M + H)⁺
   1-385; A-11; B-13; MS m/z 413 (M + H)⁺
   1-386; A-11; B-14; MS m/z 421 (M + H)⁺
   1-387; A-11; B-15; MS m/z 435 (M + H)⁺
   1-388; A-11; B-16; MS m/z 441 (M + H)⁺
   1-389; A-11; B-17; MS m/z 447 (M + H)⁺
   1-390; A-11; B-18; MS m/z 451 (M + H)⁺
   1-391: A-11; B-19; MS m/z 461 (M + H)⁺
   1-392: A-11; B-20; MS m/z 463 (M + H)⁺
   1-393; A-11; B-21; MS m/z 467 (M + H)⁺
   1-394; A-11; B-22; MS m/z 471 (M + H)⁺
   1-395; A-11; B-23; MS m/z 483 (M + H)⁺
   1-396; A-11; B-24; MS m/z 483 (M + H)⁺
   1-397; A-11; B-25; MS m/z 452 (M + H)⁺
   1-398; A-11; B-26; MS m/z 492 (M + H)⁺
   1-399; A-11; B-27; MS m/z 492 (M + H)⁺
   1-400; A-11; B-28; MS m/z 485 (M + H)⁺
   1-401; A-11; B-29; MS m/z 489 (M + H)⁺
   1-402; A-11; B-30; MS m/z 501 (M + H)⁺
   1-403; A-11; B-31; MS m/z 503 (M + H)⁺
   1-404; A-11; B-32; MS m/z 505 (M + H)⁺
   1-405; A-11; B-33; MS m/z 505 (M + H)⁺
   1-406; A-11; B-34; MS m/z 515 (M + H)⁺
   1-407; A-11; B-35; MS m/z 529 (M + H)⁺
   1-408; A-11; B-36; MS m/z 547 (M + H)⁺
   1-409; A-12; B-01; MS m/z 405 (M + H)⁺
   1-410; A-12; B-02; MS m/z 409 (M + H)⁺
   1-411; A-12; B-03; MS m/z 367 (M + H)⁺
   1-412; A-12; B-04; MS m/z 425 (M + H)⁺
   1-413; A-12; B-05; MS m/z 437 (M + H)⁺
   1-414; A-12; B-06; MS m/z 477 (M + H)⁺
   1-415; A-12; B-07; MS m/z 457 (M + H)⁺
   1-416; A-12; B-08; MS m/z 463 (M + H)⁺
   1-417; A-12; B-09; MS m/z 465 (M + H)⁺
   1-418; A-12; B-10; MS m/z 487 (M + H)⁺
   1-419; A-12; B-11; MS m/z 491 (M + H)⁺
   1-420; A-12; B-12; MS m/z 499 (M + H)⁺
   1-421; A-12; B-13; MS m/z 413 (M + H)⁺
   1-422; A-12; B-14; MS m/z 421 (M + H)⁺
   1-423; A-12; B-15;. MS m/z 435 (M + H)⁺
   1-424; A-12; B-16; MS m/z 441 (M + H)⁺
   1-425; A-12; B-17; MS m/z 447 (M + H)⁺
   1-426; A-12; B-18; MS m/z 451 (M + H)⁺
   1-427; A-12; B-19; MS m/z 461 (M + H)⁺
   1-428; A-12; B-20; MS m/z 463 (M + H)⁺
   1-429; A-12; B-21; MS m/z 467 (M + H)⁺
   1-430; A-12; B-22; MS m/z 471 (M + H)⁺
   1-431; A-12; B-23; MS m/z 483 (M + H)⁺
   1-432; A-12; B-24; MS m/z 483 (M + H)⁺
   1-433; A-12; B-25; MS m/z 452 (M + H)⁺
   1-434; A-12; B-26; MS m/z 492 (M + H)⁺
   1-435; A-12; B-27; MS m/z 492 (M + H)⁺
   1-436; A-12; B-28; MS m/z 485 (M + H)⁺
   1-437; A-12; B-29; MS m/z 489 (M + H)⁺
   1-438; A-12; B-30; MS m/z 501 (M + H)⁺
   1-439; A-12; B-31; MS m/z 503 (M + H)⁺
   1-440; A-12; B-32; MS m/z 505 (M + H)⁺
   1-441; A-12; B-33; MS m/z 505 (M + H)⁺
   1-442; A-12; B-34; MS m/z 515 (M + H)⁺
   1-443; A-12; B-35; MS m/z 529 (M + H)⁺
   1-444; A-12; B-36; MS m/z 547 (M + H)⁺
   1-445; A-13; B-01; MS m/z 413 (M + H)⁺
   1-446; A-13; B-02; MS m/z 417 (M + H)⁺
   1-448; A-13; B-03; MS m/z 375 (M + H)⁺
   1-449; A-13; B-04; MS m/z 433 (M + H)⁺
   1-450; A-13; B-05; MS m/z 445 (M + H)⁺
   1-451; A-13; B-06; MS m/z 485 (M + H)⁺
   1-452; A-13; B-07; MS m/z 465 (M + H)⁺
   1-453; A-13; B-08; MS m/z 471 (M + H)⁺
   1-454; A-13; B-09; MS m/z 473 (M + H)⁺
   1-455; A-13; B-10; MS m/z 495 (M + H)⁺
   1-456; A-13; B-11; MS m/z 499 (M + H)⁺
   1-457; A-13; B-12; MS m/z 507 (M + H)⁺
   1-458; A-13; B-13; MS m/z 421 (M + H)⁺
   1-459; A-13; B-14; MS m/z 429 (M + H)⁺
   1-460; A-13; B-15; MS m/z 443 (M + H)⁺
   1-461; A-13; B-16; MS m/z 449 (M + H)⁺
   1-462; A-13; B-17; MS m/z 455 (M + H)⁺
   1-463; A-13; B-18; MS m/z 459 (M + H)⁺
   1-464; A-13; B-19; MS m/z 469 (M + H)⁺
   1-465; A-13; B-20; MS m/z 471 (M + H)⁺
   1-466; A-13; B-21; MS m/z 475 (M + H)⁺
   1-467; A-13; B-22; MS m/z 479 (M + H)⁺
   1-468; A-13; B-23; MS m/z 491 (M + H)⁺
   1-469; A-13; B-24; MS m/z 491 (M + H)⁺
   1-470; A-13; B-25; MS m/z 460 (M + H)⁺
   1-471; A-13; B-26; MS m/z 500 (M + H)⁺
   1-472; A-13; B-27; MS m/z 500 (M + H)⁺
   1-473; A-13; B-28; MS m/z 493 (M + H)⁺
   1-474; A-13; B-29; MS m/z 497 (M + H)⁺
   1-475; A-13; B-30; MS m/z 509 (M + H)⁺
   1-476; A-13; B-31; MS m/z 511 (M + H)⁺
   1-477; A-13; B-32; MS m/z 513 (M + H)⁺
   1-478; A-13; B-33; MS m/z 513 (M + H)⁺
   1-479; A-13; B-34; MS m/z 523 (M + H)⁺
   1-480; A-13; B-35; MS m/z 537 (M + H)⁺
   1-481; A-13; B-36; MS m/z 555 (M + H)⁺
   1-482; A-13; B-37; MS m/z 415 (M + H)⁺
   1-483; A-13; B-38; MS m/z 429 (M + H)⁺
   1-484; A-13; B-39; MS m/z 429 (M + H)⁺
   1-485; A-13; B-40; MS m/z 431 (M + H)⁺
   1-486; A-13; B-41; MS m/z 403 (M + H)⁺
   1-487; A-13; B-42; MS m/z 431 (M + H)⁺
   1-488; A-13; B-43; MS m/z 431 (M + H)⁺
   1-489; A-13; B-44; MS m/z 443 (M + H)⁺
   1-490; A-13; B-45; MS m/z 445 (M + H)⁺
   1-491; A-13; B-46; MS m/z 443 (M + H)⁺
   1-492; A-13; B-47; MS m/z 445 (M + H)⁺
   1-493; A-14; B-01; MS m/z 413 (M + H)⁺
   1-494; A-14; B-02; MS m/z 417 (M + H)⁺
   1-495; A-14; B-03; MS m/z 375 (M + H)⁺
   1-496; A-14; B-04; MS m/z 433 (M + H)⁺
   1-497; A-14; B-05; MS m/z 445 (M + H)⁺
   1-498; A-14; B-06; MS m/z 485 (M + H)⁺
   1-499; A-14; B-07; MS m/z 465 (M + H)⁺
   1-500; A-14; B-08; MS m/z 471 (M + H)⁺
   1-501; A-14; B-09; MS m/z 473 (M + H)⁺
   1-502; A-14; B-10; MS m/z 495 (M + H)⁺
   1-503; A-14; B-11; MS m/z 499 (M + H)⁺
   1-504; A-14; B-12; MS m/z 507 (M + H)⁺
   1-505; A-14; B-13; MS m/z 421 (M + H)⁺
   1-506; A-14; B-14; MS m/z 429 (M + H)⁺
   1-507; A-14; B-15; MS m/z 443 (M + H)⁺
   1-508; A-14; B-16; MS m/z 449 (M + H)⁺
   1-509; A-14; B-17; MS m/z 455 (M + H)⁺
   1-510; A-14; B-18; MS m/z 459 (M + H)⁺
   1-511: A-14; B-19; MS m/z 469 (M + H)⁺
   1-512; A-14; B-20; MS m/z 471 (M + H)⁺
   1-513; A-14; B-21; MS m/z 475 (M + H)⁺
   1-514; A-14; B-22; MS m/z 479 (M + H)⁺
   1-515; A-14; B-23; MS m/z 491 (M + H)⁺
   1-516; A-14; B-24; MS m/z 491 (M + H)⁺
   1-517; A-14; B-25; MS m/z 460 (M + H)⁺
   1-518; A-14; B-26; MS m/z 500 (M + H)⁺
   1-519; A-14; B-27; MS m/z 500 (M + H)⁺
   1-520; A-14; B-28; MS m/z 493 (M + H)⁺
   1-521; A-14; B-29; MS m/z 497 (M + H)⁺
   1-522; A-14; B-30; MS m/z 509 (M + H)⁺
   1-523; A-14; B-31; MS m/z 511 (M + H)⁺
   1-524; A-14; B-32; MS m/z 513 (M + H)⁺
   1-525; A-14; B-33; MS m/z 513 (M + H)⁺
   1-526; A-14; B-34; MS m/z 523 (M + H)⁺
   1-527; A-14; B-35; MS m/z 537 (M + H)⁺
   1-528: A-14; B-36; MS m/z 555 (M + H)⁺
   1-529; A-15; B-01; MS m/z 421 (M + H)⁺
   1-530; A-15; B-02; MS m/z 425 (M + H)⁺
   1-531; A-15; B-03; MS m/z 383 (M + H)⁺
   1-532; A-15; B-04; MS m/z 441 (M + H)⁺
   1-533; A-15; B-05; MS m/z 453 (M + H)⁺
   1-534; A-15; B-06; MS m/z 493 (M + H)⁺
   1-535; A-15; B-07; MS m/z 473 (M + H)⁺
   1-536; A-15; B-08; MS m/z 479 (M + H)⁺
   1-537; A-15; B-09; MS m/z 481 (M + H)⁺
   1-538; A-15; B-10; MS m/z 503 (M + H)⁺
   1-539; A-15; B-11; MS m/z 507 (M + H)⁺
   1-540; A-15; B-12; MS m/z 515 (M + H)⁺
   1-541; A-15; B-13; MS m/z 429 (M + H)⁺
   1-542; A-15; B-14; MS m/z 437 (M + H)⁺
   1-543; A-15; B-15; MS m/z 451 (M + H)⁺
   1-544; A-15; B-16; MS m/z 457 (M + H)⁺
   1-545; A-15; B-17; MS m/z 463 (M + H)⁺
   1-546; A-15; B-18; MS m/z 467 (M + H)⁺
   1-547; A-15; B-19; MS m/z 477 (M + H)⁺
   1-548; A-15; B-20; MS m/z 479 (M + H)⁺
   1-549; A-15; B-21; MS m/z 483 (M + H)⁺
   1-550; A-15; B-22; MS m/z 487 (M + H)⁺
   1-551; A-15; B-23; MS m/z 499 (M + H)⁺
   1-552; A-15; B-24; MS m/z 499 (M + H)⁺
   1-553; A-15; B-25; MS m/z 468 (M + H)⁺
   1-554; A-15; B-26; MS m/z 508 (M + H)⁺
   1-555; A-15; B-27; MS m/z 508 (M + H)⁺
   1-556; A-15; B-28; MS m/z 501 (M + H)⁺
   1-557; A-15; B-29; MS m/z 505 (M + H)⁺
   1-558; A-15; B-30; MS m/z 517 (M + H)⁺
   1-559; A-15; B-31; MS m/z 519 (M + H)⁺
   1-560; A-15; B-32; MS m/z 521 (M + H)⁺
   1-561; A-15; B-33; MS m/z 521 (M + H)⁺
   1-562; A-15; B-34; MS m/z 531 (M + H)⁺
   1-563; A-15; B-35; MS m/z 545 (M + H)⁺
   1-564; A-15; B-36; MS m/z 563 (M + H)⁺
   1-565; A-16; B-01; MS m/z 427 (M + H)⁺
   1-566; A-16; B-02; MS m/z 431 (M + H)⁺
   1-567; A-16; B-03; MS m/z 389 (M + H)⁺
   1-568; A-16; B-04; MS m/z 447 (M + H)⁺
   1-569; A-16; B-05; MS m/z 459 (M + H)⁺
   1-570; A-16; B-06; MS m/z 499 (M + H)⁺
   1-571; A-16; B-07; MS m/z 479 (M + H)⁺
   1-572; A-16; B-08; MS m/z 485 (M + H)⁺
   1-573; A-16; B-09; MS m/z 487 (M + H)⁺
   1-574; A-16; B-10; MS m/z 509 (M + H)⁺
   1-575; A-16; B-11; MS m/z 513 (M + H)⁺
   1-576; A-16; B-12; MS m/z 521 (M + H)⁺
   1-577; A-16; B-13; MS m/z 435 (M + H)⁺
   1-578; A-16; B-14; MS m/z 443 (M + H)⁺
   1-579; A-16; B-15; MS m/z 457 (M + H)⁺
   1-580; A-16; B-16; MS m/z 463 (M + H)⁺
   1-581; A-16; B-17; MS m/z 469 (M + H)⁺
   1-582; A-16; B-18; MS m/z 473 (M + H)⁺
   1-583; A-16; B-19; MS m/z 483 (M + H)⁺
   1-584; A-16; B-20; MS m/z 485 (M + H)⁺
   1-585; A-16; B-21; MS m/z 489 (M + H)⁺
   1-586; A-16; B-22; MS m/z 493 (M + H)⁺
   1-587; A-16; B-23; MS m/z 505 (M + H)⁺
   1-588; A-16; B-24; MS m/z 505 (M + H)⁺
   1-589; A-16; B-25; MS m/z 474 (M + H)⁺
   1-590; A-16; B-26; MS m/z 514 (M + H)⁺
   1-591; A-16; B-27; MS m/z 514 (M + H)⁺
   1-592; A-16; B-28; MS m/z 507 (M + H)⁺
   1-593; A-16; B-29; MS m/z 511 (M + H)⁺
   1-594; A-16; B-30; MS m/z 523 (M + H)⁺
   1-595; A-16; B-31; MS m/z 525 (M + H)⁺
   1-596; A-16; B-32; MS m/z 527 (M + H)⁺
   1-597; A-16; B-33; MS m/z 527 (M + H)⁺
   1-598; A-16; B-34; MS m/z 537 (M + H)⁺
   1-599; A-16; B-35; MS m/z 551 (M + H)⁺
   1-600; A-16; B-36; MS m/z 569 (M + H)⁺
   1-601; A-17; B-01; MS m/z 433 (M + H)⁺
   1-602; A-17; B-02; MS m/z 437 (M + H)⁺
   1-603; A-17; B-03; MS m/z 395 (M + H)⁺
   1-604; A-17; B-04; MS m/z 453 (M + H)⁺
   1-605; A-17; B-05; MS m/z 465 (M + H)⁺
   1-606; A-17; B-06; MS m/z 505 (M + H)⁺
   1-607; A-17; B-07; MS m/z 485 (M + H)⁺
   1-608; A-17; B-08; MS m/z 491 (M + H)⁺
   1-609; A-17; B-09; MS m/z 493 (M + H)⁺
   1-610; A-17; B-10; MS m/z 515 (M + H)⁺
   1-611; A-17; B-11; MS m/z 519 (M + H)⁺
   1-612; A-17; B-12; MS m/z 527 (M + H)⁺
   1-613; A-17; B-13; MS m/z 441 (M + H)⁺
   1-614; A-17; B-14; MS m/z 449 (M + H)⁺
   1-615; A-17; B-15; MS m/z 463 (M + H)⁺
   1-616; A-17; B-16; MS m/z 469 (M + H)⁺
   1-617; A-17; B-17; MS m/z 475 (M + H)⁺
   1-618; A-17; B-18; MS m/z 479 (M + H)⁺
   1-619; A-17; B-19; MS m/z 489 (M + H)⁺
   1-620; A-17; B-20; MS m/z 491 (M + H)⁺
   1-621; A-17; B-21; MS m/z 495 (M + H)⁺
   1-622; A-17; B-22; MS m/z 499 (M + H)⁺
   1-623; A-17; B-23; MS m/z 511 (M + H)⁺
   1-624; A-17; B-24; MS m/z 511 (M + H)⁺
   1-625; A-17; B-25; MS m/z 480 (M + H)⁺
   1-626; A-17; B-26; MS m/z 520 (M + H)⁺
   1-627; A-17; B-27; MS m/z 520 (M + H)⁺
   1-628; A-17; B-28; MS m/z 513 (M + H)⁺
   1-629; A-17; B-29; MS m/z 517 (M + H)⁺
   1-630; A-17; B-30; MS m/z 529 (M + H)⁺
   1-631; A-17; B-31; MS m/z 531 (M + H)⁺
   1-632; A-17; B-32; MS m/z 533 (M + H)⁺
   1-633; A-17; B-33; MS m/z 533 (M + H)⁺
   1-634; A-17; B-34; MS m/z 543 (M + H)⁺
   1-635; A-17; B-35; MS m/z 557 (M + H)⁺
   1-636; A-17; B-36; MS m/z 575 (M + H)⁺
   1-637; A-18; B-01; MS m/z 449 (M + H)⁺
   1-638; A-18; B-02; MS m/z 453 (M + H)⁺
   1-639; A-18; B-03; MS m/z 411 (M + H)⁺
   1-640; A-18; B-04; MS m/z 469 (M + H)⁺
   1-641; A-18; B-05; MS m/z 481 (M + H)⁺
   1-642; A-18; B-06; MS m/z 521 (M + H)⁺
   1-643; A-18; B-07; MS m/z 501 (M + H)⁺
   1-644; A-18; B-08; MS m/z 507 (M + H)⁺
   1-645; A-18; B-09; MS m/z 509 (M + H)⁺
   1-646; A-18; B-10; MS m/z 531 (M + H)⁺
   1-647; A-18; B-11; MS m/z 535 (M + H)⁺
   1-648; A-18; B-12; MS m/z 543 (M + H)⁺
   1-649; A-18; B-13; MS m/z 457 (M + H)⁺
   1-650; A-18; B-14; MS m/z 465 (M + H)⁺
   1-651; A-18; B-15; MS m/z 479 (M + H)⁺
   1-652; A-18; B-16; MS m/z 485 (M + H)⁺
   1-653; A-18; B-17; MS m/z 491 (M + H)⁺
   1-654; A-18; B-18; MS m/z 495 (M + H)⁺
   1-655; A-18; B-19; MS m/z 505 (M + H)⁺
   1-656; A-18; B-20; MS m/z 507 (M + H)⁺
   1-657; A-18; B-21; MS m/z 511 (M + H)⁺
   1-658; A-18; B-22; MS m/z 515 (M + H)⁺
   1-659; A-18; B-23; MS m/z 527 (M + H)⁺
   1-660; A-18; B-24; MS m/z 527 (M + H)⁺
   1-661; A-18; B-26; MS m/z 536 (M + H)⁺
   1-663; A-18; B-27; MS m/z 536 (M + H)⁺
   1-664; A-18; B-28; MS m/z 529 (M + H)⁺
   1-665; A-18; B-29; MS m/z 533 (M + H)⁺
   1-666; A-18; B-30; MS m/z 545 (M + H)⁺
   1-667; A-18; B-31; MS m/z 547 (M + H)⁺
   1-668; A-18; B-32; MS m/z 549 (M + H)⁺
   1-669; A-18; B-33; MS m/z 549 (M + H)⁺
   1-670; A-18; B-34; MS m/z 559 (M + H)⁺
   1-671; A-18; B-35; MS m/z 573 (M + H)⁺
   1-672; A-18; B-36; MS m/z 591 (M + H)⁺
   1-673; A-19; B-01; MS m/z 425 (M + H)⁺
   1-674; A-19; B-02; MS m/z 429 (M + H)⁺
   1-675; A-19; B-03; MS m/z 387 (M + H)⁺
   1-676; A-19; B-04; MS m/z 445 (M + H)⁺
   1-677; A-19; B-05; MS m/z 457 (M + H)⁺
   1-678; A-19; B-06; MS m/z 497 (M + H)⁺
   1-679; A-19; B-07; MS m/z 477 (M + H)⁺
   1-680; A-19; B-08; MS m/z 483 (M + H)⁺
   1-681; A-19; B-09; MS m/z 485 (M + H)⁺
   1-682; A-19; B-10; MS m/z 507 (M + H)⁺
   1-683; A-19; B-11; MS m/z 511 (M + H)⁺
   1-684; A-19; B-12; MS m/z 519 (M + H)⁺
   1-685; A-19; B-13; MS m/z 433 (M + H)⁺
   1-686; A-19; B-14; MS m/z 441 (M + H)⁺
   1-687; A-19; B-15; MS m/z 455 (M + H)⁺
   1-688; A-19; B-16; MS m/z 461 (M + H)⁺
   1-689; A-19; B-17; MS m/z 467 (M + H)⁺
   1-690; A-19; B-18; MS m/z 471 (M + H)⁺
   1-691; A-19; B-19; MS m/z 481 (M + H)⁺
   1-692; A-19; B-20; MS m/z 483 (M + H)⁺
   1-693; A-19; B-21; MS m/z 487 (M + H)⁺
   1-694; A-19; B-22; MS m/z 491 (M + H)⁺
   1-695; A-19; B-23; MS m/z 503 (M + H)⁺
   1-696; A-19; B-24; MS m/z 503 (M + H)⁺
   1-697; A-19; B-25; MS m/z 4.72 (M + H)⁺
   1-698; A-19; B-26; MS m/z 512 (M + H)⁺
   1-699; A-19; B-27; MS m/z 512 (M + H)⁺
   1-700; A-19; B-28; MS m/z 505 (M + H)⁺
   1-701; A-19; B-29; MS m/z 509 (M + H)⁺
   1-702; A-19; B-30; MS m/z 521 (M + H)⁺
   1-703; A-19; B-31; MS m/z 523 (M + H)⁺
   1-704; A-19; B-32; MS m/z 525 (M + H)⁺
   1-705; A-19; B-33; MS m/z 525 (M + H)⁺
   1-706; A-19; B-34; MS m/z 535 (M + H)⁺
   1-707; A-19; B-35; MS m/z 549 (M + H)⁺
   1-708; A-19; B-36; MS m/z 567 (M + H)⁺
   1-709; A-20; B-01; MS m/z 429 (M + H)⁺
   1-710; A-20; B-02; MS m/z 433 (M + H)⁺
   1-711; A-20; B-03; MS m/z 391 (M + H)⁺
   1-712; A-20; B-04; MS m/z 449 (M + H)⁺
   1-713; A-20; B-05; MS m/z 461 (M + H)⁺
   1-714; A-20; B-06; MS m/z 501 (M + H)⁺
   1-715; A-20; B-07; MS m/z 481 (M + H)⁺
   1-716; A-20; B-08; MS m/z 487 (M + H)⁺
   1-717; A-20; B-09; MS m/z 489 (M + H)⁺
   1-718; A-20; B-10; MS m/z 511 (M + H)⁺
   1-719; A-20; B-11; MS m/z 515 (M + H)⁺
   1-720; A-20; B-12; MS m/z 523 (M + H)⁺
   1-721; A-20; B-13; MS m/z 437 (M + H)⁺
   1-722; A-20; B-14; MS m/z 445 (M + H)⁺
   1-723; A-20; B-15; MS m/z 459 (M + H)⁺
   1-724; A-20; B-16; MS m/z 465 (M + H)⁺
   1-725; A-20; B-17; MS m/z 471 (M + H)⁺
   1-726; A-20; B-18; MS m/z 475 (M + H)⁺
   1-727; A-20; B-19; MS m/z 485 (M + H)⁺
   1-728; A-20; B-20; MS m/z 487 (M + H)⁺
   1-729; A-20; B-21; MS m/z 491 (M + H)⁺
   1-730; A-20; B-22; MS m/z 495 (M + H)⁺
   1-731; A-20; B-23; MS m/z 507 (M + H)⁺
   1-732; A-20; B-24; MS m/z 507 (M + H)⁺
   1-733; A-20; B-25; MS m/z 476 (M + H)⁺
   1-734; A-20; B-26; MS m/z 516 (M + H)⁺
   1-735; A-20; B-27; MS m/z 516 (M + H)⁺
   1-736; A-20; B-28; MS m/z 509 (M + H)⁺
   1-737; A-20; B-29; MS m/z 513 (M + H)⁺
   1-738; A-20; B-30; MS m/z 525 (M + H)⁺
   1-739; A-20; B-31; MS m/z 527 (M + H)⁺
   1-740; A-20; B-32; MS m/z 529 (M + H)⁺
   1-741; A-20; B-33; MS m/z 529 (M + H)⁺
   1-742; A-20; B-34; MS m/z 539 (M + H)⁺
   1-743; A-20; B-35; MS m/z 553 (M + H)⁺
   1-744; A-20; B-36; MS m/z 571 (M + H)⁺
   1-745; A-21; B-01; MS m/z 435 (M + H)⁺
   1-746; A-21; B-02; MS m/z 439 (M + H)⁺
   1-747; A-21; B-03; MS m/z 397 (M + H)⁺
   1-748; A-21; B-04; MS m/z 455 (M + H)⁺
   1-749; A-21; B-05; MS m/z 467 (M + H)⁺
   1-750; A-21; B-06; MS m/z 507 (M + H)⁺
   1-751; A-21; B-07; MS m/z 487 (M + H)⁺
   1-752; A-21; B-08; MS m/z 493 (M + H)⁺
   1-753; A-21; B-09; MS m/z 495 (M + H)⁺
   1-754; A-21; B-10; MS m/z 517 (M + H)⁺
   1-755; A-21; B-11; MS m/z 521 (M + H)⁺
   1-756; A-21; B-12; MS m/z 529 (M + H)⁺
   1-757; A-21; B-13; MS m/z 443 (M + H)⁺
   1-758; A-21; B-14; MS m/z 451 (M + H)⁺
   1-759; A-21; B-15; MS m/z 465 (M + H)⁺
   1-760; A-21; B-16; MS m/z 471 (M + H)⁺
   1-761; A-21; B-17; MS m/z 477 (M + H)⁺
   1-762; A-21; B-18; MS m/z 481 (M + H)⁺
   1-763; A-21; B-19; MS m/z 491 (M + H)⁺
   1-764; A-21; B-20; MS m/z 493 (M + H)⁺
   1-765; A-21; B-21; MS m/z 497 (M + H)⁺
   1-766; A-21; B-22; MS m/z 501 (M + H)⁺
   1-767; A-21; B-23; MS m/z 513 (M + H)⁺
   1-768; A-21; B-24; MS m/z 513 (M + H)⁺
   1-769; A-21; B-25; MS m/z 482 (M + H)⁺
   1-770; A-21; B-26; MS m/z 522 (M + H)⁺
   1-771; A-21; B-27; MS m/z 522 (M + H)⁺
   1-772; A-21; B-28; MS m/z 515 (M + H)⁺
   1-773; A-21; B-29; MS m/z 519 (M + H)⁺
   1-774; A-21; B-30; MS m/z 531 (M + H)⁺
   1-775; A-21; B-31; MS m/z 533 (M + H)⁺
   1-776; A-21; B-32; MS m/z 535 (M + H)⁺
   1-777; A-21; B-33; MS m/z 535 (M + H)⁺
   1-778; A-21; B-34; MS m/z 545 (M + H)⁺
   1-779; A-21; B-35; MS m/z 559 (M + H)⁺
   1-780; A-21; B-36; MS m/z 577 (M + H)⁺
   1-781; A-22; B-01; MS m/z 459 (M + H)⁺
   1-782; A-22; B-02; MS m/z 463 (M + H)⁺
   1-783; A-22; B-03; MS m/z 421 (M + H)⁺
   1-784; A-22; B-04; MS m/z 479 (M + H)⁺
   1-785; A-22; B-05; MS m/z 491 (M + H)⁺
   1-786; A-22; B-06; MS m/z 531 (M + H)⁺
   1-787; A-22; B-07; MS m/z 511 (M + H)⁺
   1-788; A-22; B-08; MS m/z 517 (M + H)⁺
   1-789; A-22; B-09; MS m/z 519 (M + H)⁺
   1-790; A-22; B-10; MS m/z 541 (M + H)⁺
   1-791; A-22; B-11; MS m/z 545 (M + H)⁺
   1-792; A-22; B-12; MS m/z 553 (M + H)⁺
   1-793; A-22; B-13; MS m/z 467 (M + H)⁺
   1-794; A-22; B-14; MS m/z 475 (M + H)⁺
   1-795; A-22; B-15; MS m/z 489 (M + H)⁺
   1-796; A-22; B-16; MS m/z 495 (M + H)⁺
   1-797; A-22; B-17; MS m/z 501 (M + H)⁺
   1-798; A-22; B-18; MS m/z 505 (M + H)⁺
   1-799; A-22; B-19; MS m/z 515 (M + H)⁺
   1-800; A-22; B-20; MS m/z 517 (M + H)⁺
   1-801; A-22; B-21; MS m/z 521 (M + H)⁺
   1-802; A-22; B-22; MS m/z 525 (M + H)⁺
   1-803; A-22; B-23; MS m/z 537 (M + H)⁺
   1-804; A-22; B-24; MS m/z 537 (M + H)⁺
   1-805; A-22; B-25; MS m/z 506 (M + H)⁺
   1-806; A-22; B-26; MS m/z 546 (M + H)⁺
   1-807; A-22; B-27; MS m/z 546 (M + H)⁺
   1-808; A-22; B-28; MS m/z 539 (M + H)⁺
   1-809; A-22; B-29; MS m/z 543 (M + H)⁺
   1-810: A-22; B-30; MS m/z 555 (M + H)⁺
   1-811: A-22; B-31; MS m/z 557 (M + H)⁺
   1-812; A-22; B-32; MS m/z 559 (M + H)⁺
   1-813; A-22; B-33; MS m/z 559 (M + H)⁺
   1-814; A-22; B-34; MS m/z 569 (M + H)⁺
   1-815; A-22; B-35; MS m/z 583 (M + H)⁺
   1-816; A-22; B-36; MS m/z 601 (M + H)⁺
   1-817; A-23; B-01; MS m/z 463 (M + H)⁺
   1-818; A-23; B-02; MS m/z 467 (M + H)⁺
   1-819; A-23; B-03; MS m/z 425 (M + H)⁺
   1-820; A-23; B-04; MS m/z 483 (M + H)⁺
   1-821; A-23; B-05; MS m/z 495 (M + H)⁺
   1-822; A-23; B-06; MS m/z 535 (M + H)⁺
   1-823; A-23; B-07; MS m/z 515 (M + H)⁺
   1-824; A-23; B-08; MS m/z 521 (M + H)⁺
   1-825; A-23; B-09; MS m/z 523 (M + H)⁺
   1-826; A-23; B-10; MS m/z 545 (M + H)⁺
   1-827; A-23; B-11; MS m/z 549 (M + H)⁺
   1-828; A-23; B-12; MS m/z 557 (M + H)⁺
   1-829; A-23; B-13; MS m/z 471 (M + H)⁺
   1-830; A-23; B-14; MS m/z 479 (M + H)⁺
   1-831; A-23; B-15; MS m/z 493 (M + H)⁺
   1-832; A-23; B-16; MS m/z 499 (M + H)⁺
   1-833; A-23; B-17; MS m/z 505 (M + H)⁺
   1-834; A-23; B-18; MS m/z 509 (M + H)⁺
   1-835; A-23; B-19; MS m/z 519 (M + H)⁺
   1-836; A-23; B-20; MS m/z 521 (M + H)⁺
   1-837; A-23; B-21; MS m/z 525 (M + H)⁺
   1-838; A-23; B-22; MS m/z 529 (M + H)⁺
   1-839; A-23; B-23; MS m/z 541 (M + H)⁺
   1-840; A-23; B-24; MS m/z 541 (M + H)⁺
   1-841; A-23; B-25; MS m/z 510 (M + H)⁺
   1-842; A-23; B-26; MS m/z 550 (M + H)⁺
   1-843; A-23; B-27; MS m/z 550 (M + H)⁺
   1-844; A-23; B-28; MS m/z 543 (M + H)⁺
   1-845; A-23; B-29; MS m/z 547 (M + H)⁺
   1-846; A-23; B-30; MS m/z 559 (M + H)⁺
   1-847; A-23; B-31; MS m/z 561 (M + H)⁺
   1-848; A-23; B-32; MS m/z 563 (M + H)⁺
   1-849; A-23; B-33; MS m/z 563 (M + H)⁺
   1-850; A-23; B-34; MS m/z 573 (M + H)⁺
   1-851; A-23; B-35; MS m/z 587 (M + H)⁺
   1-852; A-23; B-36; MS m/z 605 (M + H)⁺
   1-853; A-24; B-01; MS m/z 467 (M + H)⁺
   1-854; A-24; B-02; MS m/z 471 (M + H)⁺
   1-855; A-24; B-03; MS m/z 429 (M + H)⁺
   1-856; A-24; B-04; MS m/z 487 (M + H)⁺
   1-857; A-24; B-05; MS m/z 499 (M + H)⁺
   1-858; A-24; B-06; MS m/z 539 (M + H)⁺
   1-859; A-24; B-07; MS m/z 519 (M + H)⁺
   1-860; A-24; B-08; MS m/z 525 (M + H)⁺
   1-861; A-24; B-09; MS m/z 527 (M + H)⁺
   1-862; A-24; B-10; MS m/z 549 (M + H)⁺
   1-863; A-24; B-11; MS m/z 553 (M + H)⁺
   1-864; A-24; B-12; MS m/z 561 (M + H)⁺
   1-865; A-24; B-13; MS m/z 475 (M + H)⁺
   1-866; A-24; B-14; MS m/z 483 (M + H)⁺
   1-867; A-24; B-15; MS m/z 497 (M + H)⁺
   1-868; A-24; B-16; MS m/z 503 (M + H)⁺
   1-869; A-24; B-17; MS m/z 509 (M + H)⁺
   1-870; A-24; B-18; MS m/z 513 (M + H)⁺
   1-871; A-24; B-19; MS m/z 523 (M + H)⁺
   1-872; A-24; B-20; MS m/z 525 (M + H)⁺
   1-873; A-24; B-21; MS m/z 529 (M + H)⁺
   1-874; A-24; B-22; MS m/z 533 (M + H)⁺
   1-875; A-24; B-23; MS m/z 545 (M + H)⁺
   1-876: A-24; B-24; MS m/z 545 (M + H)⁺
   1-877; A-24; B-25; MS m/z 514 (M + H)⁺
   1-878; A-24; B-26; MS m/z 554 (M + H)⁺
   1-879; A-24; B-27; MS m/z 554 (M + H)⁺
   1-880; A-24; B-28; MS m/z 547 (M + H)⁺
   1-881; A-24; B-29; MS m/z 551 (M + H)⁺
   1-882; A-24; B-30; MS m/z 563 (M + H)⁺
   1-883; A-24; B-31; MS m/z 565 (M + H)⁺
   1-884; A-24; B-32; MS m/z 567 (M + H)⁺
   1-885; A-24; B-33; MS m/z 567 (M + H)⁺
   1-886; A-24; B-34; MS m/z 577 (M + H)⁺
   1-887; A-24; B-35; MS m/z 591 (M + H)⁺
   1-888; A-24; B-36; MS m/z 609 (M + H)⁺
   1-889; A-25; B-01; MS m/z 394 (M + H)⁺
   1-890; A-25; B-02; MS m/z 398 (M + H)⁺
   1-891; A-25; B-03; MS m/z 356 (M + H)⁺
   1-892; A-25; B-04; MS m/z 414 (M + H)⁺
   1-893; A-25; B-05; MS m/z 426 (M + H)⁺
   1-894; A-25; B-06; MS m/z 466 (M + H)⁺
   1-895; A-25; B-07; MS m/z 456 (M + H)⁺
   1-896; A-25; B-08; MS m/z 452 (M + H)⁺
   1-897; A-25; B-09; MS m/z 454 (M + H)⁺
   1-898; A-25; B-10; MS m/z 476 (M + H)⁺
   1-899; A-25; B-11; MS m/z 480 (M + H)⁺
   1-900; A-25; B-12; MS m/z 488 (M + H)⁺
   1-901; A-25; B-13; MS m/z 402 (M + H)⁺
   1-902; A-25; B-14; MS m/z 410 (M + H)⁺
   1-903; A-25; B-15; MS m/z 424 (M + H)⁺
   1-904; A-25; B-16; MS m/z 430 (M + H)⁺
   1-905; A-25; B-17; MS m/z 436 (M + H)⁺
   1-906; A-25; B-18; MS m/z 440 (M + H)⁺
   1-907; A-25; B-19; MS m/z 450 (M + H)⁺
   1-908; A-25; B-20; MS m/z 452 (M + H)⁺
   1-909; A-25; B-21; MS m/z 456 (M + H)⁺
   1-910; A-25; B-22; MS m/z 460 (M + H)⁺
   1-911; A-25; B-23; MS m/z 472 (M + H)⁺
   1-912; A-25; B-24; MS m/z 472 (M + H)⁺
   1-913; A-25; B-25; MS m/z 441 (M + H)⁺
   1-914; A-25; B-26; MS m/z 481 (M + H)⁺
   1-915; A-25; B-27; MS m/z 481 (M + H)⁺
   1-916; A-25; B-28; MS m/z 474 (M + H)⁺
   1-917; A-25; B-29; MS m/z 478 (M + H)⁺
   1-918; A-25; B-30; MS m/z 490 (M + H)⁺
   1-919; A-25; B-31; MS m/z 492 (M + H)⁺
   1-920; A-25; B-32; MS m/z 494 (M + H)⁺
   1-921; A-25; B-33; MS m/z 494 (M + H)⁺
   1-922; A-25; B-34; MS m/z 504 (M + H)⁺
   1-923; A-25; B-35; MS m/z 518 (M + H)⁺
   1-924; A-25; B-36; MS m/z 536 (M + H)⁺
   1-925; A-26; B-01; MS m/z 400 (M + H)⁺
   1-926; A-26; B-02; MS m/z 404 (M + H)⁺
   1-927; A-26; B-03; MS m/z 362 (M + H)⁺
   1-928; A-26; B-04; MS m/z 420 (M + H)⁺
   1-929; A-26; B-05; MS m/z 432 (M + H)⁺
   1-930; A-26; B-06; MS m/z 472 (M + H)⁺
   1-931; A-26; B-07; MS m/z 452 (M + H)⁺
   1-932; A-26; B-08; MS m/z 458 (M + H)⁺
   1-933; A-26; B-09; MS m/z 460 (M + H)⁺
   1-934; A-26; B-10; MS m/z 482 (M + H)⁺
   1-935; A-26; B-11; MS m/z 486 (M + H)⁺
   1-936; A-26; B-12; MS m/z 494 (M + H)⁺
   1-937; A-26; B-13; MS m/z 408 (M + H)⁺
   1-938; A-26; B-14; MS m/z 416 (M + H)⁺
   1-939; A-26; B-15; MS m/z 430 (M + H)⁺
   1-940; A-26; B-16; MS m/z 436 (M + H)⁺
   1-941; A-26; B-17; MS m/z 442 (M + H)⁺
   1-942; A-26; B-18; MS m/z 446 (M + H)⁺
   1-943; A-26; B-19; MS m/z 456 (M + H)⁺
   1-944; A-26; B-20; MS m/z 458 (M + H)⁺
   1-945; A-26; B-21; MS m/z 462 (M + H)⁺
   1-946; A-26; B-22; MS m/z 466 (M + H)⁺
   1-947; A-26; B-23; MS m/z 478 (M + H)⁺
   1-948; A-26; B-24; MS m/z 478 (M + H)⁺
   1-949; A-26; B-25; MS m/z 447 (M + H)⁺
   1-950; A-26; B-26; MS m/z 487 (M + H)⁺
   1-951; A-26; B-27; MS m/z 487 (M + H)⁺
   1-952; A-26; B-28; MS m/z 480 (M + H)⁺
   1-953; A-26; B-29; MS m/z 484 (M + H)⁺
   1-954; A-26; B-30; MS m/z 496 (M + H)⁺
   1-955; A-26; B-31; MS m/z 498 (M + H)⁺
   1-956; A-26; B-32; MS m/z 500 (M + H)⁺
   1-957; A-26; B-33; MS m/z 500 (M + H)⁺
   1-958; A-26; B-34; MS m/z 510 (M + H)⁺
   1-959; A-26; B-35; MS m/z 524 (M + H)⁺
   1-960; A-26; B-36; MS m/z 542 (M + H)⁺
   1-961; A-27; B-01; MS m/z 434 (M + H)⁺
   1-962; A-27; B-02; MS m/z 438 (M + H)⁺
   1-963; A-27; B-03; MS m/z 396 (M + H)⁺
   1-964; A-27; B-04; MS m/z 454 (M + H)⁺
   1-965; A-27; B-05; MS m/z 466 (M + H)⁺
   1-966; A-27; B-06; MS m/z 506 (M + H)⁺
   1-967; A-27; B-07; MS m/z 486 (M + H)⁺
   1-968; A-27; B-08; MS m/z 492 (M + H)⁺
   1-969; A-27; B-09; MS m/z 494 (M + H)⁺
   1-970; A-27; B-10; MS m/z 516 (M + H)⁺
   1-971; A-27; B-11; MS m/z 520 (M + H)⁺
   1-972; A-27; B-12; MS m/z 528 (M + H)⁺
   1-973; A-27; B-13; MS m/z 442 (M + H)⁺
   1-974; A-27; B-14; MS m/z 450 (M + H)⁺
   1-975; A-27; B-15; MS m/z 464 (M + H)⁺
   1-976; A-27; B-16; MS m/z 470 (M + H)⁺
   1-977; A-27; B-17; MS m/z 476 (M + H)⁺
   1-978; A-27; B-18; MS m/z 480 (M + H)⁺
   1-979; A-27; B-19; MS m/z 490 (M + H)⁺
   1-980; A-27; B-20; MS m/z 492 (M + H)⁺
   1-981; A-27; B-21; MS m/z 496 (M + H)⁺
   1-982; A-27; B-22; MS m/z 500 (M + H)⁺
   1-983; A-27; B-23; MS m/z 512 (M + H)⁺
   1-984; A-27; B-24; MS m/z 512 (M + H)⁺
   1-985; A-27; B-25; MS m/z 481 (M + H)⁺
   1-986; A-27; B-26; MS m/z 521 (M + H)⁺
   1-987; A-27; B-27; MS m/z 521 (M + H)⁺
   1-988; A-27; B-28; MS m/z 514 (M + H)⁺
   1-989; A-27; B-29; MS m/z 518 (M + H)⁺
   1-990; A-27; B-30; MS m/z 530 (M + H)⁺
   1-991; A-27; B-31; MS m/z 532 (M + H)⁺
   1-992; A-27; B-32; MS m/z 534 (M + H)⁺
   1-993; A-27; B-33; MS m/z 534 (M + H)⁺
   1-994; A-27; B-34; MS m/z 544 (M + H)⁺
   1-995; A-27; B-35; MS m/z 558 (M + H)⁺
   1-996; A-27; B-36; MS m/z 576 (M + H)⁺
   1-997; A-28; B-01; MS m/z 464 (M + H)⁺
   1-998; A-28; B-02; MS m/z 468 (M + H)⁺
   1-999; A-28; B-03; MS m/z 426 (M + H)⁺
   1-1000; A-28; B-04; MS m/z 484 (M + H)⁺
   1-1001; A-28; B-05; MS m/z 496 (M + H)⁺
   1-1002; A-28; B-06; MS m/z 536 (M + H)⁺
   1-1003; A-28; B-07; MS m/z 516 (M + H)⁺
   1-1004; A-28; B-08; MS m/z 522 (M + H)⁺
   1-1005; A-28; B-09; MS m/z 524 (M + H)⁺
   1-1006; A-28; B-10; MS m/z 546 (M + H)⁺
   1-1007; A-28; B-11; MS m/z 550 (M + H)⁺
   1-1008; A-28; B-12; MS m/z 558 (M + H)⁺
   1-1009; A-28; B-13; MS m/z 472 (M + H)⁺
   1-1010; A-28; B-14; MS m/z 480 (M + H)⁺
   1-1011; A-28; B-15; MS m/z 494 (M + H)⁺
   1-1012; A-28; B-16; MS m/z 500 (M + H)⁺
   1-1013; A-28; B-17; MS m/z 506 (M + H)⁺
   1-1014; A-28; B-18; MS m/z 510 (M + H)⁺
   1-1015; A-28; B-19; MS m/z 520 (M + H)⁺
   1-1016; A-28; B-20; MS m/z 522 (M + H)⁺
   1-1017; A-28; B-21; MS m/z 526 (M + H)⁺
   1-1018; A-28; B-22; MS m/z 530 (M + H)⁺
   1-1019; A-28; B-23; MS m/z 542 (M + H)⁺
   1-1020; A-28; B-24; MS m/z 542 (M + H)⁺
   1-1021; A-28; B-25; MS m/z 511 (M + H)⁺
   1-1022; A-28; B-26; MS m/z 551 (M + H)⁺
   1-1023; A-28; B-27; MS m/z 551 (M + H)⁺
   1-1024; A-28; B-28; MS m/z 544 (M + H)⁺
   1-1025; A-28; B-29; MS m/z 548 (M + H)⁺
   1-1026; A-28; B-30; MS m/z 560 (M + H)⁺
   1-1027; A-28; B-31; MS m/z 562 (M + H)⁺
   1-1028; A-28; B-32; MS m/z 564 (M + H)⁺
   1-1029; A-28; B-33; MS m/z 564 (M + H)⁺
   1-1030; A-28; B-34; MS m/z 574 (M + H)⁺
   1-1031; A-28; B-35; MS m/z 588 (M + H)⁺
   1-1032; A-28; B-36; MS m/z 606 (M + H)⁺
   1-1033; A-29; B-01
   1-1034; A-29; B-02
   1-1035; A-29; B-03; MS m/z 368 (M + H)⁺
   1-1036; A-29; B-04
   1-1037; A-29; B-05
   1-1038; A-29; B-06
   1-1039; A-29; B-07
   1-1040; A-29; B-08
   1-1041; A-29; B-09
   1-1042; A-29; B-10
   1-1043; A-29; B-11
   1-1044; A-29; B-12
   1-1045; A-29; B-13
   1-1046; A-29; B-14
   1-1047; A-29; B-15
   1-1048; A-29; B-16
   1-1049; A-29; B-17; MS m/z 448 (M + H)⁺
   1-1050; A-29; B-18
   1-1051; A-29; B-19
   1-1052; A-29; B-20
   1-1053; A-29; B-21
   1-1054; A-29; B-22; MS m/z 472 (M + H)⁺
   1-1055; A-29; B-23
   1-1056; A-29; B-24
   1-1057; A-29; B-25
   1-1058; A-29; B-26
   1-1059; A-29; B-27
   1-1060; A-29; B-28
   1-1061; A-29; B-29
   1-1062; A-29; B-30
   1-1063; A-29; B-31
   1-1064; A-29; B-32
   1-1065; A-29; B-33
   1-1066; A-29; B-34
   1-1067; A-29; B-35
   1-1068; A-29; B-36
   1-1069; A-30; B-01; MS m/z 422 (M + H)⁺
   1-1070; A-30; B-02; MS m/z 426 (M + H)⁺
   1-1071; A-30; B-03; MS m/z 384 (M + H)⁺
   1-1072; A-30; B-04; MS m/z 442 (M + H)⁺
   1-1073; A-30; B-05; MS m/z 454 (M + H)⁺
   1-1074; A-30; B-06; MS m/z 494 (M + H)⁺
   1-1075; A-30; B-07; MS m/z 474 (M + H)⁺
   1-1076; A-30; B-08; MS m/z 480 (M + H)⁺
   1-1077; A-30; B-09; MS m/z 482 (M + H)⁺
   1-1078; A-30; B-10; MS m/z 504 (M + H)⁺
   1-1079; A-30; B-11; MS m/z 508 (M + H)⁺
   1-1080; A-30; B-12; MS m/z 516 (M + H)⁺
   1-1081; A-30; B-13; MS m/z 430 (M + H)⁺
   1-1082; A-30; B-14; MS m/z 438 (M + H)⁺
   1-1083; A-30; B-15; MS m/z 452 (M + H)⁺
   1-1084; A-30; B-16; MS m/z 458 (M + H)⁺
   1-1085; A-30; B-17; MS m/z 464 (M + H)⁺
   1-1086; A-30; B-18; MS m/z 468 (M + H)⁺
   1-1087; A-30; B-19; MS m/z 478 (M + H)⁺
   1-1088; A-30; B-20; MS m/z 480 (M + H)⁺
   1-1089; A-30; B-21; MS m/z 484 (M + H)⁺
   1-1090; A-30; B-22; MS m/z 488 (M + H)⁺
   1-1091; A-30; B-23; MS m/z 500 (M + H)⁺
   1-1092; A-30; B-24; MS m/z 500 (M + H)⁺
   1-1093; A-30; B-25; MS m/z 469 (M + H)⁺
   1-1094; A-30; B-26; MS m/z 509 (M + H)⁺
   1-1095; A-30; B-27; MS m/z 509 (M + H)⁺
   1-1096; A-30; B-28; MS m/z 502 (M + H)⁺
   1-1097; A-30; B-29; MS m/z 506 (M + H)⁺
   1-1098; A-30; B-30; MS m/z 518 (M + H)⁺
   1-1099; A-30; B-31; MS m/z 520 (M + H)⁺
   1-1100; A-30; B-32; MS m/z 522 (M + H)⁺
   1-1101; A-30; B-33; MS m/z 522 (M + H)⁺
   1-1102; A-30; B-34; MS m/z 532 (M + H)⁺
   1-1103; A-30; B-35; MS m/z 546 (M + H)⁺
   1-1104; A-30; B-36; MS m/z 564 (M + H)⁺
   1-1105; A-31; B-01
   1-1106; A-31; B-02
   1-1107; A-31; B-03; MS m/z 384 (M + H)⁺
   1-1108; A-31; B-04
   1-1109; A-31; B-05
   1-1110; A-31; B-06
   1-1111; A-31; B-07
   1-1112; A-31; B-08
   1-1113; A-31; B-09
   1-1114; A-31; B-10; MS m/z 504 (M + H)⁺
   1-1115; A-31; B-11
   1-1116; A-31; B-12; MS m/z 516 (M + H)⁺
   1-1117; A-31; B-13
   1-1118; A-31; B-14; MS m/z 438 (M + H)⁺
   1-1119; A-31; B-15; MS m/z 452 (M + H)⁺
   1-1120; A-31; B-16; MS m/z 458 (M + H)⁺
   1-1121; A-31; B-17; MS m/z 464 (M + H)⁺
   1-1122; A-31; B-18; MS m/z 468 (M + H)⁺
   1-1123; A-31; B-19
   1-1124; A-31; B-20; MS m/z 480 (M + H)⁺
   1-1125; A-31; B-21; MS m/z 484 (M + H)⁺
   1-1126; A-31; B-22; MS m/z 488 (M + H)⁺
   1-1127; A-31; B-23; MS m/z 500 (M + H)⁺
   1-1128; A-31; B-24; MS m/z 500 (M + H)⁺
   1-1129; A-31; B-25
   1-1130; A-31; B-26; MS m/z 509 (M + H)⁺
   1-1131; A-31; B-27; MS m/z 509 (M + H)⁺
   1-1132; A-31; B-28; MS m/z 502 (M + H)⁺
   1-1133; A-31; B-29; MS m/z 506 (M + H)⁺
   1-1134; A-31; B-30; MS m/z 518 (M + H)⁺
   1-1135; A-31; B-31; MS m/z 520 (M + H)⁺
   1-1136; A-31; B-32; MS m/z 522 (M + H)⁺
   1-1137; A-31; B-33; MS m/z 522 (M + H)⁺
   1-1138; A-31; B-34; MS m/z 532 (M + H)⁺
   1-1139; A-31; B-35; MS m/z 546 (M + H)⁺
   1-1140; A-31; B-36; MS m/z 564 (M + H)⁺
   1-1141; A-32; B-01; MS m/z 448 (M + H)⁺
   1-1142; A-32; B-02; MS m/z 452 (M + H)⁺
   1-1143; A-32; B-03; MS m/z 410 (M + H)⁺
   1-1144; A-32; B-04; MS m/z 468 (M + H)⁺
   1-1145; A-32; B-05; MS m/z 480 (M + H)⁺
   1-1146; A-32; B-06; MS m/z 520 (M + H)⁺
   1-1147; A-32; B-07; MS m/z 500 (M + H)⁺
   1-1148; A-32; B-08; MS m/z 506 (M + H)⁺
   1-1149; A-32; B-09; MS m/z 508 (M + H)⁺
   1-1150; A-32; B-10; MS m/z 530 (M + H)⁺
   1-1151; A-32; B-11; MS m/z 534 (M + H)⁺
   1-1152; A-32; B-12; MS m/z 542 (M + H)⁺
   1-1153; A-32; B-13; MS m/z 456 (M + H)⁺
   1-1154; A-32; B-14; MS m/z 464 (M + H)⁺
   1-1155; A-32; B-15; MS m/z 478 (M + H)⁺
   1-1156; A-32; B-16; MS m/z 484 (M + H)⁺
   1-1157; A-32; B-17; MS m/z 490 (M + H)⁺
   1-1158; A-32; B-18; MS m/z 494 (M + H)⁺
   1-1159; A-32; B-19; MS m/z 504 (M + H)⁺
   1-1160; A-32; B-20; MS m/z 506 (M + H)⁺
   1-1161; A-32; B-21; MS m/z 510 (M + H)⁺
   1-1162; A-32; B-22; MS m/z 514 (M + H)⁺
   1-1163; A-32; B-23; MS m/z 526 (M + H)⁺
   1-1164; A-32; B-24; MS m/z 526 (M + H)⁺
   1-1165; A-32; B-25; MS m/z 495 (M + H)⁺
   1-1166; A-32; B-26; MS m/z 535 (M + H)⁺
   1-1167; A-32; B-27; MS m/z 535 (M + H)⁺
   1-1168; A-32; B-28; MS m/z 528 (M + H)⁺
   1-1169; A-32; B-29; MS m/z 532 (M + H)⁺
   1-1170; A-32; B-30; MS m/z 544 (M + H)⁺
   1-1171; A-32; B-31; MS m/z 546 (M + H)⁺
   1-1172; A-32; B-32; MS m/z 548 (M + H)⁺
   1-1173; A-32; B-33; MS m/z 548 (M + H)⁺
   1-1174; A-32; B-34; MS m/z 558 (M + H)⁺
   1-1175; A-32; B-35; MS m/z 572 (M + H)⁺
   1-1176; A-32; B-36; MS m/z 590 (M + H)⁺
   1-1177; A-33; B-01; MS m/z 363 (M + H)⁺
   1-1178; A-33; B-02; MS m/z 367 (M + H)⁺
   1-1179; A-33; B-03; MS m/z 325 (M + H)⁺
   1-1180; A-33; B-04; MS m/z 383 (M + H)⁺
   1-1181; A-33; B-05; MS m/z 395 (M + H)⁺
   1-1182; A-33; B-06; MS m/z 435 (M + H)⁺
   1-1183; A-33; B-07; MS m/z 415 (M + H)⁺
   1-1184; A-33; B-08; MS m/z 421 (M + H)⁺
   1-1185; A-33; B-09; MS m/z 423 (M + H)⁺
   1-1186; A-33; B-10; MS m/z 445 (M + H)⁺
   1-1187; A-33; B-11; MS m/z 449 (M + H)⁺
   1-1188; A-33; B-12; MS m/z 457 (M + H)⁺
   1-1189; A-33; B-13; MS m/z 371 (M + H)⁺
   1-1190; A-33; B-14; MS m/z 379 (M + H)⁺
   1-1191; A-33; B-15; MS m/z 393 (M + H)⁺
   1-1192; A-33; B-16; MS m/z 399 (M + H)⁺
   1-1193; A-33; B-17; MS m/z 405 (M + H)⁺
   1-1194; A-33; B-18; MS m/z 409 (M + H)⁺
   1-1195; A-33; B-19; MS m/z 419 (M + H)⁺
   1-1196; A-33; B-20; MS m/z 421 (M + H)⁺
   1-1197; A-33; B-21; MS m/z 425 (M + H)⁺
   1-1198; A-33; B-22; MS m/z 429 (M + H)⁺
   1-1199; A-33; B-23; MS m/z 441 (M + H)⁺
   1-1200; A-33; B-24; MS m/z 441 (M + H)⁺
   1-1201; A-33; B-25; MS m/z 410 (M + H)⁺
   1-1202; A-33; B-26; MS m/z 450 (M + H)⁺
   1-1203; A-33; B-27; MS m/z 450 (M + H)⁺
   1-1204; A-33; B-28; MS m/z 443 (M + H)⁺
   1-1205; A-33; B-29; MS m/z 447 (M + H)⁺
   1-1206; A-33; B-30; MS m/z 459 (M + H)⁺
   1-1207; A-33; B-31; MS m/z 461 (M + H)⁺
   1-1208; A-33; B-32; MS m/z 463 (M + H)⁺
   1-1209; A-33; B-33; MS m/z 463 (M + H)⁺
   1-1210; A-33; B-34; MS m/z 473 (M + H)⁺
   1-1211; A-33; B-35; MS m/z 487 (M + H)⁺
   1-1212; A-33; B-36; MS m/z 505 (M + H)⁺
   1-1213; A-34; B-01
   1-1214; A-34; B-02
   1-1215; A-34; B-03
   1-1216; A-34; B-04
   1-1217; A-34; B-05
   1-1218; A-34; B-06
   1-1219; A-34; B-07
   1-1220; A-34; B-08
   1-1221; A-34; B-09
   1-1222; A-34; B-10
   1-1223; A-34; B-11
   1-1224; A-34; B-12
   1-1225; A-34; B-13
   1-1226; A-34; B-14
   1-1227; A-34; B-15
   1-1228; A-34; B-16
   1-1229; A-34; B-17
   1-1230; A-34; B-18
   1-1231; A-34; B-19
   1-1232; A-34; B-20
   1-1233; A-34; B-21
   1-1234; A-34; B-22
   1-1235; A-34; B-23
   1-1236; A-34; B-24
   1-1237; A-34; B-25
   1-1238; A-34; B-26
   1-1239; A-34; B-27
   1-1240; A-34; B-28
   1-1241; A-34; B-29
   1-1242; A-34; B-30
   1-1243; A-34; B-31
   1-1244; A-34; B-32
   1-1245; A-34; B-33
   1-1246; A-34; B-34
   1-1247; A-34; B-35
   1-1248; A-34; B-36
   1-1249; A-35; B-01; MS m/z 391 (M + H)⁺
   1-1250; A-35; B-02; MS m/z 395 (M + H)⁺
   1-1251; A-35; B-03; MS m/z 353 (M + H)⁺
   1-1252; A-35; B-04; MS m/z 411 (M + H)⁺
   1-1253; A-35; B-05; MS m/z 423 (M + H)⁺
   1-1254; A-35; B-06; MS m/z 463 (M + H)⁺
   1-1255; A-35; B-07; MS m/z 443 (M + H)⁺
   1-1256; A-35; B-08; MS m/z 449 (M + H)⁺
   1-1257; A-35; B-09; MS m/z 451 (M + H)⁺
   1-1258; A-35; B-10; MS m/z 473 (M + H)⁺
   1-1259; A-35; B-11; MS m/z 477 (M + H)⁺
   1-1260; A-35; B-12; MS m/z 485 (M + H)⁺
   1-1261; A-35; B-13; MS m/z 399 (M + H)⁺
   1-1262; A-35; B-14; MS m/z 407 (M + H)⁺
   1-1263; A-35; B-15; MS m/z 421 (M + H)⁺
   1-1264; A-35; B-16; MS m/z 427 (M + H)⁺
   1-1265; A-35; B-17; MS m/z 433 (M + H)⁺
   1-1266; A-35; B-18; MS m/z 437 (M + H)⁺
   1-1267; A-35; B-19; MS m/z 447 (M + H)⁺
   1-1268; A-35; B-20; MS m/z 449 (M + H)⁺
   1-1269; A-35; B-21; MS m/z 453 (M + H)⁺
   1-1270; A-35; B-22; MS m/z 457 (M + H)⁺
   1-1271; A-35; B-23; MS m/z 469 (M + H)⁺
   1-1272; A-35; B-24; MS m/z 469 (M + H)⁺
   1-1273; A-35; B-25; MS m/z 438 (M + H)⁺
   1-1274; A-35; B-26; MS m/z 478 (M + H)⁺
   1-1275; A-35; B-27; MS m/z 478 (M + H)⁺
   1-1276; A-35; B-28; MS m/z 471 (M + H)⁺
   1-1277; A-35; B-29; MS m/z 475 (M + H)⁺
   1-1278; A-35; B-30; MS m/z 487 (M + H)⁺
   1-1279: A-35; B-31; MS m/z 489 (M + H)⁺
   1-1280; A-35; B-32; MS m/z 491 (M + H)⁺
   1-1281; A-35; B-33; MS m/z 491 (M + H)⁺
   1-1282; A-35; B-34; MS m/z 501 (M + H)⁺
   1-1283; A-35; B-35; MS m/z 515 (M + H)⁺
   1-1284; A-35; B-36; MS m/z 533 (M + H)⁺
   1-1285; A-36; B-01; MS m/z 405 (M + H)⁺
   1-1286; A-36; B-02; MS m/z 409 (M + H)⁺
   1-1287; A-36; B-03; MS m/z 367 (M + H)⁺
   1-1288; A-36; B-04; MS m/z 425 (M + H)⁺
   1-1289; A-36; B-05; MS m/z 437 (M + H)⁺
   1-1290; A-36; B-06; MS m/z 477 (M + H)⁺
   1-1291; A-36; B-07; MS m/z 457 (M + H)⁺
   1-1292; A-36; B-08; MS m/z 463 (M + H)⁺
   1-1293; A-36; B-09; MS m/z 465 (M + H)⁺
   1-1294; A-36; B-10; MS m/z 487 (M + H)⁺
   1-1295; A-36; B-11; MS m/z 491 (M + H)⁺
   1-1296; A-36; B-12; MS m/z 499 (M + H)⁺
   1-1297; A-36; B-13; MS m/z 413 (M + H)⁺
   1-1298; A-36; B-14; MS m/z 421 (M + H)⁺
   1-1299; A-36; B-15; MS m/z 435 (M + H)⁺
   1-1300; A-36; B-16; MS m/z 441 (M + H)⁺
   1-1301; A-36; B-17; MS m/z 447 (M + H)⁺
   1-1302; A-36; B-18; MS m/z 451 (M + H)⁺
   1-1303; A-36; B-19; MS m/z 461 (M + H)⁺
   1-1304; A-36; B-20; MS m/z 463 (M + H)⁺
   1-1305; A-36; B-21; MS m/z 467 (M + H)⁺
   1-1306; A-36; B-22; MS m/z 471 (M + H)⁺
   1-1307; A-36; B-23; MS m/z 483 (M + H)⁺
   1-1308; A-36; B-24; MS m/z 483 (M + H)⁺
   1-1309; A-36; B-25; MS m/z 452 (M + H)⁺
   1-1310; A-36; B-26; MS m/z 492 (M + H)⁺
   1-1311; A-36; B-27; MS m/z 492 (M + H)⁺
   1-1312; A-36; B-28
   1-1313; A-36; B-29; MS m/z 489 (M + H)⁺
   1-1314; A-36; B-30; MS m/z 501 (M + H)⁺
   1-1315; A-36; B-31; MS m/z 503 (M + H)⁺
   1-1316; A-36; B-32; MS m/z 505 (M + H)⁺
   1-1317; A-36; B-33; MS m/z 505 (M + H)⁺
   1-1318; A-36; B-34; MS m/z 515 (M + H)⁺
   1-1319; A-36; B-35; MS m/z 529 (M + H)⁺
   1-1320; A-36; B-36; MS m/z 547 (M + H)⁺
   1-1321; A-37; B-0.1; MS m/z 455 (M + H)⁺
   1-1322; A-37; B-02; MS m/z 459 (M + H)⁺
   1-1323; A-37; B-03; MS m/z 417 (M + H)⁺
   1-1324; A-37; B-04; MS m/z 475 (M + H)⁺
   1-1325; A-37; B-05; MS m/z 487 (M + H)⁺
   1-1326; A-37; B-06; MS m/z 527 (M + H)⁺
   1-1327; A-37; B-07; MS m/z 507 (M + H)⁺
   1-1328; A-37; B-08; MS m/z 513 (M + H)⁺
   1-1329; A-37; B-09; MS m/z 515 (M + H)⁺
   1-1330; A-37; B-10; MS m/z 537 (M + H)⁺
   1-1331; A-37; B-11; MS m/z 541 (M + H)⁺
   1-1332; A-37; B-12; MS m/z 549 (M + H)⁺
   1-1333; A-37; B-13; MS m/z 463 (M + H)⁺
   1-1334; A-37; B-14; MS m/z 471 (M + H)⁺
   1-1335; A-37; B-15; MS m/z 485 (M + H)⁺
   1-1336; A-37; B-16; MS m/z 491 (M + H)⁺
   1-1337; A-37; B-17; MS m/z 497 (M + H)⁺
   1-1338; A-37; B-18; MS m/z 501 (M + H)⁺
   1-1339; A-37; B-19; MS m/z 511 (M + H)⁺
   1-1340; A-37; B-20; MS m/z 513 (M + H)⁺
   1-1341; A-37; B-21; MS m/z 517 (M + H)⁺
   1-1342; A-37; B-22; MS m/z 521 (M + H)⁺
   1-1343; A-37; B-23; MS m/z 533 (M + H)⁺
   1-1344; A-37; B-24; MS m/z 533 (M + H)⁺
   1-1345; A-37; B-25; MS m/z 502 (M + H)⁺
   1-1346; A-37; B-26; MS m/z 542 (M + H)⁺
   1-1347; A-37; B-27; MS m/z 542 (M + H)⁺
   1-1348; A-37; B-28; MS m/z 535 (M + H)⁺
   1-1349; A-37; B-29; MS m/z 539 (M + H)⁺
   1-1350; A-37; B-30; MS m/z 551 (M + H)⁺
   1-1351; A-37; B-31; MS m/z 553 (M + H)⁺
   1-1352; A-37; B-32; MS m/z 555 (M + H)⁺
   1-1353; A-37; B-33; MS m/z 555 (M + H)⁺
   1-1354; A-37; B-34; MS m/z 565 (M + H)⁺
   1-1355; A-37; B-35; MS m/z 579 (M + H)⁺
   1-1356; A-37; B-36; MS m/z 597 (M + H)⁺
   1-1357; A-38; B-01; MS m/z 392 (M + H)⁺
   1-1358; A-38; B-02; MS m/z 396 (M + H)⁺
   1-1359; A-38; B-03; MS m/z 354 (M + H)⁺
   1-1360; A-38; B-04; MS m/z 412 (M + H)⁺
   1-1361; A-38; B-05; MS m/z 424 (M + H)⁺
   1-1362; A-38; B-06; MS m/z 464 (M + H)⁺
   1-1363; A-38; B-07; MS m/z 444 (M + H)⁺
   1-1364; A-38; B-08; MS m/z 450 (M + H)⁺
   1-1365; A-38; B-09; MS m/z 452 (M + H)⁺
   1-1366; A-38; B-10; MS m/z 474 (M + H)⁺
   1-1367; A-38; B-11; MS m/z 478 (M + H)⁺
   1-1368; A-38; B-12; MS m/z 486 (M + H)⁺
   1-1369; A-38; B-13
   1-1370; A-38; B-14; MS m/z 408 (M + H)⁺
   1-1371; A-38; B-15; MS m/z 422 (M + H)⁺
   1-1372; A-38; B-16; MS m/z 428 (M + H)⁺
   1-1373; A-38; B-17; MS m/z 434 (M + H)⁺
   1-1374; A-38; B-18; MS m/z 438 (M + H)⁺
   1-1375; A-38; B-19; MS m/z 448 (M + H)⁺
   1-1376; A-38; B-20; MS m/z 450 (M + H)⁺
   1-1377; A-38; B-21; MS m/z 454 (M + H)⁺
   1-1378; A-38; B-22; MS m/z 458 (M + H)⁺
   1-1379; A-38; B-23; MS m/z 470 (M + H)⁺
   1-1380; A-38; B-24; MS m/z 470 (M + H)⁺
   1-1381; A-38; B-25
   1-1382; A-38; B-26; MS m/z 479 (M + H)⁺
   1-1383; A-38; B-27; MS m/z 479 (M + H)⁺
   1-1384; A-38; B-28; MS m/z 472 (M + H)⁺
   1-1385; A-38; B-29; MS m/z 476 (M + H)⁺
   1-1386; A-38; B-30; MS m/z 488 (M + H)⁺
   1-1387; A-38; B-31; MS m/z 490 (M + H)⁺
   1-1388; A-38; B-32; MS m/z 492 (M + H)⁺
   1-1389; A-38; B-33; MS m/z 492 (M + H)⁺
   1-1390; A-38; B-34; MS m/z 502 (M + H)⁺
   1-1391; A-38; B-35; MS m/z 516 (M + H)⁺
   1-1392; A-38; B-36; MS m/z 534 (M + H)⁺
   1-1393; A-39; B-01; MS m/z 460 (M + H)⁺
   1-1394; A-39; B-02; MS m/z 464 (M + H)⁺
   1-1395; A-39; B-03; MS m/z 422 (M + H)⁺
   1-1396; A-39; B-04; MS m/z 480 (M + H)⁺
   1-1397; A-39; B-05; MS m/z 492 (M + H)⁺
   1-1398; A-39; B-06; MS m/z 532 (M + H)⁺
   1-1399; A-39; B-07; MS m/z 512 (M + H)⁺
   1-1400; A-39; B-08; MS m/z 518 (M + H)⁺
   1-1401; A-39; B-09
   1-1402; A-39; B-10; MS m/z 542 (M + H)⁺
   1-1403; A-39; B-11; MS m/z 546 (M + H)⁺
   1-1404; A-39; B-12; MS m/z 554 (M + H)⁺
   1-1405; A-39; B-13; MS m/z 468 (M + H)⁺
   1-1406; A-39; B-14; MS m/z 476 (M + H)⁺
   1-1407; A-39; B-15; MS m/z 490 (M + H)⁺
   1-1408; A-39; B-16; MS m/z 496 (M + H)⁺
   1-1409; A-39; B-17; MS m/z 502 (M + H)⁺
   1-1410; A-39; B-18
   1-1411; A-39; B-19; MS m/z 516 (M + H)⁺
   1-1412; A-39; B-20; MS m/z 518 (M + H)⁺
   1-1413; A-39; B-21
   1-1414; A-39; B-22
   1-1415; A-39; B-23; MS m/z 538 (M + H)⁺
   1-1416; A-39; B-24; MS m/z 538 (M + H)⁺
   1-1417; A-39; B-25
   1-1418; A-39; B-26; MS m/z 547 (M + H)⁺
   1-1419; A-39; B-27; MS m/z 547 (M + H)⁺
   1-1420; A-39; B-28; MS m/z 540 (M + H)⁺
   1-1421; A-39; B-29; MS m/z 544 (M + H)⁺
   1-1422; A-39; B-30; MS m/z 556 (M + H)⁺
   1-1423; A-39; B-31; MS m/z 558 (M + H)⁺
   1-1424; A-39; B-32; MS m/z 560 (M + H)⁺
   1-1425; A-39; B-33
   1-1426; A-39; B-34
   1-1427; A-39; B-35; MS m/z 584 (M + H)⁺
   1-1428; A-39; B-36; MS m/z 602 (M + H)⁺
   1-1429; A-41; B-11; MS m/z 465 (M + H)⁺
   1-1430; A-41; B-48; MS m/z 465 (M + H)⁺
   1-1431; A-41; B-49; MS m/z 463 (M + H)⁺
   1-1432; A-41; B-50; MS m/z 449 (M + H)⁺
   1-1433; A-41; B-51; MS m/z 465 (M + H)⁺
   1-1434; A-41; B-52; MS m/z 463 (M + H)⁺
   1-1435; A-41; B-53; MS m/z 451 (M + H)⁺
   1-1436; A-41; B-54; MS m/z 479 (M + H)⁺
   1-1437; A-41; B-55; MS m/z 449 (M + H)⁺
   1-1438; A-41; B-56; MS m/z 461 (M + H)⁺
   1-1439; A-41; B-57; MS m/z 467 (M + H)⁺
   1-1440; A-41; B-58; MS m/z 445 (M + H)⁺
   1-1441; A-54; B-02; MS m/z 417 (M + H)⁺
   1-1442; A-54; B-37; MS m/z 415 (M + H)⁺
   1-1443; A-54; B-38; MS m/z 429 (M + H)⁺
   1-1444; A-54; B-39; MS m/z 429 (M + H)⁺
   1-1445; A-54; B-40; MS m/z 431 (M + H)⁺
   1-1446; A-54; B-41; MS m/z 403 (M + H)⁺
   1-1447; A-54; B-42; MS m/z 431 (M + H)⁺
   1-1448; A-54; B-43; MS m/z 431 (M + H)⁺
   1-1449; A-54; B-44; MS m/z 443 (M + H)⁺
   1-1450; A-54; B-45; MS m/z 445 (M + H)⁺
   1-1451; A-54; B-46; MS m/z 443 (M + H)⁺
   1-1452; A-54; B-47; MS m/z 445 (M + H)⁺
   1-1453; A-55; B-02; MS m/z 417 (M + H)⁺
   1-1454; A-55; B-37; MS m/z 415 (M + H)⁺
   1-1455; A-55; B-38; MS m/z 429 (M + H)⁺
   1-1456; A-55; B-39; MS m/z 429 (M + H)⁺
   1-1457; A-55; B-40; MS m/z 431 (M + H)⁺
   1-1458; A-55; B-41; MS m/z 403 (M + H)⁺
   1-1459; A-55; B-42; MS m/z 431 (M + H)⁺
   1-1460; A-55; B-43; MS m/z 431 (M + H)⁺
   1-1461; A-55; B-44; MS m/z 443 (M + H)⁺
   1-1462; A-55; B-45; MS m/z 445 (M + H)⁺
   1-1463; A-55; B-46; MS m/z 443 (M + H)⁺
   1-1464; A-55; B-47; MS m/z 445 (M + H)⁺
   1-1465; A-56; B-02; MS m/z 417 (M + H)⁺
   1-1466; A-56; B-37; MS m/z 415 (M + H)⁺
   1-1467; A-56; B-38; MS m/z 429 (M + H)⁺
   1-1468; A-56; B-39; MS m/z 429 (M + H)⁺
   1-1469; A-56; B-40; MS m/z 431 (M + H)⁺
   1-1470; A-56; B-41; MS m/z 403 (M + H)⁺
   1-1471; A-56; B-42; MS m/z 431 (M + H)⁺
   1-1472; A-56; B-43; MS m/z 431 (M + H)⁺
   1-1473; A-56; B-44; MS m/z 443 (M + H)⁺
   1-1474; A-56; B-45; MS m/z 445 (M + H)⁺
   1-1475; A-56; B-46; MS m/z 443 (M + H)⁺
   1-1476; A-56; B-47; MS m/z 445 (M + H)⁺
   1-1477; A-57; B-02; MS m/z 431 (M + H)⁺
   1-1478; A-57; B-37; MS m/z 429 (M + H)⁺
   1-1479; A-57; B-38; MS m/z 443 (M + H)⁺
   1-1480; A-57; B-39; MS m/z 443 (M + H)⁺
   1-1481; A-57; B-40; MS m/z 445 (M + H)⁺
   1-1482; A-57; B-41; MS m/z 417 (M + H)⁺
   1-1483; A-57; B-42; MS m/z 445 (M + H)⁺
   1-1484; A-57; B-43; MS m/z 445 (M + H)⁺
   1-1485; A-57; B-44; MS m/z 457 (M + H)⁺
   1-1486; A-57; B-45; MS m/z 459 (M + H)⁺
   1-1487; A-57; B-46; MS m/z 457 (M + H)⁺
   1-1488; A-57; B-47; MS m/z 459 (M + H)⁺
   1-1489: A-58; B-02; MS m/z 403 (M + H)⁺
   1-1490; A-58; B-37; MS m/z 401 (M + H)⁺
   1-1491; A-58; B-38; MS m/z 415 (M + H)⁺
   1-1492; A-58; B-39; MS m/z 415 (M + H)⁺
   1-1493; A-58; B-40; MS m/z 417 (M + H)⁺
   1-1494: A-58; B-41; MS m/z 389 (M + H)⁺
   1-1495; A-58; B-42; MS m/z 417 (M + H)⁺
   1-1496; A-58; B-43; MS m/z 417 (M + H)⁺
   1-1497; A-58; B-44; MS m/z 429 (M + H)⁺
   1-1498; A-58; B-45; MS m/z 431 (M + H)⁺
   1-1499; A-58; B-46; MS m/z 429 (M + H)⁺
   1-1500; A-58; B-47; MS m/z 431 (M + H)⁺
   1-1501; A-59; B-02; MS m/z 435 (M + H)⁺
   1-1502; A-59; B-37; MS m/z 433 (M + H)⁺
   1-1503; A-59; B-38; MS m/z 447 (M + H)⁺
   1-1504; A-59; B-39; MS m/z 447 (M + H)⁺
   1-1505; A-59; B-40; MS m/z 449 (M + H)⁺
   1-1506; A-59; B-41; MS m/z 421 (M + H)⁺
   1-1507; A-59; B-42; MS m/z 449 (M + H)⁺
   1-1508; A-59; B-43; MS m/z 449 (M + H)⁺
   1-1509; A-59; B-44; MS m/z 461 (M + H)⁺
   1-1510: A-59; B-45; MS m/z 463 (M + H)⁺
   1-1511; A-59; B-46; MS m/z 461 (M + H)⁺
   1-1512; A-59; B-47; MS m/z 463 (M + H)⁺
   1-1513; A-60; B-02; MS m/z 423 (M + H)⁺
   1-1514; A-60; B-37; MS m/z 421 (M + H)⁺
   1-1515; A-60; B-38; MS m/z 435 (M + H)⁺
   1-1516; A-60; B-39; MS m/z 435 (M + H)⁺
   1-1517; A-60; B-40; MS m/z 437 (M + H)⁺
   1-1518; A-60; B-41; MS m/z 409 (M + H)⁺
   1-1519; A-60; B-42; MS m/z 437 (M + H)⁺
   1-1520; A-60; B-43; MS m/z 437 (M + H)⁺
   1-1521; A-60; B-44; MS m/z 449 (M + H)⁺
   1-1522; A-60; B-45; MS m/z 451 (M + H)⁺
   1-1523; A-60; B-46; MS m/z 449 (M + H)⁺
   1-1524; A-60; B-47; MS m/z 451 (M + H)⁺
   1-1525; A-61; B-11; MS m/z 477 (M + H)⁺
   1-1526; A-61; B-48; MS m/z 477 (M + H)⁺
   1-1527; A-61; B-49; MS m/z 475 (M + H)⁺
   1-1528; A-61; B-50; MS m/z 461 (M + H)⁺
   1-1529; A-61; B-51; MS m/z 477 (M + H)⁺
   1-1530; A-61; B-52; MS m/z 475 (M + H)⁺
   1-1531; A-61; B-53; MS m/z 463 (M + H)⁺
   1-1532; A-61; B-54; MS m/z 491 (M + H)⁺
   1-1533; A-61; B-55; MS m/z 461 (M + H)⁺
   1-1534; A-61; B-56; MS m/z 473 (M + H)⁺
   1-1535; A-61; B-57; MS m/z 479 (M + H)⁺
   1-1536; A-61; B-58; MS m/z 457 (M + H)⁺
   1-1537; A-62; B-11; MS m/z 477 (M + H)⁺
   1-1538; A-62; B-48; MS m/z 477 (M + H)⁺
   1-1539; A-62; B-49; MS m/z 475 (M + H)⁺
   1-1540; A-62; B-50; MS m/z 461 (M + H)⁺
   1-1541; A-62; B-51; MS m/z 477 (M + H)⁺
   1-1542; A-62; B-52; MS m/z 475 (M + H)⁺
   1-1543; A-62; B-53; MS m/z 463 (M + H)⁺
   1-1544; A-62; B-54; MS m/z 491 (M + H)⁺
   1-1545; A-62; B-55; MS m/z 461 (M + H)⁺
   1-1546; A-62; B-56; MS m/z 473 (M + H)⁺
   1-1547; A-62; B-57; MS m/z 479 (M + H)⁺
   1-1548; A-62; B-58; MS m/z 457 (M + H)⁺
   1-1549; A-63; B-11; MS m/z 477 (M + H)⁺
   1-1550; A-63; B-48; MS m/z 477 (M + H)⁺
   1-1551; A-63; B-49; MS m/z 475 (M + H)⁺
   1-1552; A-63; B-50; MS m/z 461 (M + H)⁺
   1-1553; A-63; B-51; MS m/z 477 (M + H)⁺
   1-1554; A-63; B-52; MS m/z 475 (M + H)⁺
   1-1555; A-63; B-53; MS m/z 463 (M + H)⁺
   1-1556; A-63; B-54; MS m/z 491 (M + H)⁺
   1-1557; A-63; B-55; MS m/z 461 (M + H)⁺
   1-1558; A-63; B-56; MS m/z 473 (M + H)⁺
   1-1559; A-63; B-57; MS m/z 479 (M + H)⁺
   1-1560; A-63; B-58; MS m/z 457 (M + H)⁺
   1-1561; A-64; B-11; MS m/z 477 (M + H)⁺
   1-1562; A-64; B-48; MS m/z 477 (M + H)⁺
   1-1563; A-64; B-49; MS m/z 475 (M + H)⁺
   1-1564; A-64; B-50; MS m/z 461 (M + H)⁺
   1-1565; A-64; B-51; MS m/z 477 (M + H)⁺
   1-1566; A-64; B-52; MS m/z 475 (M + H)⁺
   1-1567; A-64; B-53; MS m/z 463 (M + H)⁺
   1-1568; A-64; B-54; MS m/z 491 (M + H)⁺
   1-1569; A-64; B-55; MS m/z 461 (M + H)⁺
   1-1570; A-64; B-56; MS m/z 473 (M + H)⁺
   1-1571; A-64; B-57; MS m/z 479 (M + H)⁺
   1-1572; A-64; B-58; MS m/z 457 (M + H)⁺
   1-1573; A-65; B-11; MS m/z 463 (M + H)⁺
   1-1574; A-65; B-48; MS m/z 463 (M + H)⁺
   1-1575; A-65; B-49; MS m/z 461 (M + H)⁺
   1-1576; A-65; B-50; MS m/z 447 (M + H)⁺
   1-1577; A-65; B-51; MS m/z 463 (M + H)⁺
   1-1578; A-65; B-52; MS m/z 461 (M + H)⁺
   1-1579; A-65; B-53; MS m/z 449 (M + H)⁺
   1-1580; A-65; B-54; MS m/z 477 (M + H)⁺
   1-1581; A-65; B-55; MS m/z 447 (M + H)⁺
   1-1582; A-65; B-56; MS m/z 459 (M + H)⁺
   1-1583; A-65; B-57; MS m/z 465 (M + H)⁺
   1-1584; A-65; B-58; MS m/z 443 (M + H)⁺
   1-1585; A-66; B-11; MS m/z 463 (M + H)⁺
   1-1586; A-66; B-48; MS m/z 463 (M + H)⁺
   1-1587; A-66; B-49; MS m/z 461 (M + H)⁺
   1-1588; A-66; B-50; MS m/z 447 (M + H)⁺
   1-1589; A-66; B-51; MS m/z 463 (M + H)⁺
   1-1590; A-66; B-52; MS m/z 461 (M + H)⁺
   1-1591; A-66; B-53; MS m/z 449 (M + H)⁺
   1-1592; A-66; B-54; MS m/z 477 (M + H)⁺
   1-1593; A-66; B-55; MS m/z 447 (M + H)⁺
   1-1594; A-66; B-56; MS m/z 459 (M + H)⁺
   1-1595; A-66; B-57; MS m/z 465 (M + H)⁺
   1-1596; A-66; B-58; MS m/z 443 (M + H)⁺
   1-1597; A-72; B-01; MS m/z 379 (M + H)⁺
   1-1598; A-72; B-02; MS m/z 383 (M + H)⁺
   1-1599; A-72; B-03; MS m/z 341 (M + H)⁺
   1-1600; A-72; B-04; MS m/z 399 (M + H)⁺
   1-1601; A-72; B-05; MS m/z 411 (M + H)⁺
   1-1602; A-72; B-06; MS m/z 451 (M + H)⁺
   1-1603; A-72; B-07; MS m/z 431 (M + H)⁺
   1-1604; A-72; B-08; MS m/z 437 (M + H)⁺
   1-1605; A-72; B-09; MS m/z 439 (M + H)⁺
   1-1606; A-72; B-10; MS m/z 461 (M + H)⁺
   1-1607; A-72; B-11; MS m/z 465 (M + H)⁺
   1-1608; A-72; B-12; MS m/z 473 (M + H)⁺
   1-1609; A-72; B-13; MS m/z 387 (M + H)⁺
   1-1610; A-72; B-14; MS m/z 395 (M + H)⁺
   1-1611; A-72; B-15; MS m/z 409 (M + H)⁺
   1-1612; A-72; B-16; MS m/z 415 (M + H)⁺
   1-1613; A-72; B-17; MS m/z 421 (M + H)⁺
   1-1614; A-72; B-18; MS m/z 425 (M + H)⁺
   1-1615; A-72; B-19; MS m/z 435 (M + H)⁺
   1-1616; A-72; B-20; MS m/z 437 (M + H)⁺
   1-1617; A-72; B-21; MS m/z 441 (M + H)⁺
   1-1618; A-72; B-22; MS m/z 445 (M + H)⁺
   1-1619; A-72; B-23; MS m/z 457 (M + H)⁺
   1-1620; A-72; B-24; MS m/z 457 (M + H)⁺
   1-1621; A-72; B-25; MS m/z 426 (M + H)⁺
   1-1622; A-72; B-26; MS m/z 466 (M + H)⁺
   1-1623; A-72; B-27; MS m/z 466 (M + H)⁺
   1-1624; A-72; B-28; MS m/z 459 (M + H)⁺
   1-1625; A-72; B-29; MS m/z 463 (M + H)⁺
   1-1626; A-72; B-30; MS m/z 475 (M + H)⁺
   1-1627; A-72; B-31; MS m/z 477 (M + H)⁺
   1-1628; A-72; B-32; MS m/z 479 (M + H)⁺
   1-1629; A-72; B-33; MS m/z 479 (M + H)⁺
   1-1630; A-72; B-34; MS m/z 489 (M + H)⁺
   1-1631; A-72; B-35; MS m/z 503 (M + H)⁺
   1-1632; A-72; B-36; MS m/z 521 (M + H)⁺
(b) The examples of compounds described in : 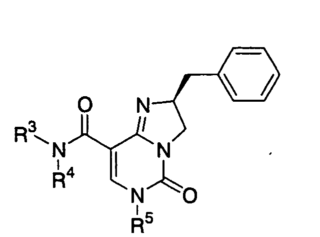 which are included in Compound (I), are listed below in order of compound number, -NR³R⁴, -R⁵ and spectrum data thereof.
   2-1; A-07; B-32; MS m/z 479 (M + H)⁺
   2-2; A-09; B-13; MS m/z 413 (M + H)⁺
   2-3; A-09; B-14; MS m/z 421 (M + H)⁺
   2-4; A-09; B-15; MS m/z 435 (M + H)⁺
   2-5; A-09; B-16; MS m/z 441 (M + H)⁺
   2-6; A-09; B-17; MS m/z 447 (M + H)⁺
   2-7; A-09; B-18; MS m/z 451 (M + H)⁺
   2-8; A-09; B-19; MS m/z 461 (M + H)⁺
   2-9; A-09; B-20; MS m/z 463 (M + H)⁺
   2-10; A-09; B-21; MS m/z 467 (M + H)⁺
   2-11; A-09; B-22; MS m/z 471 (M + H)⁺
   2-12; A-09; B-23; MS m/z 483 (M + H)⁺
   2-13; A-09; B-24; MS m/z 483 (M + H)⁺
   2-14; A-09; B-32; MS m/z 505 (M + H)⁺
   2-15; A-10; B-13; MS m/z 413 (M + H)⁺
   2-16; A-10; B-14; MS m/z 421 (M + H)⁺
   2-17; A-10; B-15; MS m/z 435 (M + H)⁺
   2-18; A-10; B-16; MS m/z 441 (M + H)⁺
   2-19; A-10; B-17; MS m/z 447 (M + H)⁺
   2-20; A-10; B-18; MS'm/z 451 (M + H)⁺
   2-21; A-10; B-19; MS m/z 461 (M + H)⁺
   2-22; A-10; B-20; MS m/z 463 (M + H)⁺
   2-23; A-10; B-21; MS m/z 467 (M + H)⁺
   2-24; A-10; B-22; MS m/z 471 (M + H)⁺
   2-25; A-10; B-23; MS m/z 483 (M + H)⁺
   2-26; A-10; B-24; MS m/z 483 (M + H)⁺
   2-27; A-11; B-13; MS m/z 413 (M + H)⁺
   2-28; A-11; B-14; MS m/z 421 (M + H)⁺
   2-29; A-11; B-15; MS m/z 435 (M + H)⁺
   2-30; A-11; B-16; MS m/z 441 (M + H)⁺
   2-31; A-11; B-17; MS m/z 447 (M + H)⁺
   2-32; A-11; B-18; MS m/z 451 (M + H)⁺
   2-33; A-11; B-19; MS m/z 461 (M + H)⁺
   2-34; A-11; B-20; MS m/z 463 (M + H)⁺
   2-35; A-11; B-21; MS m/z 467 (M + H)⁺
   2-36; A-11; B-22; MS m/z 471 (M + H)⁺
   2-37; A-11; B-23; MS m/z 483 (M + H)⁺
   2-38; A-11; B-24; MS m/z 483 (M + H)⁺
   2-39; A-11; B-31; MS m/z 503 (M + H)⁺
   2-40; A-12; B-13; MS m/z 413 (M + H)⁺
   2-41; A-12; B-14; MS m/z 421 (M + H)⁺
   2-42; A-12; B-15; MS m/z 435 (M + H)⁺
   2-43; A-12; B-16; MS m/z 441 (M + H)⁺
   2-44; A-12; B-17; MS m/z 447 (M + H)⁺
   2-45; A-12; B-18; MS m/z 451 (M + H)⁺
   2-46; A-12; B-19; MS m/z 461 (M + H)⁺
   2-47; A-12; B-20; MS m/z 463 (M + H)⁺
   2-48; A-12; B-21; MS m/z 467 (M + H)⁺
   2-49; A-12; B-22; MS m/z 471 (M + H)⁺
   2-50; A-12; B-23; MS m/z 483 (M + H)⁺
   2-51; A-12; B-24; MS m/z 483 (M + H)⁺
   2-52; A-13; B-13; MS m/z 421 (M + H)⁺
   2-53; A-13; B-14; MS m/z 429 (M + H)⁺
   2-54; A-13; B-15; MS m/z 443 (M + H)⁺
   2-55; A-13; B-16; MS m/z 449 (M + H)⁺
   2-56; A-13; B-17; MS m/z 455 (M + H)⁺
   2-57; A-13; B-18; MS m/z 459 (M + H)⁺
   2-58; A-13; B-19; MS m/z 469 (M + H)⁺
   2-59; A-13; B-20; MS m/z 471 (M + H)⁺
   2-60; A-13; B-21; MS m/z 475 (M + H)⁺
   2-61; A-13; B-22; MS m/z 479 (M + H)⁺
   2-62; A-13; B-23; MS m/z 491 (M + H)⁺
   2-63; A-13; B-24; MS m/z 491 (M + H)⁺
   2-64; A-14; B-13; MS m/z 421 (M + H)⁺
   2-65; A-14; B-14; MS m/z 429 (M + H)⁺
   2-66; A-14; B-15; MS m/z 443 (M + H)⁺
   2-67; A-14; B-16; MS m/z 449 (M + H)⁺
   2-68; A-14; B-17; MS m/z 455 (M + H)⁺
   2-69; A-14; B-18; MS m/z 459 (M + H)⁺
   2-70; A-14; B-19; MS m/z 469 (M + H)⁺
   2-71; A-14; B-20; MS m/z 471 (M + H)⁺
   2-72; A-14; B-21; MS m/z 475 (M + H)⁺
   2-73; A-14; B-22; MS m/z 479 (M + H)⁺
   2-74; A-14; B-23; MS m/z 491 (M + H)⁺
   2-75; A-14; B-24; MS m/z 491 (M + H)⁺
   2-76; A-15; B-13; MS m/z 429 (M + H)⁺
   2-77; A-15; B-14; MS m/z 437 (M + H)⁺
   2-78; A-15; B-15; MS m/z 451 (M + H)⁺
   2-79; A-15; B-16; MS m/z 457 (M + H)⁺
   2-80; A-15; B-17; MS m/z 463 (M + H)⁺
   2-81; A-15; B-18; MS m/z 467 (M + H)⁺
   2-82; A-15; B-19; MS m/z 477 (M + H)⁺
   2-83; A-15; B-20; MS m/z 479 (M + H)⁺
   2-84; A-15; B-21; MS m/z 483 (M + H)⁺
   2-85; A-15; B-22; MS m/z 487 (M + H)⁺
   2-86; A-15; B-23; MS m/z 499 (M + H)⁺
   2-87; A-15; B-24; MS m/z 499 (M + H)⁺
   2-88; A-16; B-13; MS m/z 435 (M + H)⁺
   2-89; A-16; B-14; MS m/z 443 (M + H)⁺
   2-90; A-16; B-15; MS m/z 457 (M + H)⁺
   2-91; A-16; B-16; MS m/z 463 (M + H)⁺
   2-92; A-16; B-17; MS m/z 469 (M + H)⁺
   2-93; A-16; B-18; MS m/z 473 (M + H)⁺
   2-94; A-16; B-19; MS m/z 483 (M + H)⁺
   2-95; A-16; B-20; MS m/z 485 (M + H)⁺
   2-96; A-16; B-21; MS m/z 489 (M + H)⁺
   2-97; A-16; B-22; MS m/z 493 (M + H)⁺
   2-98; A-16; B-23; MS m/z 505 (M + H)⁺
   2-99; A-16; B-24; MS m/z 505 (M + H)⁺
   2-100; A-17; B-19; MS m/z 489 (M + H)⁺
   2-101; A-18; B-08; MS m/z 507 (M + H)⁺
   2-102; A-19; B-05; MS m/z 457 (M + H)⁺
   2-103; A-19; B-16; MS m/z 461 (M + H)⁺
   2-104; A-19; B-31; MS m/z 523 (M + H)⁺
   2-105; A-24; B-09; MS m/z 527 (M + H)⁺
   2-106; A-24; B-19; MS m/z 523 (M + H)⁺
   2-107; A-24; B-32; MS m/z 567 (M + H)⁺
(c) The examples of compounds described in : 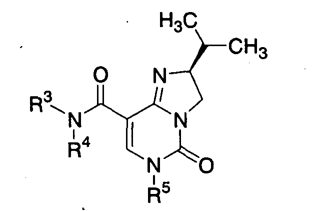 which are included in Compound (I), are listed below in order of compound number, -NR³R⁴, -R⁵ and spectrum data thereof.
   3-1; A-09; B-01
   3-2; A-09; B-02
   3-3; A-09; B-03
   3-4; A-09; B-04; MS m/z 377 (M + H)⁺
   3-5; A-09; B-05
   3-6; A-09; B-06
   3-7; A-09; B-07; MS m/z 409 (M + H)⁺
   3-8: A-09; B-08
   3-9: A-09; B-09
   3-10; A-09; B-10
   3-11; A-09; B-11
   3-12; A-09; B-12
   3-13; A-09; B-13; MS m/z 365 (M + H)⁺
   3-14; A-09; B-14; MS m/z 373 (M + H)⁺
   3-15; A-09; B-15; MS m/z 387 (M + H)⁺
   3-16; A-09; B-16; MS m/z 393 (M + H)⁺
   3-17; A-09; B-17; MS m/z 399 (M + H)⁺
   3-18; A-09; B-18; MS m/z 403 (M + H)⁺
   3-19; A-09; B-19; MS m/z 413 (M + H)⁺
   3-20; A-09; B-20; MS m/z 415 (M + H)⁺
   3-21; A-09; B-21; MS m/z 419 (M + H)⁺
   3-22; A-09; B-22; MS m/z 423 (M + H)⁺
   3-23; A-09; B-23; MS m/z 435 (M + H)⁺
   3-24; A-09; B-24; MS m/z 435 (M + H)⁺
   3-25; A-10; B-01; MS m/z 357 (M + H)⁺
   3-26; A-10; B-02; MS m/z 361 (M + H)⁺
   3-27; A-10; B-03; MS m/z 319 (M + H)⁺
   3-28; A-10; B-04; MS m/z 377 (M + H)⁺
   3-29; A-10; B-05; MS m/z 389 (M + H)⁺
   3-30; A-10; B-06; MS m/z 429 (M + H)⁺
   3-31; A-10; B-07; MS m/z 409 (M + H)⁺
   3-32; A-10; B-08; MS m/z 415 (M + H)⁺
   3-33; A-10; B-09; MS m/z 417 (M + H)⁺
   3-34; A-10; B-10; MS m/z 439 (M + H)⁺
   3-35; A-10; B-11; MS m/z 443 (M + H)⁺
   3-36; A-10; B-12; MS m/z 451 (M + H)⁺
   3-37; A-10; B-13; MS m/z 365 (M + H)⁺
   3-38; A-10; B-14; MS m/z 373 (M + H)⁺
   3-39; A-10; B-15; MS m/z 387 (M + H)⁺
   3-40; A-10; B-16; MS m/z 393 (M + H)⁺
   3-41; A-10; B-17; MS m/z 399 (M + H)⁺
   3-42; A-10; B-18; MS m/z 403 (M + H)⁺
   3-43; A-10; B-19; MS m/z 413 (M + H)⁺
   3-44; A-10; B-20; MS m/z 415 (M + H)⁺
   3-45; A-10; B-21; MS m/z 419 (M + H)⁺
   3-46; A-10; B-22; MS m/z 423 (M + H)⁺
   3-47; A-10; B-23; MS m/z 435 (M + H)⁺
   3-48; A-10; B-24; MS m/z 435 (M + H)⁺
   3-49; A-11; B-01; MS m/z 357 (M + H)⁺
   3-50; A-11; B-02; MS m/z 361 (M + H)⁺
   3-51; A-11; B-03; MS m/z 319 (M + H)⁺
   3-52; A-11; B-04; MS m/z 377 (M + H)⁺
   3-53; A-11; B-05; MS m/z 389 (M + H)⁺
   3-54; A-11; B-06; MS m/z 429 (M + H)⁺
   3-55; A-11; B-07; MS m/z 409 (M + H)⁺
   3-56; A-11; B-08; MS m/z 415 (M + H)⁺
   3-57; A-11; B-09; MS m/z 417 (M + H)⁺
   3-58; A-11; B-10; MS m/z 439 (M + H)⁺
   3-59; A-11; B-11; MS m/z 443 (M + H)⁺
   3-60; A-11; B-12; MS m/z 451 (M + H)⁺
   3-61; A-11; B-13; MS m/z 365 (M + H)⁺
   3-62; A-11; B-14; MS m/z 373 (M + H)⁺
   3-63; A-11; B-15; MS m/z 387 (M + H)⁺
   3-64; A-11; B-16; MS m/z 393 (M + H)⁺
   3-65; A-11; B-17; MS m/z 399 (M + H)⁺
   3-66; A-11; B-18; MS m/z 403 (M + H)⁺
   3-67; A-11; B-19; MS m/z 413 (M + H)⁺
   3-68; A-11; B-20; MS m/z 415 (M + H)⁺
   3-69; A-11; B-21; MS m/z 419 (M + H)⁺
   3-70; A-11; B-22; MS m/z 423 (M + H)⁺
   3-71; A-11; B-23; MS m/z 435 (M + H)⁺
   3-72; A-11; B-24; MS m/z 435 (M + H)⁺
   3-73; A-12; B-01; MS m/z 357 (M + H)⁺
   3-74; A-12; B-02; MS m/z 361 (M + H)⁺
   3-75; A-12; B-03; MS m/z 319 (M + H)⁺
   3-76; A-12; B-04; MS m/z 377 (M + H)⁺
   3-77; A-12; B-05; MS m/z 389 (M + H)⁺
   3-78; A-12; B-06; MS m/z 429 (M + H)⁺
   3-79; A-12; B-07; MS m/z 409 (M + H)⁺
   3-80; A-12; B-08; MS m/z 415 (M + H)⁺
   3-81; A-12; B-09; MS m/z 417 (M + H)⁺
   3-82; A-12; B-10; MS m/z 439 (M + H)⁺
   3-83; A-12; B-11; MS m/z 443 (M + H)⁺
   3-84; A-12; B-12; MS m/z 451 (M + H)⁺
   3-85; A-12; B-13; MS m/z 365 (M + H)⁺
   3-86; A-12; B-14; MS m/z 373 (M + H)⁺
   3-87; A-12; B-15; MS m/z 387 (M + H)⁺
   3-88; A-12; B-16; MS m/z 393 (M + H)⁺
   3-89; A-12; B-17; MS m/z 399 (M + H)⁺
   3-90; A-12; B-18; MS m/z 403 (M + H)⁺
   3-91; A-12; B-19; MS m/z 413 (M + H)⁺
   3-92; A-12; B-20; MS m/z 415 (M + H)⁺
   3-93; A-12; B-21; MS m/z 419 (M + H)⁺
   3-94; A-12; B-22; MS m/z 423 (M + H)⁺
   3-95; A-12; B-23; MS m/z 435 (M + H)⁺
   3-96; A-12; B-24; MS m/z 435 (M + H)⁺
   3-97; A-13; B-01; MS m/z 365 (M + H)⁺
   3-98; A-13; B-02; MS m/z 369 (M + H)⁺
   3-99; A-13; B-03; MS m/z 327 (M + H)⁺
   3-100; A-13; B-04; MS m/z 385 (M + H)⁺
   3-101; A-13; B-05; MS m/z 397 (M + H)⁺
   3-102; A-13; B-06; MS m/z 437 (M + H)⁺
   3-103; A-13; B-07; MS m/z 417 (M + H)⁺
   3-104; A-13; B-08; MS m/z 423 (M + H)⁺
   3-105; A-13; B-09; MS m/z 425 (M + H)⁺
   3-106; A-13; B-10; MS m/z 447 (M + H)⁺
   3-107; A-13; B-11; MS m/z 451 (M + H)⁺
   3-108; A-13; B-12; MS m/z 459 (M + H)⁺
   3-109; A-13; B-13; MS m/z 373 (M + H)⁺
   3-110: A-13; B-14; MS m/z 381 (M + H)⁺
   3-111; A-13; B-15; MS m/z 395 (M + H)⁺
   3-112; A-13; B-16; MS m/z 401 (M + H)⁺
   3-113; A-13; B-17; MS m/z 407 (M + H)⁺
   3-114; A-13; B-18; MS m/z 411 (M + H)⁺
   3-115; A-13; B-19; MS m/z 421 (M + H)⁺
   3-116; A-13; B-20; MS m/z 423 (M + H)⁺
   3-117; A-13; B-21; MS m/z 427 (M + H)⁺
   3-118; A-13; B-22; MS m/z 431 (M + H)⁺
   3-119; A-13; B-23; MS m/z 443 (M + H)⁺
   3-120; A-13; B-24; MS m/z 443 (M + H)⁺
   3-121; A-14; B-01; MS m/z 365 (M + H)⁺
   3-122; A-14; B-02; MS m/z 369 (M + H)⁺
   3-123; A-14; B-03; MS m/z 327 (M + H)⁺
   3-124; A-14; B-04; MS m/z 385 (M + H)⁺
   3-125; A-14; B-05; MS m/z 397 (M + H)⁺
   3-126; A-14; B-06; MS m/z 437 (M + H)⁺
   3-127; A-14; B-07; MS m/z 417 (M + H)⁺
   3-128; A-14; B-08; MS m/z 423 (M + H)⁺
   3-129; A-14; B-09; MS m/z 425 (M + H)⁺
   3-130; A-14; B-10; MS m/z 447 (M + H)⁺
   3-131; A-14; B-11; MS m/z 451 (M + H)⁺
   3-132; A-14; B-12; MS m/z 459 (M + H)⁺
   3-133; A-14; B-13; MS m/z 373 (M + H)⁺
   3-134; A-14; B-14; MS m/z 381 (M + H)⁺
   3-135; A-14; B-15; MS m/z 395 (M + H)⁺
   3-136; A-14; B-16; MS m/z 401 (M + H)⁺
   3-137; A-14; B-17; MS m/z 407 (M + H)⁺
   3-138; A-14; B-18; MS m/z 411 (M + H)⁺
   3-139; A-14; B-19; MS m/z 421 (M + H)⁺
   3-140; A-14; B-20; MS m/z 423 (M + H)⁺
   3-141; A-14; B-21; MS m/z 427 (M + H)⁺
   3-142; A-14; B-22; MS m/z 431 (M + H)⁺
   3-143; A-14; B-23; MS m/z 443 (M + H)⁺
   3-144; A-14; B-24; MS m/z 443 (M + H)⁺
   3-145; A-15; B-01; MS m/z 373 (M + H)⁺
   3-146; A-15; B-02; MS m/z 377 (M + H)⁺
   3-147; A-15; B-03; MS m/z 335 (M + H)⁺
   3-148; A-15; B-04; MS m/z 393 (M + H)⁺
   3-149; A-15; B-05; MS m/z 405 (M + H)⁺
   3-150; A-15; B-06
   3-151; A-15; B-07; MS m/z 425 (M + H)⁺
   3-152; A-15; B-08; MS m/z 431 (M + H)⁺
   3-153; A-15; B-09; MS m/z 433 (M + H)⁺
   3-154; A-15; B-10; MS m/z 455 (M + H)⁺
   3-155; A-15; B-11; MS m/z 459 (M + H)⁺
   3-156; A-15; B-12; MS m/z 467 (M + H)⁺
   3-157; A-15; B-13; MS m/z 381 (M + H)⁺
   3-158; A-15; B-14; MS m/z 389 (M + H)⁺
   3-159; A-15; B-15; MS m/z 403 (M + H)⁺
   3-160; A-15; B-16; MS m/z 409 (M + H)⁺
   3-161; A-15; B-17; MS m/z 415 (M + H)⁺
   3-162; A-15; B-18; MS m/z 419 (M + H)⁺
   3-163; A-15; B-19; MS m/z 429 (M + H)⁺
   3-164; A-15; B-20; MS m/z 431 (M + H)⁺
   3-165; A-15; B-21; MS m/z 435 (M + H)⁺
   3-166; A-15; B-22; MS m/z 439 (M + H)⁺
   3-167; A-15; B-23; MS m/z 451 (M + H)⁺
   3-168; A-15; B-24; MS m/z 451 (M + H)⁺
   3-169; A-16; B-01; MS m/z 379 (M + H)⁺
   3-170; A-16; B-02; MS m/z 383 (M + H)⁺
   3-171; A-16; B-03; MS m/z 341 (M + H)⁺
   3-172; A-16; B-04; MS m/z 399 (M + H)⁺
   3-173; A-16; B-05; MS m/z 411 (M + H)⁺
   3-174; A-16; B-06; MS m/z 451 (M + H)⁺
   3-175; A-16; B-07; MS m/z 431 (M + H)⁺
   3-176; A-16; B-08; MS m/z 437 (M + H)⁺
   3-177; A-16; B-09; MS m/z 439 (M + H)⁺
   3-178; A-16; B-10; MS m/z 461 (M + H)⁺
   3-179; A-16; B-11; MS m/z 465 (M + H)⁺
   3-180; A-16; B-12; MS m/z 473 (M + H)⁺
   3-181; A-16; B-13; MS m/z 387 (M + H)⁺
   3-182; A-16; B-14; MS m/z 395 (M + H)⁺
   3-183; A-16; B-15; MS m/z 409 (M + H)⁺
   3-184; A-16; B-16; MS m/z 415 (M + H)⁺
   3-185; A-16; B-17; MS m/z 421 (M + H)⁺
   3-186; A-16; B-18; MS m/z 425 (M + H)⁺
   3-187; A-16; B-19; MS m/z 435 (M + H)⁺
   3-188; A-16; B-20; MS m/z 437 (M + H)⁺
   3-189; A-16; B-21; MS m/z 441 (M + H)⁺
   3-190; A-16; B-22; MS m/z 445 (M + H)⁺
   3-191; A-16; B-23; MS m/z 457 (M + H)⁺
   3-192; A-16; B-24; MS m/z 457 (M + H)⁺
   3-193; A-19; B-08; MS m/z 435 (M + H)⁺
   3-194; A-19; B-19; MS m/z 433 (M + H)⁺
(d) The examples of compounds described in : 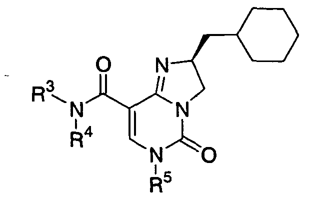 which are included in Compound (I), are listed below in order of compound number, -NR³R⁴, -R⁵ and spectrum data thereof.
   4-1; A-09; B-01; MS m/z 411 (M + H)⁺
   4-2; A-09; B-02; MS m/z 415 (M + H)⁺
   4-3; A-09; B-03; MS m/z 373 (M + H)⁺
   4-4; A-09; B-04; MS m/z 431 (M + H)⁺
   4-5; A-09; B-05; MS m/z 443 (M + H)⁺
   4-6; A-09; B-06; MS m/z 483 (M + H)⁺
   4-7; A-09; B-07; MS m/z 463 (M + H)⁺
   4-8; A-09; B-08; MS m/z 469 (M + H)⁺
   4-9; A-09; B-09; MS m/z 471 (M + H)⁺
   4-10; A-09; B-10; MS m/z 493 (M + H)⁺
   4-11; A-09; B-11; MS m/z 497 (M + H)⁺
   4-12; A-09; B-12; MS m/z 505 (M + H)⁺
   4-13; A-10; B-01; MS m/z 411 (M + H)⁺
   4-14; A-10; B-02; MS m/z 415 (M + H)⁺
   4-15; A-10; B-03; MS m/z 373 (M + H)⁺
   4-16; A-10; B-04; MS m/z 431 (M + H)⁺
   4-17; A-10; B-05; MS m/z 443 (M + H)⁺
   4-18; A-10; B-06; MS m/z 483 (M + H)⁺
   4-19; A-10; B-07; MS m/z 463 (M + H)⁺
   4-20; A-10; B-08; MS m/z 469 (M + H)⁺
   4-21; A-10; B-09; MS m/z 471 (M + H)⁺
   4-22; A-10; B-10; MS m/z 493 (M + H)⁺
   4-23; A-10; B-11; MS m/z 497 (M + H)⁺
   4-24; A-10; B-12; MS m/z 505 (M + H)⁺
   4-25; A-11; B-01; MS m/z 411 (M + H)⁺
   4-26; A-11; B-02; MS m/z 415 (M + H)⁺
   4-27; A-11; B-03; MS m/z 373 (M + H)⁺
   4-28; A-11; B-04; MS m/z 431 (M + H)⁺
   4-29; A-11; B-05; MS m/z 443 (M + H)⁺
   4-30; A-11; B-06; MS m/z 483 (M + H)⁺
   4-31; A-11; B-07; MS m/z 463 (M + H)⁺
   4-32; A-11; B-08; MS m/z 469 (M + H)⁺
   4-33; A-11; B-09; MS m/z 471 (M + H)⁺
   4-34; A-11; B-10; MS m/z 493 (M + H)⁺
   4-35; A-11; B-11; MS m/z 497 (M + H)⁺
   4-36; A-11; B-12; MS m/z 505 (M + H)⁺
   4-37; A-12; B-01; MS m/z 411 (M + H)⁺
   4-38; A-12; B-02; MS m/z 415 (M + H)⁺
   4-39; A-12; B-03; MS m/z 373 (M + H)⁺
   4-40; A-12; B-04; MS m/z 431 (M + H)⁺
   4-41; A-12; B-05; MS m/z 443 (M + H)⁺
   4-42; A-12; B-06; MS m/z 483 (M + H)⁺
   4-43; A-12; B-07; MS m/z 463 (M + H)⁺
   4-44; A-12; B-08; MS m/z 469 (M + H)⁺
   4-45; A-12; B-09; MS m/z 471 (M + H)⁺
   4-46; A-12; B-10; MS m/z 493 (M + H)⁺
   4-47; A-12; B-11; MS m/z 497 (M + H)⁺
   4-48; A-12; B-12; MS m/z 505 (M + H)⁺
   4-49; A-13; B-01; MS m/z 419 (M + H)⁺
   4-50; A-13; B-02; MS m/z 423 (M + H)⁺
   4-51; A-13; B-03; MS m/z 381 (M + H)⁺
   4-52; A-13; B-04; MS m/z 439 (M + H)⁺
   4-53; A-13; B-05; MS m/z 451 (M + H)⁺
   4-54; A-13; B-06; MS m/z 491 (M + H)⁺
   4-55; A-13; B-07; MS m/z 471 (M + H)⁺
   4-56; A-13; B-08; MS m/z 477 (M + H)⁺
   4-57; A-13; B-09; MS m/z 479 (M + H)⁺
   4-58; A-13; B-10; MS m/z 501 (M + H)⁺
   4-59; A-13; B-11; MS m/z 505 (M + H)⁺
   4-60; A-13; B-12; MS m/z 513 (M + H)⁺
   4-61; A-14; B-01; MS m/z 419 (M + H)⁺
   4-62; A-14; B-02; MS m/z 423 (M + H)⁺
   4-63; A-14; B-03; MS m/z 381 (M + H)⁺
   4-64; A-14; B-04; MS m/z 439 (M + H)⁺
   4-65; A-14; B-05; MS m/z 451 (M + H)⁺
   4-66; A-14; B-06; MS m/z 491 (M + H)⁺
   4-67; A-14; B-07; MS m/z 471 (M + H)⁺
   4-68; A-14; B-08; MS m/z 477 (M + H)⁺
   4-69; A-14; B-09; MS m/z 479 (M + H)⁺
   4-70; A-14; B-10; MS m/z 501 (M + H)⁺
   4-71; A-14; B-11; MS m/z 505 (M + H)⁺
   4-72; A-14; B-12; MS m/z 513 (M + H)⁺
   4-73; A-15; B-01; MS m/z 427 (M + H)⁺
   4-74; A-15; B-02; MS m/z 431 (M + H)⁺
   4-75; A-15; B-03; MS m/z 389 (M + H)⁺
   4-76; A-15; B-04; MS m/z 447 (M + H)⁺
   4-77; A-15; B-05; MS m/z 459 (M + H)⁺
   4-78; A-15; B-06; MS m/z 499 (M + H)⁺
   4-79; A-15; B-07; MS m/z 479 (M + H)⁺
   4-80; A-15; B-08; MS m/z 485 (M + H)⁺
   4-81; A-15; B-09; MS m/z 487 (M + H)⁺
   4-82; A-15; B-10; MS m/z 509 (M + H)⁺
   4-83; A-15; B-11; MS m/z 513 (M + H)⁺
   4-84; A-15; B-12; MS m/z 521 (M + H)⁺
   4-85; A-16; B-01; MS m/z 433 (M + H)⁺
   4-86; A-16; B-02; MS m/z 437 (M + H)⁺
   4-87; A-16; B-03; MS m/z 395 (M + H)⁺
   4-88; A-16; B-04; MS m/z 453 (M + H)⁺
   4-89; A-16; B-05; MS m/z 465 (M + H)⁺
   4-90; A-16; B-06; MS m/z 505 (M + H)⁺
   4-91; A-16; B-07; MS m/z 485 (M + H)⁺
   4-92; A-16; B-08; MS m/z 491 (M + H)⁺
   4-93; A-16; B-09; MS m/z 493 (M + H)⁺
   4-94; A-16; B-10; MS m/z 515 (M + H)⁺
   4-95; A-16; B-11; MS m/z 519 (M + H)⁺
   4-96; A-16; B-12; MS m/z 527 (M + H)⁺
   4-97; A-25; B-01; MS m/z 400 (M + H)⁺
   4-98; A-25; B-02; MS m/z 404 (M + H)⁺
   4-99; A-25; B-03; MS m/z 362 (M + H)⁺
   4-100; A-25; B-04; MS m/z 420 (M + H)⁺
   4-101; A-25; B-05; MS m/z 432 (M + H)⁺
   4-102; A-25; B-06; MS m/z 472 (M + H)⁺
   4-103; A-25; B-07; MS m/z 452 (M + H)⁺
   4-104; A-25; B-08; MS m/z 458 (M + H)⁺
   4-105; A-25; B-09; MS m/z 460 (M + H)⁺
   4-106; A-25; B-10; MS m/z 482 (M + H)⁺
   4-107; A-25; B-11; MS m/z 486 (M + H)⁺
   4-108; A-25; B-12; MS m/z 494 (M + H)⁺
   4-109; A-26; B-01; MS m/z 406 (M + H)⁺
   4-110; A-26; B-02; MS m/z 410 (M + H)⁺
   4-111; A-26; B-03; MS m/z 368 (M + H)⁺
   4-112; A-26; B-04; MS m/z 426 (M + H)⁺
   4-113; A-26; B-05; MS m/z 438 (M + H)⁺
   4-114; A-26; B-06; MS m/z 478 (M + H)⁺
   4-115; A-26; B-07; MS m/z 458 (M + H)⁺
   4-116; A-26; B-08; MS m/z 464 (M + H)⁺
   4-117; A-26; B-09; MS m/z 466 (M + H)⁺
   4-118; A-26; B-10; MS m/z 488 (M + H)⁺
   4-119; A-26; B-11; MS m/z 492 (M + H)⁺
   4-120; A-26; B-12; MS m/z 500 (M + H)⁺
   4-121; A-27; B-01; MS m/z 440 (M + H)⁺
   4-122; A-27; B-02; MS m/z 444 (M + H)⁺
   4-123; A-27; B-03; MS m/z 402 (M + H)⁺
   4-124; A-27; B-04; MS m/z 460 (M + H)⁺
   4-125; A-27; B-05; MS m/z 472 (M + H)⁺
   4-126; A-27; B-06; MS m/z 512 (M + H)⁺
   4-127; A-27; B-07; MS m/z 492 (M + H)⁺
   4-128; A-27; B-08; MS m/z 498 (M + H)⁺
   4-129; A-27; B-09; MS m/z 500 (M + H)⁺
   4-130; A-27; B-10; MS m/z 522 (M + H)⁺
   4-131; A-27; B-11; MS m/z 526 (M + H)⁺
   4-132; A-27; B-12; MS m/z 534 (M + H)⁺
   4-133; A-28; B-01; MS m/z 470 (M + H)⁺
   4-134; A-28; B-02; MS m/z 474 (M + H)⁺
   4-135; A-28; B-03; MS m/z 432 (M + H)⁺
   4-136; A-28; B-04; MS m/z 490 (M + H)⁺
   4-137; A-28; B-05; MS m/z 502 (M + H)⁺
   4-138; A-28; B-06; MS m/z 542 (M + H)⁺
   4-139; A-28; B-07; MS m/z 522 (M + H)⁺
   4-140; A-28; B-08; MS m/z 528 (M + H)⁺
   4-141; A-28; B-09; MS m/z 530 (M + H)⁺
   4-142; A-28; B-10; MS m/z 552 (M + H)⁺
   4-143; A-28; B-11; MS m/z 556 (M + H)⁺
   4-144; A-28; B-12; MS m/z 564 (M + H)⁺
   4-145; A-29; B-01; MS m/z 385 (M + H)⁺
   4-146; A-29; B-02; MS m/z 389 (M + H)⁺
   4-147; A-29; B-03; MS m/z 347 (M + H)⁺
   4-148; A-29; B-04; MS m/z 405 (M + H)⁺
   4-149; A-29; B-05; MS m/z 417 (M + H)⁺
   4-150; A-29; B-06; MS m/z 457 (M + H)⁺
   4-151; A-29; B-07; MS m/z 437 (M + H)⁺
   4-152; A-29; B-08; MS m/z 443 (M + H)⁺
   4-153; A-29; B-09; MS m/z 445 (M + H)⁺
   4-154; A-29; B-10; MS m/z 467 (M + H)⁺
   4-155; A-29; B-11; MS m/z 471 (M + H)⁺
   4-156; A-29; B-12; MS m/z 479 (M + H)⁺
   4-157; A-30; B-01; MS m/z 428 (M + H)⁺
   4-158; A-30; B-02; MS m/z 432 (M + H)⁺
   4-159; A-30; B-03; MS m/z 390 (M + H)⁺
   4-160; A-30; B-04; MS m/z 448 (M + H)⁺
   4-161; A-30; B-05; MS m/z 460 (M + H)⁺
   4-162; A-30; B-06; MS m/z 500 (M + H)⁺
   4-163; A-30; B-07; MS m/z 480 (M + H)⁺
   4-164; A-30; B-08; MS m/z 486 (M + H)⁺
   4-165; A-30; B-09; MS m/z 488 (M + H)⁺
   4-166; A-30; B-10; MS m/z 510 (M + H)⁺
   4-167; A-30; B-11; MS m/z 514 (M + H)⁺
   4-168; A-30; B-12; MS m/z 522 (M + H)⁺
   4-169; A-31; B-01; MS m/z 428 (M + H)⁺
   4-170; A-31; B-02; MS m/z 432 (M + H)⁺
   4-171; A-31; B-03; MS m/z 390 (M + H)⁺
   4-172; A-31; B-04; MS m/z 448 (M + H)⁺
   4-173; A-31; B-05; MS m/z 460 (M + H)⁺
   4-174; A-31; B-06; MS m/z 500 (M + H)⁺
   4-175; A-31; B-07; MS m/z 480 (M + H)⁺
   4-176; A-31; B-08; MS m/z 486 (M + H)⁺
   4-177; A-31; B-09; MS m/z 488 (M + H)⁺
   4-178; A-31; B-10; MS m/z 510 (M + H)⁺
   4-179; A-31; B-11; MS m/z 514 (M + H)⁺
   4-180; A-31; B-12; MS m/z 522 (M + H)⁺
   4-181; A-32; B-01; MS m/z 454 (M + H)⁺
   4-182; A-32; B-02; MS m/z 458 (M + H)⁺
   4-183; A-32; B-03; MS m/z 416 (M + H)⁺
   4-184; A-32; B-04; MS m/z 474 (M + H)⁺
   4-185; A-32; B-05; MS m/z 486 (M + H)⁺
   4-186; A-32; B-06; MS m/z 526 (M + H)⁺
   4-187; A-32; B-07; MS m/z 506 (M + H)⁺
   4-188; A-32; B-08; MS m/z 512 (M + H)⁺
   4-189; A-32; B-09; MS m/z 514 (M + H)⁺
   4-190; A-32; B-10; MS m/z 536 (M + H)⁺
   4-191; A-32; B-11; MS m/z 540 (M + H)⁺
   4-192; A-32; B-12; MS m/z 548 (M + H)⁺
(e) The examples of compounds described in : 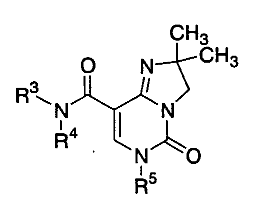 which are included in Compound (I), are listed below in order of compound number, -NR³R⁴, -R⁵ and spectrum data thereof.
   5-1; A-09; B-01; MS m/z 343 (M + H)⁺
   5-2; A-09; B-02; MS m/z 347 (M + H)⁺
   5-3; A-09; B-03; MS m/z 305 (M + H)⁺
   5-4; A-09; B-04; MS m/z 363 (M + H)⁺
   5-5; A-09; B-05; MS m/z 375 (M + H)⁺
   5-6; A-09; B-06; MS m/z 415 (M + H)⁺
   5-7; A-09; B-07; MS m/z 395 (M + H)⁺
   5-8; A-09; B-08; MS m/z 401 (M + H)⁺
   5-9; A-09; B-09; MS m/z 403 (M + H)⁺
   5-10; A-09; B-10; MS m/z 425 (M + H)⁺
   5-11; A-09; B-11; MS m/z 429 (M + H)⁺
   5-12; A-09; B-12; MS m/z 437 (M + H)⁺
   5-13; A-10; B-01; MS m/z 343 (M + H)⁺
   5-14; A-10; B-02; MS m/z 347 (M + H)⁺
   5-15; A-10; B-03; MS m/z 305 (M + H)⁺
   5-16; A-10; B-04; MS m/z 363 (M + H)⁺
   5-17; A-10; B-05; MS m/z 375 (M + H)⁺
   5-18; A-10; B-06; MS m/z 415 (M + H)⁺
   5-19; A-10; B-07; MS m/z 395 (M + H)⁺
   5-20; A-10; B-08; MS m/z 401 (M + H)⁺
   5-21; A-10; B-09; MS m/z 403 (M + H)⁺
   5-22; A-10; B-10; MS m/z 425 (M + H)⁺
   5-23; A-10; B-11; MS m/z 429 (M + H)⁺
   5-24; A-10; B-12; MS m/z 437 (M + H)⁺
   5-25; A-11; B-01; MS m/z 343 (M + H)⁺
   5-26; A-11; B-02; MS m/z 347 (M + H)⁺
   5-27; A-11; B-03; MS m/z 305 (M + H)⁺
   5-28; A-11; B-04; MS m/z 363 (M + H)⁺
   5-29; A-11; B-05; MS m/z 375 (M + H)⁺
   5-30; A-11; B-06; MS m/z 415 (M + H)⁺
   5-31; A-11; B-07; MS m/z 395 (M + H)⁺
   5-32; A-11; B-08; MS m/z 401 (M + H)⁺
   5-33; A-11; B-09; MS m/z 403 (M + H)⁺
   5-34; A-11; B-10; MS m/z 425 (M + H)⁺
   5-35; A-11; B-11; MS m/z 429 (M + H)⁺
   5-36; A-11; B-12; MS m/z 437 (M + H)⁺
   5-37; A-12; B-01; MS m/z 343 (M + H)⁺
   5-38; A-12; B-02; MS m/z 347 (M + H)⁺
   5-39; A-12; B-03; MS m/z 305 (M + H)⁺
   5-40; A-12; B-04; MS m/z 363 (M + H)⁺
   5-41; A-12; B-05; MS m/z 375 (M + H)⁺
   5-42; A-12; B-06; MS m/z 415 (M + H)⁺
   5-43; A-12; B-07; MS m/z 395 (M + H)⁺
   5-44; A-12; B-08; MS m/z 401 (M + H)⁺
   5-45; A-12; B-09; MS m/z 403 (M + H)⁺
   5-46; A-12; B-10; MS m/z 425 (M + H)⁺
   5-47; A-12; B-11; MS m/z 429 (M + H)⁺
   5-48; A-12; B-12; MS m/z 437 (M + H)⁺
   5-49; A-13; B-01; MS m/z 351 (M + H)⁺
   5-50; A-13; B-02; MS m/z 355 (M + H)⁺
   5-51; A-13; B-03; MS m/z 313 (M + H)⁺
   5-52; A-13; B-04; MS m/z 371 (M + H)⁺
   5-53; A-13; B-05; MS m/z 383 (M + H)⁺
   5-54; A-13; B-06; MS m/z 423 (M + H)⁺
   5-55; A-13; B-07; MS m/z 403 (M + H)⁺
   5-56; A-13; B-08; MS m/z 409 (M + H)⁺
   5-57; A-13; B-09; MS m/z 411 (M + H)⁺
   5-58; A-13; B-10; MS m/z 433 (M + H)⁺
   5-59; A-13; B-11; MS m/z 437 (M + H)⁺
   5-60; A-13; B-12; MS m/z 445 (M + H)⁺
   5-61; A-14; B-01; MS m/z 351 (M + H)⁺
   5-62; A-14; B-02; MS m/z 355 (M + H)⁺
   5-63; A-14; B-03; MS m/z 313 (M + H)⁺
   5-64; A-14; B-04; MS m/z 371 (M + H)⁺
   5-65; A-14; B-05; MS m/z 383 (M + H)⁺
   5-66; A-14; B-06; MS m/z 423 (M + H)⁺
   5-67; A-14; B-07; MS m/z 403 (M + H)⁺
   5-68; A-14; B-08; MS m/z 409 (M + H)⁺
   5-69; A-14; B-09; MS m/z 411 (M + H)⁺
   5-70; A-14; B-10; MS m/z 433 (M + H)⁺
   5-71; A-14; B-11; MS m/z 437 (M + H)⁺
   5-72; A-14; B-12; MS m/z 445 (M + H)⁺
   5-73; A-15; B-01; MS m/z 359 (M + H)⁺
   5-74; A-15; B-02; MS m/z 363 (M + H)⁺
   5-75; A-15; B-03; MS m/z 321 (M + H)⁺
   5-76; A-15; B-04; MS m/z 379 (M + H)⁺
   5-77; A-15; B-05; MS m/z 391 (M + H)⁺
   5-78; A-15; B-06; MS m/z 431 (M + H)⁺
   5-79; A-15; B-07; MS m/z 411 (M + H)⁺
   5-80; A-15; B-08; MS m/z 417 (M + H)⁺
   5-81; A-15; B-09; MS m/z 419 (M + H)⁺
   5-82; A-15; B-10; MS m/z 441 (M + H)⁺
   5-83; A-15; B-11; MS m/z 445 (M + H)⁺
   5-84; A-15; B-12; MS m/z 453 (M + H)⁺
   5-85; A-16; B-01; MS m/z 365 (M + H)⁺
   5-86; A-16; B-02; MS m/z 369 (M + H)⁺
   5-87; A-16; B-03; MS m/z 327 (M + H)⁺
   5-88; A-16; B-04; MS m/z 385 (M + H)⁺
   5-89; A-16; B-05; MS m/z 397 (M + H)⁺
   5-90; A-16; B-06; MS m/z 437 (M + H)⁺
   5-91; A-16; B-07; MS m/z 417 (M + H)⁺
   5-92; A-16; B-08; MS m/z 423 (M + H)⁺
   5-93; A-16; B-09; MS m/z 425 (M + H)⁺
   5-94; A-16; B-10; MS m/z 447 (M + H)⁺
   5-95; A-16; B-11; MS m/z 451 (M + H)⁺
   5-96; A-16; B-12; MS m/z 459 (M + H)⁺
   5-97; A-17; B-01; MS m/z 371 (M + H)⁺
   5-98; A-17; B-02; MS m/z 375 (M + H)⁺
   5-99; A-17; B-03; MS m/z 333 (M + H)⁺
   5-100; A-17; B-04; MS m/z 391 (M + H)⁺
   5-101; A-17; B-05; MS m/z 403 (M + H)⁺
   5-102; A-17; B-06; MS m/z 443 (M + H)⁺
   5-103; A-17; B-07; MS m/z 423 (M + H)⁺
   5-104; A-17; B-08; MS m/z 429 (M + H)⁺
   5-105; A-17; B-09; MS m/z 431 (M + H)⁺
   5-106; A-17; B-10; MS m/z 453 (M + H)⁺
   5-107; A-17; B-11; MS m/z 457 (M + H)⁺
   5-108; A-17; B-12; MS m/z 465 (M + H)⁺
   5-109; A-18; B-01; MS m/z 387 (M + H)⁺
   5-110; A-18; B-02; MS m/z 391 (M + H)⁺
   5-111; A-18; B-03; MS m/z 349 (M + H)⁺
   5-112; A-18; B-04; MS m/z 407 (M + H)⁺
   5-113; A-18; B-05; MS m/z 419 (M + H)⁺
   5-114; A-18; B-06; MS m/z 459 (M + H)⁺
   5-115; A-18; B-07; MS m/z 439 (M + H)⁺
   5-116; A-18; B-08; MS m/z 445 (M + H)⁺
   5-117; A-18; B-09; MS m/z 447 (M + H)⁺
   5-118; A-18; B-10; MS m/z 469 (M + H)⁺
   5-119; A-18; B-11; MS m/z 473 (M + H)⁺
   5-120; A-18; B-12; MS m/z 481 (M + H)⁺
   5-121; A-19; B-01; MS m/z 363 (M + H)⁺
   5-122; A-19; B-02; MS m/z 367 (M + H)⁺
   5-123; A-19; B-03; MS m/z 325 (M + H)⁺
   5-124; A-19; B-04; MS m/z 383 (M + H)⁺
   5-125; A-19; B-05; MS m/z 395 (M + H)⁺
   5-126; A-19; B-06; MS m/z 435 (M + H)⁺
   5-127; A-19; B-07; MS m/z 415 (M + H)⁺
   5-128; A-19; B-08; MS m/z 421 (M + H)⁺
   5-129; A-19; B-09; MS m/z 423 (M + H)⁺
   5-130; A-19; B-10; MS m/z 445 (M + H)⁺
   5-131; A-19; B-11; MS m/z 449 (M + H)⁺
   5-132; A-19; B-12; MS m/z 457 (M + H)⁺
   5-133; A-20; B-01; MS m/z 367 (M + H)⁺
   5-134; A-20; B-02; MS m/z 371 (M + H)⁺
   5-135; A-20; B-03; MS m/z 329 (M + H)⁺
   5-136; A-20; B-04; MS m/z 387 (M + H)⁺
   5-137; A-20; B-05; MS m/z 399 (M + H)⁺
   5-138; A-20; B-06; MS m/z 439 (M + H)⁺
   5-139; A-20; B-07; MS m/z 419 (M + H)⁺
   5-140; A-20; B-08; MS m/z 425 (M + H)⁺
   5-141; A-20; B-09; MS m/z 427 (M + H)⁺
   5-142; A-20; B-10; MS m/z 449 (M + H)⁺
   5-143; A-20; B-11; MS m/z 453 (M + H)⁺
   5-144; A-20; B-12; MS m/z 461 (M + H)⁺
   5-145; A-21; B-01; MS m/z 373 (M + H)⁺
   5-146; A-21; B-02; MS m/z 377 (M + H)⁺
   5-147; A-21; B-03; MS m/z 335 (M + H)⁺
   5-148; A-21; B-04; MS m/z 393 (M + H)⁺
   5-149; A-21; B-05; MS m/z 405 (M + H)⁺
   5-150; A-21; B-06; MS m/z 445 (M + H)⁺
   5-151; A-21; B-07; MS m/z 425 (M + H)⁺
   5-152; A-21; B-08; MS m/z 431 (M + H)⁺
   5-153; A-21; B-09; MS m/z 433 (M + H)⁺
   5-154; A-21; B-10; MS m/z 455 (M + H)⁺
   5-155; A-21; B-11; MS m/z 459 (M + H)⁺
   5-156; A-21; B-12; MS m/z 467 (M + H)⁺
   5-157; A-22; B-01; MS m/z 397 (M + H)⁺
   5-158; A-22; B-02; MS m/z 401 (M + H)⁺
   5-159; A-22; B-03; MS m/z 359 (M + H)⁺
   5-160; A-22; B-04; MS m/z 417 (M + H)⁺
   5-161; A-22; B-05; MS m/z 429 (M + H)⁺
   5-162; A-22; B-06; MS m/z 469 (M + H)⁺
   5-163; A-22; B-07; MS m/z 449 (M + H)⁺
   5-164; A-22; B-08; MS m/z 455 (M + H)⁺
   5-165; A-22; B-09; MS m/z 457 (M + H)⁺
   5-166; A-22; B-10; MS m/z 479 (M + H)⁺
   5-167; A-22; B-11; MS m/z 483 (M + H)⁺
   5-168; A-22; B-12; MS m/z 491 (M + H)⁺
   5-169; A-23; B-01; MS m/z 401 (M + H)⁺
   5-170; A-23; B-02; MS m/z 405 (M + H)⁺
   5-171; A-23; B-03; MS m/z 363 (M + H)⁺
   5-172; A-23; B-04; MS m/z 421 (M + H)⁺
   5-173; A-23; B-05; MS m/z 433 (M + H)⁺
   5-174; A-23; B-06; MS m/z 473 (M + H)⁺
   5-175; A-23; B-07; MS m/z 453 (M + H)⁺
   5-176; A-23; B-08; MS m/z 459 (M + H)⁺
   5-177; A-23; B-09; MS m/z 461 (M + H)⁺
   5-178; A-23; B-10; MS m/z 483 (M + H)⁺
   5-179; A-23; B-11; MS m/z 487 (M + H)⁺
   5-180; A-23; B-12; MS m/z 495 (M + H)⁺
   5-181; A-24; B-01; MS m/z 405 (M + H)⁺
   5-182; A-24; B-02; MS m/z 409 (M + H)⁺
   5-183; A-24; B-03; MS m/z 367 (M + H)⁺
   5-184; A-24; B-04; MS m/z 425 (M + H)⁺
   5-185; A-24; B-05; MS m/z 437 (M + H)⁺
   5-186; A-24; B-06; MS m/z 477 (M + H)⁺
   5-187; A-24; B-07; MS m/z 457 (M + H)⁺
   5-188; A-24; B-08; MS m/z 463 (M + H)⁺
   5-189; A-24; B-09; MS m/z 465 (M + H)⁺
   5-190; A-24; B-10; MS m/z 487 (M + H)⁺
   5-191; A-24; B-11; MS m/z 491 (M + H)⁺
   5-192; A-24; B-12; MS m/z 499 (M + H)⁺
(f) The examples of compounds described in : 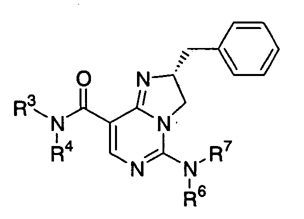 which are included in Compound (I), are listed below in order of compound number, -NR³R⁴, -NR⁶R⁷ and spectrum data thereof.
   6-1; A-09; C-25; MS m/z 437 (M + H)⁺
   6-2; A-09; C-26
   6-3; A-09; C-27; MS m/z 457 (M + H)⁺
   6-4; A-09; C-28; MS m/z 560 (M + H)⁺
   6-5; A-09; C-29; MS m/z 463 (M + H)⁺
   6-6; A-09; C-30; MS m/z 479 (M + H)⁺
   6-7; A-09; C-31; MS m/z 479 (M + H)⁺
   6-8; A-09; C-32; MS m/z 505 (M + H)⁺
   6-9; A-09; C-33; MS m/z 517 (M + H)⁺
   6-10; A-09; C-34; MS m/z 525 (M + H)⁺
   6-11; A-09; C-35; MS m/z 534 (M + H)⁺
   6-12; A-09; C-36; MS m/z 529 (M + H)⁺
   6-13; A-10; C-25; MS m/z 437 (M + H)⁺
   6-14; A-10; C-26
   6-15; A-10; C-27; MS m/z 457 (M + H)⁺
   6-16; A-10; C-28; MS m/z 560 (M + H)⁺
   6-17; A-10; C-29; MS m/z 463 (M + H)⁺
   6-18; A-10; C-30; MS m/z 479 (M + H)⁺
   6-19; A-10; C-31; MS m/z 479 (M + H)⁺
   6-20; A-10; C-32; MS m/z 505 (M + H)⁺
   6-21; A-10; C-33; MS m/z 517 (M + H)⁺
   6-22; A-10; C-34
   6-23; A-10; C-35; MS m/z 534 (M + H)⁺
   6-24; A-10; C-36; MS m/z 529 (M + H)⁺
   6-25; A-11; C-25; MS m/z 437 (M + H)⁺
   6-26; A-11; C-26
   6-27; A-11; C-27; MS m/z 457 (M + H)⁺
   6-28; A-11; C-28; MS m/z 560 (M + H)⁺
   6-29; A-11; C-29; MS m/z 463 (M + H)⁺
   6-30; A-11; C-30; MS m/z 479 (M + H)⁺
   6-31; A-11; C-31; MS m/z 479 (M + H)⁺
   6-32; A-11; C-32; MS m/z 505 (M + H)⁺
   6-33; A-11; C-33; MS m/z 517 (M + H)⁺
   6-34; A-11; C-34; MS m/z 525 (M + H)⁺
   6-35; A-11; C-35; MS m/z 534 (M + H)⁺
   6-36; A-11; C-36; MS m/z 529 (M + H)⁺
   6-37; A-12; C-25; MS m/z 437 (M + H)⁺
   6-38; A-12; C-26
   6-39; A-12; C-27; MS m/z 457 (M + H)⁺
   6-40; A-12; C-28; MS m/z 560 (M + H)⁺
   6-41; A-12; C-29; MS m/z 463 (M + H)⁺
   6-42; A-12; C-30; MS m/z 479 (M + H)⁺
   6-43; A-12; C-31; MS m/z 479 (M + H)⁺
   6-44; A-12; C-32; MS m/z 505 (M + H)⁺
   6-45; A-12; C-33; MS m/z 517 (M + H)⁺
   6-46; A-12; C-34
   6-47; A-12; C-35; MS m/z 534 (M + H)⁺
   6-48; A-12; C-36; MS m/z 529 (M + H)⁺
   6-49; A-13; C-25; MS m/z 445 (M + H)⁺
   6-50; A-13; C-26
   6-51; A-13; C-27; MS m/z 465 (M + H)⁺
   6-52; A-13; C-28; MS m/z 568 (M + H)⁺
   6-53; A-13; C-29; MS m/z 471 (M + H)⁺
   6-54; A-13; C-30; MS m/z 487 (M + H)⁺
   6-55; A-13; C-31; MS m/z 487 (M + H)⁺
   6-56; A-13; C-32; MS m/z 513 (M + H)⁺
   6-57; A-13; C-33; MS m/z 525 (M + H)⁺
   6-58; A-13; C-34; MS m/z 533 (M + H)⁺
   6-59; A-13; C-35; MS m/z 542 (M + H)⁺
   6-60; A-13; C-36; MS m/z 537 (M + H)⁺
   6-61; A-14; C-25; MS m/z 445 (M + H)⁺
   6-62; A-14; C-26
   6-63; A-14; C-27; MS m/z 465 (M + H)⁺
   6-64; A-14; C-28; MS m/z 568 (M + H)⁺
   6-65; A-14; C-29; MS m/z 471 (M + H)⁺
   6-66; A-14; C-30; MS m/z 487 (M + H)⁺
   6-67; A-14; C-31; MS m/z 487 (M + H)⁺
   6-68; A-14; C-32; MS m/z 513 (M + H)⁺
   6-69; A-14; C-33; MS m/z 525 (M + H)⁺
   6-70; A-14; C-34
   6-71; A-14; C-35; MS m/z 542 (M + H)⁺
   6-72; A-14; C-36; MS m/z 537 (M + H)⁺
   6-73; A-15; C-25; MS m/z 453 (M + H)⁺
   6-74; A-15; C-26
   6-75; A-15; C-27; MS m/z 473 (M + H)⁺
   6-76; A-15; C-28: MS m/z 576 (M + H)⁺
   6-77; A-15; C-29; MS m/z 479 (M + H)⁺
   6-78; A-15; C-30; MS m/z 495 (M + H)⁺
   6-79; A-15; C-31; MS m/z 495 (M + H)⁺
   6-80; A-15; C-32; MS m/z 521 (M + H)⁺
   6-81; A-15; C-33; MS m/z 533 (M + H)⁺
   6-82; A-15; C-34
   6-83; A-15; C-35; MS m/z 550 (M + H)⁺
   6-84; A-15; C-36; MS m/z 545 (M + H)⁺
   6-85; A-16; C-25; MS m/z 459 (M + H)⁺
   6-86; A-16; C-26
   6-87; A-16; C-27; MS m/z 479 (M + H)⁺
   6-88; A-16; C-28; MS m/z 582 (M + H)⁺
   6-89; A-16; C-29; MS m/z 485 (M + H)⁺
   6-90; A-16; C-30; MS m/z 501 (M + H)⁺
   6-91; A-16; C-31; MS m/z 501 (M + H)⁺
   6-92; A-16; C-32; MS m/z 527 (M + H)⁺
   6-93; A-16; C-33; MS m/z 539 (M + H)⁺
   6-94; A-16; C-34
   6-95; A-16; C-35; MS m/z 556 (M + H)⁺
   6-96; A-16; C-36; MS m/z 551 (M + H)⁺
(g) The examples of compounds described in : 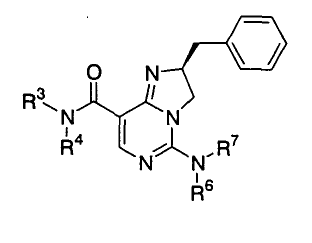 which are included in Compound (I), are listed below in order of compound number, -NR³R⁴, -NR⁶R⁷ and spectrum data thereof.
   7-1; A-01; C-01; MS m/z 354 (M + H)⁺
   7-2; A-01; C-02; MS m/z 366 (M + H)⁺
   7-3; A-01; C-03; MS m/z 378 (M + H)⁺
   7-4; A-01; C-04; MS m/z 382 (M + H)⁺
   7-5; A-01; C-05; MS m/z 384 (M + H)⁺
   7-6; A-01; C-06; MS m/z 392 (M + H)⁺
   7-7; A-01; C-07; MS m/z 394 (M + H)⁺
   7-8; A-01; C-08; MS m/z 410 (M + H)⁺
   7-9; A-01; C-09; MS m/z 416 (M + H)⁺
   7-10; A-01; C-10; MS m/z 422 (M + H)⁺
   7-11; A-01; C-11; MS m/z 430 (M + H)⁺
   7-12; A-01; C-12; MS m/z 436 (M + H)⁺
   7-13; A-01; C-13; MS m/z 446 (M + H)⁺
   7-14; A-01; C-14; MS m/z 452 (M + H)⁺
   7-15; A-01; C-16; MS m/z 462 (M + H)⁺
   7-16; A-01: C-17; MS m/z 470 (M + H)⁺
   7-17; A-01; C-18; MS m/z 434 (M + H)⁺
   7-18; A-01; C-19; MS m/z 382 (M + H)⁺
   7-19; A-01: C-20; MS m/z 475 (M + H)⁺
   7-20; A-01: C-21; MS m/z 397 (M + H)⁺
   7-21; A-01; C-22; MS m/z 423 (M + H)⁺
   7-22; A-01; C-23; MS m/z 437 (M + H)⁺
   7-23; A-01; C-24; MS m/z 458 (M + H)⁺
   7-24; A-01; C-25; MS m/z 383 (M + H)⁺
   7-25; A-01; C-26; MS m/z 395 (M + H)⁺
   7-26; A-01; C-27; MS m/z 403 (M + H)⁺
   7-27; A-01; C-28; MS m/z 506 (M + H)⁺
   7-28; A-01; C-29; MS m/z 409 (M + H)⁺
   7-29; A-01; C-30; MS m/z 425 (M + H)⁺
   7-30; A-01; C-31; MS m/z 425 (M + H)⁺
   7-31; A-01; C-32; MS m/z 451 (M + H)⁺
   7-32; A-01; C-33; MS m/z 463 (M + H)⁺
   7-33; A-01; C-34; MS m/z 471 (M + H)⁺
   7-34; A-01; C-35; MS m/z 480 (M + H)⁺
   7-35; A-01; C-36; MS m/z 475 (M + H)⁺
   7-36; A-01; C-48; MS m/z 456 (M + H)⁺
   7-37; A-02; C-01; MS m/z 366 (M + H)⁺
   7-38; A-02; C-02; MS m/z 378 (M + H)⁺
   7-39; A-02; C-03; MS m/z 390 (M + H)⁺
   7-40; A-02; C-04; MS m/z 394 (M + H)⁺
   7-41; A-02; C-05; MS m/z 396 (M + H)⁺
   7-42; A-02; C-06; MS m/z 404 (M + H)⁺
   7-43; A-02; C-07; MS m/z 406 (M + H)⁺
   7-44; A-02; C-08; MS m/z 422 (M + H)⁺
   7-45; A-02; C-09; MS m/z 428 (M + H)⁺
   7-46; A-02; C-10; MS m/z 434 (M + H)⁺
   7-47; A-02; C-11; MS m/z 442 (M + H)⁺
   7-48; A-02; C-12; MS m/z 448 (M + H)⁺
   7-49;.A-02; C-13; MS m/z 458 (M + H)⁺
   7-50; A-02; C-14; MS m/z 464 (M + H)⁺
   7-51; A-02; C-16; MS m/z 474 (M + H)⁺
   7-52; A-02; C-17; MS m/z 482 (M + H)⁺
   7-53; A-02; C-18; MS m/z 446 (M + H)⁺
   7-54; A-02; C-19; MS m/z 394 (M + H)⁺
   7-55; A-02; C-20; MS m/z 487 (M + H)⁺
   7-56; A-02; C-21; MS m/z 409 (M + H)⁺
   7-57; A-02; C-22; MS m/z 435 (M + H)⁺
   7-58; A-02; C-23; MS m/z 449 (M + H)⁺
   7-59; A-02; C-24; MS m/z 470 (M + H)⁺
   7-60; A-02; C-25; MS m/z 395 (M + H)⁺
   7-61; A-02; C-26; MS m/z 407 (M + H)⁺
   7-62; A-02; C-27; MS m/z 415 (M + H)⁺
   7-63; A-02; C-28; MS m/z 518 (M + H)⁺
   7-64; A-02; C-29; MS m/z 421 (M + H)⁺
   7-65; A-02; C-30; MS m/z 437 (M + H)⁺
   7-66; A-02; C-31; MS m/z 437 (M + H)⁺
   7-67; A-02; C-32; MS m/z 463 (M + H)⁺
   7-68; A-02; C-33; MS m/z 475 (M + H)⁺
   7-69; A-02; C-34; MS m/z 483 (M + H)⁺
   7-70; A-02; C-35; MS m/z 492 (M + H)⁺
   7-71; A-02; C-36; MS m/z 487 (M + H)⁺
   7-72; A-02; C-48; MS m/z 468 (M + H)⁺
   7-73; A-03; C-01; MS m/z 366 (M + H)⁺
   7-74; A-03; C-02; MS m/z 378 (M + H)⁺
   7-75; A-03; C-03; MS m/z 390 (M + H)⁺
   7-76; A-03; C-04; MS m/z 394 (M + H)⁺
   7-77; A-03; C-05; MS m/z 396 (M + H)⁺
   7-78; A-03; C-06; MS m/z 404 (M + H)⁺
   7-79; A-03; C-07; MS m/z 406 (M + H)⁺
   7-80; A-03; C-08; MS m/z 422 (M + H)⁺
   7-81; A-03; C-09; MS m/z 428 (M + H)⁺
   7-82; A-03; C-10; MS m/z 434 (M + H)⁺
   7-83; A-03; C-11; MS m/z 442 (M + H)⁺
   7-84; A-03; C-12; MS m/z 448 (M + H)⁺
   7-85; A-03; C-13; MS m/z 458 (M + H)⁺
   7-86; A-03; C-14; MS m/z 464 (M + H)⁺
   7-87; A-03; C-16; MS m/z 474 (M + H)⁺
   7-88; A-03; C-17; MS m/z 482 (M + H)⁺
   7-89; A-03; C-18; MS m/z 446 (M + H)⁺
   7-90; A-03; C-19; MS m/z 394 (M + H)⁺
   7-91; A-03; C-20; MS m/z 487 (M + H)⁺
   7-92; A-03; C-21; MS m/z 409 (M + H)⁺
   7-93; A-03; C-22; MS m/z 435 (M + H)⁺
   7-94; A-03; C-23; MS m/z 449 (M + H)⁺
   7-95; A-03; C-24; MS m/z 470 (M + H)⁺
   7-96; A-03; C-25; MS m/z 395 (M + H)⁺
   7-97; A-03; C-26; MS m/z 407 (M + H)⁺
   7-98; A-03; C-27; MS m/z 415 (M + H)⁺
   7-99; A-03; C-28; MS m/z 518 (M + H)⁺
   7-100; A-03; C-29; MS m/z 421 (M + H)⁺
   7-101; A-03; C-30; MS m/z 437 (M + H)⁺
   7-102; A-03; C-31; MS m/z 437 (M + H)⁺
   7-103; A-03; C-32; MS m/z 463 (M + H)⁺
   7-104; A-03; C-33; MS m/z 475 (M + H)⁺
   7-105; A-03; C-34; MS m/z 483 (M + H)⁺
   7-106; A-03; C-35; MS m/z 492 (M + H)⁺
   7-107; A-03; C-36; MS m/z 487 (M + H)⁺
   7-108; A-03; C-48; MS m/z 468 (M + H)⁺
   7-109; A-04; C-01; MS m/z 368 (M + H)⁺
   7-110; A-04; C-02; MS m/z 380 (M + H)⁺
   7-111; A-04; C-03; MS m/z 392 (M + H)⁺
   7-112; A-04; C-04; MS m/z 396 (M + H)⁺
   7-113; A-04; C-05; MS m/z 398 (M + H)⁺
   7-114; A-04; C-06; MS m/z 406 (M + H)⁺
   7-115; A-04; C-07; MS m/z 408 (M + H)⁺
   7-116; A-04; C-08; MS m/z 424 (M + H)⁺
   7-117; A-04; C-09; MS m/z 430 (M + H)⁺
   7-118; A-04; C-10; MS m/z 436 (M + H)⁺
   7-119; A-04; C-11; MS m/z 444 (M + H)⁺
   7-120; A-04; C-12; MS m/z 450 (M + H)⁺
   7-121; A-04; C-13; MS m/z 460 (M + H)⁺
   7-122; A-04; C-14; MS m/z 466 (M + H)⁺
   7-123; A-04; C-16; MS m/z 476 (M + H)⁺
   7-124; A-04; C-17; MS m/z 484 (M + H)⁺
   7-125; A-04; C-18; MS m/z 448 (M + H)⁺
   7-126; A-04; C-19; MS m/z 396 (M + H)⁺
   7-127; A-04; C-20; MS m/z 489 (M + H)⁺
   7-128; A-04; C-21; MS m/z 411 (M + H)⁺
   7-129; A-04; C-22; MS m/z 437 (M + H)⁺
   7-130; A-04; C-23; MS m/z 451 (M + H)⁺
   7-131; A-04; C-24; MS m/z 472 (M + H)⁺
   7-132; A-04; C-25; MS m/z 397 (M + H)⁺
   7-133; A-04; C-26; MS m/z 409 (M + H)⁺
   7-134; A-04; C-27; MS m/z 417 (M + H)⁺
   7-135; A-04; C-28; MS m/z 520 (M + H)⁺
   7-136; A-04; C-29; MS m/z 423 (M + H)⁺
   7-137; A-04; C-30; MS m/z 439 (M + H)⁺
   7-138; A-04; C-31; MS m/z 439 (M + H)⁺
   7-139; A-04; C-32; MS m/z 465 (M + H)⁺
   7-140; A-04; C-33; MS m/z 477 (M + H)⁺
   7-141; A-04; C-34; MS m/z 485 (M + H)⁺
   7-142; A-04; C-35; MS m/z 494 (M + H)⁺
   7-143; A-04; C-36; MS m/z 489 (M + H)⁺
   7-144; A-04; C-48; MS m/z 470 (M + H)⁺
   7-145; A-05; C-01; MS m/z 382 (M + H)⁺
   7-146; A-05; C-02; MS m/z 394 (M + H)⁺
   7-147; A-05; C-03; MS m/z 406 (M + H)⁺
   7-148; A-05; C-04; MS m/z 410 (M + H)⁺
   7-149; A-05; C-05; MS m/z 412 (M + H)⁺
   7-150; A-05; C-06; MS m/z 420 (M + H)⁺
   7-151; A-05; C-07; MS m/z 422 (M + H)⁺
   7-152; A-05; C-08; MS m/z 438 (M + H)⁺
   7-153; A-05; C-09; MS m/z 444 (M + H)⁺
   7-154; A-05; C-10; MS m/z 450 (M + H)⁺
   7-155; A-05; C-11; MS m/z 458 (M + H)⁺
   7-156; A-05; C-12; MS m/z 464 (M + H)⁺
   7-157; A-05; C-13; MS m/z 474 (M + H)⁺
   7-158; A-05; C-14; MS m/z 480 (M + H)⁺
   7-159; A-05; C-16; MS m/z 490 (M + H)⁺
   7-160; A-05; C-17; MS m/z 498 (M + H)⁺
   7-161; A-05; C-18; MS m/z 462 (M + H)⁺
   7-162; A-05; C-19; MS m/z 410 (M + H)⁺
   7-163; A-05;.C-20; MS m/z 503 (M + H)⁺
   7-164; A-05; C-21; MS m/z 425 (M + H)⁺
   7-165; A-05; C-22; MS m/z 451 (M + H)⁺
   7-166; A-05; C-23; MS m/z 465 (M + H)⁺
   7-167; A-05; C-24; MS m/z 486 (M + H)⁺
   7-168; A-05; C-25; MS m/z 411 (M + H)⁺
   7-169; A-05; C-26; MS m/z 423 (M + H)⁺
   7-170; A-05; C-27; MS m/z 431 (M + H)⁺
   7-171; A-05; C-28; MS m/z 534 (M + H)⁺
   7-172; A-05; C-29; MS m/z 437 (M + H)⁺
   7-173; A-05; C-30; MS m/z 453 (M + H)⁺
   7-174; A-05; C-31; MS m/z 453 (M + H)⁺
   7-175; A-05; C-32; MS m/z 479 (M + H)⁺
   7-176; A-05; C-33; MS m/z 491 (M + H)⁺
   7-177; A-05; C-34; MS m/z 499 (M + H)⁺
   7-178; A-05; C-35; MS m/z 508 (M + H)⁺
   7-179; A-05; C-36; MS m/z 503 (M + H)⁺
   7-180; A-05; C-37; MS m/z 467 (M + H)⁺
   7-181; A-05; C-38; MS m/z 480 (M + H)⁺
   7-182; A-05; C-39; MS m/z 453 (M + H)⁺
   7-183; A-05; C-40; MS m/z 451 (M + H)⁺
   7-184; A-05; C-41; MS m/z 467 (M + H)⁺
   7-185; A-05; C-42; MS m/z 451 (M + H)⁺
   7-186; A-05; C-43; MS m/z 451 (M + H)⁺
   7-187; A-05; C-44; MS m/z 439 (M + H)⁺
   7-188; A-05; C-45; MS m/z 451 (M + H)⁺
   7-189; A-05; C-46; MS m/z 448 (M + H)⁺
   7-190; A-05; C-47; MS m/z 440 (M + H)⁺
   7-191; A-05; C-48; MS m/z 484 (M + H)⁺
   7-192; A-06; C-01; MS m/z 384 (M + H)⁺
   7-193; A-06; C-02; MS m/z 396 (M + H)⁺
   7-194; A-06; C-03; MS m/z 408 (M + H)⁺
   7-195; A-06; C-04; MS m/z 412 (M + H)⁺
   7-196; A-06; C-05; MS m/z 414 (M + H)⁺
   7-197; A-06; C-06; MS m/z 422 (M + H)⁺
   7-198; A-06; C-07; MS m/z 424 (M + H)⁺
   7-199; A-06; C-08; MS m/z 440 (M + H)⁺
   7-200; A-06; C-09; MS m/z 446 (M + H)⁺
   7-201; A-06; C-10; MS m/z 452 (M + H)⁺
   7-202; A-06; C-11; MS m/z 460 (M + H)⁺
   7-203; A-06; C-12; MS m/z 466 (M + H)⁺
   7-204; A-06; C-13; MS m/z 476 (M + H)⁺
   7-205; A-06; C-14; MS m/z 482 (M + H)⁺
   7-206; A-06; C-16; MS m/z 492 (M + H)⁺
   7-207; A-06; C-17; MS m/z 500 (M + H)⁺
   7-208; A-06; C-18; MS m/z 464 (M + H)⁺
   7-209; A-06; C-19; MS m/z 412 (M + H)⁺
   7-210; A-06; C-20; MS m/z 505 (M + H)⁺
   7-211; A-06; C-21; MS m/z 427 (M + H)⁺
   7-212; A-06; C-22; MS m/z 453 (M + H)⁺
   7-213; A-06; C-23; MS m/z 467 (M + H)⁺
   7-214; A-06; C-24; MS m/z 488 (M + H)⁺
   7-215; A-06; C-25; MS m/z 413 (M + H)⁺
   7-216; A-06; C-26; MS m/z 425 (M + H)⁺
   7-217; A-06; C-27; MS m/z 433 (M + H)⁺
   7-218; A-06; C-28; MS m/z 536 (M + H)⁺
   7-219; A-06; C-29; MS m/z 439 (M + H)⁺
   7-220; A-06; C-30; MS m/z 455 (M + H)⁺
   7-221; A-06; C-31; MS m/z 455 (M + H)⁺
   7-222; A-06; C-32; MS m/z 481 (M + H)⁺
   7-223; A-06; C-33; MS m/z 493 (M + H)⁺
   7-224; A-06; C-34; MS m/z 501 (M + H)⁺
   7-225; A-06; C-35; MS m/z 510 (M + H)⁺
   7-226; A-06; C-36; MS m/z 505 (M + H)⁺
   7-227; A-06; C-48; MS m/z 486 (M + H)⁺
   7-228; A-07; C-01; MS m/z 382 (M + H)⁺
   7-229; A-07; C-02; MS m/z 394 (M + H)⁺
   7-230; A-07; C-03; MS m/z 406 (M + H)⁺
   7-231; A-07; C-04; MS m/z 410 (M + H)⁺
   7-232; A-07; C-05; MS m/z 412 (M + H)⁺
   7-233; A-07; C-06; MS m/z 420 (M + H)⁺
   7-234; A-07; C-07; MS m/z 422 (M + H)⁺
   7-235; A-07; C-08; MS m/z 438 (M + H)⁺
   7-236; A-07; C-09; MS m/z 444 (M + H)⁺
   7-237; A-07; C-10; MS m/z 450 (M + H)⁺
   7-238; A-07; C-11; MS m/z 458 (M + H)⁺
   7-239; A-07; C-12; MS m/z 464 (M + H)⁺
   7-240; A-07; C-13; MS m/z 474 (M + H)⁺
   7-241; A-07; C-14; MS m/z 480 (M + H)⁺
   7-242; A-07; C-16; MS m/z 490 (M + H)⁺
   7-243; A-07; C-17; MS m/z 498 (M + H)⁺
   7-244; A-07; C-18; MS m/z 462 (M + H)⁺
   7-245; A-07; C-19; MS m/z 410 (M + H)⁺
   7-246; A-07; C-20; MS m/z 503 (M + H)⁺
   7-247; A-07; C-21; MS m/z 425 (M + H)⁺
   7-248; A-07; C-22; MS m/z 451 (M + H)⁺
   7-249; A-07; C-23; MS m/z 465 (M + H)⁺
   7-250; A-07; C-24; MS m/z 486 (M + H)⁺
   7-251; A-07; C-25; MS m/z 411 (M + H)⁺
   7-252; A-07; C-26; MS m/z 423 (M + H)⁺
   7-253; A-07; C-27; MS m/z 431 (M + H)⁺
   7-254; A-07; C-28; MS m/z 534 (M + H)⁺
   7-255; A-07; C-29; MS m/z 437 (M + H)⁺
   7-256; A-07; C-30; MS m/z 453 (M + H)⁺
   7-257; A-07; C-31; MS m/z 453 (M + H)⁺
   7-258; A-07; C-32; MS m/z 479 (M + H)⁺
   7-259; A-07; C-33; MS m/z 491 (M + H)⁺
   7-260; A-07; C-34; MS m/z 499 (M + H)⁺
   7-261; A-07; C-35; MS m/z 508 (M + H)⁺
   7-262; A-07; C-36; MS m/z 503 (M + H)⁺
   7-263; A-07; C-48; MS m/z 484 (M + H)⁺
   7-264; A-08; C-01; MS m/z 384 (M + H)⁺
   7-265; A-08; C-02; MS m/z 396 (M + H)⁺
   7-266; A-08; C-03; MS m/z 408 (M + H)⁺
   7-267; A-08; C-04; MS m/z 412 (M + H)⁺
   7-268; A-08; C-05; MS m/z 414 (M + H)⁺
   7-269; A-08; C-06; MS m/z 422 (M + H)⁺
   7-270; A-08; C-07; MS m/z 424 (M + H)⁺
   7-271; A-08; C-08; MS m/z 440 (M + H)⁺
   7-272; A-08; C-09; MS m/z 446 (M + H)⁺
   7-273; A-08; C-10; MS m/z 452 (M + H)⁺
   7-274; A-08; C-11; MS m/z 460 (M + H)⁺
   7-275; A-08; C-12; MS m/z 466 (M + H)⁺
   7-276; A-08; C-13; MS m/z 476 (M + H)⁺
   7-277; A-08; C-14; MS m/z 482 (M + H)⁺
   7-278; A-08; C-16; MS m/z 492 (M + H)⁺
   7-279; A-08; C-17; MS m/z 500 (M + H)⁺
   7-280; A-08; C-18; MS m/z 464 (M + H)⁺
   7-281; A-08; C-19; MS m/z 412 (M + H)⁺
   7-282; A-08; C-20; MS m/z 505 (M + H)⁺
   7-283; A-08; C-21; MS m/z 427 (M + H)⁺
   7-284; A-08; C-22; MS m/z 453 (M + H)⁺
   7-285; A-08; C-23; MS m/z 467 (M + H)⁺
   7-286; A-08; C-24; MS m/z 488 (M + H)⁺
   7-287; A-08; C-25; MS m/z 413 (M + H)⁺
   7-288; A-08; C-26; MS m/z 425 (M + H)⁺
   7-289; A-08; C-27; MS m/z 433 (M + H)⁺
   7-290; A-08; C-28; MS m/z 536 (M + H)⁺
   7-291; A-08; C-29; MS m/z 439 (M + H)⁺
   7-292; A-08; C-30; MS m/z 455 (M + H)⁺
   7-293; A-08; C-31; MS m/z 455 (M + H)⁺
   7-294; A-08; C-32; MS m/z 481 (M + H)⁺
   7-295; A-08; C-33; MS m/z 493 (M + H)⁺
   7-296; A-08; C-34; MS m/z 501 (M + H)⁺
   7-297; A-08; C-35; MS m/z 510 (M + H)⁺
   7-298; A-08; C-36; MS m/z 505 (M + H)⁺
   7-299; A-08; C-48; MS m/z 486 (M + H)⁺
   7-300; A-09; C-01; MS m/z 408 (M + H)⁺
   7-301; A-09; C-02; MS m/z 420 (M + H)⁺
   7-302; A-09; C-03; MS m/z 432 (M + H)⁺
   7-303; A-09; C-04; MS m/z 436 (M + H)⁺
   7-304; A-09; C-05; MS m/z 438 (M + H)⁺
   7-305; A-09; C-06; MS m/z 446 (M + H)⁺
   7-306; A-09; C-07; MS m/z 448 (M + H)⁺
   7-307; A-09; C-08; MS m/z 464 (M + H)⁺
   7-308; A-09; C-09; MS m/z 470 (M + H)⁺
   7-309; A-09; C-10; MS m/z 476 (M + H)⁺
   7-310; A-09; C-11; MS m/z 484 (M + H)⁺
   7-311; A-09; C-12; MS m/z 490 (M + H)⁺
   7-312; A-09; C-13; MS m/z 500 (M + H)⁺
   7-313; A-09; C-14; MS m/z 506 (M + H)⁺
   7-314; A-09; C-15; MS m/z 524 (M + H)⁺
   7-315; A-09; C-16; MS m/z 516 (M + H)⁺
   7-316; A-09; C-17; MS m/z 524 (M + H)⁺
   7-317; A-09; C-18; MS m/z 436 (M + H)⁺
   7-318; A-09; C-19; MS m/z 488 (M + H)⁺
   7-319; A-09; C-20; MS m/z 529 (M + H)⁺
   7-320; A-09; C-21; MS m/z 451 (M + H)⁺
   7-321; A-09; C-22; MS m/z 477 (M + H)⁺
   7-322; A-09; C-23; MS m/z 491 (M + H)⁺
   7-323; A-09; C-24; MS m/z 512 (M + H)⁺
   7-324; A-09; C-25; MS m/z 437 (M + H)⁺
   7-325; A-09; C-26; MS m/z 449 (M + H)⁺
   7-326; A-09; C-27; MS m/z 457 (M + H)⁺
   7-327; A-09; C-28; MS m/z 560 (M + H)⁺
   7-328; A-09; C-29; MS m/z 463 (M + H)⁺
   7-329; A-09; C-30; MS m/z 479 (M + H)⁺
   7-330; A-09; C-31; MS m/z 479 (M + H)⁺
   7-331; A-09; C-32; MS m/z 505 (M + H)⁺
   7-332; A-09; C-33; MS m/z 517 (M + H)⁺
   7-333; A-09; C-34; MS m/z 525 (M + H)⁺
   7-334; A-09; C-35; MS m/z 534 (M + H)⁺
   7-335; A-09; C-36; MS m/z 529 (M + H)⁺
   7-336; A-10; C-01; MS m/z 408 (M + H)⁺
   7-337; A-10; C-02; MS m/z 420 (M + H)⁺
   7-338; A-10; C-03; MS m/z 432 (M + H)⁺
   7-339; A-10; C-04; MS m/z 436 (M + H)⁺
   7-340; A-10; C-05; MS m/z 438 (M + H)⁺
   7-341; A-10; C-06; MS m/z 446 (M + H)⁺
   7-342; A-10; C-07; MS m/z 448 (M + H)⁺
   7-343; A-10; C-08; MS m/z 464 (M + H)⁺
   7-344; A-10; C-09; MS m/z 470 (M + H)⁺
   7-345; A-10; C-10; MS m/z 476 (M + H)⁺
   7-346; A-10; C-11; MS m/z 484 (M + H)⁺
   7-347; A-10; C-12; MS m/z 490 (M + H)⁺
   7-348; A-10; C-13; MS m/z 500 (M + H)⁺
   7-349; A-10; C-14; MS m/z 506 (M + H)⁺
   7-350; A-10; C-15; MS m/z 524 (M + H)⁺
   7-351; A-10; C-16; MS m/z 516 (M + H)⁺
   7-352; A-10; C-17; MS m/z 524 (M + H)⁺
   7-353; A-10; C-18; MS m/z 436 (M + H)⁺
   7-354; A-10; C-19; MS m/z 488 (M + H)⁺
   7-355; A-10; C-20; MS m/z 529 (M + H)⁺
   7-356; A-10; C-21; MS m/z 451 (M + H)⁺
   7-357; A-10; C-22; MS m/z 477 (M + H)⁺
   7-358; A-10; C-23; MS m/z 491 (M + H)⁺
   7-359; A-10; C-24; MS m/z 512 (M + H)⁺
   7-360; A-10; C-25; MS m/z 437 (M + H)⁺
   7-361; A-10; C-26; MS m/z 449 (M + H)⁺
   7-362; A-10; C-27; MS m/z 457 (M + H)⁺
   7-363; A-10; C-28; MS m/z 560 (M + H)⁺
   7-364; A-10; C-29; MS m/z 463 (M + H)⁺
   7-365; A-10; C-30; MS m/z 479 (M + H)⁺
   7-366; A-10; C-31; MS m/z 479 (M + H)⁺
   7-367; A-10; C-32; MS m/z 505 (M + H)⁺
   7-368; A-10; C-33; MS m/z 517 (M + H)⁺
   7-369; A-10; C-34; MS m/z 525 (M + H)⁺
   7-370; A-10; C-35; MS m/z 534 (M + H)⁺
   7-371; A-10; C-36; MS m/z 529 (M + H)⁺
   7-372; A-11; C-01; MS m/z 408 (M + H)⁺
   7-373; A-11; C-02; MS m/z 420 (M + H)⁺
   7-374; A-11; C-03; MS m/z 432 (M + H)⁺
   7-375; A-11; C-04; MS m/z 436 (M + H)⁺
   7-376; A-11; C-05; MS m/z 438 (M + H)⁺
   7-377; A-11; C-06; MS m/z 446 (M + H)⁺
   7-378; A-11; C-07; MS m/z 448 (M + H)⁺
   7-379; A-11; C-08; MS m/z 464 (M + H)⁺
   7-380; A-11; C-09; MS m/z 470 (M + H)⁺
   7-381; A-11; C-10; MS m/z 476 (M + H)⁺
   7-382; A-11; C-11; MS m/z 484 (M + H)⁺
   7-383; A-11; C-12; MS m/z 490 (M + H)⁺
   7-384; A-11; C-13; MS m/z 500 (M + H)⁺
   7-385; A-11; C-14; MS m/z 506 (M + H)⁺
   7-386; A-11; C-15; MS m/z 524 (M + H)⁺
   7-387; A-11; C-16; MS m/z 516 (M + H)⁺
   7-388; A-11; C-17; MS m/z 524 (M + H)⁺
   7-389; A-11; C-18; MS m/z 436 (M + H)⁺
   7-390; A-11; C-19; MS m/z 488 (M + H)⁺
   7-391; A-11; C-20; MS m/z 529 (M + H)⁺
   7-392; A-11; C-21; MS m/z 451 (M + H)⁺
   7-393; A-11; C-22; MS m/z 477 (M + H)⁺
   7-394; A-11; C-23; MS m/z 491 (M + H)⁺
   7-395; A-11; C-24; MS m/z 512 (M + H)⁺
   7-396; A-11; C-25; MS m/z 437 (M + H)⁺
   7-397; A-11; C-26; MS m/z 449 (M + H)⁺
   7-398; A-11; C-27; MS m/z 457 (M + H)⁺
   7-399; A-11; C-28; MS m/z 560 (M + H)⁺
   7-400; A-11; C-29; MS m/z 463 (M + H)⁺
   7-401; A-11; C-30; MS m/z 479 (M + H)⁺
   7-402; A-11; C-31; MS m/z 479 (M + H)⁺
   7-403; A-11; C-32; MS m/z 505 (M + H)⁺
   7-404; A-11; C-33; MS m/z 517 (M + H)⁺
   7-405; A-11; C-34; MS m/z 525 (M + H)⁺
   7-406; A-11; C-35; MS m/z 534 (M + H)⁺
   7-407; A-11; C-36; MS m/z 529 (M + H)⁺
   7-408; A-12; C-01; MS m/z 408 (M + H)⁺
   7-409; A-12; C-02; MS m/z 420 (M + H)⁺
   7-410; A-12; C-03; MS m/z 432 (M + H)⁺
   7-411; A-12; C-04; MS m/z 436 (M + H)⁺
   7-412; A-12; C-05; MS m/z 438 (M + H)⁺
   7-413; A-12; C-06; MS m/z 446 (M + H)⁺
   7-414; A-12; C-07; MS m/z 448 (M + H)⁺
   7-415; A-12; C-08; MS m/z 464 (M + H)⁺
   7-416; A-12; C-09; MS m/z 470 (M + H)⁺
   7-417; A-12; C-10; MS m/z 476 (M + H)⁺
   7-418; A-12; C-11; MS m/z 484 (M + H)⁺
   7-419; A-12; C-12; MS m/z 490 (M + H)⁺
   7-420; A-12; C-13; MS m/z 500 (M + H)⁺
   7-421; A-12; C-14; MS m/z 506 (M + H)⁺
   7-422; A-12; C-15; MS m/z 524 (M + H)⁺
   7-423; A-12; C-16; MS m/z 516 (M + H)⁺
   7-424; A-12; C-17; MS m/z 524 (M + H)⁺
   7-425; A-12; C-18; MS m/z 436 (M + H)⁺
   7-426; A-12; C-19; MS m/z 488 (M + H)⁺
   7-427; A-12; C-20; MS m/z 529 (M + H)⁺
   7-428; A-12; C-21; MS m/z 451 (M + H)⁺
   7-429; A-12; C-22; MS m/z 477 (M + H)⁺
   7-430; A-12; C-23; MS m/z 491 (M + H)⁺
   7-431; A-12; C-24; MS m/z 512 (M + H)⁺
   7-432; A-12; C-25; MS m/z 437 (M + H)⁺
   7-433; A-12; C-26; MS m/z 449 (M + H)⁺
   7-434; A-12; C-27; MS m/z 457 (M + H)⁺
   7-435; A-12; C-28; MS m/z 560 (M + H)⁺
   7-436; A-12; C-29; MS m/z 463 (M + H)⁺
   7-437; A-12; C-30; MS m/z 479 (M + H)⁺
   7-438; A-12; C-31; MS m/z 479 (M + H)⁺
   7-439; A-12; C-32; MS m/z 505 (M + H)⁺
   7-440; A-12; C-33; MS m/z 517 (M + H)⁺
   7-441; A-12; C-34; MS m/z 525 (M + H)⁺
   7-442; A-12; C-35; MS m/z 534 (M + H)⁺
   7-443; A-12; C-36; MS m/z 529 (M + H)⁺
   7-444; A-13; C-01; MS m/z 416 (M + H)⁺
   7-445; A-13; C-02; MS m/z 428 (M + H)⁺
   7-446; A-13; C-03; MS m/z 440 (M + H)⁺
   7-447; A-13; C-04; MS m/z 444 (M + H)⁺
   7-448; A-13; C-05; MS m/z 446 (M + H)⁺
   7-449; A-13; C-06; MS m/z 454 (M + H)⁺
   7-450; A-13; C-07; MS m/z 456 (M + H)⁺
   7-451; A-13; C-08; MS m/z 472 (M + H)⁺
   7-452; A-13; C-09; MS m/z 478 (M + H)⁺
   7-453; A-13; C-10; MS m/z 484 (M + H)⁺
   7-454; A-13; C-11; MS m/z 492 (M + H)⁺
   7-455; A-13; C-12; MS m/z 498 (M + H)⁺
   7-456; A-13; C-13; MS m/z 508 (M + H)⁺
   7-457; A-13; C-14; MS m/z 514 (M + H)⁺
   7-458; A-13; C-15; MS m/z 532 (M + H)⁺
   7-459; A-13; C-16; MS m/z 524 (M + H)⁺
   7-460; A-13; C-17; MS m/z 532 (M + H)⁺
   7-461; A-13; C-18; MS m/z 444 (M + H)⁺
   7-462; A-13; C-19; MS m/z 496 (M + H)⁺
   7-463; A-13; C-20; MS m/z 537 (M + H)⁺
   7-464; A-13; C-21; MS m/z 459 (M + H)⁺
   7-465; A-13; C-22; MS m/z 485 (M + H)⁺
   7-466; A-13; C-23; MS m/z 499 (M + H)⁺
   7-467; A-13; C-24; MS m/z 520 (M + H)⁺
   7-468; A-13; C-25; MS m/z 445 (M + H)⁺
   7-469; A-13; C-26; MS m/z 457 (M + H)⁺
   7-470; A-13; C-27; MS m/z 465 (M + H)⁺
   7-471; A-13; C-28; MS m/z 568 (M + H)⁺
   7-472; A-13; C-29; MS m/z 471 (M + H)⁺
   7-473; A-13; C-30; MS m/z 487 (M + H)⁺
   7-474; A-13; C-31; MS m/z 487 (M + H)⁺
   7-475; A-13; C-32; MS m/z 513 (M + H)⁺
   7-476; A-13; C-33; MS m/z 525 (M + H)⁺
   7-477; A-13; C-34; MS m/z 533 (M + H)⁺
   7-478; A-13; C-35; MS m/z 542 (M + H)⁺
   7-479; A-13; C-36; MS m/z 537 (M + H)⁺
   7-480; A-14; C-01: MS m/z 416 (M + H)⁺
   7-481; A-14; C-02; MS m/z 428 (M + H)⁺
   7-482; A-14; C-03; MS m/z 440 (M + H)⁺
   7-483; A-14; C-04; MS m/z 444 (M + H)⁺
   7-484; A-14; C-05; MS m/z 446 (M + H)⁺
   7-485; A-14; C-06; MS m/z 454 (M + H)⁺
   7-486; A-14; C-07; MS m/z 456 (M + H)⁺
   7-487; A-14; C-08; MS m/z 472 (M + H)⁺
   7-488; A-14; C-09; MS m/z 478 (M + H)⁺
   7-489; A-14; C-10; MS m/z 484 (M + H)⁺
   7-490; A-14; C-11; MS m/z 492 (M + H)⁺
   7-491; A-14; C-12; MS m/z 498 (M + H)⁺
   7-492; A-14; C-13; MS m/z 508 (M + H)⁺
   7-493; A-14; C-14; MS m/z 514 (M + H)⁺
   7-494; A-14; C-15; MS m/z 532 (M + H)⁺
   7-495; A-14; C-16; MS m/z 524 (M + H)⁺
   7-496; A-14; C-17; MS m/z 532 (M + H)⁺
   7-497; A-14; C-18; MS m/z 444 (M + H)⁺
   7-498; A-14; C-19; MS m/z 496 (M + H)⁺
   7-499; A-14; C-20; MS m/z 537 (M + H)⁺
   7-500; A-14; C-21; MS m/z 459 (M + H)⁺
   7-501; A-14; C-22; MS m/z 485 (M + H)⁺
   7-502; A-14; C-23; MS m/z 499 (M + H)⁺
   7-503; A-14; C-24; MS m/z 520 (M + H)⁺
   7-504; A-14; C-25; MS m/z 445 (M + H)⁺
   7-505; A-14; C-26; MS m/z 457 (M + H)⁺
   7-506; A-14; C-27; MS m/z 465 (M + H)⁺
   7-507; A-14; C-28; MS m/z 568 (M + H)⁺
   7-508; A-14; C-29; MS m/z 471 (M + H)⁺
   7-509; A-14; C-30; MS m/z 487 (M + H)⁺
   7-510; A-14; C-31; MS m/z 487 (M + H)⁺
   7-511; A-14; C-32; MS m/z 513 (M + H)⁺
   7-512; A-14; C-33; MS m/z 525 (M + H)⁺
   7-513; A-14; C-34; MS m/z 533 (M + H)⁺
   7-514; A-14; C-35; MS m/z 542 (M + H)⁺
   7-515; A-14; C-36; MS m/z 537 (M + H)⁺
   7-516; A-14; C-37; MS m/z 501 (M + H)⁺
   7-517; A-14; C-38; MS m/z 514 (M + H)⁺
   7-518; A-14; C-39; MS m/z 487 (M + H)⁺
   7-519; A-14; C-40; MS m/z 485 (M + H)⁺
   7-520; A-14; C-41; MS m/z 501 (M + H)⁺
   7-521; A-14; C-42; MS m/z 485 (M + H)⁺
   7-522; A-14; C-43; MS m/z 485 (M + H)⁺
   7-523; A-14; C-44; MS m/z 473 (M + H)⁺
   7-524; A-14; C-45; MS m/z 485 (M + H)⁺
   7-525; A-14; C-46; MS m/z 482 (M + H)⁺
   7-526; A-14; C-47; MS m/z 474 (M + H)⁺
   7-527; A-15; C-01; MS m/z 424 (M + H)⁺
   7-528; A-15; C-02; MS m/z 436 (M + H)⁺
   7-529; A-15; C-03; MS m/z 448 (M + H)⁺
   7-530; A-15; C-04; MS m/z 452 (M + H)⁺
   7-531; A-15; C-05; MS m/z 454 (M + H)⁺
   7-532; A-15; C-06; MS m/z 462 (M + H)⁺
   7-533; A-15; C-07; MS m/z 464 (M + H)⁺
   7-534; A-15; C-08; MS m/z 480 (M + H)⁺
   7-535; A-15; C-09; MS m/z 486 (M + H)⁺
   7-536; A-15; C-10; MS m/z 492 (M + H)⁺
   7-537; A-15; C-11; MS m/z 500 (M + H)⁺
   7-538; A-15; C-12; MS m/z 506 (M + H)⁺
   7-539; A-15; C-13; MS m/z 516 (M + H)⁺
   7-540; A-15; C-14; MS m/z 522 (M + H)⁺
   7-541; A-15; C-15; MS m/z 540 (M + H)⁺
   7-542; A-15; C-16; MS m/z 532 (M + H)⁺
   7-543; A-15; C-17; MS m/z 540 (M + H)⁺
   7-544; A-15; C-18; MS m/z 452 (M + H)⁺
   7-545; A-15; C-19; MS m/z 504 (M + H)⁺
   7-546; A-15; C-20; MS m/z 545 (M + H)⁺
   7-547; A-15; C-21; MS m/z 467 (M + H)⁺
   7-548; A-15; C-22; MS m/z 493 (M + H)⁺
   7-549; A-15; C-23; MS m/z 507 (M + H)⁺
   7-550; A-15; C-24; MS m/z 528 (M + H)⁺
   7-551; A-15; C-25; MS m/z 453 (M + H)⁺
   7-552; A-15; C-26; MS m/z 465 (M + H)⁺
   7-553; A-15; C-27; MS m/z 473 (M + H)⁺
   7-554; A-15; C-28; MS m/z 576 (M + H)⁺
   7-555; A-15; C-29; MS m/z 479 (M + H)⁺
   7-556; A-15; C-30; MS m/z 495 (M + H)⁺
   7-557; A-15; C-31; MS m/z 495 (M + H)⁺
   7-558; A-15; C-32; MS m/z 521 (M + H)⁺
   7-559; A-15; C-33; MS m/z 533 (M + H)⁺
   7-560; A-15; C-34; MS m/z 541 (M + H)⁺
   7-561; A-15; C-35; MS m/z 550 (M + H)⁺
   7-562; A-15; C-36; MS m/z 545 (M + H)⁺
   7-563; A-16; C-01; MS m/z 430 (M + H)⁺
   7-564; A-16; C-02; MS m/z 442 (M + H)⁺
   7-565; A-16; C-03; MS m/z 454 (M + H)⁺
   7-566; A-16; C-04; MS m/z 458 (M + H)⁺
   7-567; A-16; C-05; MS m/z 460 (M + H)⁺
   7-568; A-16; C-06; MS m/z 468 (M + H)⁺.
   7-569; A-16; C-07; MS m/z 470 (M + H)⁺
   7-570; A-16; C-08; MS m/z 486 (M + H)⁺
   7-571; A-16; C-09; MS m/z 492 (M + H)⁺
   7-572; A-16; C-10; MS m/z 498 (M + H)⁺
   7-573; A-16; C-11; MS m/z 506 (M + H)⁺
   7-574; A-16; C-12; MS m/z 512 (M + H)⁺
   7-575; A-16; C-13; MS m/z 522 (M + H)⁺
   7-576; A-16; C-14; MS m/z 528 (M + H)⁺
   7-577; A-16; C-15; MS m/z 546 (M + H)⁺
   7-578; A-16; C-16; MS m/z 538 (M + H)⁺
   7-579; A-16; C-17; MS m/z 546 (M + H)⁺
   7-580; A-16; C-18; MS m/z 458 (M + H)⁺
   7-581; A-16; C-19; MS m/z 510 (M + H)⁺
   7-582; A-16; C-20; MS m/z 551 (M + H)⁺
   7-583; A-16; C-21; MS m/z 473 (M + H)⁺
   7-584; A-16; C-22; MS m/z 499 (M + H)⁺
   7-585; A-16; C-23; MS m/z 513 (M + H)⁺
   7-586; A-16; C-24; MS m/z 534 (M + H)⁺
   7-587; A-16; C-25; MS m/z 459 (M + H)⁺
   7-588; A-16; C-26; MS m/z 471 (M + H)⁺
   7-589; A-16; C-27; MS m/z 479 (M + H)⁺
   7-590; A-16; C-28; MS m/z 582 (M + H)⁺
   7-591; A-16; C-29; MS m/z 485 (M + H)⁺
   7-592; A-16; C-30; MS m/z 501 (M + H)⁺
   7-593; A-16; C-31; MS m/z 501 (M + H)⁺
   7-594; A-16; C-32; MS m/z 527 (M + H)⁺
   7-595; A-16; C-33; MS m/z 539 (M + H)⁺
   7-596; A-16; C-34; MS m/z 547 (M + H)⁺
   7-597; A-16; C-35; MS m/z 556 (M + H)⁺
   7-598; A-16; C-36; MS m/z 551 (M + H)⁺
   7-599; A-17; C-01; MS m/z 436 (M + H)⁺
   7-600; A-17; C-02; MS m/z 448 (M + H)⁺
   7-601; A-17; C-03; MS m/z 460 (M + H)⁺
   7-602; A-17; C-04; MS m/z 464 (M + H)⁺
   7-603; A-17; C-05; MS m/z 466 (M + H)⁺
   7-604; A-17; C-06; MS m/z 474 (M + H)⁺
   7-605; A-17; C-07; MS m/z 476 (M + H)⁺
   7-606; A-17; C-08; MS m/z 492 (M + H)⁺
   7-607; A-17; C-09; MS m/z 498 (M + H)⁺
   7-608; A-17; C-10; MS m/z 504 (M + H)⁺
   7-609; A-17; C-11; MS m/z 512 (M + H)⁺
   7-610; A-17; C-12; MS m/z 518 (M + H)⁺
   7-611; A-17; C-13; MS m/z 528 (M + H)⁺
   7-612; A-17; C-14; MS m/z 534 (M + H)⁺
   7-613; A-17; C-15; MS m/z 552 (M + H)⁺
   7-614; A-17; C-16; MS m/z 544 (M + H)⁺
   7-615; A-17; C-17; MS m/z 552 (M + H)⁺
   7-616; A-17; C-18; MS m/z 464 (M + H)⁺
   7-617; A-17; C-19; MS m/z 516 (M + H)⁺
   7-618; A-17; C-20; MS m/z 557 (M + H)⁺
   7-619; A-17; C-21; MS m/z 479 (M + H)⁺
   7-620; A-17; C-22; MS m/z 505 (M + H)⁺
   7-621; A-17; C-23; MS m/z 519 (M + H)⁺
   7-622; A-17; C-24; MS m/z 540 (M + H)⁺
   7-623; A-17; C-25; MS m/z 465 (M + H)⁺
   7-624; A-17; C-26; MS m/z 477 (M + H)⁺
   7-625; A-17; C-27; MS m/z 485 (M + H)⁺
   7-626; A-17; C-28; MS m/z 588 (M + H)⁺
   7-627; A-17; C-29; MS m/z 491 (M + H)⁺
   7-628; A-17; C-30; MS m/z 507 (M + H)⁺
   7-629; A-17; C-31; MS m/z 507 (M + H)⁺
   7-630; A-17; C-32; MS m/z 533 (M + H)⁺
   7-631; A-17; C-33; MS m/z 545 (M + H)⁺
   7-632; A-17; C-34; MS m/z 553 (M + H)⁺
   7-633; A-17; C-35; MS m/z 562 (M + H)⁺
   7-634; A-17; C-36; MS m/z 557 (M + H)⁺
   7-635; A-17; C-37; MS m/z 521 (M + H)⁺
   7-636; A-17; C-38; MS m/z 534 (M + H)⁺
   7-637; A-17; C-39; MS m/z 507 (M + H)⁺
   7-638; A-17; C-40; MS m/z 505 (M + H)⁺
   7-639; A-17; C-41; MS m/z 521 (M + H)⁺
   7-640; A-17; C-42; MS m/z 505 (M + H)⁺
   7-641; A-17; C-43; MS m/z 505 (M + H)⁺
   7-642; A-17; C-44; MS m/z 493 (M + H)⁺
   7-643; A-17; C-45; MS m/z 505 (M + H)⁺
   7-644; A-17; C-46; MS m/z 502 (M + H)⁺
   7-645; A-17; C-47; MS m/z 494 (M + H)⁺
   7-646; A-18; C-01; MS m/z 452 (M + H)⁺
   7-647; A-18; C-02; MS m/z 464 (M + H)⁺
   7-648; A-18; C-03; MS m/z 476 (M + H)⁺
   7-649; A-18; C-04; MS m/z 480 (M + H)⁺
   7-650; A-18; C-05; MS m/z 482 (M + H)⁺
   7-651; A-18; C-06; MS m/z 490 (M + H)⁺
   7-652; A-18; C-07; MS m/z 492 (M + H)⁺
   7-653; A-18; C-08; MS m/z 508 (M + H)⁺
   7-654; A-18; C-09; MS m/z 514 (M + H)⁺
   7-655; A-18; C-10; MS m/z 520 (M + H)⁺
   7-656; A-18; C-11; MS m/z 528 (M + H)⁺
   7-657; A-18; C-12; MS m/z 534 (M + H)⁺
   7-658; A-18; C-13; MS m/z 544 (M + H)⁺
   7-659; A-18; C-14; MS m/z 550 (M + H)⁺
   7-660; A-18; C-15; MS m/z 568 (M + H)⁺
   7-661; A-18; C-16; MS m/z 560 (M + H)⁺
   7-662; A-18; C-17; MS m/z 568 (M + H)⁺
   7-663; A-18; C-18; MS m/z 480 (M + H)⁺
   7-664; A-18; C-19; MS m/z 532 (M + H)⁺
   7-665; A-18; C-20; MS m/z 573 (M + H)⁺
   7-666; A-18; C-21; MS m/z 495 (M + H)⁺
   7-667; A-18; C-22; MS m/z 521 (M + H)⁺
   7-668; A-18; C-23; MS m/z 535 (M + H)⁺
   7-669; A-18; C-24; MS m/z 556 (M + H)⁺
   7-670; A-18; C-25; MS m/z 481 (M + H)⁺
   7-671; A-18; C-26; MS m/z 493 (M + H)⁺
   7-672; A-18; C-27; MS m/z 501 (M + H)⁺
   7-673; A-18; C-28; MS m/z 604 (M + H)⁺
   7-674; A-18; C-29; MS m/z 507 (M + H)⁺
   7-675; A-18; C-30; MS m/z 523 (M + H)⁺
   7-676; A-18; C-31; MS m/z 523 (M + H)⁺
   7-677; A-18; C-32; MS m/z 549 (M + H)⁺
   7-678; A-18; C-33; MS m/z 561 (M + H)⁺
   7-679; A-18; C-34; MS m/z 569 (M + H)⁺
   7-680; A-18; C-35; MS m/z 578 (M + H)⁺
   7-681; A-18; C-36; MS m/z 573 (M + H)⁺
   7-682; A-19; C-01; MS m/z 428 (M + H)⁺
   7-683; A-19; C-02; MS m/z 440 (M + H)⁺
   7-684; A-19; C-03; MS m/z 452 (M + H)⁺
   7-685; A-19; C-04; MS m/z 456 (M + H)⁺
   7-686; A-19; C-05; MS m/z 458 (M + H)⁺
   7-687; A-19; C-06; MS m/z 466 (M + H)⁺
   7-688; A-19; C-07; MS m/z 468 (M + H)⁺
   7-689; A-19; C-08; MS m/z 484 (M + H)⁺
   7-690; A-19; C-09; MS m/z 490 (M + H)⁺
   7-691; A-19; C-10; MS m/z 496 (M + H)⁺
   7-692; A-19; C-11; MS m/z 504 (M + H)⁺
   7-693; A-19; C-12; MS m/z 510 (M + H)⁺
   7-694; A-19; C-13; MS m/z 520 (M + H)⁺
   7-695; A-19; C-14; MS m/z 526 (M + H)⁺
   7-696; A-19; C-15; MS m/z 544 (M + H)⁺
   7-697; A-19; C-16; MS m/z 536 (M + H)⁺
   7-698; A-19; C-17; MS m/z 544 (M + H)⁺
   7-699; A-19; C-18; MS m/z 456 (M + H)⁺
   7-700; A-19; C-19; MS m/z 508 (M + H)⁺
   7-701; A-19; C-20; MS m/z 549 (M + H)⁺
   7-702; A-19; C-21; MS m/z 471 (M + H)⁺
   7-703; A-19; C-22; MS m/z 497 (M + H)⁺
   7-704; A-19; C-23; MS m/z 511 (M + H)⁺
   7-705; A-19; C-24; MS m/z 532 (M + H)⁺
   7-706; A-19; C-25; MS m/z 457 (M + H)⁺
   7-707; A-19; C-26; MS m/z 469 (M + H)⁺
   7-708; A-19; C-27; MS m/z 477 (M + H)⁺
   7-709; A-19; C-28; MS m/z 580 (M + H)⁺
   7-710; A-19; C-29; MS m/z 483 (M + H)
   7-711; A-19; C-30; MS m/z 499 (M + H)⁺
   7-712; A-19; C-31; MS m/z 499 (M + H)⁺
   7-713; A-19; C-32; MS m/z 525 (M + H)⁺
   7-714; A-19; C-33; MS m/z 537 (M + H)⁺
   7-715; A-19; C-34; MS m/z 545 (M + H)⁺
   7-716; A-19; C-35; MS m/z 554 (M + H)⁺
   7-717; A-19; C-36; MS m/z 549 (M + H)⁺
   7-718; A-20; C-01; MS m/z 432 (M + H)⁺
   7-719; A-20; C-02; MS m/z 444 (M + H)⁺
   7-720; A-20; C-03; MS m/z 456 (M + H)⁺
   7-721; A-20; C-04; MS m/z 460 (M + H)⁺
   7-722; A-20; C-05; MS m/z 462 (M + H)⁺
   7-723; A-20; C-06; MS m/z 470 (M + H)⁺
   7-724; A-20; C-07; MS m/z 472 (M + H)⁺
   7-725; A-20; C-08; MS m/z 488 (M + H)⁺
   7-726; A-20; C-09; MS m/z 494 (M + H)⁺
   7-727; A-20; C-10; MS m/z 500 (M + H)⁺
   7-728; A-20; C-11; MS m/z 508 (M + H)⁺
   7-729; A-20; C-12; MS m/z 514 (M + H)⁺
   7-730; A-20; C-13; MS m/z 524 (M + H)⁺
   7-731; A-20; C-14; MS m/z 530 (M + H)⁺
   7-732; A-20; C-15; MS m/z 548 (M + H)⁺
   7-733; A-20; C-16; MS m/z 540 (M + H)⁺
   7-734; A-20; C-17; MS m/z 548 (M + H)⁺
   7-735; A-20; C-18; MS m/z 460 (M + H)⁺
   7-736; A-20; C-19; MS m/z 512 (M + H)⁺
   7-737; A-20; C-20; MS m/z 553 (M + H)⁺
   7-738; A-20; C-21; MS m/z 475 (M + H)⁺
   7-739; A-20; C-22; MS m/z 501 (M + H)⁺
   7-740; A-20; C-23; MS m/z 515 (M + H)⁺
   7-741; A-20; C-24; MS m/z 536 (M + H)⁺
   7-742; A-20; C-25; MS m/z 461 (M + H)⁺
   7-743; A-20; C-26; MS m/z 473 (M + H)⁺
   7-744; A-20; C-27; MS m/z 481 (M + H)⁺
   7-745; A-20; C-28; MS m/z 584 (M + H)⁺
   7-746; A-20; C-29; MS m/z 487 (M + H)⁺
   7-747; A-20; C-30; MS m/z 503 (M + H)⁺
   7-748; A-20; C-31; MS m/z 503 (M + H)⁺
   7-749; A-20; C-32; MS m/z 529 (M + H)⁺
   7-750; A-20; C-33; MS m/z 541 (M + H)⁺
   7-751; A-20; C-34; MS m/z 549 (M + H)⁺
   7-752; A-20; C-35; MS m/z 558 (M + H)⁺
   7-753; A-20; C-36; MS m/z 553 (M + H)⁺
   7-754; A-21; C-01; MS m/z 438 (M + H)⁺
   7-755; A-21; C-02; MS m/z 450 (M + H)⁺
   7-756; A-21; C-03; MS m/z 462 (M + H)⁺
   7-757; A-21; C-04; MS m/z 466 (M + H)⁺
   7-758; A-21; C-05; MS m/z 468 (M + H)⁺
   7-759; A-21; C-06; MS m/z 476 (M + H)⁺
   7-760; A-21; C-07; MS m/z 478 (M + H)⁺
   7-761; A-21; C-08; MS m/z 494 (M + H)⁺
   7-762; A-21; C-09; MS m/z 500 (M + H)⁺
   7-763; A-21; C-10; MS m/z 506 (M + H)⁺
   7-764; A-21; C-11; MS m/z 514 (M + H)⁺
   7-765; A-21; C-12; MS m/z 520 (M + H)⁺
   7-766; A-21; C-13; MS m/z 530 (M + H)⁺
   7-767; A-21; C-14; MS m/z 536 (M + H)⁺
   7-768; A-21; C-15; MS m/z 554 (M + H)⁺
   7-769; A-21; C-16; MS m/z 546 (M + H)⁺
   7-770; A-21; C-17; MS m/z 554 (M + H)⁺
   7-771; A-21; C-18; MS m/z 466 (M + H)⁺
   7-772; A-21; C-19; MS m/z 518 (M + H)⁺
   7-773; A-21; C-20; MS m/z 559 (M + H)⁺
   7-774; A-21; C-21; MS m/z 481 (M + H)⁺
   7-775; A-21; C-22; MS m/z 507 (M + H)⁺
   7-776; A-21; C-23; MS m/z 521 (M + H)⁺
   7-777; A-21; C-24; MS m/z 542 (M + H)⁺
   7-778; A-21; C-25; MS m/z 467 (M + H)⁺
   7-779; A-21; C-26; MS m/z 479 (M + H)⁺
   7-780; A-21; C-27; MS m/z 487 (M + H)⁺
   7-781; A-21; C-28; MS m/z 590 (M + H)⁺
   7-782; A-21; C-29; MS m/z 493 (M + H)⁺
   7-783; A-21; C-30; MS m/z 509 (M + H)⁺
   7-784; A-21; C-31; MS m/z 509 (M + H)⁺
   7-785; A-21; C-32; MS m/z 535 (M + H)⁺
   7-786; A-21; C-33; MS m/z 547 (M + H)⁺
   7-787; A-21; C-34; MS m/z 555 (M + H)⁺
   7-788; A-21; C-35; MS m/z 564 (M + H)⁺
   7-789; A-21; C-36; MS m/z 559 (M + H)⁺
   7-790; A-22; C-01; MS m/z 462 (M + H)⁺
   7-791; A-22; C-02; MS m/z 474 (M + H)⁺
   7-792; A-22; C-03; MS m/z 486 (M + H)⁺
   7-793; A-22; C-04; MS m/z 490 (M + H)⁺
   7-794; A-22; C-05; MS m/z 492 (M + H)⁺
   7-795; A-22; C-06; MS m/z 500 (M + H)⁺
   7-796; A-22; C-07; MS m/z 502 (M + H)⁺
   7-797; A-22; C-08; MS m/z 518 (M + H)⁺
   7-798; A-22; C-09; MS m/z 524 (M + H)⁺
   7-799; A-22; C-10; MS m/z 530 (M + H)⁺
   7-800; A-22; C-11; MS m/z 538 (M + H)⁺
   7-801; A-22; C-12; MS m/z 544 (M + H)⁺
   7-802; A-22; C-13; MS m/z 554 (M + H)⁺
   7-803; A-22; C-14; MS m/z 560 (M + H)⁺
   7-804; A-22; C-15; MS m/z 578 (M + H)⁺
   7-805; A-22; C-16; MS m/z 570 (M + H)⁺
   7-806; A-22; C-17; MS m/z 578 (M + H)⁺
   7-807; A-22; C-18; MS m/z 490 (M + H)⁺
   7-808; A-22; C-19; MS m/z 542 (M + H)⁺
   7-809; A-22; C-20; MS m/z 583 (M + H)⁺
   7-810; A-22; C-21; MS m/z 505 (M + H)⁺
   7-811; A-22; C-22; MS m/z 531 (M + H)⁺
   7-812; A-22; C-23; MS m/z 545 (M + H)⁺
   7-813; A-22; C-24; MS m/z 566 (M + H)⁺
   7-814; A-22; C-25; MS m/z 491 (M + H)⁺
   7-815; A-22; C-26; MS m/z 503 (M + H)⁺
   7-816; A-22; C-27; MS m/z 511 (M + H)⁺
   7-817; A-22; C-28; MS m/z 614 (M + H)⁺
   7-818; A-22; C-29; MS m/z 517 (M + H)⁺
   7-819; A-22; C-30; MS m/z 533 (M + H)⁺
   7-820; A-22; C-31; MS m/z 533 (M + H)⁺
   7-821; A-22; C-32; MS m/z 559 (M + H)⁺
   7-822; A-22; C-33; MS m/z 571 (M + H)⁺
   7-823; A-22; C-34; MS m/z 579 (M + H)⁺
   7-824; A-22; C-35; MS m/z 588 (M + H)⁺
   7-825; A-22; C-36; MS m/z 583 (M + H)⁺
   7-826; A-23; C-01; MS m/z 466 (M + H)⁺
   7-827; A-23; C-02; MS m/z 478 (M + H)⁺
   7-828; A-23; C-03; MS m/z 490 (M + H)⁺
   7-829; A-23; C-04; MS m/z 494 (M + H)⁺
   7-830; A-23; C-05; MS m/z 496 (M + H)⁺
   7-831; A-23; C-06; MS m/z 504 (M + H)⁺
   7-832; A-23; C-07; MS m/z 506 (M + H)⁺
   7-833; A-23; C-08; MS m/z 522 (M + H)⁺
   7-834; A-23; C-09; MS m/z 528 (M + H)⁺
   7-835; A-23; C-10; MS m/z 534 (M + H)⁺
   7-836; A-23; C-11; MS m/z 542 (M + H)⁺
   7-837; A-23; C-12; MS m/z 548 (M + H)⁺
   7-838; A-23; C-13; MS m/z 558 (M + H)⁺
   7-839; A-23; C-14; MS m/z 564 (M + H)⁺
   7-840; A-23; C-15; MS m/z 582 (M + H)⁺
   7-841; A-23; C-16; MS m/z 574 (M + H)⁺
   7-842; A-23; C-17; MS m/z 582 (M + H)⁺
   7-843; A-23; C-18; MS m/z 494 (M + H)⁺
   7-844; A-23; C-19; MS m/z 546 (M + H)⁺
   7-845; A-23; C-20; MS m/z 587 (M + H)⁺
   7-846; A-23; C-21; MS m/z 509 (M + H)⁺
   7-847; A-23; C-22; MS m/z 535 (M + H)⁺
   7-848; A-23; C-23; MS m/z 549 (M + H)⁺
   7-849; A-23; C-24; MS m/z 570 (M + H)⁺
   7-850; A-23; C-25; MS m/z 495 (M + H)⁺
   7-851; A-23; C-26; MS m/z 507 (M + H)⁺
   7-852; A-23; C-27; MS m/z 515 (M + H)⁺
   7-853; A-23; C-28; MS m/z 618 (M + H)⁺
   7-854; A-23; C-29; MS m/z 521 (M + H)⁺
   7-855; A-23; C-30; MS m/z 537 (M + H)⁺
   7-856; A-23; C-31; MS m/z 537 (M + H)⁺
   7-857; A-23; C-32; MS m/z 563 (M + H)⁺
   7-858; A-23; C-33; MS m/z 575 (M + H)⁺
   7-859; A-23; C-34; MS m/z 583 (M + H)⁺
   7-860; A-23; C-35; MS m/z 592 (M + H)⁺
   7-861; A-23; C-36; MS m/z 587 (M + H)⁺
   7-862; A-24; C-01; MS m/z 470 (M + H)⁺
   7-863; A-24; C-02; MS m/z 482 (M + H)⁺
   7-864; A-24; C-03; MS m/z 494 (M + H)⁺
   7-865; A-24; C-04; MS m/z 498 (M + H)⁺
   7-866; A-24; C-05; MS m/z 500 (M + H)⁺
   7-867; A-24; C-06; MS m/z 508 (M + H)⁺
   7-868; A-24; C-07; MS m/z 510 (M + H)⁺
   7-869; A-24; C-08; MS m/z 526 (M + H)⁺
   7-870; A-24; C-09; MS m/z 532 (M + H)⁺
   7-871; A-24; C-10; MS m/z 538 (M + H)⁺
   7-872; A-24; C-11; MS m/z 546 (M + H)⁺
   7-873; A-24; C-12; MS m/z 552 (M + H)⁺
   7-874; A-24; C-13; MS m/z 562 (M + H)⁺
   7-875; A-24; C-14; MS m/z 568 (M + H)⁺
   7-876; A-24; C-15; MS m/z 586 (M + H)⁺
   7-877; A-24; C-16; MS m/z 578 (M + H)⁺
   7-878; A-24; C-17; MS m/z 586 (M + H)⁺
   7-879; A-24; C-18; MS m/z 498 (M + H)⁺
   7-880; A-24; C-19; MS m/z 550 (M + H)⁺
   7-881; A-24; C-20; MS m/z 591 (M + H)⁺
   7-882; A-24; C-21; MS m/z 513 (M + H)⁺
   7-883; A-24; C-22; MS m/z 539 (M + H)⁺
   7-884; A-24; C-23; MS m/z 553 (M + H)⁺
   7-885; A-24; C-24; MS m/z 574 (M + H)⁺
   7-886; A-24; C-25; MS m/z 499 (M + H)⁺
   7-887; A-24; C-26; MS m/z 511 (M + H)⁺
   7-888; A-24; C-27; MS m/z 519 (M + H)⁺
   7-889; A-24; C-28; MS m/z 622 (M + H)⁺
   7-890; A-24; C-29; MS m/z 525 (M + H)⁺
   7-891; A-24; C-30; MS m/z 541 (M + H)⁺
   7-892; A-24; C-31; MS m/z 541 (M + H)⁺
   7-893; A-24; C-32; MS m/z 567 (M + H)⁺
   7-894; A-24; C-33; MS m/z 579 (M + H)⁺
   7-895; A-24; C-34; MS m/z 587 (M + H)⁺
   7-896; A-24; C-35; MS m/z 596 (M + H)⁺
   7-897; A-24; C-36; MS m/z 591 (M + H)⁺
   7-898; A-25; C-01; MS m/z 397 (M + H)⁺
   7-899; A-25; C-02; MS m/z 409 (M + H)⁺
   7-900; A-25; C-03; MS m/z 421 (M + H)⁺
   7-901; A-25; C-04; MS m/z 425 (M + H)⁺
   7-902; A-25; C-05; MS m/z 427 (M + H)⁺
   7-903; A-25; C-06; MS m/z 435 (M + H)⁺
   7-904; A-25; C-07; MS m/z 437 (M + H)⁺
   7-905; A-25; C-08; MS m/z 453 (M + H)⁺
   7-906; A-25; C-09; MS m/z 459 (M + H)⁺
   7-907; A-25; C-10; MS m/z 465 (M + H)⁺
   7-908; A-25; C-11; MS m/z 473 (M + H)⁺
   7-909; A-25; C-12; MS m/z 479 (M + H)⁺
   7-910; A-25; C-13; MS m/z 489 (M + H)⁺
   7-911; A-25; C-14; MS m/z 495 (M + H)⁺
   7-912; A-25; C-15; MS m/z 513 (M + H)⁺
   7-913; A-25; C-16; MS m/z 505 (M + H)⁺
   7-914; A-25; C-17; MS m/z 513 (M + H)⁺
   7-915; A-25; C-18; MS m/z 425 (M + H)⁺
   7-916; A-25; C-19; MS m/z 477 (M + H)⁺
   7-917; A-25; C-20; MS m/z 518 (M + H)⁺
   7-918; A-25; C-21; MS m/z 440 (M + H)⁺
   7-919; A-25; C-22; MS m/z 466 (M + H)⁺
   7-920; A-25; C-23; MS m/z 480 (M + H)⁺
   7-921; A-25; C-24; MS m/z 501 (M + H)⁺
   7-922; A-25; C-25; MS m/z 426 (M + H)⁺
   7-923; A-25; C-26; MS m/z 438 (M + H)⁺
   7-924; A-25; C-27; MS m/z 446 (M + H)⁺
   7-925; A-25; C-28; MS m/z 549 (M + H)⁺
   7-926; A-25; C-29; MS m/z 452 (M + H)⁺
   7-927; A-25; C-30; MS m/z 468 (M + H)⁺
   7-928; A-25; C-31; MS m/z 468 (M + H)⁺
   7-929; A-25; C-32; MS m/z 494 (M + H)⁺
   7-930; A-25; C-33; MS m/z 506 (M + H)⁺
   7-931; A-25; C-34; MS m/z 514 (M + H)⁺
   7-932; A-25; C-35; MS m/z 523 (M + H)⁺
   7-933; A-25; C-36; MS m/z 518 (M + H)⁺
   7-934; A-27; C-01; MS m/z 437 (M + H)⁺
   7-935; A-27; C-02; MS m/z 449 (M + H)⁺
   7-936; A-27; C-03; MS m/z 461 (M + H)⁺
   7-937; A-27; C-04; MS m/z 465 (M + H)⁺
   7-938; A-27; C-05; MS m/z 467 (M + H)⁺
   7-939; A-27; C-06; MS m/z 475 (M + H)⁺
   7-940; A-27; C-07; MS m/z 477 (M + H)⁺
   7-941; A-27; C-08; MS m/z 493 (M + H)⁺
   7-942; A-27; C-09; MS m/z 499 (M + H)⁺
   7-943; A-27; C-10; MS m/z 505 (M + H)⁺
   7-944; A-27; C-11; MS m/z 513 (M + H)⁺
   7-945; A-27; C-12; MS m/z 519 (M + H)⁺
   7-946; A-27; C-13; MS m/z 529 (M + H)⁺
   7-947; A-27; C-14; MS m/z 535 (M + H)⁺
   7-948; A-27; C-15; MS m/z 553 (M + H)⁺
   7-949; A-27; C-16; MS m/z 545 (M + H)⁺
   7-950; A-27; C-17; MS m/z 553 (M + H)⁺
   7-951; A-27; C-18; MS m/z 465 (M + H)⁺
   7-952; A-27; C-19; MS m/z 517 (M + H)⁺
   7-953; A-27; C-20; MS m/z 558 (M + H)⁺
   7-954; A-27; C-21; MS m/z 480 (M + H)⁺
   7-955; A-27; C-22; MS m/z 506 (M + H)⁺
   7-956; A-27; C-23; MS m/z 520 (M + H)⁺
   7-957; A-27; C-24; MS m/z 541 (M + H)⁺
   7-958; A-27; C-25; MS m/z 466 (M + H)⁺
   7-959; A-27; C-26; MS m/z 478 (M + H)⁺
   7-960; A-27; C-27; MS m/z 486 (M + H)⁺
   7-961; A-27; C-28; MS m/z 589 (M + H)⁺
   7-962; A-27; C-29; MS m/z 492 (M + H)⁺
   7-963; A-27; C-30; MS m/z 508 (M + H)⁺
   7-964; A-27; C-31; MS m/z 508 (M + H)⁺
   7-965; A-27; C-32; MS m/z 534 (M + H)⁺
   7-966; A-27; C-33; MS m/z 546 (M + H)⁺
   7-967; A-27; C-34; MS m/z 554 (M + H)⁺
   7-968; A-27; C-35; MS m/z 563 (M + H)⁺
   7-969; A-27; C-36; MS m/z 558 (M + H)⁺
   7-970; A-28; C-01; MS m/z 467 (M + H)⁺
   7-971; A-28; C-02; MS m/z 479 (M + H)⁺
   7-972; A-28; C-03; MS m/z 491 (M + H)⁺
   7-973; A-28; C-04; MS m/z 495 (M + H)⁺
   7-974; A-28; C-05; MS m/z 497 (M + H)⁺
   7-975; A-28; C-06; MS m/z 505 (M + H)⁺
   7-976; A-28; C-07; MS m/z 507 (M + H)⁺
   7-977; A-28; C-08; MS m/z 523 (M + H)⁺
   7-978; A-28; C-09; MS m/z 529 (M + H)⁺
   7-979; A-28; C-10; MS m/z 535 (M + H)⁺
   7-980; A-28; C-11; MS m/z 543 (M + H)⁺
   7-981; A-28; C-12; MS m/z 549 (M + H)⁺
   7-982; A-28; C-13; MS m/z 559 (M + H)⁺
   7-983; A-28; C-14; MS m/z 565 (M + H)⁺
   7-984; A-28; C-15; MS m/z 583 (M + H)⁺
   7-985; A-28; C-16; MS m/z 575 (M + H)⁺
   7-986; A-28; C-17; MS m/z 583 (M + H)⁺
   7-987; A-28; C-18; MS m/z 495 (M + H)⁺
   7-988; A-28; C-19; MS m/z 547 (M + H)⁺
   7-989; A-28; C-20; MS m/z 588 (M + H)⁺
   7-990; A-28; C-21; MS m/z 510 (M + H)⁺
   7-991; A-28; C-22; MS m/z 536 (M + H)⁺
   7-992; A-28; C-23; MS m/z 550 (M + H)⁺
   7-993; A-28; C-24; MS m/z 571 (M + H)⁺
   7-994; A-28; C-25; MS m/z 496 (M + H)⁺
   7-995; A-28; C-26; MS m/z 508 (M + H)⁺
   7-996; A-28; C-27; MS m/z 516 (M + H)⁺
   7-997; A-28; C-28; MS m/z 619 (M + H)⁺
   7-998; A-28; C-29; MS m/z 522 (M + H)⁺
   7-999; A-28; C-30; MS m/z 538 (M + H)⁺
   7-1000; A-28; C-31; MS m/z 538 (M + H)⁺
   7-1001; A-28; C-32; MS m/z 564 (M + H)⁺
   7-1002; A-28; C-33; MS m/z 576 (M + H)⁺
   7-1003; A-28; C-34; MS m/z 584 (M + H)⁺
   7-1004; A-28; C-35; MS m/z 593 (M + H)⁺
   7-1005; A-28; C-36; MS m/z 588 (M + H)⁺
   7-1006; A-33; C-01; MS m/z 366 (M + H)⁺
   7-1007; A-33; C-02; MS m/z 378 (M + H)⁺
   7-1008; A-33; C-03; MS m/z 390 (M + H)⁺
   7-1009; A-33; C-04; MS m/z 394 (M + H)⁺
   7-1010; A-33; C-05; MS m/z 396 (M + H)⁺
   7-1011; A-33; C-06; MS m/z 404 (M + H)⁺
   7-1012; A-33; C-07; MS m/z 406 (M + H)⁺
   7-1013; A-33; C-08; MS m/z 422 (M + H)⁺
   7-1014; A-33; C-09; MS m/z 428 (M + H)⁺
   7-1015; A-33; C-10; MS m/z 434 (M + H)⁺
   7-1016; A-33; C-11; MS m/z 442 (M + H)⁺
   7-1017; A-33; C-12; MS m/z 448 (M + H)⁺
   7-1018; A-33; C-13; MS m/z 458 (M + H)⁺
   7-1019; A-33; C-14; MS m/z 464 (M + H)⁺
   7-1020; A-33; C-15; MS m/z 482 (M + H)⁺
   7-1021; A-33; C-16; MS m/z 474 (M + H)⁺
   7-1022; A-33; C-17; MS m/z 482 (M + H)⁺
   7-1023; A-33; C-18; MS m/z 394 (M + H)⁺
   7-1024; A-33; C-19; MS m/z 446 (M + H)⁺
   7-1025; A-33; C-20; MS m/z 487 (M + H)⁺
   7-1026; A-33; C-21; MS m/z 409 (M + H)⁺
   7-1027; A-33; C-22; MS m/z 435 (M + H)⁺
   7-1028; A-33; C-23; MS m/z 449 (M + H)⁺
   7-1029; A-33; C-24; MS m/z 470 (M + H)⁺
   7-1030; A-33; C-25; MS m/z 395 (M + H)⁺
   7-1031; A-33; C-26; MS m/z 407 (M + H)⁺
   7-1032; A-33; C-27; MS m/z 415 (M + H)⁺
   7-1033; A-33; C-28; MS m/z 518 (M + H)⁺
   7-1034; A-33; C-29; MS m/z 421 (M + H)⁺
   7-1035; A-33; C-30; MS m/z 437 (M + H)⁺
   7-1036; A-33; C-31; MS m/z 437 (M + H)⁺
   7-1037; A-33; C-32; MS m/z 463 (M + H)⁺
   7-1038; A-33; C-33; MS m/z 475 (M + H)⁺
   7-1039; A-33; C-34; MS m/z 483 (M + H)⁺
   7-1040; A-33; C-35; MS m/z 492 (M + H)⁺
   7-1041; A-33; C-36; MS m/z 487 (M + H)⁺
   7-1042; A-34; C-01; MS m/z 379 (M + H)⁺
   7-1043; A-34; C-02; MS m/z 391 (M + H)⁺
   7-1044; A-34; C-03; MS m/z 403 (M + H)⁺
   7-1045; A-34; C-04; MS m/z 407 (M + H)⁺
   7-1046; A-34; C-05; MS m/z 409 (M + H)⁺
   7-1047; A-34; C-06; MS m/z 417 (M + H)⁺
   7-1048; A-34; C-07; MS m/z 419 (M + H)⁺
   7-1049; A-34; C-08; MS m/z 435 (M + H)⁺
   7-1050; A-34; C-09; MS m/z 441 (M + H)⁺
   7-1051; A-34; C-10: MS m/z 447 (M + H)⁺
   7-1052; A-34; C-11; MS m/z 455 (M + H)⁺
   7-1053; A-34; C-12; MS m/z 461 (M + H)⁺
   7-1054; A-34; C-13; MS m/z 471 (M + H)⁺
   7-1055; A-34; C-14; MS m/z 477 (M + H)⁺
   7-1056; A-34; C-15; MS m/z 495 (M + H)⁺
   7-1057; A-34; C-16; MS m/z 487 (M + H)⁺
   7-1058; A-34; C-17; MS m/z 495 (M + H)⁺
   7-1059; A-34; C-18; MS m/z 407 (M + H)⁺
   7-1060; A-34; C-19; MS m/z 459 (M + H)⁺
   7-1061; A-34; C-20; MS m/z 500 (M + H)⁺
   7-1062; A-34; C-21; MS m/z 422 (M + H)⁺
   7-1063; A-34; C-22; MS m/z 448 (M + H)⁺
   7-1064; A-34; C-23; MS m/z 462 (M + H)⁺
   7-1065; A-34; C-24; MS m/z 483 (M + H)⁺
   7-1066; A-34; C-25; MS m/z 408 (M + H)⁺
   7-1067; A-34; C-26; MS m/z 420 (M + H)⁺
   7-1068; A-34; C-27; MS m/z 428 (M + H)⁺
   7-1069; A-34; C-28; MS m/z 531 (M + H)⁺
   7-1070; A-34; C-29; MS m/z 434 (M + H)⁺
   7-1071; A-34; C-30; MS m/z 450 (M + H)⁺
   7-1072; A-34; C-31; MS m/z 450 (M + H)⁺
   7-1073; A-34; C-32; MS m/z 476 (M + H)⁺
   7-1074; A-34; C-33; MS m/z 488 (M + H)⁺
   7-1075; A-34; C-34; MS m/z 496 (M + H)⁺
   7-1076; A-34; C-35; MS m/z 505 (M + H)⁺
   7-1077; A-34; C-36; MS m/z 500 (M + H)⁺
   7-1078; A-35; C-01; MS m/z 394 (M + H)⁺
   7-1079; A-35; C-02; MS m/z 406 (M + H)⁺
   7-1080; A-35; C-03; MS m/z 418 (M + H)⁺
   7-1081; A-35; C-04; MS m/z 422 (M + H)⁺
   7-1082; A-35; C-05; MS m/z 424 (M + H)⁺
   7-1083; A-35; C-06; MS m/z 432 (M + H)⁺
   7-1084; A-35; C-07; MS m/z 434 (M + H)⁺
   7-1085; A-35; C-08; MS m/z 450 (M + H)⁺
   7-1086; A-35; C-09; MS m/z 456 (M + H)⁺
   7-1087; A-35; C-10; MS m/z 462 (M + H)⁺
   7-1088; A-35; C-11; MS m/z 470 (M + H)⁺
   7-1089; A-35; C-12; MS m/z 476 (M + H)⁺
   7-1090; A-35; C-13; MS m/z 486 (M + H)⁺
   7-1091; A-35; C-14; MS m/z 492 (M + H)⁺
   7-1092; A-35; C-15; MS m/z 510 (M + H)⁺
   7-1093; A-35; C-16; MS m/z 502 (M + H)⁺
   7-1094; A-35; C-17; MS m/z 510 (M + H)⁺
   7-1095; A-35; C-18; MS m/z 422 (M + H)⁺
   7-1096; A-35; C-19; MS m/z 474 (M + H)⁺
   7-1097; A-35; C-20; MS m/z 515 (M + H)⁺
   7-1098; A-35; C-21; MS m/z 437 (M + H)⁺
   7-1099; A-35; C-22; MS m/z 463 (M + H)⁺
   7-1100; A-35; C-23; MS m/z 477 (M + H)⁺
   7-1101; A-35; C-24; MS m/z 498 (M + H)⁺
   7-1102; A-35; C-25; MS m/z 423 (M + H)⁺
   7-1103; A-35; C-26; MS m/z 435 (M + H)⁺
   7-1104; A-35; C-27; MS m/z 443 (M + H)⁺
   7-1105; A-35; C-28; MS m/z 546 (M + H)⁺
   7-1106; A-35; C-29; MS m/z 449 (M + H)⁺
   7-1107; A-35; C-30; MS m/z 465 (M + H)⁺
   7-1108; A-35; C-31; MS m/z 465 (M + H)⁺
   7-1109; A-35; C-32; MS m/z 491 (M + H)⁺
   7-1110; A-35; C-33; MS m/z 503 (M + H)⁺
   7-1111; A-35; C-34; MS m/z 511 (M + H)⁺
   7-1112; A-35; C-35; MS m/z 520 (M + H)⁺
   7-1113; A-35; C-36; MS m/z 515 (M + H)⁺
   7-1114; A-36; C-01; MS m/z 408 (M + H)⁺
   7-1115; A-36; C-02; MS m/z 420 (M + H)⁺
   7-1116; A-36; C-03; MS m/z 432 (M + H)⁺
   7-1117; A-36; C-04; MS m/z 436 (M + H)⁺
   7-1118; A-36; C-05; MS m/z 438 (M + H)⁺
   7-1119; A-36; C-06; MS m/z 446 (M + H)⁺
   7-1120; A-36; C-07; MS m/z 448 (M + H)⁺
   7-1121; A-36; C-08; MS m/z 464 (M + H)⁺
   7-1122; A-36; C-09; MS m/z 470 (M + H)⁺
   7-1123; A-36; C-10; MS m/z 476 (M + H)⁺
   7-1124; A-36; C-11; MS m/z 484 (M + H)⁺
   7-1125; A-36; C-12; MS m/z 490 (M + H)⁺
   7-1126; A-36; C-13; MS m/z 500 (M + H)⁺
   7-1127; A-36; C-14; MS m/z 506 (M + H)⁺
   7-1128; A-36; C-15; MS m/z 524 (M + H)⁺
   7-1129; A-36; C-16; MS m/z 516 (M + H)⁺
   7-1130; A-36; C-17; MS m/z 524 (M + H)⁺
   7-1131; A-36; C-18; MS m/z 436 (M + H)⁺
   7-1132; A-36; C-19; MS m/z 488 (M + H)⁺
   7-1133; A-36; C-20; MS m/z 529 (M + H)⁺
   7-1134; A-36; C-21; MS m/z 451 (M + H)⁺
   7-1135; A-36; C-22; MS m/z 477 (M + H)⁺
   7-1136; A-36; C-23; MS m/z 491 (M + H)⁺
   7-1137; A-36; C-24; MS m/z 512 (M + H)⁺
   7-1138; A-36; C-25; MS m/z 437 (M + H)⁺
   7-1139; A-36; C-26; MS m/z 449 (M + H)⁺
   7-1140; A-36; C-27; MS m/z 457 (M + H)⁺
   7-1141; A-36; C-28; MS m/z 560 (M + H)⁺
   7-1142; A-36; C-29; MS m/z 463 (M + H)⁺
   7-1143; A-36; C-30; MS m/z 479 (M + H)⁺
   7-1144; A-36; C-31; MS m/z 479 (M + H)⁺
   7-1145; A-36; C-32; MS m/z 505 (M + H)⁺
   7-1146; A-36; C-33; MS m/z 517 (M + H)⁺
   7-1147; A-36; C-34; MS m/z 525 (M + H)⁺
   7-1148; A-36; C-35; MS m/z 534 (M + H)⁺
   7-1149; A-36; C-36; MS m/z 529 (M + H)⁺
   7-1150; A-37; C-01; MS m/z 458 (M + H)⁺
   7-1151; A-37; C-02; MS m/z 470 (M + H)⁺
   7-1152; A-37; C-03; MS m/z 482 (M + H)⁺
   7-1153; A-37; C-04; MS m/z 486 (M + H)⁺
   7-1154; A-37; C-05; MS m/z 488 (M + H)⁺
   7-1155; A-37; C-06; MS m/z 496 (M + H)⁺
   7-1156; A-37; C-07; MS m/z 498 (M + H)⁺
   7-1157; A-37; C-08; MS m/z 514 (M + H)⁺
   7-1158; A-37; C-09; MS m/z 520 (M + H)⁺
   7-1159; A-37; C-10; MS m/z 526 (M + H)⁺
   7-1160; A-37; C-11; MS m/z 534 (M + H)⁺
   7-1161; A-37; C-12; MS m/z 540 (M + H)⁺
   7-1162; A-37; C-13; MS m/z 550 (M + H)⁺
   7-1163; A-37; C-14; MS m/z 556 (M + H)⁺
   7-1164; A-37; C-15; MS m/z 574 (M + H)⁺
   7-1165; A-37; C-16; MS m/z 566 (M + H)⁺
   7-1166; A-37; C-17; MS m/z 574 (M + H)⁺
   7-1167; A-37; C-18; MS m/z 486 (M + H)⁺
   7-1168; A-37; C-19; MS m/z 538 (M + H)⁺
   7-1169; A-37; C-20; MS m/z 579 (M + H)⁺
   7-1170; A-37; C-21; MS m/z 501 (M + H)⁺
   7-1171; A-37; C-22; MS m/z 527 (M + H)⁺
   7-1172; A-37; C-23; MS m/z 541 (M + H)⁺
   7-1173; A-37; C-24; MS m/z 562 (M + H)⁺
   7-1174; A-37; C-25; MS m/z 487 (M + H)⁺
   7-1175; A-37; C-26; MS m/z 499 (M + H)⁺
   7-1176; A-37; C-27; MS m/z 507 (M + H)⁺
   7-1177; A-37; C-28; MS m/z 610 (M + H)⁺
   7-1178; A-37; C-29; MS m/z 513 (M + H)⁺
   7-1179; A-37; C-30; MS m/z 529 (M + H)⁺
   7-1180; A-37; C-31; MS m/z 529 (M + H)⁺
   7-1181; A-37; C-32; MS m/z 555 (M + H)⁺
   7-1182; A-37; C-33; MS m/z 567 (M + H)⁺
   7-1183; A-37; C-34; MS m/z 575 (M + H)⁺
   7-1184; A-37; C-35; MS m/z 584 (M + H)⁺
   7-1185; A-37; C-36; MS m/z 579 (M + H)⁺
   7-1186; A-40; C-01; MS m/z 423 (M + H)⁺
   7-1187; A-40; C-02; MS m/z 435 (M + H)⁺
   7-1188; A-40; C-03; MS m/z 447 (M + H)⁺
   7-1189; A-40; C-04; MS m/z 451 (M + H)⁺
   7-1190; A-40; C-05; MS m/z 453 (M + H)⁺
   7-1191; A-40; C-06; MS m/z 461 (M + H)⁺
   7-1192; A-40; C-07; MS m/z 463 (M + H)⁺
   7-1193; A-40; C-08; MS m/z 479 (M + H)⁺
   7-1194; A-40; C-09; MS m/z 485 (M + H)⁺
   7-1195; A-40; C-10; MS m/z 491 (M + H)⁺
   7-1196; A-40; C-11; MS m/z 499 (M + H)⁺
   7-1197; A-40; C-12; MS m/z 505 (M + H)⁺
   7-1198; A-40; C-13; MS m/z 515 (M + H)⁺
   7-1199; A-40; C-14; MS m/z 521 (M + H)⁺
   7-1200; A-40; C-15; MS m/z 539 (M + H)⁺
   7-1201; A-40; C-16; MS m/z 531 (M + H)⁺
   7-1202; A-40; C-17; MS m/z 539 (M + H)⁺
   7-1203; A-40; C-18; MS m/z 451 (M + H)⁺
   7-1204; A-40; C-19; MS m/z 503 (M + H)⁺
   7-1205; A-40; C-20; MS m/z 544 (M + H)⁺
   7-1206; A-40; C-21; MS m/z 466 (M + H)⁺
   7-1207; A-40; C-22; MS m/z 492 (M + H)⁺
   7-1208; A-40; C-23; MS m/z 506 (M + H)⁺
   7-1209; A-40; C-24; MS m/z 527 (M + H)⁺
   7-1210; A-40; C-25; MS m/z 452 (M + H)⁺
   7-1211; A-40; C-26; MS m/z 464 (M + H)⁺
   7-1212; A-40; C-27; MS m/z 472 (M + H)⁺
   7-1213; A-40; C-28; MS m/z 575 (M + H)⁺
   7-1214; A-40; C-29; MS m/z 478 (M + H)⁺
   7-1215; A-40; C-30; MS m/z 494 (M + H)⁺
   7-1216; A-40; C-31; MS m/z 494 (M + H)⁺
   7-1217; A-40; C-32; MS m/z 520 (M + H)⁺
   7-1218; A-40; C-33; MS m/z 532 (M + H)⁺
   7-1219; A-40; C-34; MS m/z 540 (M + H)⁺
   7-1220; A-40; C-35; MS m/z 549 (M + H)⁺
   7-1221; A-40; C-36; MS m/z 544 (M + H)⁺
   7-1222; A-41; C-32; MS m/z 479 (M + H)⁺
   7-1223; A-41; C-37; MS m/z 467 (M + H)⁺
   7-1224; A-41; C-38; MS m/z 480 (M + H)⁺
   7-1225; A-41; C-39; MS m/z 453 (M + H)⁺
   7-1226; A-41; C-40; MS m/z 451 (M + H)⁺
   7-1227; A-41; C-41; MS m/z 467 (M + H)⁺
   7-1228; A-41; C-42; MS m/z 451 (M + H)⁺
   7-1229; A-41; C-43; MS m/z 451 (M + H)⁺
   7-1230; A-41; C-44; MS m/z 439 (M + H)⁺
   7-1231; A-41; C-45; MS m/z 451 (M + H)⁺
   7-1232; A-41; C-46; MS m/z 448 (M + H)⁺
   7-1233; A-41; C-47; MS m/z 440 (M + H)⁺
   7-1234; A-42; C-01; MS m/z 382 (M + H)⁺
   7-1235; A-42; C-02; MS m/z 394 (M + H)⁺
   7-1236; A-42; C-03; MS m/z 406 (M + H)⁺
   7-1237; A-42; C-04; MS m/z 410 (M + H)⁺
   7-1238; A-42; C-05; MS m/z 412 (M + H)⁺
   7-1239; A-42; C-06; MS m/z 420 (M + H)⁺
   7-1240; A-42; C-07; MS m/z 422 (M + H)⁺
   7-1241; A-42; C-08; MS m/z 438 (M + H)⁺
   7-1242; A-42; C-09; MS m/z 444 (M + H)⁺
   7-1243; A-42; C-10; MS m/z 450 (M + H)⁺
   7-1244; A-42; C-11; MS m/z 458 (M + H)⁺
   7-1245; A-42; C-12; MS m/z 464 (M + H)⁺
   7-1246; A-42; C-13; MS m/z 474 (M + H)⁺
   7-1247; A-42; C-14; MS m/z 480 (M + H)⁺
   7-1248; A-42; C-15; MS m/z 498 (M + H)⁺
   7-1249; A-42; C-16; MS m/z 490 (M + H)⁺
   7-1250; A-42; C-17; MS m/z 498 (M + H)⁺
   7-1251; A-42; C-18; MS m/z 410 (M + H)⁺
   7-1252; A-42; C-19; MS m/z 462 (M + H)⁺
   7-1253; A-42; C-20; MS m/z 503 (M + H)⁺
   7-1254; A-42; C-21; MS m/z 425 (M + H)⁺
   7-1255; A-42; C-22; MS m/z 451 (M + H)⁺
   7-1256; A-42; C-23; MS m/z 465 (M + H)⁺
   7-1257; A-42; C-24; MS m/z 486 (M + H)⁺
   7-1258; A-42; C-25; MS m/z 411 (M + H)⁺
   7-1259; A-42; C-26; MS m/z 423 (M + H)⁺
   7-1260; A-42; C-27; MS m/z 431 (M + H)⁺
   7-1261; A-42; C-28; MS m/z 534 (M + H)⁺
   7-1262; A-42; C-29; MS m/z 437 (M + H)⁺
   7-1263; A-42; C-30; MS m/z 453 (M + H)⁺
   7-1264; A-42; C-31; MS m/z 453 (M + H)⁺
   7-1265; A-42; C-32; MS m/z 479 (M + H)⁺
   7-1266; A-42; C-33; MS m/z 491 (M + H)⁺
   7-1267; A-42; C-34; MS m/z 499 (M + H)⁺
   7-1268; A-42; C-35; MS m/z 508 (M + H)⁺
   7-1269; A-42; C-36; MS m/z 503 (M + H)⁺
   7-1270; A-42; C-37; MS m/z 467 (M + H)⁺
   7-1271; A-42; C-38; MS m/z 480 (M + H)⁺
   7-1272; A-42; C-39; MS m/z 453 (M + H)⁺
   7-1273; A-42; C-40; MS m/z 451 (M + H)⁺
   7-1274; A-42; C-41; MS m/z 467 (M + H)⁺
   7-1275; A-42; C-42; MS m/z 451 (M + H)⁺
   7-1276; A-42; C-43: MS m/z 451 (M + H)⁺
   7-1277; A-42; C-44; MS m/z 439 (M + H)⁺
   7-1278; A-42; C-45; MS m/z 451 (M + H)⁺
   7-1279; A-42; C-46; MS m/z 448 (M + H)⁺
   7-1280; A-42; C-47; MS m/z 440 (M + H)⁺
   7-1281; A-43; C-32; MS m/z 465 (M + H)⁺
   7-1282; A-43; C-37; MS m/z 453 (M + H)⁺
   7-1283; A-43; C-38; MS m/z 466 (M + H)⁺
   7-1284; A-43; C-39; MS m/z 439 (M + H)⁺
   7-1285; A-43; C-40; MS m/z 437 (M + H)⁺
   7-1286; A-43; C-41; MS m/z 453 (M + H)⁺
   7-1287; A-43; C-42; MS m/z 437 (M + H)⁺
   7-1288; A-43; C-43; MS m/z 437 (M + H)⁺
   7-1289; A-43; C-44; MS m/z 425 (M + H)⁺
   7-1290; A-43; C-45; MS m/z 437 (M + H)⁺
   7-1291; A-43; C-46; MS m/z 434 (M + H)⁺
   7-1292; A-43; C-47; MS m/z 426 (M + H)⁺
   7-1293; A-44; C-32; MS m/z 493 (M + H)⁺
   7-1294; A-44; C-37; MS m/z 481 (M + H)⁺
   7-1295; A-44; C-38; MS m/z 494 (M + H)⁺
   7-1296; A-44; C-39; MS m/z 467 (M + H)⁺
   7-1297; A-44; C-40; MS m/z 465 (M + H)⁺
   7-1298; A-44; C-41; MS m/z 481 (M + H)⁺
   7-1299; A-44; C-42; MS m/z 465 (M + H)⁺
   7-1300; A-44; C-43; MS m/z 465 (M + H)⁺
   7-1301; A-44; C-44; MS m/z 453 (M + H)⁺
   7-1302; A-44; C-45; MS m/z 465 (M + H)⁺
   7-1303; A-44; C-46; MS m/z 462 (M + H)⁺
   7-1304; A-44; C-47; MS m/z 454 (M + H)⁺
   7-1305; A-45; C-32
   7-1306; A-45; C-37; MS m/z 467 (M + H)⁺
   7-1307; A-45; C-38
   7-1308; A-45; C-39; MS m/z 453 (M + H)⁺
   7-1309; A-45; C-40; MS m/z 451 (M + H)⁺
   7-1310; A-45; C-41; MS m/z 467 (M + H)⁺
   7-1311; A-45; C-42; MS m/z 451 (M + H)⁺
   7-1312; A-45; C-43
   7-1313; A-45; C-44; MS m/z 439 (M + H)⁺
   7-1314; A-45; C-45
   7-1315; A-45; C-46; MS m/z 448 (M + H)⁺
   7-1316; A-45; C-47; MS m/z 440 (M + H)⁺
   7-1317; A-46; C-32; MS m/z 489 (M + H)⁺
   7-1318; A-46; C-37; MS m/z 477 (M + H)⁺
   7-1319; A-46; C-38
   7-1320; A-46; C-39; MS m/z 463 (M + H)⁺
   7-1321; A-46; C-40; MS m/z 461 (M + H)⁺
   7-1322; A-46; C-41; MS m/z 477 (M + H)⁺
   7-1323; A-46; C-42; MS m/z 461 (M + H)⁺
   7-1324; A-46; C-43; MS m/z 461 (M + H)⁺
   7-1325; A-46; C-44; MS m/z 449 (M + H)⁺
   7-1326; A-46; C-45
   7-1327; A-46; C-46; MS m/z 458 (M + H)⁺
   7-1328; A-46; C-47; MS m/z 450 (M + H)⁺
   7-1329; A-47; C-32; MS m/z 479 (M + H)⁺
   7-1330; A-47; C-37; MS m/z 467 (M + H)⁺
   7-1331; A-47; C-38; MS m/z 480 (M + H)⁺
   7-1332; A-47; C-39; MS m/z 453 (M + H)⁺
   7-1333; A-47; C-40; MS m/z 451 (M + H)⁺
   7-1334; A-47; C-41; MS m/z 467 (M + H)⁺
   7-1335; A-47; C-42; MS m/z 451 (M + H)⁺
   7-1336; A-47; C-43; MS m/z 451 (M + H)⁺
   7-1337; A-47; C-44; MS m/z 439 (M + H)⁺
   7-1338; A-47; C-45; MS m/z 451 (M + H)⁺
   7-1339; A-47; C-46; MS m/z 448 (M + H)⁺
   7-1340; A-47; C-47; MS m/z 440 (M + H)⁺
   7-1341; A-48; C-32; MS m/z 533 (M + H)⁺
   7-1342; A-48; C-37; MS m/z 521 (M + H)⁺
   7-1343; A-48; C-38; MS m/z 534 (M + H)⁺
   7-1344; A-48; C-39; MS m/z 507 (M + H)⁺
   7-1345; A-48; C-40; MS m/z 505 (M + H)⁺
   7-1346; A-48; C-41; MS m/z 521 (M + H)⁺
   7-1347; A-48; C-42; MS m/z 505 (M + H)⁺
   7-1348; A-48; C-43; MS m/z 505 (M + H)⁺
   7-1349; A-48; C-44; MS m/z 493 (M + H)⁺
   7-1350; A-48; C-45; MS m/z 505 (M + H)⁺
   7-1351; A-48; C-46; MS m/z 502 (M + H)⁺
   7-1352; A-48; C-47; MS m/z 494 (M + H)⁺
   7-1353; A-49; C-32; MS m/z 531 (M + H)⁺
   7-1354; A-49; C-37; MS m/z 519 (M + H)⁺
   7-1355; A-49; C-38; MS m/z 532 (M + H)⁺
   7-1356; A-49; C-39; MS m/z 505 (M + H)⁺
   7-1357; A-49; C-40; MS m/z 503 (M + H)⁺
   7-1358; A-49; C-41; MS m/z 519 (M + H)⁺
   7-1359; A-49; C-42; MS m/z 503 (M + H)⁺
   7-1360; A-49; C-43; MS m/z 503 (M + H)⁺
   7-1361; A-49; C-44; MS m/z 491 (M + H)⁺
   7-1362; A-49; C-45; MS m/z 503 (M + H)⁺
   7-1363; A-49; C-46; MS m/z 500 (M + H)⁺
   7-1364; A-49; C-47; MS m/z 492 (M + H)⁺
   7-1365; A-50; C-32; MS m/z 529 (M + H)⁺
   7-1366; A-50; C-37; MS m/z 517 (M + H)⁺
   7-1367; A-50; C-38; MS m/z 530 (M + H)⁺
   7-1368; A-50; C-39; MS m/z 503 (M + H)⁺
   7-1369; A-50; C-40; MS m/z 501 (M + H)⁺
   7-1370; A-50; C-41; MS m/z 517 (M + H)⁺
   7-1371; A-50; C-42; MS m/z 501 (M + H)⁺
   7-1372; A-50; C-43; MS m/z 501 (M + H)⁺
   7-1373; A-50; C-44; MS m/z 489 (M + H)⁺
   7-1374; A-50; C-45; MS m/z 501 (M + H)⁺
   7-1375; A-50; C-46; MS m/z 498 (M + H)⁺
   7-1376; A-50; C-47; MS m/z 490 (M + H)⁺
   7-1377; A-51; C-32; MS m/z 547 (M + H)⁺
   7-1378; A-51; C-37; MS m/z 535 (M + H)⁺
   7-1379; A-51; C-38
   7-1380; A-51; C-39; MS m/z 521 (M + H)⁺
   7-1381; A-51; C-40; MS m/z 519 (M + H)⁺
   7-1382; A-51; C-41; MS m/z 535 (M + H)⁺
   7-1383; A-51; C-42; MS m/z 519 (M + H)⁺
   7-1384; A-51; C-43; MS m/z 519 (M + H)⁺
   7-1385; A-51; C-44; MS m/z 507 (M + H)⁺
   7-1386; A-51; C-45; MS m/z 519 (M + H)⁺
   7-1387; A-51; C-46; MS m/z 516 (M + H)⁺
   7-1388; A-51; C-47; MS m/z 508 (M + H)⁺
   7-1389; A-52; C-32; MS m/z 535 (M + H)⁺
   7-1390; A-52; C-37; MS m/z 523 (M + H)⁺
   7-1391; A-52; C-38
   7-1392; A-52; C-39; MS m/z 509 (M + H)⁺
   7-1393; A-52; C-40; MS m/z 507 (M + H)⁺
   7-1394; A-52; C-41; MS m/z 523 (M + H)⁺
   7-1395; A-52; C-42; MS m/z 507 (M + H)⁺
   7-1396; A-52; C-43; MS m/z 507 (M + H)⁺
   7-1397; A-52; C-44; MS m/z 495 (M + H)⁺
   7-1398; A-52; C-45; MS m/z 507 (M + H)⁺
   7-1399; A-52; C-46; MS m/z 504 (M + H)⁺
   7-1400; A-52; C-47; MS m/z 496 (M + H)⁺
   7-1401; A-53; C-32; MS m/z 517 (M + H)⁺
   7-1402; A-53; C-37; MS m/z 505 (M + H)⁺
   7-1403; A-53; C-38
   7-1404; A-53; C-39; MS m/z 491 (M + H)⁺
   7-1405; A-53; C-40; MS m/z 489 (M + H)⁺
   7-1406; A-53; C-41; MS m/z 505 (M + H)⁺
   7-1407; A-53; C-42; MS m/z 489 (M + H)⁺
   7-1408; A-53; C-43; MS m/z 489 (M + H)⁺
   7-1409; A-53; C-44; MS m/z 477 (M + H)⁺
   7-1410; A-53; C-45; MS m/z 489 (M + H)⁺
   7-1411; A-53; C-46; MS m/z 486 (M + H)⁺
   7-1412; A-53; C-47; MS m/z 478 (M + H)⁺
   7-1413; A-59; C-01; MS m/z 434 (M + H)⁺
   7-1414; A-59; C-02; MS m/z 446 (M + H)⁺
   7-1415; A-59; C-03; MS m/z 458 (M + H)⁺
   7-1416; A-59; C-04; MS m/z 462 (M + H)⁺
   7-1417; A-59; C-05; MS m/z 464 (M + H)⁺
   7-1418; A-59; C-06; MS m/z 472 (M + H)⁺
   7-1419; A-59; C-07; MS m/z 474 (M + H)⁺
   7-1420; A-59; C-08; MS m/z 490 (M + H)⁺
   7-1421; A-59; C-09; MS m/z 496 (M + H)⁺
   7-1422; A-59; C-10; MS m/z 502 (M + H)⁺
   7-1423; A-59; C-11; MS m/z 510 (M + H)⁺
   7-1424; A-59; C-12; MS m/z 516 (M + H)⁺
   7-1425; A-59; C-13; MS m/z 526 (M + H)⁺
   7-1426; A-59; C-14; MS m/z 532 (M + H)⁺
   7-1427; A-59; C-15; MS m/z 550 (M + H)⁺
   7-1428; A-59; C-16; MS m/z 542 (M + H)⁺
   7-1429; A-59; C-17; MS m/z 550 (M + H)⁺
   7-1430; A-59; C-18; MS m/z 462 (M + H)⁺
   7-1431; A-59; C-19; MS m/z 514 (M + H)⁺
   7-1432; A-59; C-20; MS m/z 555 (M + H)⁺
   7-1433; A-59; C-21; MS m/z 477 (M + H)⁺
   7-1434; A-59; C-22; MS m/z 503 (M + H)⁺
   7-1435; A-59; C-23; MS m/z 517 (M + H)⁺
   7-1436; A-59; C-24; MS m/z 538 (M + H)⁺
   7-1437; A-59; C-25; MS m/z 463 (M + H)⁺
   7-1438; A-59; C-26; MS m/z 475 (M + H)⁺
   7-1439; A-59; C-27; MS m/z 483 (M + H)⁺
   7-1440; A-59; C-28; MS m/z 586 (M + H)⁺
   7-1441; A-59; C-29; MS m/z 489 (M + H)⁺
   7-1442; A-59; C-30; MS m/z 505 (M + H)⁺
   7-1443; A-59; C-31; MS m/z 505 (M + H)⁺
   7-1444; A-59; C-32; MS m/z 531 (M + H)⁺
   7-1445; A-59; C-33; MS m/z 543 (M + H)⁺
   7-1446; A-59; C-34; MS m/z 551 (M + H)⁺
   7-1447; A-59; C-35; MS m/z 560 (M + H)⁺
   7-1448; A-59; C-36; MS m/z 555 (M + H)⁺
   7-1449; A-67; C-01; MS m/z 446 (M + H)⁺
   7-1450; A-67; C-02; MS m/z 458 (M + H)⁺
   7-1451; A-67; C-03; MS m/z 470 (M + H)⁺
   7-1452; A-67; C-04; MS m/z 474 (M + H)⁺
   7-1453; A-67; C-05; MS m/z 476 (M + H)⁺
   7-1454; A-67; C-06; MS m/z 484 (M + H)⁺
   7-1455; A-67; C-07; MS m/z 486 (M + H)⁺
   7-1456; A-67; C-08; MS m/z 502 (M + H)⁺
   7-1457; A-67; C-09; MS m/z 508 (M + H)⁺
   7-1458; A-67; C-10; MS m/z 514 (M + H)⁺
   7-1459; A-67; C-11; MS m/z 522 (M + H)⁺
   7-1460; A-67; C-12; MS m/z 528 (M + H)⁺
   7-1461; A-67; C-13; MS m/z 538 (M + H)⁺
   7-1462; A-67; C-14; MS m/z 544 (M + H)⁺
   7-1463; A-67; C-15; MS m/z 562 (M + H)⁺
   7-1464; A-67; C-16; MS m/z 554 (M + H)⁺
   7-1465; A-67; C-17; MS m/z 562 (M + H)⁺
   7-1466; A-67; C-18; MS m/z 474 (M + H)⁺
   7-1467; A-67; C-19; MS m/z 526 (M + H)⁺
   7-1468; A-67; C-20; MS m/z 567 (M + H)⁺
   7-1469; A-67; C-21; MS m/z 489 (M + H)⁺
   7-1470; A-67; C-22; MS m/z 515 (M + H)⁺
   7-1471; A-67; C-23; MS m/z 529 (M + H)⁺
   7-1472; A-67; C-24; MS m/z 550 (M + H)⁺
   7-1473; A-67; C-25; MS m/z 475 (M + H)⁺
   7-1474; A-67; C-26; MS m/z 487 (M + H)⁺
   7-1475; A-67; C-27; MS m/z 495 (M + H)⁺
   7-1476; A-67; C-28; MS m/z 598 (M + H)⁺
   7-1477; A-67; C-29; MS m/z 501 (M + H)⁺
   7-1478; A-67; C-30; MS m/z 517 (M + H)⁺
   7-1479; A-67; C-31; MS m/z 517 (M + H)⁺
   7-1480; A-67; C-32; MS m/z 543 (M + H)⁺
   7-1481; A-67; C-33; MS m/z 555 (M + H)⁺
   7-1482; A-67; C-34; MS m/z 563 (M + H)⁺
   7-1483; A-67; C-35; MS m/z 572 (M + H)⁺
   7-1484; A-67; C-36; MS m/z 567 (M + H)⁺
   7-1485; A-68; C-01; MS m/z 416 (M + H)⁺
   7-1486; A-68; C-02; MS m/z 428 (M + H)⁺
   7-1487; A-68; C-03; MS m/z 440 (M + H)⁺
   7-1488; A-68; C-04; MS m/z 444 (M + H)⁺
   7-1489; A-68; C-05; MS m/z 446 (M + H)⁺
   7-1490; A-68; C-06; MS m/z 454 (M + H)⁺
   7-1491; A-68; C-07; MS m/z 456 (M + H)⁺
   7-1492; A-68; C-08; MS m/z 472 (M + H)⁺
   7-1493; A-68; C-09; MS m/z 478 (M + H)⁺
   7-1494; A-68; C-10; MS m/z 484 (M + H)⁺
   7-1495; A-68; C-11; MS m/z 492 (M + H)⁺
   7-1496; A-68; C-12; MS m/z 498 (M + H)⁺
   7-1497; A-68; C-13; MS m/z 508 (M + H)⁺
   7-1498; A-68; C-14; MS m/z 514 (M + H)⁺
   7-1499; A-68; C-15; MS m/z 532 (M + H)⁺
   7-1500; A-68; C-16; MS m/z 524 (M + H)⁺
   7-1501; A-68; C-17; MS m/z 532 (M + H)⁺
   7-1502; A-68; C-18; MS m/z 444 (M + H)⁺
   7-1503; A-68; C-19; MS m/z 496 (M + H)⁺
   7-1504; A-68; C-20; MS m/z 537 (M + H)⁺
   7-1505; A-68; C-21; MS m/z 459 (M + H)⁺
   7-1506; A-68; C-22; MS m/z 485 (M + H)⁺
   7-1507; A-68; C-23; MS m/z 499 (M + H)⁺
   7-1508; A-68; C-24; MS m/z 520 (M + H)⁺
   7-1509; A-68; C-25; MS m/z 445 (M + H)⁺
   7-1510; A-68; C-26; MS m/z 457 (M + H)⁺
   7-1511; A-68; C-27; MS m/z 465 (M + H)⁺
   7-1512; A-68; C-28; MS m/z 568 (M + H)⁺
   7-1513; A-68; C-29; MS m/z 471 (M + H)⁺
   7-1514; A-68; C-30; MS m/z 487 (M + H)⁺
   7-1515; A-68; C-31; MS m/z 487 (M + H)⁺
   7-1516; A-68; C-32; MS m/z 513 (M + H)⁺
   7-1517; A-68; C-33; MS m/z 525 (M + H)⁺
   7-1518; A-68; C-34; MS m/z 533 (M + H)⁺
   7-1519; A-68; C-35; MS m/z 542 (M + H)⁺
   7-1520; A-68; C-36; MS m/z 537 (M + H)⁺
   7-1521; A-69; C-01; MS m/z 446 (M + H)⁺
   7-1522; A-69; C-02; MS m/z 458 (M + H)⁺
   7-1523; A-69; C-03; MS m/z 470 (M + H)⁺
   7-1524; A-69; C-04; MS m/z 474 (M + H)⁺
   7-1525; A-69; C-05; MS m/z 476 (M + H)⁺
   7-1526; A-69; C-06; MS m/z 484 (M + H)⁺
   7-1527; A-69; C-07; MS m/z 486 (M + H)⁺
   7-1528; A-69; C-08; MS m/z 502 (M + H)⁺
   7-1529; A-69; C-09; MS m/z 508 (M + H)⁺
   7-1530; A-69; C-10; MS m/z 514 (M + H)⁺
   7-1531; A-69; C-11; MS m/z 522 (M + H)⁺
   7-1532; A-69; C-12; MS m/z 528 (M + H)⁺
   7-1533; A-69; C-13; MS m/z 538 (M + H)⁺
   7-1534; A-69; C-14; MS m/z 544 (M + H)⁺
   7-1535; A-69; C-15; MS m/z 562 (M + H)⁺
   7-1536; A-69; C-16; MS m/z 554 (M + H)⁺
   7-1537; A-69; C-17; MS m/z 562 (M + H)⁺
   7-1538; A-69; C-18; MS m/z 474 (M + H)⁺
   7-1539; A-69; C-19; MS m/z 526 (M + H)⁺
   7-1540; A-69; C-20; MS m/z 567 (M + H)⁺
   7-1541; A-69; C-21; MS m/z 489 (M + H)⁺
   7-1542; A-69; C-22; MS m/z 515 (M + H)⁺
   7-1543; A-69; C-23; MS m/z 529 (M + H)⁺
   7-1544; A-69; C-24; MS m/z 550 (M + H)⁺
   7-1545; A-69; C-25; MS m/z 475 (M + H)⁺
   7-1546; A-69; C-26; MS m/z 487 (M + H)⁺
   7-1547; A-69; C-27; MS m/z 495 (M + H)⁺
   7-1548; A-69; C-28; MS m/z 598 (M + H)⁺
   7-1549; A-69; C-29; MS m/z 501 (M + H)⁺
   7-1550; A-69; C-30;. MS m/z 517 (M + H)⁺
   7-1551; A-69; C-31; MS m/z 517 (M + H)⁺
   7-1552; A-69; C-32; MS m/z 543 (M + H)⁺
   7-1553; A-69; C-33; MS m/z 555 (M + H)⁺
   7-1554; A-69; C-34; MS m/z 563 (M + H)⁺
   7-1555; A-69; C-35; MS m/z 572 (M + H)⁺
   7-1556; A-69; C-36; MS m/z 567 (M + H)⁺
   7-1557; A-70; C-01; MS m/z 378 (M + H)⁺
   7-1558; A-70; C-02; MS m/z 390 (M + H)⁺
   7-1559; A-70; C-03; MS m/z 402 (M + H)⁺
   7-1560; A-70; C-04; MS m/z 406 (M + H)⁺
   7-1561; A-70; C-05; MS m/z 408 (M + H)⁺
   7-1562; A-70; C-06; MS m/z 416 (M + H)⁺
   7-1563; A-70; C-07; MS m/z 418 (M + H)⁺
   7-1564; A-70; C-08; MS m/z 434 (M + H)⁺
   7-1565; A-70; C-09; MS m/z 440 (M + H)⁺
   7-1566; A-70; C-10; MS m/z 446 (M + H)⁺
   7-1567; A-70; C-11; MS m/z 454 (M + H)⁺
   7-1568; A-70; C-12; MS m/z 460 (M + H)⁺
   7-1569; A-70; C-13; MS m/z 470 (M + H)⁺
   7-1570; A-70; C-14; MS m/z 476 (M + H)⁺
   7-1571; A-70; C-15; MS m/z 494 (M + H)⁺
   7-1572; A-70; C-16; MS m/z 486 (M + H)⁺
   7-1573; A-70; C-17; MS m/z 494 (M + H)⁺
   7-1574; A-70; C-18; MS m/z 406 (M + H)⁺
   7-1575; A-70; C-19; MS m/z 458 (M + H)⁺
   7-1576; A-70; C-20; MS m/z 499 (M + H)⁺
   7-1577; A-70; C-21; MS m/z 421 (M + H)⁺
   7-1578; A-70; C-22; MS m/z 447 (M + H)⁺
   7-1579; A-70; C-23; MS m/z 461 (M + H)⁺
   7-1580; A-70; C-24; MS m/z 482 (M + H)⁺
   7-1581; A-70; C-25; MS m/z 407 (M + H)⁺
   7-1582; A-70; C-26; MS m/z 419 (M + H)⁺
   7-1583; A-70; C-27; MS m/z 427 (M + H)⁺
   7-1584; A-70; C-28; MS m/z 530 (M + H)⁺
   7-1585; A-70; C-29; MS m/z 433 (M + H)⁺
   7-1586; A-70; C-30; MS m/z 449 (M + H)⁺
   7-1587; A-70; C-31; MS m/z 449 (M + H)⁺
   7-1588; A-70; C-32; MS m/z 475 (M + H)⁺
   7-1589; A-70; C-33; MS m/z 487 (M + H)⁺
   7-1590; A-70; C-34; MS m/z 495 (M + H)⁺
   7-1591; A-70; C-35; MS m/z 504 (M + H)⁺
   7-1592; A-70; C-36; MS m/z 499 (M + H)⁺
   7-1593; A-71; C-01; MS m/z 452 (M + H)⁺
   7-1594; A-71; C-02; MS m/z 464 (M + H)⁺
   7-1595; A-71; C-03; MS m/z 476 (M + H)⁺
   7-1596; A-71; C-04; MS m/z 480 (M + H)⁺
   7-1597; A-71; C-05; MS m/z 482 (M + H)⁺
   7-1598; A-71; C-06; MS m/z 490 (M + H)⁺
   7-1599; A-71; C-07; MS m/z 492 (M + H)⁺
   7-1600; A-71; C-08; MS m/z 508 (M + H)⁺
   7-1601; A-71; C-09; MS m/z 514 (M + H)⁺
   7-1602; A-71; C-10; MS m/z 520 (M + H)⁺
   7-1603; A-71; C-11; MS m/z 528 (M + H)⁺
   7-1604; A-71; C-12; MS m/z 534 (M + H)⁺
   7-1605; A-71; C-13; MS m/z 544 (M + H)⁺
   7-1606; A-71; C-14; MS m/z 550 (M + H)⁺
   7-1607; A-71; C-15; MS m/z 568 (M + H)⁺
   7-1608; A-71; C-16; MS m/z 560 (M + H)⁺
   7-1609; A-71; C-17; MS m/z 568 (M + H)⁺
   7-1610; A-71; C-18; MS m/z 480 (M + H)⁺
   7-1611; A-71; C-19; MS m/z 532 (M + H)⁺
   7-1612; A-71; C-20; MS m/z 573 (M + H)⁺
   7-1613; A-71; C-21; MS m/z 495 (M + H)⁺
   7-1614; A-71; C-22; MS m/z 521 (M + H)⁺
   7-1615; A-71; C-23; MS m/z 535 (M + H)⁺
   7-1616; A-71; C-24; MS m/z 556 (M + H)⁺
   7-1617; A-71; C-25; MS m/z 481 (M + H)⁺
   7-1618; A-71; C-26; MS m/z 493 (M + H)⁺
   7-1619; A-71; C-27; MS m/z 501 (M + H)⁺
   7-1620; A-71; C-28; MS m/z 604 (M + H)⁺
   7-1621; A-71; C-29; MS m/z 507 (M + H)⁺
   7-1622; A-71; C-30; MS m/z 523 (M + H)⁺
   7-1623; A-71; C-31; MS m/z 523 (M + H)⁺
   7-1624; A-71; C-32; MS m/z 549 (M + H)⁺
   7-1625; A-71; C-33; MS m/z 561 (M + H)⁺
   7-1626; A-71; C-34; MS m/z 569 (M + H)⁺
   7-1627; A-71; C-35; MS m/z 578 (M + H)⁺
   7-1628; A-71; C-36; MS m/z 573 (M + H)⁺
(h) The examples of compounds described in : 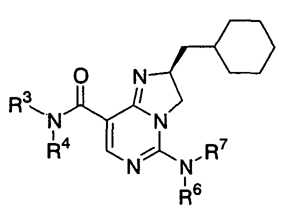 which are included in Compound (I), are listed below in order of compound number, -NR³R⁴, -NR⁶R⁷ and spectrum data thereof.
   8-1; A-25; C-13; MS m/z 495 (M + H)⁺
   8-2; A-25; C-14; MS m/z 501 (M + H)⁺
   8-3; A-25; C-15; MS m/z 519 (M + H)⁺
   8-4; A-25; C-16; MS m/z 511 (M + H)⁺,
   8-5; A-25; C-17; MS m/z 519 (M + H)⁺
   8-6; A-25; C-18; MS m/z 431 (M + H)⁺
   8-7; A-25; C-19; MS m/z 483 (M + H)⁺
   8-8; A-25; C-20; MS m/z 524 (M + H)⁺
   8-9; A-25; C-21; MS m/z 446 (M + H)⁺
   8-10; A-25; C-22; MS m/z 472 (M + H)⁺
   8-11; A-25; C-23; MS m/z 486 (M + H)⁺
   8-12; A-25; C-24; MS m/z 507 (M + H)⁺
   8-13; A-27; C-13; MS m/z 535 (M + H)⁺
   8-14; A-27; C-14; MS m/z 541 (M + H)⁺
   8-15; A-27; C-15; MS m/z 559 (M + H)⁺
   8-16; A-27; C-16; MS m/z 551 (M + H)⁺
   8-17; A-27; C-17; MS m/z 559 (M + H)⁺
   8-18; A-27; C-18; MS m/z 471 (M + H)⁺
   8-19; A-27; C-19; MS m/z 523 (M + H)⁺
   8-20; A-27; C-20
   8-21; A-27; C-21; MS m/z 486 (M + H)⁺
   8-22; A-27; C-22
   8-23; A-27; C-23; MS m/z 526 (M + H)⁺
   8-24; A-27; C-24; MS m/z 547 (M + H)⁺
   8-25; A-28; C-13; MS m/z 565 (M + H)⁺
   8-26; A-28; C-14; MS m/z 571 (M + H)⁺
   8-27; A-28; C-15; MS m/z 589 (M + H)⁺
   8-28; A-28; C-16; MS m/z 581 (M + H)⁺
   8-29; A-28; C-17; MS m/z 589 (M + H)⁺
   8-30; A-28; C-18; MS m/z 501 (M + H)⁺
   8-31; A-28; C-19; MS m/z 553 (M + H)⁺
   8-32; A-28; C-20; MS m/z 594 (M + H)⁺
   8-33; A-28; C-21; MS m/z 516 (M + H)⁺
   8-34; A-28; C-22
   8-35; A-28; C-23; MS m/z 556 (M + H)⁺
   8-36; A-28; C-24; MS m/z 577 (M + H)⁺
   8-37; A-42; C-13; MS m/z 480 (M + H)⁺
   8-38; A-42; C-14; MS m/z 486 (M + H)⁺
   8-39; A-42; C-15; MS m/z 504 (M + H)⁺
   8-40; A-42; C-16; MS m/z 496 (M + H)⁺
   8-41; A-42; C-17; MS m/z 504 (M + H)⁺
   8-42; A-42; C-18; MS m/z 416 (M + H)⁺
   8-43; A-42; C-19; MS m/z 468 (M + H)⁺
   8-44; A-42; C-20; MS m/z 509 (M + H)⁺
   8-45; A-42; C-21; MS m/z 431 (M + H)⁺
   8-46; A-42; C-22
   8-47; A-42; C-23; MS m/z 471 (M + H)⁺
   8-48; A-42; C-24; MS m/z 492 (M + H)⁺
   8-49; A-59; C-13; MS m/z 532 (M + H)⁺
   8-50; A-59; C-14; MS m/z 538 (M + H)⁺
   8-51; A-59; C-15; MS m/z 556 (M + H)⁺
   8-52; A-59; C-16; MS m/z 548 (M + H)⁺
   8-53; A-59; C-17; MS m/z 556 (M + H)⁺
   8-54; A-59; C-18; MS m/z 468 (M + H)⁺
   8-55; A-59; C-19; MS m/z 520 (M + H)⁺
   8-56; A-59; C-20
   8-57; A-59; C-21; MS m/z 483 (M + H)⁺
   8-58; A-59; C-22
   8-59; A-59; C-23; MS m/z 523 (M + H)⁺
   8-60; A-59; C-24; MS m/z 544 (M + H)⁺
   8-61; A-67; C-13; MS m/z 544 (M + H)⁺
   8-62; A-67; C-14; MS m/z 550 (M + H)⁺
   8-63; A-67; C-15; MS m/z 568 (M + H)⁺
   8-64; A-67; C-16; MS m/z 560 (M + H)⁺
   8-65; A-67; C-17; MS m/z 568 (M + H)⁺
   8-66; A-67; C-18; MS m/z 480 (M + H)⁺
   8-67; A-67; C-19; MS m/z 532 (M + H)⁺
   8-68; A-67; C-20
   8-69; A-67; C-21; MS m/z 495 (M + H)⁺
   8-70; A-67; C-22
   8-71; A-67; C-23; MS m/z 535 (M + H)⁺
   8-72; A-67; C-24; MS m/z 556 (M + H)⁺
   8-73; A-68; C-13; MS m/z 514 (M + H)⁺
   8-74; A-68; C-14; MS m/z 520 (M + H)⁺
   8-75; A-68; C-15; MS m/z 538 (M + H)⁺
   8-76; A-68; C-16; MS m/z 530 (M + H)⁺
   8-77; A-68; C-17; MS m/z 538 (M + H)⁺
   8-78; A-68; C-18; MS m/z 450 (M + H)⁺
   8-79; A-68; C-19; MS m/z 502 (M + H)⁺
   8-80; A-68; C-20
   8-81; A-68; C-21; MS m/z 465 (M + H)⁺
   8-82; A-68; C-22
   8-83; A-68; C-23; MS m/z 505 (M + H)⁺
   8-84; A-68; C-24; MS m/z 526 (M + H)⁺
   8-85; A-69; C-13; MS m/z 544 (M + H)⁺
   8-86; A-69; C-14; MS m/z 550 (M + H)⁺
   8-87; A-69; C-15; MS m/z 568 (M + H)⁺
   8-88; A-69; C-16; MS m/z 560 (M + H)⁺
   8-89; A-69; C-17; MS m/z 568 (M + H)⁺
   8-90; A-69; C-18; MS m/z 480 (M + H)⁺
   8-91; A-69; C-19; MS m/z 532 (M + H)⁺
   8-92; A-69; C-20
   8-93; A-69; C-21; MS m/z 495 (M + H)⁺
   8-94; A-69; C-22
   8-95; A-69; C-23; MS m/z 535 (M + H)⁺
   8-96; A-69; C-24; MS m/z 556 (M + H)⁺
(i) The examples of compounds describe in : 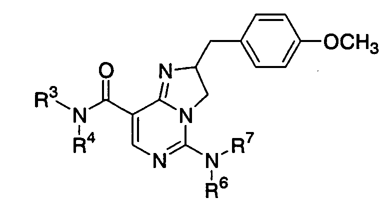 which are included in Compound (I), are listed below in order of compound number, -NR³R⁴, -NR⁶R⁷ and spectrum data thereof.
   9-1; A-09; C-01; MS m/z 438 (M + H)⁺
   9-2; A-09; C-02; MS m/z 450 (M + H)⁺
   9-3; A-09; C-03; MS m/z 462 (M + H)⁺
   9-4; A-09; C-04; MS m/z 466 (M + H)⁺
   9-5; A-09; C-05; MS m/z 468 (M + H)⁺
   9-6; A-09; C-06; MS m/z 476 (M + H)⁺
   9-7; A-09; C-07; MS m/z 478 (M + H)⁺
   9-8; A-09; C-08; MS m/z 494 (M + H)⁺
   9-9; A-09; C-09; MS m/z 500 (M + H)⁺
   9-10; A-09; C-10; MS m/z 506 (M + H)⁺
   9-11; A-09; C-11; MS m/z 514 (M + H)⁺
   9-12; A-09; C-12; MS m/z 520 (M + H)⁺
   9-13; A-10; C-01; MS m/z 438 (M + H)⁺
   9-14; A-10; C-02; MS m/z 450 (M + H)⁺
   9-15; A-10; C-03; MS m/z 462 (M + H)⁺
   9-16; A-10; C-04; MS m/z 466 (M + H)⁺
   9-17; A-10; C-05; MS m/z 468 (M + H)⁺
   9-18; A-10; C-06; MS m/z 476 (M + H)⁺
   9-19; A-10; C-07; MS m/z 478 (M + H)⁺
   9-20; A-10; C-08; MS m/z 494 (M + H)⁺
   9-21; A-10; C-09; MS m/z 500 (M + H)⁺
   9-22; A-10; C-10; MS m/z 506 (M + H)⁺
   9-23; A-10; C-11; MS m/z 514 (M + H)⁺
   9-24; A-10; C-12; MS m/z 520 (M + H)⁺
   9-25; A-11; C-01; MS m/z 438 (M + H)⁺
   9-26; A-11; C-02; MS m/z 450 (M + H)⁺
   9-27; A-11; C-03; MS m/z 462 (M + H)⁺
   9-28; A-11; C-04; MS m/z 466 (M + H)⁺
   9-29; A-11; C-05; MS m/z 468 (M + H)⁺
   9-30; A-11; C-06; MS m/z 476 (M + H)⁺
   9-31; A-11; C-07; MS m/z 478 (M + H)⁺
   9-32; A-11; C-08; MS m/z 494 (M + H)⁺
   9-33; A-11; C-09; MS m/z 500 (M + H)⁺
   9-34; A-11; C-10; MS m/z 506 (M + H)⁺
   9-35; A-11; C-11; MS m/z 514 (M + H)⁺
   9-36; A-11; C-12; MS m/z 520 (M + H)⁺
   9-37; A-12; C-01; MS m/z 438 (M + H)⁺
   9-38; A-12; C-02; MS m/z 450 (M + H)⁺
   9-39; A-12; C-03; MS m/z 462 (M + H)⁺
   9-40; A-12; C-04; MS m/z 466 (M + H)⁺
   9-41; A-12; C-05; MS m/z 468 (M + H)⁺
   9-42; A-12; C-06; MS m/z 476 (M + H)⁺
   9-43; A-12; C-07; MS m/z 478 (M + H)⁺
   9-44; A-12; C-08; MS m/z 494 (M + H)⁺
   9-45; A-12; C-09; MS m/z 500 (M + H)⁺
   9-46; A-12; C-10; MS m/z 506 (M + H)⁺
   9-47; A-12; C-11; MS m/z 514 (M + H)⁺
   9-48; A-12; C-12; MS m/z 520 (M + H)⁺
   9-49; A-13; C-01; MS m/z 446 (M + H)⁺
   9-50; A-13; C-02; MS m/z 458 (M + H)⁺
   9-51; A-13; C-03; MS m/z 470 (M + H)⁺
   9-52; A-13; C-04; MS m/z 474 (M + H)⁺
   9-53; A-13; C-05; MS m/z 476 (M + H)⁺
   9-54; A-13; C-06; MS m/z 484 (M + H)⁺
   9-55; A-13; C-07; MS m/z 486 (M + H)⁺
   9-56; A-13; C-08; MS m/z 502 (M + H)⁺
   9-57; A-13; C-09; MS m/z 508 (M + H)⁺
   9-58; A-13; C-10; MS m/z 514 (M + H)⁺
   9-59; A-13; C-11; MS m/z 522 (M + H)⁺
   9-60; A-13; C-12; MS m/z 528 (M + H)⁺
   9-61; A-14; C-01; MS m/z 446 (M + H)⁺
   9-62; A-14; C-02; MS m/z 458 (M + H)⁺
   9-63; A-14; C-03; MS m/z 470 (M + H)⁺
   9-64; A-14; C-04; MS m/z 474 (M + H)⁺
   9-65; A-14; C-05; MS m/z 476 (M + H)⁺
   9-66; A-14; C-06; MS m/z 484 (M + H)⁺
   9-67; A-14; C-07; MS m/z 486 (M + H)⁺
   9-68; A-14; C-08; MS m/z 502 (M + H)⁺
   9-69; A-14; C-09; MS m/z 508 (M + H)⁺
   9-70; A-14; C-10; MS m/z 514 (M + H)⁺
   9-71; A-14; C-11; MS m/z 522 (M + H)⁺
   9-72; A-14; C-12; MS m/z 528 (M + H)⁺
   9-73; A-15; C-01; MS m/z 454 (M + H)⁺
   9-74; A-15; C-02; MS m/z 466 (M + H)⁺
   9-75; A-15; C-03; MS m/z 478 (M + H)⁺
   9-76; A-15; C-04; MS m/z 482 (M + H)⁺
   9-77; A-15; C-05; MS m/z 484 (M + H)⁺
   9-78; A-15; C-06; MS m/z 492 (M + H)⁺
   9-79; A-15; C-07; MS m/z 494 (M + H)⁺
   9-80; A-15; C-08; MS m/z 510 (M + H)⁺
   9-81; A-15; C-09; MS m/z 516 (M + H)⁺
   9-82; A-15; C-10; MS m/z 522 (M + H)⁺
   9-83; A-15; C-11; MS m/z 530 (M + H)⁺
   9-84; A-15; C-12; MS m/z 536 (M + H)⁺
   9-85; A-16; C-01; MS m/z 460 (M + H)⁺
   9-86; A-16; C-02; MS m/z 472 (M + H)⁺
   9-87; A-16; C-03; MS m/z 484 (M + H)⁺
   9-88; A-16; C-04; MS m/z 488 (M + H)⁺
   9-89; A-16; C-05; MS m/z 490 (M + H)⁺
   9-90; A-16; C-06; MS m/z 498 (M + H)⁺
   9-91; A-16; C-07; MS m/z 500 (M + H)⁺
   9-92; A-16; C-08; MS m/z 516 (M + H)⁺
   9-93; A-16; C-09; MS m/z 522 (M + H)⁺
   9-94; A-16; C-10; MS m/z 528 (M + H)⁺
   9-95; A-16; C-11; MS m/z 536 (M + H)⁺
   9-96; A-16; C-12; MS m/z 542 (M + H)⁺
(j) The examples of compounds described in : 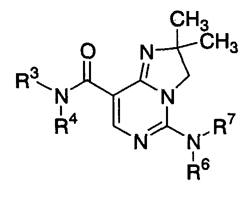 which are included in Compound (I), are listed below in order of compound number, -NR³R⁴, -NR⁶R⁷ and spectrum data thereof.
   10-1; A-25; C-01; MS m/z 335 (M + H)⁺
   10-2; A-25; C-02; MS m/z 347 (M + H)⁺
   10-3; A-25; C-03; MS m/z 359 (M + H)⁺
   10-4; A-25; C-04; MS m/z 363 (M + H)⁺
   10-5; A-25; C-05; MS m/z 365 (M + H)⁺
   10-6; A-25; C-06; MS m/z 373 (M + H)⁺
   10-7; A-25; C-07
   10-8; A-25; C-08; MS m/z 391 (M + H)⁺
   10-9; A-25; C-09; MS m/z 397 (M + H)⁺
   10-10; A-25; C-10; MS m/z 403 (M + H)⁺
   10-11; A-25; C-11; MS m/z 411 (M + H)⁺
   10-12; A-25; C-12; MS m/z 417 (M + H)⁺
   10-13; A-27; C-01; MS m/z 375 (M + H)⁺
   10-14; A-27; C-02; MS m/z 387 (M + H)⁺
   10-15; A-27; C-03; MS m/z 399 (M + H)⁺
   10-16; A-27; C-04; MS m/z 403 (M + H)⁺
   10-17; A-27; C-05; MS m/z 405 (M + H)⁺
   10-18; A-27; C-06; MS m/z 413 (M + H)⁺
   10-19; A-27; C-07; MS m/z 415 (M + H)⁺
   10-20; A-27; C-08; MS m/z 431 (M + H)⁺
   10-21; A-27; C-09; MS m/z 437 (M + H)⁺
   10-22; A-27; C-10; MS m/z 443 (M + H)⁺
   10-23; A-27; C-11; MS m/z 451 (M + H)⁺
   10-24; A-27; C-12; MS m/z 457 (M + H)⁺
   10-25; A-28; C-01; MS m/z 405 (M + H)⁺
   10-26; A-28; C-02; MS m/z 417 (M + H)⁺
   10-27; A-28; C-03; MS m/z 429 (M + H)⁺
   10-28; A-28; C-04; MS m/z 433 (M + H)⁺
   10-29; A-28; C-05; MS m/z 435 (M + H)⁺
   10-30; A-28; C-06; MS m/z 443 (M + H)⁺
   10-31; A-28; C-07; MS m/z 445 (M + H)⁺
   10-32; A-28; C-08; MS m/z 461 (M + H)⁺
   10-33; A-28; C-09; MS m/z 467 (M + H)⁺
   10-34; A-28; C-10; MS m/z 473 (M + H)⁺
   10-35; A-28; C-11; MS m/z 481 (M + H)⁺
   10-36; A-28; C-12; MS m/z 487 (M + H)⁺
   10-37; A-42; C-01; MS m/z 320 (M + H)⁺
   10-38; A-42; C-02; MS m/z 332 (M + H)⁺
   10-39; A-42; C-03; MS m/z 344 (M + H)⁺
   10-40; A-42; C-04; MS m/z 348 (M + H)⁺
   10-41; A-42; C-05; MS m/z 350 (M + H)⁺
   10-42; A-42; C-06; MS m/z 358 (M + H)⁺
   10-43; A-42; C-07; MS m/z 360 (M + H)⁺
   10-44; A-42; C-08; MS m/z 376 (M + H)⁺
   10-45; A-42; C-09; MS m/z 382 (M + H)⁺
   10-46; A-42; C-10; MS m/z 388 (M + H)⁺
   10-47; A-42; C-11; MS m/z 396 (M + H)⁺
   10-48; A-42; C-12; MS m/z 402 (M + H)⁺
   10-49; A-59; C-01; MS m/z 372 (M + H)⁺
   10-50; A-59; C-02; MS m/z 384 (M + H)⁺
   10-51; A-59; C-03; MS m/z 396 (M + H)⁺
   10-52; A-59; C-04; MS m/z 400 (M + H)⁺
   10-53; A-59; C-05; MS m/z 402 (M + H)⁺
   10-54; A-59; C-06; MS m/z 410 (M + H)⁺
   10-55; A-59; C-07; MS m/z 412 (M + H)⁺
   10-56; A-59; C-08; MS m/z 428 (M + H)⁺
   10-57; A-59; C-09; MS m/z 434 (M + H)⁺
   10-58; A-59; C-10; MS m/z 440 (M + H)⁺
   10-59; A-59; C-11; MS m/z 448 (M + H)⁺
   10-60; A-59; C-12; MS m/z 454 (M + H)⁺
   10-61; A-67; C-01; MS m/z 384 (M + H)⁺
   10-62; A-67; C-02; MS m/z 396 (M + H)⁺
   10-63; A-67; C-03; MS m/z 408 (M + H)⁺
   10-64; A-67; C-04; MS m/z 412 (M + H)⁺
   10-65; A-67; C-05; MS m/z 414 (M + H)⁺
   10-66; A-67; C-06; MS m/z 422 (M + H)⁺
   10-67; A-67; C-07; MS m/z 424 (M + H)⁺
   10-68; A-67; C-08; MS m/z 440 (M + H)⁺
   10-69; A-67; C-09; MS m/z 446 (M + H)⁺
   10-70; A-67; C-10; MS m/z 452 (M + H)⁺
   10-71; A-67; C-11; MS m/z 460 (M + H)⁺
   10-72; A-67; C-12; MS m/z 466 (M + H)⁺
   10-73; A-68; C-01; MS m/z 354 (M + H)⁺
   10-74; A-68; C-02; MS m/z 366 (M + H)⁺
   10-75; A-68; C-03; MS m/z 378 (M + H)⁺
   10-76; A-68; C-04; MS m/z 382 (M + H)⁺
   10-77; A-68; C-05; MS m/z 384 (M + H)⁺
   10-78; A-68; C-06; MS m/z 392 (M + H)⁺
   10-79; A-68; C-07; MS m/z 394 (M + H)⁺
   10-80; A-68; C-08; MS m/z 410 (M + H)⁺
   10-81; A-68; C-09; MS m/z 416 (M + H)⁺
   10-82; A-68; C-10; MS m/z 422 (M + H)⁺
   10-83; A-68; C-11; MS m/z 430 (M + H)⁺
   10-84; A-68; C-12; MS m/z 436 (M + H)⁺
   10-85; A-69; C-01; MS m/z 384 (M + H)⁺
   10-86; A-69; C-02; MS m/z 396 (M + H)⁺
   10-87; A-69; C-03; MS m/z 408 (M + H)⁺
   10-88; A-69; C-04; MS m/z 412 (M + H)⁺
   10-89; A-69; C-05; MS m/z 414 (M + H)⁺
   10-90; A-69; C-06; MS m/z 422 (M + H)⁺
   10-91; A-69; C-07; MS m/z 424 (M + H)⁺
   10-92; A-69; C-08; MS m/z 440 (M + H)⁺
   10-93; A-69; C-09; MS m/z 446 (M + H)⁺
   10-94; A-69; C-10; MS m/z 452 (M + H)⁺
   10-95; A-69; C-11; MS m/z 460 (M + H)⁺
   10-96; A-69; C-12; MS m/z 466 (M + H)⁺
(k) The examples of compounds described in : 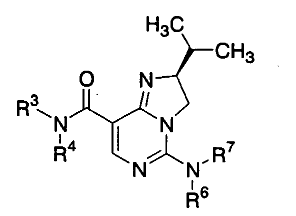 which are included in Compound (I), are listed below in order of compound number, -NR³R⁴, -NR⁶R⁷ and spectrum data thereof.
   11-1; A-19; C-13; MS m/z 472 (M + H)⁺
   11-2; A-25; C-01; MS m/z 349 (M + H)⁺
   11-3; A-25; C-02; MS m/z 361 (M + H)⁺
   11-4; A-25; C-03; MS m/z 373 (M + H)⁺
   11-5; A-25; C-04; MS m/z 377 (M + H)⁺
   11-6; A-25; C-05; MS m/z 379 (M + H)⁺
   11-7; A-25; C-06; MS m/z 387 (M + H)⁺
   11-8; A-25; C-07; MS m/z 389 (M + H)⁺
   11-9; A-25; C-08; MS m/z 405 (M + H)⁺
   11-10; A-25; C-09; MS m/z 411 (M + H)⁺
   11-11; A-25; C-10; MS m/z 417 (M + H)⁺
   11-12; A-25; C-11; MS m/z 425 (M + H)⁺
   11-13; A-25; C-12; MS m/z 431 (M + H)⁺
   11-14; A-27; C-01; MS m/z 389 (M + H)⁺
   11-15; A-27; C-02; MS m/z 401 (M + H)⁺
   11-16; A-27; C-03; MS m/z 413 (M + H)⁺
   11-17; A-27; C-04; MS m/z 417 (M + H)⁺
   11-18; A-27; C-05; MS m/z 419 (M + H)⁺
   11-19; A-27; C-06; MS m/z 427 (M + H)⁺
   11-20; A-27; C-07; MS m/z 429 (M + H)⁺
   11-21; A-27; C-08; MS m/z 445 (M + H)⁺
   11-22; A-27; C-09; MS m/z 451 (M + H)⁺
   11-23; A-27; C-10; MS m/z 457 (M + H)⁺
   11-24; A-27; C-11; MS m/z 465 (M + H)⁺
   11-25; A-27; C-12; MS m/z 471 (M + H)⁺
   11-26; A-28; C-01; MS m/z 419 (M + H)⁺
   11-27; A-28; C-02; MS m/z 431 (M + H)⁺
   11-28; A-28; C-03; MS m/z 443 (M + H)⁺
   11-29; A-28; C-04; MS m/z 447 (M + H)⁺
   11-30; A-28; C-05; MS m/z 449 (M + H)⁺
   11-31; A-28; C-06; MS m/z 457 (M + H)⁺
   11-32; A-28; C-07; MS m/z 459 (M + H)⁺
   11-33; A-28; C-08; MS m/z 475 (M + H)⁺
   11-34; A-28; C-09; MS m/z 481 (M + H)⁺
   11-35; A-28; C-10; MS m/z 487 (M + H)⁺
   11-36; A-28; C-11; MS m/z 495 (M + H)⁺
   11-37; A-28; C-12; MS m/z 501 (M + H)⁺
   11-38; A-42; C-01; MS m/z 334 (M + H)⁺
   11-39; A-42; C-02; MS m/z 346 (M + H)⁺
   11-40; A-42; C-03; MS m/z 358 (M + H)⁺
   11-41; A-42; C-04; MS m/z 362 (M + H)⁺
   11-42; A-42; C-05; MS m/z 364 (M + H)⁺
   11-43; A-42; C-06; MS m/z 372 (M + H)⁺
   11-44; A-42; C-07; MS m/z 374 (M + H)⁺
   11-45; A-42; C-08; MS m/z 390 (M + H)⁺
   11-46; A-42; C-09; MS m/z 396 (M + H)⁺
   11-47; A-42; C-10; MS m/z 402 (M + H)⁺
   11-48; A-42; C-11; MS m/z 410 (M + H)⁺
   11-49; A-42; C-12; MS m/z 416 (M + H)⁺
   11-50; A-59; C-01; MS m/z 386 (M + H)⁺
   11-51; A-59; C-02; MS m/z 398 (M + H)⁺
   11-52; A-59; C-03; MS m/z 410 (M + H)⁺
   11-53; A-59; C-04; MS m/z 414 (M + H)⁺
   11-54; A-59; C-05; MS m/z 416 (M + H)⁺
   11-55; A-59; C-06; MS m/z 424 (M + H)⁺
   11-56; A-59; C-07; MS m/z 426 (M + H)⁺
   11-57; A-59; C-08; MS m/z 442 (M + H)⁺
   11-58; A-59; C-09; MS m/z 448 (M + H)⁺
   11-59; A-59; C-10; MS m/z 454 (M + H)⁺
   11-60; A-59; C-11; MS m/z 462 (M + H)⁺
   11-61; A-59; C-12; MS m/z 468 (M + H)⁺
   11-62; A-67; C-01; MS m/z 398 (M + H)⁺
   11-63; A-67; C-02; MS m/z 410 (M + H)⁺
   11-64; A-67; C-03; MS m/z 422 (M + H)⁺
   11-65; A-67; C-04; MS m/z 426 (M + H)⁺
   11-66; A-67; C-05; MS m/z 428 (M + H)⁺
   11-67; A-67; C-06; MS m/z 436 (M + H)⁺
   11-68; A-67; C-07; MS m/z 438 (M + H)⁺
   11-69; A-67; C-08; MS m/z 454 (M + H)⁺
   11-70; A-67; C-09; MS m/z 460 (M + H)⁺
   11-71; A-67; C-10; MS m/z 466 (M + H)⁺
   11-72; A-67; C-11; MS m/z 474 (M + H)⁺
   11-73; A-67; C-12; MS m/z 480 (M + H)⁺
   11-74; A-68; C-01; MS m/z 368 (M + H)⁺
   11-75; A-68; C-02; MS m/z 380 (M + H)⁺
   11-76; A-68; C-03; MS m/z 392 (M + H)⁺
   11-77; A-68; C-04; MS m/z 396 (M + H)⁺
   11-78; A-68; C-05; MS m/z 398 (M + H)⁺
   11-79; A-68; C-06; MS m/z 406 (M + H)⁺
   11-80; A-68; C-07; MS m/z 408 (M + H)⁺
   11-81; A-68; C-08; MS m/z 424 (M + H)⁺
   11-82; A-68; C-09; MS m/z 430 (M + H)⁺
   11-83; A-68; C-10; MS m/z 436 (M + H)⁺
   11-84; A-68; C-11; MS m/z 444 (M + H)⁺
   11-85; A-68; C-12; MS m/z 450 (M + H)⁺
   11-86; A-69; C-01; MS m/z 398 (M + H)⁺
   11-87; A-69; C-02; MS m/z 410 (M + H)⁺
   11-88; A-69; C-03; MS m/z 422 (M + H)⁺
   11-89; A-69; C-04; MS m/z 426 (M + H)⁺
   11-90; A-69; C-05; MS m/z 428 (M + H)⁺
   11-91; A-69; C-06; MS m/z 436 (M + H)⁺
   11-92; A-69; C-07; MS m/z 438 (M + H)⁺
   11-93; A-69; C-08; MS m/z 454 (M + H)⁺
   11-94; A-69; C-09; MS m/z 460 (M + H)⁺
   11-95; A-69; C-10; MS m/z 466 (M + H)⁺
   11-96; A-69; C-11; MS m/z 474 (M + H)⁺
   11-97; A-69; C-12; MS m/z 480 (M + H)⁺
(1) The examples of compounds described in : 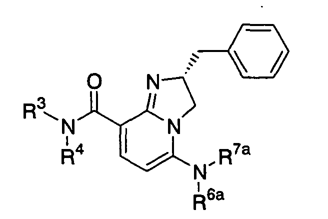 which are included in Compound (I), are listed below in order of compound number, -NR³R⁴, -NR^{6a}R^{7a} and spectrum data thereof.
   12-1; A-05; C-32; MS m/z 478 (M + H)⁺
   12-2; A-05; C-37; MS m/z 466 (M + H)⁺
   12-3; A-05; C-38; MS m/z 479 (M + H)⁺
   12-4; A-05; C-39; MS m/z 452 (M + H)⁺
   12-5; A-05; C-40; MS m/z 450 (M + H)⁺
   12-6; A-05; C-41; MS m/z 466 (M + H)⁺
   12-7; A-05; C-42; MS m/z 450 (M + H)⁺
   12-8; A-05; C-43; MS m/z 450 (M + H)⁺
   12-9; A-05; C-44; MS m/z 438 (M + H)⁺
   12-10; A-05; C-45; MS m/z 450 (M + H)⁺
   12-11; A-05; C-46; MS m/z 447 (M + H)⁺
   12-12; A-05; C-47; MS m/z 439 (M + H)⁺
   12-13; A-14; C-32; MS m/z 512 (M + H)⁺
   12-14; A-14; C-37; MS m/z 500 (M + H)⁺
   12-15; A-14; C-38; MS m/z 513 (M + H)⁺
   12-16; A-14; C-39; MS m/z 486 (M + H)⁺
   12-17; A-14; C-40; MS m/z 484 (M + H)⁺
   12-18; A-14; C-41; MS m/z 500 (M + H)⁺
   12-19; A-14; C-42; MS m/z 484 (M + H)⁺
   12-20; A-14; C-43; MS m/z 484 (M + H)⁺
   12-21; A-14; C-44; MS m/z 472 (M + H)⁺
   12-22; A-14; C-45; MS m/z 484 (M + H)⁺
   12-23; A-14; C-46; MS m/z 481 (M + H)⁺
   12-24; A-14; C-47; MS m/z 473 (M + H)⁺
   12-25; A-17; C-32; MS m/z 532 (M + H)⁺
   12-26; A-17; C-37; MS m/z 520 (M + H)⁺
   12-27; A-17; C-38; MS m/z 533 (M + H)⁺
   12-28; A-17; C-39; MS m/z 506 (M + H)⁺
   12-29; A-17; C-40; MS m/z 504 (M + H)⁺
   12-30; A-17; C-41; MS m/z 520 (M + H)⁺
   12-31; A-17; C-42; MS m/z 504 (M + H)⁺
   12-32; A-17; C-43; MS m/z 504 (M + H)⁺
   12-33; A-17; C-44; MS m/z 492 (M + H)⁺
   12-34; A-17; C-45; MS m/z 504 (M + H)⁺
   12-35; A-17; C-46; MS m/z 501 (M + H)⁺
   12-36; A-17; C-47; MS m/z 493 (M + H)⁺
   12-37; A-41; C-32; MS m/z 478 (M + H)⁺
   12-38; A-41; C-37; MS m/z 466 (M + H)⁺
   12-39; A-41; C-38; MS m/z 479 (M + H)⁺
   12-40; A-41; C-39; MS m/z 452 (M + H)⁺
   12-41; A-41; C-40; MS m/z 450 (M + H)⁺
   12-42; A-41; C-41; MS m/z 466 (M + H)⁺
   12-43; A-41; C-42; MS m/z 450 (M + H)⁺
   12-44; A-41; C-43; MS m/z 450 (M + H)⁺
   12-45; A-41; C-44; MS m/z 438 (M + H)⁺
   12-46; A-41; C-45; MS m/z 450 (M + H)⁺
   12-47; A-41; C-46; MS m/z 447 (M + H)⁺
   12-48; A-41; C-47; MS m/z 439 (M + H)⁺
   12-49; A-42; C-32; MS m/z 478 (M + H)⁺
   12-50; A-42; C-37; MS m/z 466 (M + H)⁺
   12-51; A-42; C-38; MS m/z 479 (M + H)⁺
   12-52; A-42; C-39; MS m/z 452 (M + H)⁺
   12-53; A-42; C-40; MS m/z 450 (M + H)⁺
   12-54; A-42; C-41; MS m/z 466 (M + H)⁺
   12-55; A-42; C-42; MS m/z 450 (M + H)⁺
   12-56; A-42; C-43; MS m/z 450 (M + H)⁺
   12-57; A-42; C-44; MS m/z 438 (M + H)⁺
   12-58; A-42; C-45; MS m/z 450 (M + H)⁺
   12-59; A-42; C-46; MS m/z 447 (M + H)⁺
   12-60; A-42; C-47; MS m/z 439 (M + H)⁺
   12-61; A-43; C-32; MS m/z 464 (M + H)⁺
   12-62; A-43; C-37; MS m/z 452 (M + H)⁺
   12-63; A-43; C-38; MS m/z 465 (M + H)⁺
   12-64; A-43; C-39; MS m/z 438 (M + H)⁺
   12-65; A-43; C-40; MS m/z 436 (M + H)⁺
   12-66; A-43; C-41; MS m/z 452 (M + H)⁺
   12-67; A-43; C-42; MS m/z 436 (M + H)⁺
   12-68; A-43; C-43; MS m/z 436 (M + H)⁺
   12-69; A-43; C-44; MS m/z 424 (M + H)⁺
   12-70; A-43; C-45; MS m/z 436 (M + H)⁺
   12-71; A-43; C-46; MS m/z 433 (M + H)⁺
   12-72; A-43; C-47; MS m/z 425 (M + H)⁺
   12-73; A-44; C-32; MS m/z 492 (M + H)⁺
   12-74; A-44; C-37; MS m/z 480 (M + H)⁺
   12-75; A-44; C-38; MS m/z 493 (M + H)⁺
   12-76; A-44; C-39; MS m/z 466 (M + H)⁺
   12-77; A-44; C-40; MS m/z 464 (M + H)⁺
   12-78; A-44; C-41; MS m/z 480 (M + H)⁺
   12-79; A-44; C-42; MS m/z 464 (M + H)⁺
   12-80; A-44; C-43; MS m/z 464 (M + H)⁺
   12-81; A-44; C-44; MS m/z 452 (M + H)⁺
   12-82; A-44; C-45; MS m/z 464 (M + H)⁺
   12-83; A-44; C-46; MS m/z 461 (M + H)⁺
   12-84; A-44; C-47; MS m/z 453 (M + H)⁺
   12-85; A-45; C-32
   12-86; A-45; C-37; MS m/z 466 (M + H)⁺
   12-87; A-45; C-38
   12-88; A-45; C-39; MS m/z 450 (M - H)⁺
   12-89; A-45; C-40; MS m/z 450 (M + H)⁺
   12-90; A-45; C-41
   12-91; A-45; C-42; MS m/z 450 (M + H)⁺
   12-92; A-45; C-43
   12-93; A-45; C-44
   12-94; A-45; C-45
   12-95; A-45; C-46
   12-96; A-45; C-47
   12-97; A-46; C-32
   12-98; A-46; C-37; MS m/z 476 (M + H)⁺
   12-99; A-46; C-38
   12-100; A-46; C-39; MS m/z 462 (M + H)⁺
   12-101; A-46; C-40; MS m/z 460 (M + H)⁺
   12-102; A-46; C-41
   12-103; A-46; C-42; MS m/z 460 (M + H)⁺
   12-104; A-46; C-43
   12-105; A-46; C-44; MS m/z 448 (M + H)⁺
   12-106; A-46; C-45
   12-107; A-46; C-46; MS m/z 457 (M + H)⁺
   12-108; A-46; C-47; MS m/z 449 (M + H)⁺
   12-109; A-47; C-32
   12-110; A-47; C-37; MS m/z 466 (M + H)⁺
   12-111; A-47; C-38
   12-112; A-47; C-39; MS m/z 452 (M + H)⁺
   12-113; A-47; C-40; MS m/z 450 (M + H)⁺
   12-114; A-47; C-41; MS m/z 466 (M + H)⁺
   12-115; A-47; C-42; MS m/z 450 (M + H)⁺
   12-116; A-47; C-43
   12-117; A-47; C-44; MS m/z 438 (M + H)⁺
   12-118; A-47; C-45; MS m/z 450 (M + H)⁺
   12-119; A-47; C-46; MS m/z 447 (M + H)⁺
   12-120; A-47; C-47; MS m/z 439 (M + H)⁺
   12-121; A-48; C-32; MS m/z 532 (M + H)⁺
   12-122; A-48; C-37; MS m/z 520 (M + H)⁺
   12-123; A-48; C-38; MS m/z 533 (M + H)⁺
   12-124; A-48; C-39; MS m/z 506 (M + H)⁺
   12-125; A-48; C-40; MS m/z 504 (M + H)⁺
   12-126; A-48; C-41; MS m/z 520 (M + H)⁺
   12-127; A-48; C-42; MS m/z 504 (M + H)⁺
   12-128; A-48; C-43; MS m/z 504 (M + H)⁺
   12-129; A-48; C-44; MS m/z 492 (M + H)⁺
   12-130; A-48; C-45; MS m/z 504 (M + H)⁺
   12-131; A-48; C-46; MS m/z 501 (M + H)⁺
   12-132; A-48; C-47; MS m/z 493 (M + H)⁺
   12-133; A-49; C-32; MS m/z 530 (M + H)⁺
   12-134; A-49; C-37; MS m/z 518 (M + H)⁺
   12-135; A-49; C-38; MS m/z 531 (M + H)⁺
   12-136; A-49; C-39; MS m/z 504 (M + H)⁺
   12-137; A-49; C-40; MS m/z 502 (M + H)⁺
   12-138; A-49; C-41; MS m/z 518 (M + H)⁺
   12-139; A-49; C-42; MS m/z 502 (M + H)⁺
   12-140; A-49; C-43; MS m/z 502 (M + H)⁺
   12-141; A-49; C-44; MS m/z 490 (M + H)⁺
   12-142; A-49; C-45; MS m/z 502 (M + H)⁺
   12-143; A-49; C-46; MS m/z 499 (M + H)⁺
   12-144; A-49; C-47; MS m/z 491 (M + H)⁺
   12-145; A-50; C-32; MS m/z 528 (M + H)⁺
   12-146; A-50; C-37; MS m/z 516 (M + H)⁺
   12-147; A-50; C-38; MS m/z 529 (M + H)⁺
   12-148; A-50; C-39; MS m/z 502 (M + H)⁺
   12-149; A-50; C-40; MS m/z 500 (M + H)⁺
   12-150; A-50; C-41; MS m/z 516 (M + H)⁺
   12-151; A-50; C-42; MS m/z 500 (M + H)⁺
   12-152; A-50; C-43; MS m/z 500 (M + H)⁺
   12-153; A-50; C-44; MS m/z 488 (M + H)⁺
   12-154; A-50; C-45; MS m/z 500 (M + H)⁺
   12-155; A-50; C-46; MS m/z 497 (M + H)⁺
   12-156; A-50; C-47; MS m/z 489 (M + H)⁺
   12-157; A-51; C-32; MS m/z 546 (M + H)⁺
   12-158; A-51; C-37; MS m/z 534 (M + H)⁺
   12-159; A-51; C-38; MS m/z 547 (M + H)⁺
   12-160; A-51; C-39; MS m/z 520 (M + H)⁺
   12-161; A-51; C-40; MS m/z 518 (M + H)⁺
   12-162; A-51; C-41; MS m/z 534 (M + H)⁺
   12-163; A-51; C-42; MS m/z 518 (M + H)⁺
   12-164; A-51; C-43; MS m/z 518 (M + H)⁺
   12-165; A-51; C-44; MS m/z 506 (M + H)⁺
   12-166; A-51; C-45; MS m/z 518 (M + H)⁺
   12-167; A-51; C-46; MS m/z 515 (M + H)⁺
   12-168; A-51; C-47; MS m/z 507 (M + H)⁺
   12-169; A-52; C-32; MS m/z 534 (M + H)⁺
   12-170; A-52; C-37; MS m/z 522 (M + H)⁺
   12-171; A-52; C-38; MS m/z 535 (M + H)⁺
   12-172; A-52; C-39; MS m/z 508 (M + H)⁺
   12-173; A-52; C-40; MS m/z 506 (M + H)⁺
   12-174; A-52; C-41; MS m/z 522 (M + H)⁺
   12-175; A-52; C-42; MS m/z 506 (M + H)⁺
   12-176; A-52; C-43; MS m/z 506 (M + H)⁺
   12-177; A-52; C-44; MS m/z 494 (M + H)⁺
   12-178; A-52; C-45; MS m/z 506 (M + H)⁺
   12-179; A-52; C-46; MS m/z 503 (M + H)⁺
   12-180; A-52; C-47; MS m/z 495 (M + H)⁺
   12-181; A-53; C-32; MS m/z 516 (M + H)⁺
   12-182; A-53; C-37; MS m/z 504 (M + H)⁺
   12-183; A-53; C-38; MS m/z 517 (M + H)⁺
   12-184; A-53; C-39; MS m/z 490 (M + H)⁺
   12-185; A-53; C-40; MS m/z 488 (M + H)⁺
   12-186; A-53; C-41; MS m/z 504 (M + H)⁺
   12-187; A-53; C-42; MS m/z 488 (M + H)⁺
   12-188; A-53; C-43; MS m/z 488 (M + H)⁺
   12-189; A-53; C-44; MS m/z 476 (M + H)⁺
   12-190; A-53; C-45; MS m/z 488 (M + H)⁺
   12-191; A-53; C-46; MS m/z 485 (M + H)⁺
   12-192; A-53; C-47; MS m/z 477 (M + H)⁺
(m) The examples of compounds described in : 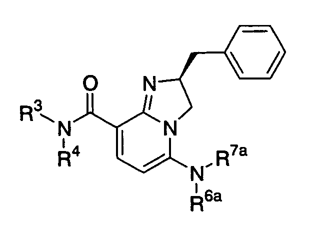 which are included in Compound (I), are listed below in order of compound number, -NR³R⁴, -NR^{6a}R^{7a} and spectrum data thereof.
   13-1; A-05; C-32; MS m/z 478 (M + H)⁺
   13-2; A-05; C-37; MS m/z 466 (M + H)⁺
   13-3; A-05; C-38; MS m/z 479 (M + H)⁺
   13-4; A-05; C-39; MS m/z 452 (M + H)⁺
   13-5; A-05; C-40; MS m/z 450 (M + H)⁺
   13-6; A-05; C-41; MS m/z 466 (M + H)⁺
   13-7; A-05; C-42; MS m/z 450 (M + H)⁺
   13-8; A-05; C-43; MS m/z 450 (M + H)⁺
   13-9; A-05; C-44; MS m/z 438 (M + H)⁺
   13-10; A-05; C-45; MS m/z 450 (M + H)⁺
   13-11; A-05; C-46; MS m/z 447 (M + H)⁺
   13-12; A-05; C-47; MS m/z 439 (M + H)⁺
   13-13; A-14; C-32; MS m/z 512 (M + H)⁺
   13-14; A-14; C-37; MS m/z 500 (M + H)⁺
   13-15; A-14; C-38; MS m/z 513 (M + H)⁺
   13-16; A-14; C-39; MS m/z 486 (M + H)⁺
   13-17; A-14; C-40; MS m/z 484 (M + H)⁺
   13-18; A-14; C-41; MS m/z 500 (M + H)⁺
   13-19; A-14; C-42; MS m/z 484 (M + H)⁺
   13-20; A-14; C-43; MS m/z 484 (M + H)⁺
   13-21; A-14; C-44; MS m/z 472 (M + H)⁺
   13-22; A-14; C-45; MS m/z 484 (M + H)⁺
   13-23; A-14; C-46; MS m/z 481 (M + H)⁺
   13-24; A-14; C-47; MS m/z 473 (M + H)⁺
   13-25; A-17; C-32; MS m/z 532 (M + H)⁺
   13-26; A-17; C-37; MS m/z 520 (M + H)⁺
   13-27; A-17; C-38; MS m/z 533 (M + H)⁺
   13-28; A-17; C-39; MS m/z 506 (M + H)⁺
   13-29; A-17; C-40; MS m/z 504 (M + H)⁺
   13-30; A-17; C-41; MS m/z 520 (M + H)⁺
   13-31; A-17; C-42; MS m/z 504 (M + H)⁺
   13-32; A-17; C-43; MS m/z 504 (M + H)⁺
   13-33; A-17; C-44; MS m/z 492 (M + H)⁺
   13-34; A-17; C-45; MS m/z 504 (M + H)⁺
   13-35; A-17; C-46; MS m/z 501 (M + H)⁺
   13-36; A-17; C-47; MS m/z 493 (M + H)⁺
   13-37; A-41; C-32; MS m/z 478 (M + H)⁺
   13-38; A-41; C-37; MS m/z 466 (M + H)⁺
   13-39; A-41; C-38; MS m/z 479 (M + H)⁺
   13-40; A-41; C-39; MS m/z 452 (M + H)⁺
   13-41; A-41; C-40; MS m/z 450 (M + H)⁺
   13-42; A-41; C-41; MS m/z 466 (M + H)⁺
   13-43; A-41; C-42; MS m/z 450 (M + H)⁺
   13-44; A-41; C-43; MS m/z 450 (M + H)⁺
   13-45; A-41; C-44; MS m/z 438 (M + H)⁺
   13-46; A-41; C-45; MS m/z 450 (M + H)⁺
   13-47; A-41; C-46; MS m/z 447 (M + H)⁺
   13-48; A-41; C-47; MS m/z 439 (M + H)⁺
   13-49; A-42; C-32; MS m/z 478 (M + H)⁺
   13-50; A-42; C-37; MS m/z 466 (M + H)⁺
   13-51; A-42; C-38; MS m/z 479 (M + H)⁺
   13-52; A-42; C-39; MS m/z 452 (M + H)⁺
   13-53; A-42; C-40; MS m/z 450 (M + H)⁺
   13-54; A-42; C-41; MS m/z 466 (M + H)⁺
   13-55; A-42; C-42; MS m/z 450 (M + H)⁺
   13-56; A-42; C-43; MS m/z 450 (M + H)⁺
   13-57; A-42; C-44; MS m/z 438 (M + H)⁺
   13-58; A-42; C-45; MS m/z 450 (M + H)⁺
   13-59; A-42; C-46; MS m/z 447 (M + H)⁺
   13-60; A-42; C-47; MS m/z 439 (M + H)⁺
   13-61; A-43; C-32; MS m/z 464 (M + H)⁺
   13-62; A-43; C-37; MS m/z 452 (M + H)⁺
   13-63; A-43; C-38; MS m/z 465 (M + H)⁺
   13-64; A-43; C-39; MS m/z 438 (M + H)⁺
   13-65; A-43; C-40; MS m/z 436 (M + H)⁺
   13-66; A-43; C-41; MS m/z 452 (M + H)⁺
   13-67; A-43; C-42; MS m/z 436 (M + H)⁺
   13-68; A-43; C-43; MS m/z 436 (M + H)⁺
   13-69; A-43; C-44; MS m/z 424 (M + H)⁺
   13-70; A-43; C-45; MS m/z 436 (M + H)⁺
   13-71; A-43; C-46; MS m/z 433 (M + H)⁺
   13-72; A-43; C-47; MS m/z 425 (M + H)⁺
   13-73; A-44; C-32; MS m/z 492 (M + H)⁺
   13-74; A-44; C-37; MS m/z 480 (M + H)⁺
   13-75; A-44; C-38; MS m/z 493 (M + H)⁺
   13-76; A-44; C-39; MS m/z 466 (M + H)⁺
   13-77; A-44; C-40; MS m/z 464 (M + H)⁺
   13-78; A-44; C-41; MS m/z 480 (M + H)⁺
   13-79; A-44; C-42; MS m/z 464 (M + H)⁺
   13-80; A-44; C-43; MS m/z 464 (M + H)⁺
   13-81; A-44; C-44; MS m/z 452 (M + H)⁺
   13-82; A-44; C-45; MS m/z 464 (M + H)⁺
   13-83; A-44; C-46; MS m/z 461 (M + H)⁺
   13-84; A-44; C-47; MS m/z 453 (M + H)⁺
   13-85; A-45; C-32
   13-86; A-45; C-37; MS m/z 466 (M + H)⁺
   13-87; A-45; C-38; MS m/z 477 (M - H)⁺
   13-88; A-45; C-39
   13-89; A-45; C-40
   13-90; A-45; C-41
   13-91; A-45; C-42; MS m/z 450 (M + H)⁺
   13-92; A-45; C-43
   13-93; A-45; C-44
   13-94; A-45; C-45
   13-95; A-45; C-46
   13-96; A-45; C-47
   13-97; A-46; C-32
   13-98; A-46; C-37; MS m/z 476 (M + H)⁺
   13-99; A-46; C-38
   13-100; A-46; C-39
   13-101; A-46; C-40; MS m/z 460 (M + H)⁺
   13-102; A-46; C-41
   13-103; A-46; C-42; MS m/z 460 (M + H)⁺
   13-104; A-46; C-43; MS m/z 460 (M + H)⁺
   13-105; A-46; C-44; MS m/z 448 (M + H)⁺
   13-106; A-46; C-45
   13-107; A-46; C-46
   13-108; A-46; C-47; MS m/z 449 (M + H)⁺
   13-109; A-47; C-32
   13-110; A-47; C-37; MS m/z 466 (M + H)⁺
   13-111; A-47; C-38
   13-112; A-47; C-39
   13-113; A-47; C-40; MS m/z 450 (M + H)⁺
   13-114; A-47; C-41
   13-115; A-47; C-42; MS m/z 450 (M + H)⁺
   13-116; A-47; C-43
   13-117; A-47; C-44
   13-118; A-47; C-45; MS m/z 450 (M + H)⁺
   13-119; A-47; C-46
   13-120; A-47; C-47
   13-121; A-48; C-32; MS m/z 532 (M + H)⁺
   13-122; A-48; C-37; MS m/z 520 (M + H)⁺
   13-123; A-48; C-38; MS m/z 533 (M + H)⁺
   13-124; A-48; C-39; MS m/z 506 (M + H)⁺
   13-125; A-48; C-40; MS m/z 504 (M + H)⁺
   13-126; A-48; C-41; MS m/z 520 (M + H)⁺
   13-127; A-48; C-42; MS m/z 504 (M + H)⁺
   13-128; A-48; C-43; MS m/z 504 (M + H)⁺
   13-129; A-48; C-44; MS m/z 492 (M + H)⁺
   13-130; A-48; C-45; MS m/z 504 (M + H)⁺
   13-131; A-48; C-46; MS m/z 501 (M + H)⁺
   13-132; A-48; C-47; MS m/z 493 (M + H)⁺
   13-133; A-49; C-32; MS m/z 530 (M + H)⁺
   13-134; A-49; C-37; MS m/z 518 (M + H)⁺
   13-135; A-49; C-38; MS m/z 531 (M + H)⁺
   13-136; A-49; C-39; MS m/z 504 (M + H)⁺
   13-137; A-49; C-40; MS m/z 502 (M + H)⁺
   13-138; A-49; C-41; MS m/z 518 (M + H)⁺
   13-139; A-49; C-42; MS m/z 502 (M + H)⁺
   13-140; A-49; C-43; MS m/z 502 (M + H)⁺
   13-141; A-49; C-44; MS m/z 490 (M + H)⁺
   13-142; A-49; C-45; MS m/z 502 (M + H)⁺
   13-143; A-49; C-46; MS m/z 499 (M + H)⁺
   13-144; A-49; C-47; MS m/z 491 (M + H)⁺
   13-145; A-50; C-32; MS m/z 528 (M + H)⁺
   13-146; A-50; C-37; MS m/z 516 (M + H)⁺
   13-147; A-50; C-38; MS m/z 529 (M + H)⁺
   13-148; A-50; C-39; MS m/z 502 (M + H)⁺
   13-149; A-50; C-40; MS m/z 500 (M + H)⁺
   13-150; A-50; C-41; MS m/z 516 (M + H)⁺
   13-151; A-50; C-42; MS m/z 500 (M + H)⁺
   13-152; A-50; C-43; MS m/z 500 (M + H)⁺
   13-153; A-50; C-44; MS m/z 488 (M + H)⁺
   13-154; A-50; C-45; MS m/z 500 (M + H)⁺
   13-155; A-50; C-46; MS m/z 497 (M + H)⁺
   13-156; A-50; C-47; MS m/z 489 (M + H)⁺
   13-157; A-51; C-32; MS m/z 546 (M + H)⁺
   13-158; A-51; C-37; MS m/z 534 (M + H)⁺
   13-159; A-51; C-38; MS m/z 547 (M + H)⁺
   13-160; A-51; C-39; MS m/z 520 (M + H)⁺
   13-161; A-51; C-40; MS m/z 518 (M + H)⁺
   13-162; A-51; C-41; MS m/z 534 (M + H)⁺
   13-163; A-51; C-42; MS m/z 518 (M + H)⁺
   13-164; A-51; C-43; MS m/z 518 (M + H)⁺
   13-165; A-51; C-44; MS m/z 506 (M + H)⁺
   13-166; A-51; C-45; MS m/z 518 (M + H)⁺
   13-167; A-51; C-46; MS m/z 515 (M + H)⁺
   13-168; A-51; C-47; MS m/z 507 (M + H)⁺
   13-169; A-52; C-32; MS m/z 534 (M + H)⁺
   13-170; A-52; C-37; MS m/z 522 (M + H)⁺
   13-171; A-52; C-38; MS m/z 535 (M + H)⁺
   13-172; A-52; C-39; MS m/z 508 (M + H)⁺
   13-173; A-52; C-40; MS m/z 506 (M + H)⁺
   13-174; A-52; C-41; MS m/z 522 (M + H)⁺
   13-175; A-52; C-42; MS m/z 506 (M + H)⁺
   13-176; A-52; C-43; MS m/z 506 (M + H)⁺
   13-177; A-52; C-44; MS m/z 494 (M + H)⁺
   13-178; A-52; C-45; MS m/z 506 (M + H)⁺
   13-179; A-52; C-46; MS m/z 503 (M + H)⁺
   13-180; A-52; C-47; MS m/z 495 (M + H)⁺
   13-181; A-53; C-32; MS m/z 516 (M + H)⁺
   13-182; A-53; C-37; MS m/z 504 (M + H)⁺
   13-183; A-53; C-38; MS m/z 517 (M + H)⁺
   13-184; A-53; C-39; MS m/z 490 (M + H)⁺
   13-185; A-53; C-40; MS m/z 488 (M + H)⁺
   13-186; A-53; C-41; MS m/z 504 (M + H)⁺
   13-187; A-53; C-42; MS m/z 488 (M + H)⁺
   13-188; A-53; C-43; MS m/z 488 (M + H)⁺
   13-189; A-53; C-44; MS m/z 476 (M + H)⁺
   13-190; A-53; C-45; MS m/z 488 (M + H)⁺
   13-191; A-53; C-46; MS m/z 485 (M + H)⁺
   13-192; A-53; C-47; MS m/z 477 (M + H)⁺

The use of Compound (I) of the present invention is not limited to an active ingredient of a medicament but can also be used as an active ingredient of other medicaments or as an intermediate in the manufacture of other compounds. Other applications like these are included in the use of Compound (I) as well. Examples of the Compound (I) which encompass the present invention include any salt including pharmaceutically acceptable salts described above, in addition to any hydrate or solvate. Any solvent can be used, so long as it forms solvate. Examples of the solvent that forms solvate include ethanol, tetrahydrofuran, dioxane and the like, although there is no limitation thereto. When one or more asymmetric carbon atom exists in the structure of Compound (I), pure isomers such as optical isomers and diastereoisomers, mixtures of isomers, and racemic mixtures are included in the present invention.

The pharmacological activity of Compound (I) will now be described.

### Test Example 1: Insulin secretion promoting activity for cultured β-cells

The established pancreas β-cell, MIN6 cell, reported by Miyazaki *et al.* (Endocrinology, vol.127, p.126-131, 1990), exhibits insulin content and insulin secretion amount by stimulation with glucose similar to those of pancreas β-cells in living bodies, and well preserves characteristics of pancreas β-cells in living bodies from a view point that it shows increase of insulin secretion in a glucose concentration-dependent manner (the above reference, and Diabetologia, vol.36, p.1139-1145, 1993). Further, the insulin secretion of the MIN6 cell is prompted in response to sulfonylurea-agents such as glibenclamide, which are used as a medicament for treatment of diabetes (Cellular Signalling, vol.5, p.777-786, 1993).

Culture of the MIN6 cells above and insulin secretion test utilizing the MIN6 cells were performed according to the methods described in Diabetologia, vol.36, p.1139-1145, 1993. The effect of a compound on the insulin secretion in the presence of 14.5 mmol/L glucose was determined by measuring insulin amounts in cell culture supernatants collected as follows. MIN6 cells cultured on a 24-well plate were washed twice by using 1 mL of Buffer A (119 mmol/L sodium chloride, 4.74 mmol/L potassium chloride, 2.54 mmol/L calcium chloride, 1.19 mmol/L magnesium sulfate, 1.19 mmol/L potassium dihydrogenphosphate, 10 mmol/L 2-[4-(2-hydroxyethyl)-1-piperazinyl]ethanesulfonic acid, and 0.1% bovine serum albumin, pH 7.3) containing 2 mmol/L glucose, and then were incubated in 1 mL of Buffer A containing 2 mmol/L glucose at 37°C for 45 minutes. After the incubation, the culture supernatant was changed to Buffer A (0.9 mL) containing a test compound at various concentrations and 2 mmol/L glucose, and the cells were further incubated at 37°C for 15 minutes. The MIN6 cells were stimulated with glucose by the addition of Buffer A (0.1 mL) containing 127 mmol/L glucose to the culture (final glucose concentration: 14.5 mmol/L). After the stimulation, the cells were further incubated at 37°C for 45 minutes, and then the culture supernatant was collected.

Separately, the effect of a compound on the insulin secretion in the presence of 5 mmol/L glucose was determined by measuring insulin amounts in cell culture supernatants collected as follows. MIN6 cells cultured on a 24-well plate were washed twice by using 1mL of Buffer A containing 5 mmol/L glucose, and then the culture supernatant was changed to Buffer A (0.9 mL) containing a test compound at various concentrations and 5 mmol/L glucose. Then, the cells were incubated at 37°C for 45 minutes (final glucose concentration: 5 mmol/L), and the culture supernatant was collected.

After the culture supernatant was diluted with a phosphate buffer containing 1% bovine serum albumin, 0.1% Tween 20, 0.12% disodium ethylenediaminetetraacetate (EDTA), and 0.1% sodium azide, antibody-reactive insulin secreted in the culture supernatant was qualified by enzyme immunoassay or radio immunoassay. The insulin level was indicated as the amount of human insulin (ng/mL). The results are indicated as average (avg) of 3 to 4 samples with standard error values (se).

The results are shown in Table 4.

**Table 4 (1)**

| (In the presence of 14.5 mmol/L of glucose) | | | |
|---|---|---|---|
| Compound No. | Drug Concentration | Insulin secretion amount(ng/mL) | |
| | (µ mol/L) | Avg | se |
| none | - | 148.4 | 4.8 |
| 1-447 | 1 | 174 | 5.4 |
| 1-482 | 1 | 170.7 | 4.2 |
| 1-484 | 1 | 173.5 | 8.8 |
| 1-485 | 1 | 172 | 1.6 |
| 1-486 | 1 | 176.6 | 3.2 |
| 1-487 | 1 | 185.1 | 5.3 |
| 1-1259 | 1 | 171 | 4.1 |
| 1-1279 | 1 | 171.8 | 2.9 |
| 1-1490 | 1 | 184 | 16.2 |
| 1-1494 | 1 | 202 | 3.4 |
| 1-1496 | 1 | 173.5 | 5 |
| 1-1477 | 1 | 191.1 | 5 |
| 1-1506 | 1 | 170.5 | 5 |
| 1-1515 | 1 | 172.6 | 6 |
| 1-1516 | 1 | 176.8 | 8.4 |
| 2-41 | 1 | 188.5 | 6.9 |
| 2-42 | 1 | 179.6 | 1.6 |
| 2-45 | 1 | 177.7 | 6.5 |
| 7-175 | 1 | 182.8 | 8.1 |
| 7-183 | 1 | 171.3 | 4.7 |
| 7-185 | 1 | 172.4 | 8.5 |
| 7-187 | 1 | 175.5 | 1.8 |
| 7-188 | 1 | 182.4 | 5.4 |
| 7-258 | 1 | 171.1 | 6.6 |
| 7-523 | 1 | 173.3 | 15.8 |
| 7-630 | 1 | 193.5 | 17 |
| 7-635 | 1 | 187.1 | 0.7 |
| 7-640 | 1 | 188.3 | 9.1 |
| 7-641 | 1 | 176.6 | 10.8 |
| 7-642 | 1 | 192 | 11.8 |
| 7-713 | 1 | 196 | 8.2 |
| 7-785 | 1 | 198 | 2.7 |
| 7-892 | 1 | 176.9 | 9 |
| 7-893 | 1 | 193.7 | 12.7 |
| 7-1223 | 1 | 172.4 | 3.5 |

**Table 4 (2)**

| (In the presence of 14.5 mmol/L of glucose) | | | |
|---|---|---|---|
| Compound No. | Drug Concentration | Insulin secretion amount (ng/mL) | |
| | (µ mol/L) | avg | se |
| 7-1270 | 1 | 177.2 | 4.1 |
| 7-1275 | 1 | 180 | 10.6 |
| 7-1277 | 1 | 179.6 | 6.7 |
| 7-1278 | 1 | 192.7 | 12.2 |
| 7-1281 | 1 | 182.4 | 2.7 |
| 7-1290 | 1 | 205.3 | 10 |
| 7-1454 | 1 | 174.7 | 9.8 |
| 7-1374 | 1 | 193.8 | 2 |
| 7-1389 | 1 | 194 | 6.1 |
| 7-1397 | 1 | 219 | 7.2 |
| 7-1398 | 1 | 164.5 | 14 |
| 7-1410 | 1 | 206.9 | 3.9 |
| 11-60 | 1 | 175.9 | 2.3 |
| 11-70 | 1 | 176.7 | 3.2 |
| AY4166 | 10 | 195.1 | 4.3 |
| Glibenclamide | 0.1 | 177.8 | 3.3 |

**Table 4 (3)**

| (In the presence of 5 mmol/L of glucose) | | | |
|---|---|---|---|
| Compound No. | Drug Concentration | Insulin secretion amount(ng/mL) | |
| | (µ mol/L) | Avg | se |
| None | - | 51.2 | 11.3 |
| 7-640 | 10 | 138 | 4.3 |
| 7-1290 | 10 | 88.5 | 2.8 |
| 7-1398 | 10 | 118.2 | 5.9 |
| AY-4166 | 10 | 170.8 | 4.2 |
| Glibenclamide | 0.1 | 156.8 | 8.4 |

As shown in Table 4, it was revealed that the compounds of the present invention had insulin secretion action. Whilst as shown in Table 4, in the presence of glucose at a low concentration (5 mmol/L), these compounds did not show marked secretion promoting action even at a 10 times higher concentration. Glibenclamide (Pharmacotherapy, vol.5, p.43, 1985) and AY-4166 (Yakuri to Rinsho, vol.7, p.121, 1997), used as controls for comparison showed marked secretion promoting action even at a low glucose concentration.

Test Example 2: Hyperglycemia suppressing action after glucose loading in normal rats

Wistar male rats (body weight: about 280 g) were used for the experiment after starvation for 24 hours. A test compound was orally administered to the rats 30 minutes before oral administration of glucose (2 g/kg). Blood was collected from a tail vein before the administration of the test compound, and at 30, 60, 120, and 180 minutes after the glucose loading. Blood glucose level was measured by using a simplified blood sugar level measuring apparatus.

The results are shown in Table 5.

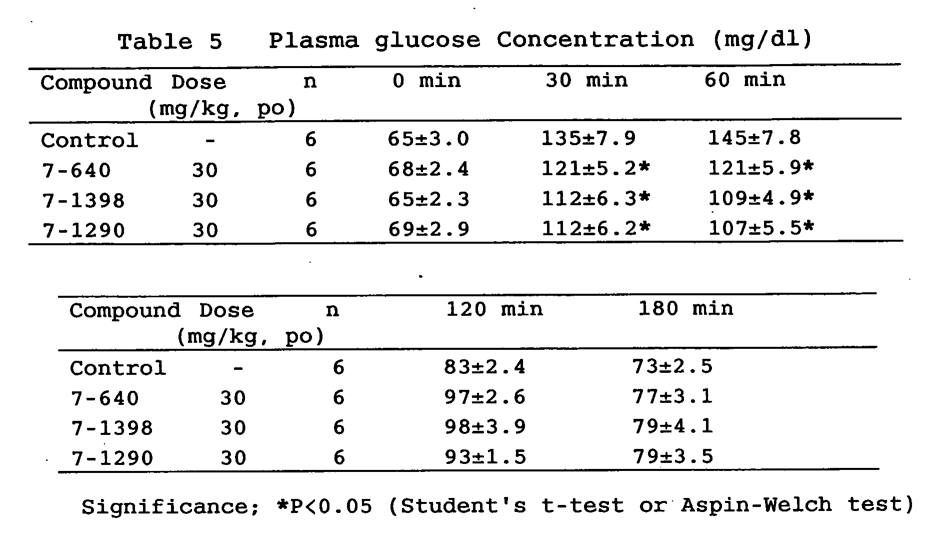

Every compound was used in the form of trihydrochloride in the experiment.

As clearly shown in Table 5, the compound of the present invention exhibited hyperglycemia suppressing action at 30 minutes and 60 minutes after the glucose loading.

However, the compound exhibited no hypoglycemic action at 120 minutes and 180 minutes after the glucose loading.

Compound (I) or a pharmaceutically acceptable salt thereof has insulin secretion promoting action in cultured β-cells and shows hypoglycemic action in rats, and accordingly, Compound (I) or a pharmaceutically acceptable salt thereof is useful as an active ingredient of a medicament for preventing and/or treating diabetes. Further, Compound (I) or a pharmaceutically acceptable salt thereof is also useful as an active ingredient of a medicament for preventing and/or treating various complications of diabetes, for example, retinopathy, nephropathy, neuropathy or the like. As the active ingredient of these medicaments, one or more compounds selected from the group consisting of Compound (I) and pharmaceutically acceptable salts thereof, as well as hydrates thereof and solvates thereof can be used. Although the aforementioned substances, per se, can also be administered, it is generally desirable to provide the medicament in a form of a pharmaceutical composition comprising the aforementioned substance as the active ingredient and one or more additives for pharmaceutical preparations. These medicaments can be administered to humans and mammals other than humans.

The form of the pharmaceutical composition is not particularly limited and an appropriate form most suitable for a purpose of therapeutic or preventive treatment can be selected from forms of pharmaceutical preparations for oral or parenteral administration. Examples of pharmaceutical preparations suitable for oral administration include, for example, tablets and the like. Examples of pharmaceutical preparations suitable for parenteral administration include, for example, injections and the like. However, the preparations are not limited to these examples.

For the preparation of solid preparations such as tablets, excipients such as lactose, mannitol and the like, disintegrating agents such as starch and the like, lubricants such as magnesium stearate and the like, binders such as hydroxypropylcellulose and the like, surface active agents such as fatty acid esters and the like, and plasticizers such as glycerin and the like may be used.

Pharmaceutical preparations for injection, which are suitable for parenteral administration, contain the aforementioned substance as the active ingredient preferably in a sterilized aqueous medium isotonic with blood of a recipient in a dissolved or suspended state. For example, as for injections, a solution can be prepared by using an aqueous medium consisting of saline, a glucose solution, a mixture of saline and a glucose solution, and the like. To these pharmaceutical preparations for parenteral administration, one or more auxiliary ingredients selected from glycols, oils, flavors, preservatives, excipients, disintegrating agents, lubricants, binders, surface active agents, plasticizers, and the like may also be added.

Dose and frequency of administration of Compound (I) may preferably be increased or decreased depending on various factors such as type and severity of diseases, dosage form, conditions of a patient such as age and body weight, and presence or absence of complications. In general, Compound (I) may preferably be administered in an amount of 1 to 1,000 mg/kg per day for an adult dividedly as 3 or 4 times of administrations.

### Best Mode for Carrying Out the Invention

The present invention will be more specifically explained with reference to the following Examples. However, the scope of the present invention is not limited to the following Examples. Compound numbers in the following Examples correspond to the aforementioned compound numbers defined in (a) to (m) as preferable compounds.

Physicochemical data of each compound in following Examples and Reference Examples were measured by following apparatuses:
¹H-NMR: JEOL JNM-EX270 (270 MHz) or JEOL JNM-GX270 (270 MHz)
MS: Micromass LCT or Micromass Quatro (measured by an APCI method or an ESI method)

### Example 1

Compounds shown in above (a) to (e) were synthesized as follows:

### Step 1: Synthesis of Compound (VIII)

To a mixture of Compound (VI) (0.5 mL, 0.1 mol/L chloroform suspension, 0.05 mmol) obtained in Reference Examples 6 to 9 and 1-hydroxybenzotriazole (0.2 mL, 0.25 mol/L solution in chloroform : tetrahydrofuran = 3:1 mixture, 0.05 mmol) was added primary or secondary amine [Compound (VII)] (0.065 mL, 1 mol/L chloroform solution, 0.065 mmol) corresponding to R³R⁴N moiety. Polymer-bound *N*-ethyl-*N*'-[3-(dimethylamino)propyl]carbodiimide (70 mg, 0.1 mmol) was then added and the mixture was stirred at 55°C overnight. The resulting reaction solution was filtered and then concentrated. Before filtering the solution, if necessary, methanol or tetrahydrofuran was added to dissolve the precipitates. To the resulting residue, ethanol (0.6 mL) and Bio-Rad AG1-X8 OH-form resin (90 mg, 0.18 mmol) were added and the mixture was stirred at 70°C for 2 hours. Resin obtained by removing the solution was washed with methanol (0.3 ml × 3). The resulting resin was then suspended in a chloroform-methanol mixture (1:1, 0.5 mL), followed with addition of 4 mol/L solution of hydrogen chloride in ethyl acetate (0.1 mL) and stirred at room temperature for 2 hours. The resulting reaction solution was filtered and concentrated. The resulting residue was dissolved in chloroform (0.5 mL), and to the mixture was added 4-polyvinylpyridine (23 mg, 0.22 mmol) or Bio-Rad AG1-X8 OH⁻ form resin (90 mg, 0.18 mmol) and was stirred at room temperature overnight. The reaction solution was filtered and concentrated to obtain Compound (VIII).

### Step 2: Synthesis of Compound (I-A)

Compound (VIII) obtained in Step 1 was dissolved in tetrahydrofuran (0.2 mL). To the solution were added triphenylphosphine (0.08 mL, 1 mol/L tetrahydrofuran solution, 0.08 mmol) and alcohol [Compound (IX)] (0.08 mL, 1 mol/L tetrahydrofuran solution, 0.08 mmol) corresponding to R⁵ moiety, then diethyl azodicarboxylate (0.08 mL, 1 mol/L tetrahydrofuran solution, 0.08 mmol) was added and the mixture was stirred at room temperature overnight. The resulting reaction solution was passed through a Bondesil SCX column (Varian Inc.) and then eluted impurities with a chloroform-methanol mixture (50% v/v, 2.2 mL). Further, the target compound was eluted by 2 mol/L ammonia-methanol solution (1.6 mL) and the resulting solution was concentrated to obtain Compound (I-A) with yield of 30 to 40% in total.

The resulting compounds were identified by mass spectrometry. The structures and analytical results of each compound are the same as aforementioned (a) to (e).

The proton nuclear magnetic resonance spectra of typical compounds are shown below:

### Compound 1-447

¹H-NMR (CDCl₃) δ (ppm) (main peaks): 0.94 (t, *J* = 7.4 Hz, 3H), 1.6-1.8 (m, 2H), 2.5-3.1 (m, 4H), 3.5-3.7 (m, 6H), 4.4-4.6 (m, 1H), 7.1-7.3 (m, 10H).

### Compound 1-1259

¹H-NMR (CDCl₃) δ (ppm): 0.95 (d, *J* = 6.1 Hz, 3H), 1.5-1.7 (m, 5H), 2.8-2.95 (m, 2H), 3.25-3.35 (m, 2H), 3.7-3.85 (m, 2H), 3.85-4.0 (m, 2H), 5.1-5.25 (m, 1H), 5.25-5.45 (m, 2H), 7.2-7.4 (m, 9H), 7.4-7.5 (br d, 1H).

### Compound 1-1279

¹H-NMR (CDCl₃) δ (ppm): 0.96 (d, *J* = 6.3 Hz, 3H), 1.5-1.75 (m, 8H), 1.68 (s, 3H), 1.75 (s, 3H), 2.1-2.2 (br s, 4H), 3.1-3.25 (m, 2H), 3.4-3.55 (m, 2H), 4.0-4.2 (m, 3H), 4.4-4.5 (m, 2H), 4.8-4.95 (m, 1H), 4.95-5.1 (m, 1H), 5.1-5.2 (m, 1H), 7.2-7.4 (m, 5H), 7.78 (s, 1H).

### Compound 2-1

¹H-NMR (CDCl₃) δ (ppm): 0.93 (d, *J* = 6.6 Hz, 3H), 0.94 (d, *J* = 6.6 Hz, 6H), 1.1-1.25 (m, 1H), 1.25-1.4 (m, 1H), 1.4-1.5 (m, 4H), 1.5-1.7 (m, 2H), 1.60 (s, 3H), 1.67 (s, 3H), 1.9-2.05 (m, 2H), 2.82 (dd, *J* = 7.1., 13.7 Hz, 1H), 3.02 (dd, *J* = 6.7, 13.7 Hz, 1H), 3.25-3.55 (m, 2H), 3.6-3.8 (m, 3H), 3.90 (t, *J* = 10.8 Hz, 1H), 4.45-4.6 (m, 1H), 5.0-5.1 (m, 1H), 7.15-7.35 (m, 5H), 8.0 (br d, 1H), 9.20 (br s, 1H);

### Compound 2-41

¹H-NMR (CDCl₃) δ (ppm): 0.3-0.4 (m, 2H), 0.55-0.65 (m, 2H), 1.05-1.2 (m, 1H), 1.45-1.7 (m, 10H), 1.8-2.0 (m, 2H), 2.85 (dd, *J* = 6.6, 13.6 Hz, 1H), 2.98 (dd, *J* = 7.1, 13.6 Hz, 1H), 3.5-3.7 (m, 3H), 3.85-4.0 (m, 1H), 4.0-4.15'(m, 1H), 4.45-4.6 (m, 1H), 7.15-7.35 (m, 5H), 7.98 (s, 1H), 9.2-9.35 (m, 1H).

### Compound 2-42

¹H-NMR (CDCl₃) δ (ppm): 1.45-1.65 (m, 10H), 1.75 (s, 6H), 1.8-2.0 (m, 2H), 2.84 (dd, *J* = 6.6, 13.5 Hz, 1H), 2.96 (dd, *J* = 6.6, 13.5 Hz, 1H), 3.55-3.7 (m, 1H), 3.85-3.95 (m, 1H), 4.0-4.15 (m, 1H), 4.28 (d, *J* = 7.3 Hz, 2H), 4.45-4.6 (m, 1H), 5.15-5.25 (m, 1H), 7.2-7.35 (m, 5H), 7.88 (s, 1H), 9.26 (br d, *J* = 7.3 Hz, 1H).

### Compound 2-45

¹H-NMR (CDCl₃) δ (ppm): 1.35-1.7 (m, 10H), 1.75-2.0 (m, 4H), 2.5-2.65 (m, 1H), 2.80 (dd, *J* = 6.6, 13.5 Hz, 1H), 2.91 (dd, *J* = 7.1, 13.5 Hz, 1H), 3.46 (dd, *J* = 5.3, 8.9 Hz, 1H), 3.55-3.75 (m, 5H), 3.8-3.9 (m, 2H), 3.95-4.05 (m, 1H), 4.4-4.55 (m, 1H), 7.1-7.3 (m, 5H), 7.85 (br d, 1H), 9.19 (d, *J* = 7.9 Hz, 1H).

### Compound 3-91

¹H-NMR (CDCl₃) δ (ppm): 0.96 (d, *J* = 6.8 Hz, 3H), 1.02 (d, *J* = 6.8 Hz, 3H), 1.2-1.4 (m, 2H), 2.0-2.15 (m, 10H), 2.15-2.35 (m, 2H), 2.35-2.5 (m, 2H), 2.5-2.6 (m, 4H), 3.5-3.7 (m, 3H), 3.8-4.2 (m, 3H), 4.55-4.65 (m, 2H), 7.86 (s, 1H), 9.41 (s, 1H).

### Compound 3-193

¹H-NMR (CDCl₃) δ (ppm): 0.8-1.0 (m, 8H), 1.1-1.35 (m, 4H), 1.6-1.8 (m, 8H), 1.8-2.0 (m, 1H), 2.55-2.7 (m, 1H), 2.8-3.1 (m, 2H), 3.45-3.7 (m, 2H), 3.8-4.0 (m, 1H), 5.45-5.6 (m, 1H), 7.2-7.4 (m, 4H), 7.9-8.0 (m, 1H), 9.7-9.8 (m, 1H).

### Compound 3-194

¹H-NMR (CDCl₃) δ (ppm): 0.8-0.95 (m, 6H), 1.2-1.45 (m, 3H), 1.65-1.8 (m, 4H), 2.0-2.15 (m, 4H), 2.55-2.7 (m, 1H), 2.8-3.1 (m, 2H), 3.5-3.75 (m, 2H), 3.8-4.0 (m, 1H), 5.45-5.6 (m, 1H), 5.6-5.75 (m, 2H), 7.2-7.4 (m, 4H), 7.9-8.0 (m, 1H), 9.7-9.8 (m, 1H).

### Example 2

Compounds shown in above (f) to (k) were synthesized by the method described below:

### Step 1: Synthesis of Compound (XI)

To a mixture of Compound (X) (0.5 mL, 0.1 mol/L solution in chloroform : tetrahydrofuran = 2:1 mixture, 0.05 mmol) obtained in Reference Examples 1 to 5 and 1-hydroxybenzotriazole (0.2 mL, 0.25 mol/L solution in chloroform : tetrahydrofuran = 2:1 mixture, 0.05 mmol) was added primary or secondary amine [Compound (VII)] (0.065 mL, 1 mol/L chloroform solution, 0.065 mmol) corresponding to R³R⁴N moiety. Polymer-bound *N*-ethyl-*N*'-[3-(dimethylamino)propyl]carbodiimide (70 mg, 0.1 mmol) was then added and the mixture was stirred at room temperature for 24 hours. The resulting reaction solution was filtered and then concentrated. Before filtering the solution, if necessary, methanol was added to dissolve the precipitates. The resulting residue was dissolved in chloroform (0.6 mL), and then 4-polyvinylpyridine (23 mg, 0.22 mmol) and polymer-bound benzoyl chloride (23 mg, 0.05 mmol) were added. The mixture was stirred at room temperature overnight. The reaction solution was filtered and concentrated to obtain Compound (XI).

### Step 2: Synthesis of Compound (XII)

Compound (XI) obtained in Step 1 was dissolved in chloroform (0.2 mL), and to the solution was added thionyl chloride (0.1 mL, 1 mol/L chloroform solution, 0.1 mmol).

The solution was hermetically sealed and stirred at 60°C for 6 hours. To the resulting residue obtained by concentrating the reaction solution were added chloroform (0.6 mL) and 4-polyvinylpyridine (23 mg, 0.22 mmol) and the mixture was stirred at 65°C overnight. The resulting reaction solution was filtered and concentrated to obtain Compound (XII).

### Step 3: Synthesis of Compound (I-B)

Compound (XII) obtained in Step 2 was dissolved in tetrahydrofuran (0.2 mL) and to the solution was added primary or secondary amine [Compound (XIII)] (0.06 mL, 1 mol/L chloroform solution, 0.06 mmol) corresponding to R⁶R⁷N moiety and the mixture was stirred at a temperature between 70°C to 75°C for 3 days. Further, to the solution were added, chloroform (0.4 mL), 4-polyvinylpyridine (23 mg, 0.22 mmol) and polymer-bound benzoyl chloride (23 mg, 0.05 mmol), and the mixture was stirred at room temperature overnight. If a precipitate was observed, methanol was added to the solution to completely dissolve the precipitate and then the reaction solution was filtered and concentrated to obtain Compound (I-B) with yield of 50 to 60% in total.

The resulting compounds were identified by mass spectrometry. The structures and analytical results of each compound are the same as aforementioned (f) to (k). The proton nuclear magnetic resonance spectra of typical compounds are shown below:

### Compound 7-31

¹H-NMR (CDCl₃) δ (ppm): 0.89 (t, *J* = 6.6 Hz, 3H), 1.2-1.4 (m, 7H), 1.45-1.7 (m, 4H), 1.75-1.95 (m, 2H), 1.95-2.3 (m, 6H), 2.8-3.4 (m, 5H), 3.45-3.75 (m, 4H), 4.55-4.7 (m, 2H), 4.85-5.0 (m, 1H), 7.15-7.4 (m, 5H), 8.60 (br s, 1H), 8.92 (br s, 1H), 9.29 (br s, 1H).

### Compound 7-251

¹H-NMR (CDCl₃) δ (ppm): 0.92 (d, *J* = 6.2 Hz, 6H), 1.2-1.3 (m, 3H), 1.3-1.7 (m, 5H), 1.75-1.95 (m, 2H), 1.95-2.3 (m, 6H), 2.8-3.4 (m, 5H), 3.45-3.75 (m, 4H), 4.55-4.7 (m, 2H), 4.8-5.0 (m, 1H), 7.15-7.4 (m, 5H), 8.58 (br s, 1H), 8.92 (br s, 1H), 9.29 (br s, 1H).

### Compound 7-258

¹H-NMR (CDCl₃) δ (ppm): 1.2-1.3 (m, 3H), 1.3-1.4 (m, 1H), 1.45-1.55 (m, 2H), 1.7-1.9 (m, 4H), 1.9-2.1 (m, 1H), 2.1-2.3 (m, 2H), 2.9-3.2 (m, 5H), 3.4-3.75 (m, 4H), 4.4-4.5 (m, 2H), 4.5-4.7 (m, 2H), 4.8-5.0 (m, 1H), 7.15-7.35 (m, 8H), 7.41 (s, 1H), 8.87 (br s, 1H), 9.39 (br s, 1H), 9.55 (br s, 1H).

### Compound 7-713

¹H-NMR (CDCl₃) δ (ppm): 1.2-1.3 (m, 3H), 1.3-1.4 (m, 1H), 1.45-1.55 (m, 2H), 1.65-2.05 (m, 6H), 2.05-2.3 (m, 4H), 2.45-2.6 (m, 1H), 2.8-3.25 (m, 3H), 3.5-3.75 (m, 4H), 4.5-4.7 (m, 2H), 4.8-5.0 (m, 1H), 5.45-5.6 (m, 1H), 7.1-7.4 (m, 9H), 8.0-8.2 (m, 1H), 8.85 (br s, 1H), 9.0-9.2 (m, 1H).

### Compound 7-785

¹H-NMR (CDCl₃) δ (ppm): 1.2-1.3 (m, 3H), 1.3-1.4 (m, 1H), 1.45-1.55 (m, 2H), 1.7-1.9 (m, 4H), 1.9-2.1 (m, 1H), 2.1-2.3 (m, 2H), 2.9-3.2 (m, 3H), 3.5-3.75 (m, 4H), 4.5-4.7 (m, 4H), 4.85-5.0 (m, 1H), 6.8-6.9 (m, 2H), 7.15-7.35 (m, 6H), 8.45-8.6 (m, 1H), 8.80 (s, 1H), 9.10 (br s, 1H).

### Compound 7-892

¹H-NMR (CDCl₃) δ (ppm): 1.2-1.45 (m, 6H), 1.65-1.85 (m, 2H), 2.15-2.35 (m, 2H), 2.9-3.2 (m, 4H), 3.25-3.45 (m, 1H), 3.6-3.8 (m, 3H), 4.55-4.75 (m, 4H), 4.85-5.0 (m, 1H), 7.15-7.35 (m, 6H), 7.45-7.6 (m, 2H), 7.63 (d, *J* = 7.6 Hz, 1H), 8.75-8.9 (m, 2H), 9.22 (br s, 1H).

### Compound 7-893

¹H-NMR (CDCl₃) δ (ppm): 1.2-1.3 (m, 3H), 1.3-1.4 (m, 1H), 1.5-1.6 (m, 2H), 1.7-1.9 (m, 4H), 1.95-2.1 (m, 1H), 2.15-2.35 (m, 2H), 2.9-3.25 (m, 3H), 3.5-3.8 (m, 4H), 4.55-4.75 (m, 4H), 4.8-5.0 (m, 1H), 7.15-7.35 (m, 6H), 7.4-7.55 (m, 2H), 7.62 (d, *J* = 7.6 Hz, 1H), 8.82 (br s, 1H), 8.95-9.1 (m, 1H), 9.27 (br s, 1H).

### Compound 7-1454

¹H-NMR (CDCl₃) δ (ppm): 2.8-2.9 (m, 1H), 2.94 (t, *J* = 6.3 Hz, 2H), 3.15-3.25 (m, 1H), 3.55-3.65 (m, 2H), 3.65-3.9 (m, 2H), 3.89 (s, 3H), 4.55-4.65 (m, 1H), 4.75-4.85 (m, 2H), 6.28 (br s, 1H), 6.34 (br s, 1H), 6.9-7.0 (m, 3H), 7.15-7.35 (m, 7H), 8.33 (s, 1H), 8.68 (br s, 1H).

### Compound 11-60

¹H-NMR (CDCl₃) δ (ppm): 0.8-0.95 (m, 6H), 2.3-2.4 (m, 1H), 2.33 (s, 3H), 2.85-2.95 (m, 4H), 3.5-3.75 (m, 6H), 3.95-4.1 (m, 1H), 6.8-7.3 (m, 8H), 8.85 (s, 1H).

### Compound 11-70

¹H-NMR (CDCl₃) δ (ppm): 0.8-1.0 (m, 6H), 2.3-2.4 (m, 1H), 2.37 (s, 3H), 2.90 (t, *J* = 6.9 Hz, 2H), 3.5-3.85 (m, 4H), 3.82 (s, 3H), 4.0-4.15 (m, 1H), 4.66 (s, 2H), 6.8-6.9 (m, 2H), 7.15-7.35 (m, 6H), 8.31 (s, 1H).

### Example 3

Compounds shown in above (1) and (m) were synthesized by the method described below:

### Step 1: Synthesis of Compound (XVI)

To compound (XV) (0.1 mol/L chloroform solution, 0.50 mL, 0.050 mmol) obtained in Reference Example 10 or 11 were added, Compound (VII) (1 mmol/L chloroform solution, 0.065 mL, 0.065 mmol) represented by R³R⁴NH, 1-hydroxybenzotriazole (0.20 mL, 0.25 mol/L solution in chloroform : tetrahydrofuran = 3:1 mixture, 0.050 mmol) and polymer-bound *N*-ethyl-*N*'-[3-(dimethylamino)propyl]carbodiimide (70 mg, 0.1 mmol), and the mixture was stirred at a temperature between 50°C and 55°C for 20 hours. The resin in the reaction mixture was separated by filtration and after resulting resin was washed with chloroform (1.8 mL), organic layers were all collected together, and the solvent was then evaporated. The residue was dissolved in chloroform (0.60 mL), and then polyvinylpyridine (23 mg, 0.22 mmol) and polymer-bound benzoyl chloride (23 mg, 0.05 mmol) were added. The mixture was stirred at room temperature for one day.

The resin in the reaction mixture was separated by filtration and the resulting resin was washed with a chloroform-methanol mixture (3:1, 1.8 mL). Organic layers were all collected together, and the solvent was then evaporated to obtain Compound (XVI).

### Step 2: Synthesis of Compound (XVII)

Total amount of compound (XVI) obtained in step 1 was dissolved in chloroform (0.50 mL), and to the solution was added thionyl chloride (0.005 mL). The solution was stirred at room temperature overnight. After confirming the completion of reaction by thin layer chromatography, the solvent was evaporated to obtain Compound (XVII).

### Step 3: Synthesis of Compound (I-C)

Total amount of compound (XVII) obtained in Step 2 was dissolved in chloroform (0.50 mL), and to the solution were added (cyanomethylene)triphenylphosphorane (1 mmol/L toluene solution, 0.10 mL, 0.10 mmol) and Compound (XIIIa) (1 mol/L chloroform solution, 0.10 mL, 0.10 mmol) represented by R^{6a}R^{7a}NH. The mixture was stirred at 50°C overnight. After confirming the completion of reaction by thin layer chromatography, the solvent was evaporated. The residue was dissolved in chloroform (0.70 mL), and to the solution were added polyvinylpyridine (23 mg, 0.22 mmol) and polymer-bound benzoyl chloride (23 mg, 0.05 mmol). The solution was hermetically sealed and stirred at room temperature for one day. The resin in the reaction mixture was separated by filtration and after resulting resin was washed with a chloroform-methanol mixture (3:1, 1.8 mL), organic layers were all collected together, and the solvent was then evaporated to obtain Compound (I-C).

The resulting compounds were identified by mass spectrometry. The structures and analytical results of each compound are the same as aforementioned (1) and (m).

### Reference Example 1

### (S)-4-(1-hydroxymethyl-2-phenylethylamino)-2-methylthiopyrimidine-5-carboxylic acid

To a solution of ethyl 4-chloro-2-methylthiopyrimidine-5-carboxylate (25.0 g, 107 mmol) in tetrahydrofuran (100 mL) was added triethylamine (29.9 mL, 215 mmol) under icecooling, then a solution of (*S*)-(-)-2-amino-3-phenyl-1-propanol (16.6g, 110 mmol) in tetrahydrofuran (150 mL) was added. The reaction solution was stirred at room temperature overnight. Ethyl ester of the titled compound (37.8 g, quantitative yield) was obtained by adding the reaction solution to water (1,000 mL), stirring the solution to collect deposited salt by filtration and drying under reduced pressure.

The resulting ethyl ester (30.0 g, 86.4 mmol) was dissolved in a mixed solvent of ethanol (300 mL) and tetrahydrofuran (150 mL), then aqueous sodium hydroxide solution (2 mol/L, 100 mL) was added and stirred at room temperature for 2 hours. The residue obtained by evaporating the solvent under reduced pressure was dissolved in water and was adjusted to pH 5 with 2 mol/L hydrochloric acid. The deposited crystals were collected by filtration and dried under reduced pressure to obtain the titled compound (27.9 g, quantitative yield).
¹H-NMR (DMSO-d₆) δ (ppm): 2.49 (s, 3H), 2.85-2.95 (m, 2H), 3.45-3.55 (m, 2H), 4.4-4.55 (m, 1H), 4.85-5.05 (m, 1H), 7.1-7.3 (m, 5H), 8.49 (s, 1H), 8.64 (d, *J* = 8.3 Hz, 1H).
IR (KBr tablet): 1658, 1583, 1495, 1373, 1284, 1271, 1176 cm⁻¹
Melting point (H₂O): 162-164°C
APCI-MS m/z 320 ([M+H]⁺).

Elemental analysis Calcd. for C₁₅H₁₇N₃O₃S 0.5 H₂O: C, 54.86; H, 5.52; N, 12.80. Found: C, 54.89; H, 5.44; N, 12.73.

The other enantiomer [(*R*)-form] of this compound was synthesized by using the corresponding amine [(*R*)-form] in the same manner. The analytical data of (*R*)-form of title compound was completely identical to those of (*S*)-form.

In Reference Examples 2 to 5, target compounds were obtained in substantially the same manner as Reference Example 1 by using corresponding amines instead of (*S*)-(-)-2-amino-3-phenyl-1-propanol.

### Reference Example 2

### (s)-4-(2-cyclohexyl-1-hydroxymethylethylamino)-2-methylthiopyrimidine-5-carboxylic acid

¹H-NMR (DMSO-d₆) δ (ppm): 0.8-1.05 (m, 2H), 1.05-1.35 (m, 4H), 1.35-1.55 (m, 2H), 1.55-1.75 (m, 4H), 1.75-1.9 (m, 1H), 2.46 (s, 3H), 3.45-3.65 (m, 2H), 4.3-4.5 (m, 1H), 4.7-4.9 (m, 1H), 8.44 (d, *J* = 8.2 Hz, 1H), 8.50 (s, 1H).
IR (KBr tablet): 1683, 1581, 1383, 1172 cm⁻¹
Melting point (H₂O): 170-173° C
APCI-MS m/z 326 ([M+H]⁺).
Elemental analysis Calcd. for C₁₅H₂₃N₃O₃S 0.3 H₂O: C, 54.46; H, 7.19; N, 12.70. Found: C, 54.55; H, 6.99; N, 12.64.

### Reference Example 3

### 4-[1-hydroxymethyl-2-(4-methoxyphenyl)ethylamino]-2-methylthiopyrimidine-5-carboxylic acid

¹H-NMR (DMSO-d₆) δ (ppm): 2.48 (s, 3H), 2.75-2.85 (m, 2H), 3.4-3.5 (m, 2H), 3.71 (s, 3H), 4.3-4.45 (m, 1H), 5.02 (br s, 1H), 6.83 (d, *J* = 8.6 Hz, 2H), 7.15 (d, *J* = 8.6 Hz, 2H), 8.49 (s, 1H), 8.59 (d, *J* = 8.2 Hz, 1H).
IR (KBr tablet): 1606, 1576, 1512, 1379, 1248, 1198 cm⁻¹
Melting point (H₂O-CH₃OH): 165-167°C
APCI-MS m/z 350 ([M+H]⁺).
Elemental analysis Calcd. for C₁₆H₁₉N₃O₄S 2.4 H₂O: C, 48.94; H, 6.11; N, 10.70. Found: C, 48.88; H, 5.61; N, 10.70.

### Reference Example 4

### 4-(2-hydroxy-1,1-dimethylethylamino)-2-methylthiopyrimidine-5-carboxylic acid

¹H-NMR (DMSO-d₆) δ (ppm): 1.44 (s, 6H), 2.50 (s, 3H), 3.62 (s, 2H), 4.88 (br s, 1H), 8.51 (s, 1H), 8.70 (s, 1H).
IR (KBr tablet): 1653, 1628, 1596, 1576, 1296, 1173 cm⁻¹
Melting point (H₂O): 227-229°C
APCI-MS m/z 258 ([M+H]⁺).
Elemental analysis Calcd. for C₁₀H₁₅N₃O₃S 0.5 H₂O: C, 45.10; H, 6.06; N, 15.78. Found: C, 45.08; H, 5.85; N, 15.58.

### Reference Example 5

### (R)-4-(1-hydroxymethyl-2-methylpropylamino)-2-methylthiopyrimidine-5-carboxylic acid

¹H-NMR (CDCl₃) δ (ppm): 0.85-1.05 (m, 6H), 1.9-2.1 (m, 1H), 2.54 (s, 3H), 3.7-3.8 (m, 1H), 3.9-4.0 (m, 1H), 4.05-4.2 (m, 1H), 8.78 (br s, 1H), 9.28 (br s, 1H).
IR (KBr tablet): 1693, 1604, 1597, 1390, 1281, 1174 cm⁻¹
Melting point (CH₃OH): 123-125°C
APCI-MS m/z 272 ([M+H]⁺).
Elemental analysis Calcd. for C₁₁H₁₇N₃O₃S 2.3 H₂O: C, 42.24; H, 6.96; N, 13.43. Found: C, 42.10; H, 5.74; N, 13.27.

### Reference Example 6

### (S)-2-benzyl-5-oxo-2,3,5,6-tetrahydroimidazo[1,2-c]pyrimidine-8-carboxylic acid

To a solution of ethyl ester of (*S*)-4-(1-hydroxymethyl-2-phenylethylamino)-2-methylthiopyrimidine-5-carboxylic acid (27.4 g, 79.0 mmol), obtained in the initial stage of Reference Example 1, in chloroform (270 mL) was added thionyl chloride (17.3 mL, 237 mmol), and the mixture was heated under reflux for 7.5 hours. The resulting reaction solution was concentrated under reduced pressure. To the resulting residue were added, chloroform (220 mL), water (60 mL) and potassium carbonate (43.7 g, 316 mmol), and the mixture was then heated under reflux for 24 hours. The chloroform layer was separated from the resulting reaction solution and then concentrated under reduced pressure. To the resulting residue were added, the aqueous layer separated from the reaction solution, ethanol (200 mL) and potassium carbonate (10.9 g, 79.0 mmol), and the mixture was then heated under reflux for 20 hours. Potassium carbonate (10.9 g, 79.0 mmol) was further added to the reaction solution, and the mixture was then heated under reflux for 5 hours. The resulting reaction solution was then concentrated under reduced pressure. The resulting residue was dissolved in water and washed with chloroform. The aqueous layer was adjusted to pH 5 to collect deposited crystals by filtration and then was dried under reduced pressure to obtain the titled compound (18.8 g, a total yield of 88%).
¹H-NMR (DMSO-d₆) δ (ppm): 2.93 (dd, *J* = 7.9, 13.5 Hz, 1H), 3.10 (dd, *J* = 4.3, 13.5 Hz, 1H), 3.77 (dd, *J* = 6.3, 12.2 Hz, 1H), 3.96 (dd, *J* = 10.3, 12.2 Hz, 1H), 4.4-4.5 (m, 1H), 7.2-7.4 (m, 5H), 8.40 (s, 1 H).

The other enantiomer [(*R*)-form] of this compound was synthesized by using an ethyl ester prepared from the corresponding amine [(*R*)-form] as in the initial stage of Reference Example 1. The analytical data of the (*R*)-form was completely identical to those of (*S*)-form.

In following Reference Examples 7 to 9, target compounds were obtained in substantially the same manner as in Reference Example 6 by using corresponding esters instead of ethyl ester of (*S*)-4-(1-hydroxymethyl-2-phenylethylamino)-2-methylthiopyrimidine-5-carboxylic acid.

### Reference Example 7

### (S)-2-isopropyl-5-oxo-2,3,5,6-tetrahydroimidazo[1,2-c]pyrimidine-8-carboxylic acid

¹H-NMR (DMSO-d₆) δ (ppm): 0.89 (d, *J* = 6.9 Hz, 3H), 0.94 (d, *J* = 6.9 Hz, 3H), 1.95-2.1 (m, 1H), 3.81 (dd, *J* = 11.9, 17.2
Hz, 1H), 4.0-4.15 (m, 2H), 8.47 (s, 1H).

### Reference Example 8

### (s)-2-(cyclohexylmethyl)-5-oxo-2,3,5,6-tetrahydroimidazo[1,2-c]pyrimidine-8-carboxylic acid

¹H-NMR (DMSO-d₆) δ (ppm): 0.85-1.05 (m, 2H), 1.1-1.3 (m, 3H), 1.3-1.55 (m, 2H), 1.55-1.8 (m, 6H), 3.55-3.75 (m, 1H), 4.1-4.3 (m, 2H), 8.45 (s, 1H).

### Reference Example 9

### 2,2-dimethyl-5-oxo-2,3,5,6-tetrahydroimidazo[1,2-c]pyrimidine-8-carboxylic acid

¹H-NMR (DMSO-d₆) δ (ppm): 1.42 (s, 6H), 3.79 (s, 2H), 8.45 (s, 1H).

### Reference Example 10

### (S)-6-chloro-2-(1-hydroxymethyl-2-phenylethylamino)nicotinic acid

Commercially available 2,6-dichloronicotinic acid (30.0 g, 156 mmol) was dissolved in dioxane (500 mL), and then to the solution were added (*S*)-(-)-2-amino-3-phenyl-1-propanol (35.43 g, 234 mmol, 1.5 equivalent) and triethylamine (65.3 mL, 469 mmol). The mixture was heated under reflux for 3 days. To the reaction mixture was added water, and the mixture was extracted with chloroform, then resulting organic layer was washed with water and dried over anhydrous sodium sulfate. The resulting residue, obtained after the evaporation of the solvent, was purified with silica gel column chromatography (chloroform : methanol : acetic acid = 10:1:0.1) to obtain yellow crystals of the titled compound (28.1 g. 91.6 mmol, a yield of 59%).
¹H-NMR (DMSO-d₆) δ (ppm): 2.82 (m, 2H), 3.2-3.7 (2H, overlapped peak with others), 4.14 (m, 1H), 4.41 (br s, 1H), 6.44 (d, *J* = 7.8 Hz, 1H), 7.1-7.4 (m, 5H), 8.06 (d, *J* = 7.9
Hz, 1H), 9.38 (br s, 1H).

### Reference Example 11

### (R)-6-chloro-2-(1-hydroxymethyl-2-phenylethylamino)nicotinic acid

Commercially available 2,6-dichloronicotinic acid (30.0 g, 156 mmol) was dissolved in dioxane (500 mL), and then to the solution were added (*R*)-(+)-2-amino-3-phenyl-1-propanol (35.43 g, 234 mmol, 1.5 equivalents) and triethylamine (65.3 mL, 469 mmol). The mixture was heated under reflux for 6 days. To the reaction mixture was added water, and the mixture was extracted with chloroform, then resulting organic layer was washed with water and dried over anhydrous sodium sulfate. After the solvent was evaporated, *n*-hexane:ethyl acetate (3:1) was added and evaporated again to obtain yellow crystals of the titled compound (35.8 g. 117 mmol, a yield of 75%).
¹H-NMR (DMSO-d₆) δ (ppm): 2.86 (m, 2H), 3.1-3.6 (2H, overlapped peak with others), 4.24 (m, 1H), 5.0 (br s, 1H), 6.59 (d, *J* = 8.1 Hz, 1H), 7.1-7.3 (m, 5H), 8.02 (d, *J* = 8.1 Hz, 1H), 10.45 (d, *J* = 7.9 Hz, 1H).

### Industrial Applicability

According to the present invention, heterobicyclic compounds or pharmaceutically acceptable salts thereof, which have glucose concentration-dependent insulin secretion promoting action or hypoglycemic action and is useful as a therapeutic agent for diabetes or the like, are provided.

## Claims

1. A compound represented by formula (I): 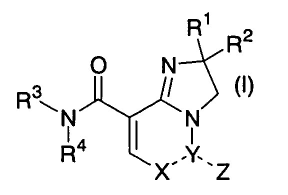 {wherein X--Y--Z represents R⁵N-C=O (wherein R⁵ represents a hydrogen atom, substituted or unsubstituted lower alkyl, substituted or unsubstituted aralkyl, substituted or unsubstituted lower alkenyl, or substituted or unsubstituted lower alkynyl), N=C-NR⁶R⁷ [wherein R⁶ and R⁷ may be the same or different and each represents a hydrogen atom, substituted or unsubstituted lower alkyl, substituted or unsubstituted aralkyl, substituted or unsubstituted lower alkenyl, substituted or unsubstituted lower alkynyl, substituted or unsubstituted aryl, or a substituted or unsubstituted heterocyclic group; or R⁶ and R⁷ are combined together with the adjacent nitrogen atom thereto to form: 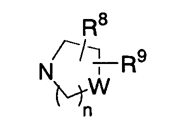 (wherein n represents an integer of 1 to 3; W represents -CH₂-, -CH=CH-, -NH-, a sulfur atom, or an oxygen atom; and R⁸ and R⁹ may be the same or different and each represents a hydrogen atom, substituted or unsubstituted lower alkyl, substituted or unsubstituted aralkyl, substituted or unsubstituted aryl, a substituted or unsubstituted heterocyclic group, substituted or unsubstituted lower alkoxycarbonyl, substituted or unsubstituted lower alkanoyl, substituted or unsubstituted aroyl, substituted or unsubstituted heteroaroyl, a halogen atom, amino, nitro, cyano, hydroxy, oxo, carboxy, carbamoyl, mono or di(substituted or unsubstituted lower alkyl)amino, substituted or unsubstituted lower alkanoylamino, substituted or unsubstituted lower alkoxy, substituted or unsubstituted aralkyloxy, or substituted or unsubstituted lower alkanoyloxy)], or C=C-NR^{6a}R^{7a} (wherein R^{6a} and R^{7a} have the same meanings as R⁶ and R⁷ defined above, respectively); R¹ and R² may be the same or different and each represents a hydrogen atom, substituted or unsubstituted lower alkyl, substituted or unsubstituted aralkyl, substituted or unsubstituted lower alkenyl, substituted or unsubstituted lower alkynyl, substituted or unsubstituted aryl, a substituted or unsubstituted heterocyclic group, a halogen atom, carboxy, substituted or unsubstituted lower alkoxycarbonyl, or C(=O)-NR^{6b}R^{7b} (wherein R^{6b} and R^{7b} have the same meanings as R⁶ and R⁷ defined above, respectively); and R³ and R⁴ may be the same or different and each represents a hydrogen atom, substituted or unsubstituted lower alkyl, substituted or unsubstituted lower alkenyl, substituted or unsubstituted aralkyl, or a substituted or unsubstituted heterocyclic group; or R³ and R⁴ are combined together with the adjacent nitrogen atom thereto to form: 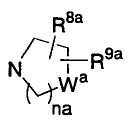 (wherein na, W^{a}, R^{8a} and R^{9a} have the same meanings as n, W, R⁸ and R⁹ defined above, respectively)}, or a pharmaceutically acceptable salt thereof.

2. The compound or the pharmaceutically acceptable salt thereof according to claim 1, wherein X--Y--Z is R⁵N-C=O (wherein R⁵ has the same meaning as defined above).

3. The compound or the pharmaceutically acceptable salt thereof according to claim 1, wherein X--Y--Z is N=C-NR⁶R⁷ (wherein R⁶ and R⁷ have the same meanings as defined above, respectively).

4. The compound or the pharmaceutically acceptable salt thereof according to claim 3, wherein R⁶ and R⁷ are not hydrogen atoms simultaneously.

5. The compound or the pharmaceutically acceptable salt thereof according to claim 3, wherein R⁶ is a hydrogen atom and R⁷ is substituted or unsubstituted pyrrolidinyl.

6. The compound or the pharmaceutically acceptable salt thereof according to claim 1, wherein X--Y--Z is C=C-NR^{6a}R^{7a} (wherein R^{6a} and R^{7a} have the same meanings as defined above, respectively).

7. The compound or the pharmaceutically acceptable salt thereof according to claim 6, wherein R^{6a} and R^{7a} are not hydrogen atoms simultaneously.

8. The compound or the pharmaceutically acceptable salt thereof according to any one of claims 1 to 7, wherein either R¹ or R² is a hydrogen atom and the other is substituted or unsubstituted lower alkyl, or substituted or unsubstituted aralkyl; or R¹ and R² may be the same or different and each is substituted or unsubstituted lower alkyl.

9. The compound or the pharmaceutically acceptable salt thereof according to any one of claims 1 to 7, wherein R¹ is a hydrogen atom and R² is aralkyl.

10. The compound or the pharmaceutically acceptable salt thereof according to any one of claims 1 to 9, wherein R³ is a hydrogen atom and R⁴ is lower alkyl.

11. The compound or the pharmaceutically acceptable salt thereof according to any one of claims 1 to 9, wherein R³ is a hydrogen atom and R⁴ is substituted or unsubstituted aralkyl.

12. A pharmaceutical composition which comprises the compound or the pharmaceutically acceptable salt thereof according to any one of claims 1 to 11.

13. A preventive and/or therapeutic agent for diabetes, which comprises the compound or the pharmaceutically acceptable salt thereof according to any one of claims 1 to 11.

14. A preventive and/or therapeutic agent for complication of diabetes, which comprises the compound or the pharmaceutically acceptable salt thereof according to any one of claims 1 to 11.

15. An insulin secretion promoter which comprises the compound or the pharmaceutically acceptable salt thereof according to any one of claims 1 to 11.

16. A hypoglycemic agent which comprises the compound or the pharmaceutically acceptable salt thereof according to any one of claims 1 to 11.

17. A method for preventing and/or treating diabetes, which comprises administering an effective amount of the compound or the pharmaceutically acceptable salt thereof according to any one of claims 1 to 11.

18. A method for preventing and/or treating a complication of diabetes, which comprises administering an effective amount of the compound or the pharmaceutically acceptable salt thereof according to any one of claims 1 to 11.

19. A method for promoting insulin secretion, which comprises administering an effective amount of the compound or the pharmaceutically acceptable salt thereof according to any one of claims 1 to 11.

20. A method for decreasing blood glucose level, which comprises administering an effective amount of the compound or the pharmaceutically acceptable salt thereof according to any one of claims 1 to 11.

21. Use of the compound or the pharmaceutically acceptable salt thereof according to any one of claims 1 to 11 for the manufacture of a preventive and/or therapeutic agent for diabetes.

22. Use of the compound or the pharmaceutically acceptable salt thereof according to any one of claims 1 to 11 for the manufacture of a preventive and/or therapeutic agent for a complication of diabetes.

23. Use of the compound or the pharmaceutically acceptable salt thereof according to any one of claims 1 to 11 for the manufacture of an insulin secretion promoter.

24. Use of the compound or the pharmaceutically acceptable salt thereof according to any one of claims 1 to 11 for the manufacture of a hypoglycemic agent.
